# EUROPEAN PATENT APPLICATION

(11) **EP 4 692 115 A1**
(43) Date of publication of application: **11.02.2026**
(21) Application number: 24784938.3
(22) Date of filing: 03.04.2024
(51) Int. Cl.: C07K 16/00

(54) **ANTIBODY-FUNCTIONAL SUBSTANCE CONJUGATE, ANTIBODY DERIVATIVE, AND COMPOUND, OR SALTS THEREOF**

(30) Priority: 04.04.2023 US 202363494102 P
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); WATANABE, Tomohiro, Kawasaki-shi, Kanagawa 210-8681 (JP); FUJII, Tomohiro, Kawasaki-shi, Kanagawa 210-8681 (JP); CHOI, Seungyong Sean, Princeton, New Jersey 08540 (US); MANDHAPATI, Appi Reddy, Princeton, New Jersey 08540 (US); BHATTARAI, Deepak, Princeton, New Jersey 08540 (US); MARKOTAN, Thomas, Princeton, New Jersey 08540 (US); MCFARLANE, Gery, Princeton, New Jersey 08540 (US)
(74) Representative: Grünecker Patent- und Rechtsanwälte PartG mbB
(86) International application number: PCT/JP2024/013787
(87) International publication number: WO 2024/210154

(57) **Abstract**

A conjugate of an antibody and a functional substance, or a salt thereof, that has excellent desired properties is provided. More specifically, a conjugate of an antibody and a functional substance, or a salt thereof, the conjugate comprising a structural unit represented by the following formula (1): 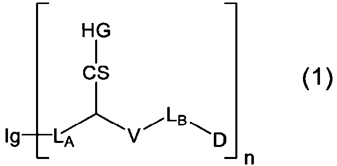 wherein Ig represents an immunoglobulin unit comprising two heavy chains and two light chains, and is bonded to L_{A} adjacent to Ig via a functional group in the side chain of a specific amino acid residue in Ig, HG represents a hydrophilic group or a monovalent group comprising a hydrophilic group, CS represents a divalent group comprising a cleavable site, V represents an oxygen atom, a sulfur atom, or an amino group (NH), L_{A} and L_{B} each independently represent a divalent group, D represents a functional substance, and the average number (n) of said bonds per the immunoglobulin unit is 1.5 or more, and related substances thereof, is provided.

## Description

### TECHNICAL FIELD

The present invention relates to conjugates of antibodies and functional substances, or salts thereof, as well as antibody derivatives and compounds, or salts thereof, used for the production of the conjugates, and the like.

### BACKGROUND ART

Recently, research and development of antibody-drug conjugates (ADCs) has been active. ADCs, as implied by the name, are medicines in which a drug (e.g., an anti-cancer agent) is conjugated with an antibody and has direct cytotoxic activities on cancer cells or the like. A typical ADC is T-DM1 (product name: Kadcyla^{®}), which was jointly developed by ImmunoGene, Inc. and F. Hoffmann-La Roche Ltd.

ADCs are produced by binding a functional group in the side chain of a specific amino acid residue present in an antibody to a drug. An example of such a functional group utilized in the production of ADCs is the amino group in the side chain of a lysine residue present in the antibody. Several technologies have been reported for modifying lysine groups in antibodies (e.g., lysine residues at positions 246/248, 288/290, or 317) (e.g., Patent Documents 1 to 4).

Another example of such a functional group utilized in the production of ADCs is a thiol group. Antibodies have disulfide groups because the heavy-heavy chains and the heavy-light chains are linked by disulfide bonds. For example, an IgG antibody consisting of two heavy chains and two light chains has four disulfide groups because the heavy-heavy chains and the heavy-light chains are linked by four disulfide bonds. Such disulfide bonds can be cleaved by a reducing agent. For example, if all four disulfide bonds in an IgG are cleaved, an IgG antibody with eight thiol groups is generated. Even if all four interchain disulfide bonds are cleaved, the heavy and light chains of the antibody do not dissociate. This is because non-covalent bonds between the chains can maintain the higher-order structure of the antibody. This non-covalent bonding also maintains the antibody properties (binding ability to the target). For example, trastuzumab deruxtecan (Patent Document 5), known as Enhertu^{®}, is an ADC with a drug-to-antibody ratio (DAR) of 8, which is produced by cleaving (reducing) all four interchain disulfide bonds and then conjugating the drug. Such ADCs are known to act as antigen-specific agents by maintaining their antibody properties.

In an ADC, an antibody and a drug are linked via a linker. Various linkers exist for ADCs. For example, ADCs used as anticancer agents contain a linker comprising a dipeptide consisting of valine-citrulline (Val-Cit: VC structure), which is stable in human plasma and has a structure that can be cleaved by a specific enzyme to release the drug within cancer cells. As shown in (A) below, a linker containing such a dipeptide is stable in human plasma. As shown in (B) below, however, the VC structure is recognized by cathepsin B in the lysosomes of human cancer cells, and the amide bond on the carboxy-terminal side of citrulline is cleaved. Therefore, an ADC having a linker containing such a dipeptide can release the drug within human cancer cells to exhibit its drug efficacies.
(A) Stable in human plasma 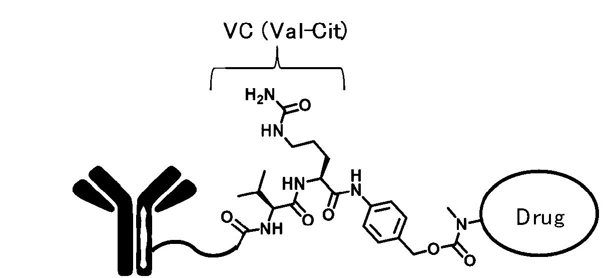
(B) Cleaved in lysosomes in human cancer cells

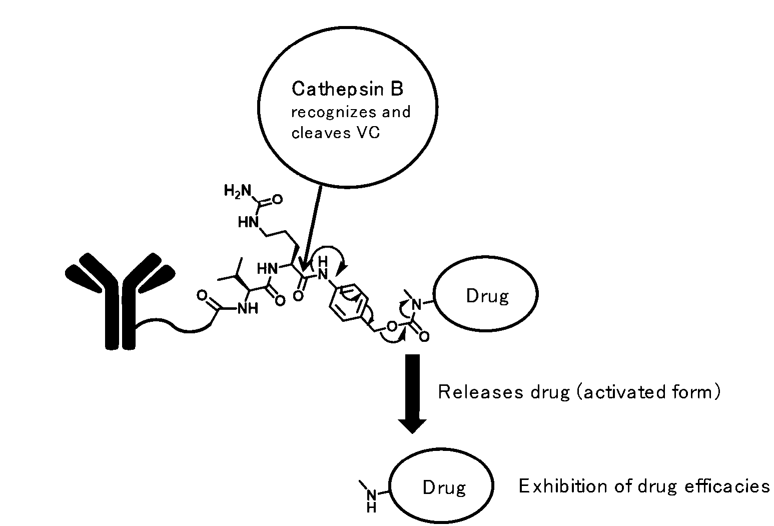

However, ADCs with linkers containing the dipeptides as described above are unstable in mouse plasma (Non-Patent Documents 1 and 2). This is because mouse plasma contains Ceslc, a carboxylase that recognizes the VC structure and cleaves the amide bond on the carboxy-terminal side of citrulline, causing the linker containing the dipeptide as described above to be cleaved by Ceslc in the plasma. Therefore, ADCs with linkers containing the dipeptides as described above exhibit significantly different pharmacokinetics between mice and humans. For this reason, mice disadvantageously make it difficult to evaluate the efficacy in humans.
(C) Undesired reaction in mouse plasma

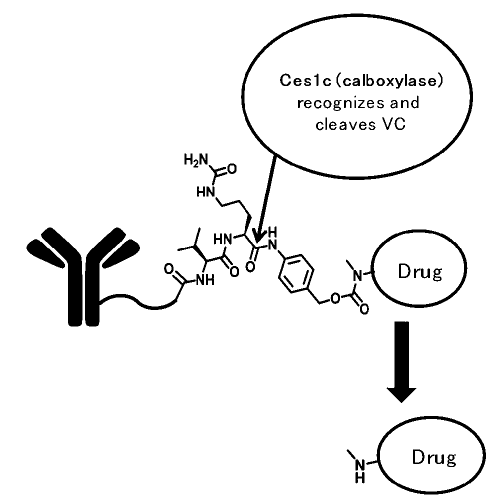

To improve the instability in mouse plasma of ADCs having the structure of "antibody-spacer-VC structure-spacer-drug" as described above, stabilization of ADCs by modifying the linker (i.e., spacer-VC structure-spacer) has been attempted. From this viewpoint, ADCs in which an antibody and a drug or its mimic are linked via the aforementioned linker have been reported. For example, the following ADC has been reported as an ADC containing the linker not in the main chain connecting the antibody and the drug or its mimic, but in the side chain of that main chain (Patent Document 6). 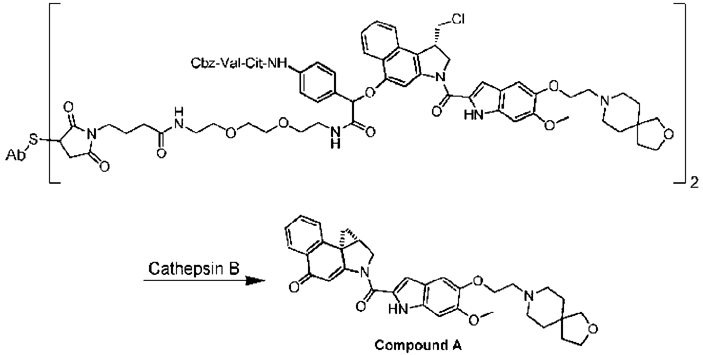
Ab: Antibody
Cbz: Benzyloxycarbonyl
Val: Valine residue
Cit: Citrulline residue

Incidentally, Non-patent Document 3 discloses that higher degrees of hydrophobicity of ADCs result in faster plasma clearance, and that the degrees of hydrophobicity of ADCs can be evaluated by Hydrophobic Interaction Chromatography (HIC)-HPLC.

### PRIOR ART DOCUMENTS

### Patent documents

Patent document 1: WO2018/199337
Patent document 2: WO2019/240288
Patent document 3: WO2019/240287
Patent document 4: WO2020/090979
Patent document 5: WO2014/057687
Patent document 6: WO2015/038426

### Non-patent documents

Non-patent document 1: Dorywalska et al., Bioconjugate Chem., 2015, 26 (4), 650-659
Non-patent document 2: Dorywalska et al., Mol Cancer Ther., 2016, 15 (5), 958-70
Non-patent document 3: Lyon et al., Nat Biotechnol., 2015, 33 (7), 733-5

### SUMMARY OF THE INVENTION

### PROBLEMS TO BE ACHIEVED BY THE INVENTION

An aspect of the present invention is to provide conjugates of antibodies and functional substances, or salts thereof, that are excellent in desired properties.

The present inventors have conducted intensive studies and found that a conjugate comprising a linker having a specific structure that contains both a hydrophilic group and a cleavable site, in the side chain of the main chain connecting an antibody and a drug (or drug mimic), and in which the linker is connected to the antibody (immunoglobulin unit), has excellent properties. For example, such a conjugate or a salt thereof can have excellent clearance (long invivo residence time) and a low aggregation rate (high monomer ratio). In addition, by using a preferred example of the linker with the specific structure as described above, it is possible to retain the hydrophilic group on the antibody after cleavage. That is, before cleavage, the drug is linked to the hydrophilic group in the form of a conjugate with an antibody, and thus the hydrophobicity of the drug can be inhibited (masking by the hydrophilic group), while after cleavage, the hydrophilic group dissociates from the drug, allowing the drug to exert its original hydrophobicity. Therefore, for example, when the drug comprised in the conjugate is a hydrophobic compound from which cell permeability (so-called bystander effect) can be expected, the present invention using the linker as described above has the advantage of making it easy for the drug to exert its cell permeability. In this specification, ADCs having such a linker may be referred to as exo-type ADCs.

The present inventors have also succeeded in developing antibody derivatives and compounds useful for the preparation of such conjugates. The conjugates, antibody derivatives, and compounds of the present invention represented by structures such as formulas (1) to (7) have the technical feature of sharing the partial structural unit of the structural unit represented by formula (5), excluding X and Y. The present inventors have succeeded in developing a series of inventions having such technical features, and have completed the present invention. The prior art neither describes nor suggests the chemical structure of the conjugate of the present invention, nor the relationship between such a chemical structure and the excellent properties as described above. The prior art also neither describes nor suggests the antibody derivatives and compounds of the present invention that can be utilized for the preparation of such conjugates.

That is, the present invention is as follows.

In the first embodiment, the present invention provides a conjugate of an antibody and a functional substance, or a salt thereof, comprising a structural unit represented by the following formula (1): 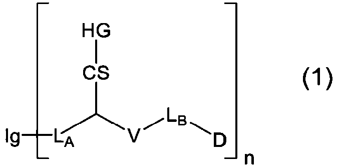 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

In a specific embodiment, the structural unit represented by formula (1) may be a structural unit represented by the following formula (1'): 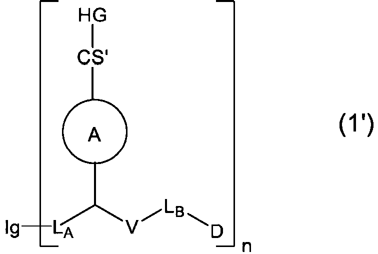 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

In a specific embodiment, the structural unit represented by formula (1') may be a structural unit represented by the following formula (1''): 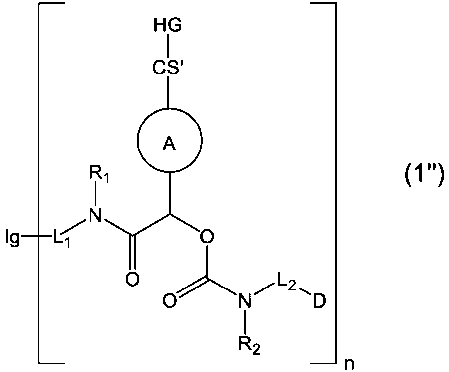 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
D represents a functional substance, and
the average number (n) of said bonds per the immunoglobulin unit is 1.5 or more.

In a preferred embodiment, the conjugate may show an aggregation rate of 2.6% or less as determined by size exclusion chromatography.

In a specific embodiment, the functional group in the side chain of a specific amino acid residue may be the amino group in the side chain of a lysine residue, and n may be 1.5 to 2.5. In this case, L_{A} may have a carbonyl group, and the bond may be achieved by an amide bond generated by the bonding between the amino group in the side chain of a lysine residue and the carbonyl group in L_{A}.

In a preferred embodiment, the lysine residue may be present at position 246/248, 288/290, or 317 according to Eu numbering.

In a preferred embodiment, the structural unit may further comprise a modified moiety represented by the following formula (I): 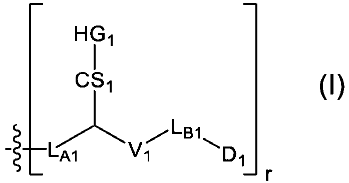 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

In a preferred embodiment, the modified moiety represented by formula (I) may be a modified moiety represented by the following formula (I'): 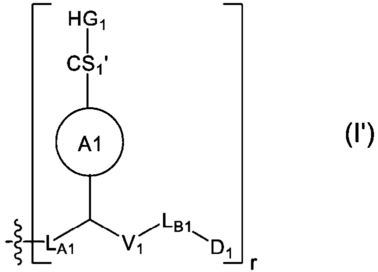 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

In a preferred embodiment, the modified moiety represented by formula (I') may be a modified moiety represented by the following formula (I''): 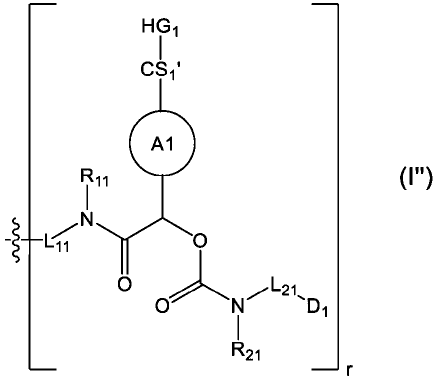 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
D₁ represents a functional substance, and
the average number (r) of said bonds per the immunoglobulin unit is 1.5 to 2.5.

In a specific embodiment, the functional group in the side chain of a specific amino acid residue may be the thiol group in the side chain of a cysteine residue, and n may be 2.0 to 8.0. n may preferably be 6.0 to 8.0, more preferably 7.0 to 8.0.

In a second embodiment, the present invention provides an antibody derivative having a bioorthogonal functional group, or a salt thereof, the antibody derivative comprising a structural unit represented by the following formula (2): 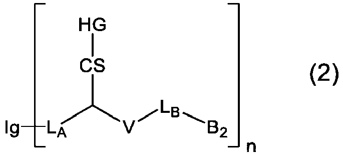 wherein
Ig represents an immunoglobulin unit comprising two heavy chains and two light chains, and is bonded to L_{A} that adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
B₂ represents a bioorthogonal functional group,
the average number (n) of said bonds per the immunoglobulin unit is 1.5 or more.

In a specific embodiment, the structural unit represented by formula (2) may be a structural unit represented by the following formula (2'): 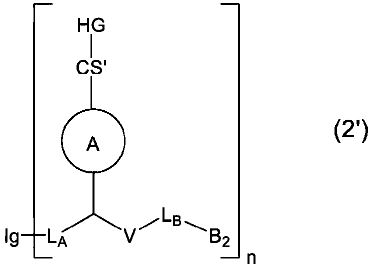 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₂ represents a bioorthogonal functional group, and
the average number (n) of said bonds per the immunoglobulin unit is 1.5 or more.

In a specific embodiment, the structural unit represented by formula (2') may be a structural unit represented by the following formula (2"): 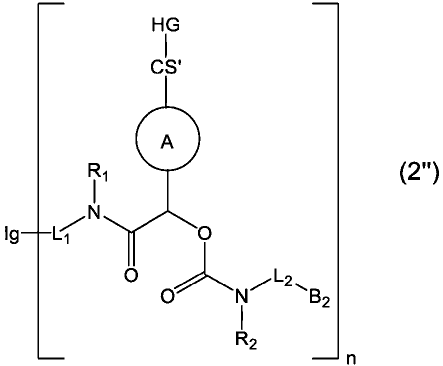 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₂ represents a bioorthogonal functional group, and
the average number (n) of said bonds per the immunoglobulin unit is 1.5 or more.

In a specific embodiment, the functional group in the side chain of a specific amino acid residue may be the amino group in the side chain of a lysine residue, and n may be 1.5 to 2.5. In this case, L_{A} may have a carbonyl group, and the bond may be achieved by an amide bond generated by the bonding between the amino group in the side chain of a lysine residue and the carbonyl group in L_{A}.

In a preferred embodiment, the lysine residue may be present at position 246/248, 288/290, or 317 according to Eu numbering.

In a preferred embodiment, the structural unit may further comprise a modified moiety represented by the following formula (II): 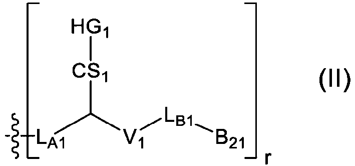 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

In a preferred embodiment, the modified moiety represented by formula (II) may be a modified moiety represented by the following formula (II'): 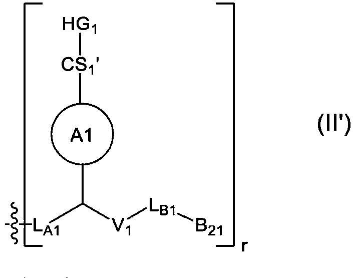 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

In a preferred embodiment, the modified moiety represented by formula (II') may be a modified moiety represented by the following formula (II"): 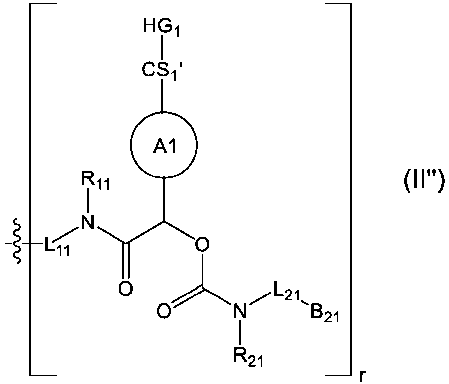 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
B₂₁ represents a bioorthogonal functional group, and
the average number (r) of said bonds per the immunoglobulin unit is 1.5 to 2.5.

In a specific embodiment, the functional group in the side chain of a specific amino acid residue may be the thiol group in the side chain of a cysteine residue, and n may be 2.0 to 8.0. n may preferably be 6.0 to 8.0, more preferably 7.0 to 8.0.

In a third embodiment, the present invention provides a compound having a bioorthogonal functional group and a functional substance, or a salt thereof, represented by the following formula (3'): 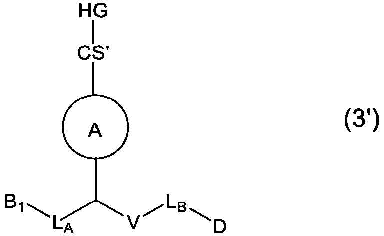 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a bioorthogonal functional group, and
D represents a functional substance.

In a specific embodiment, the structural unit represented by formula (3') may be represented by the following formula (3"): 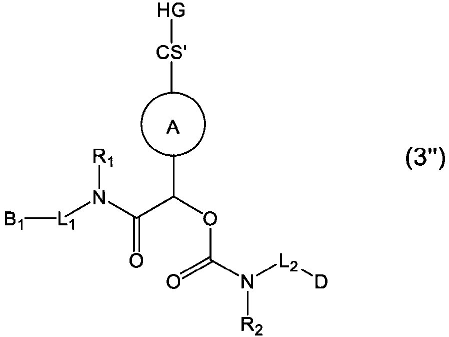 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a bioorthogonal functional group, and
D represents a functional substance.

The present invention also provides a reagent for derivatizing an antibody, the reagent comprising a compound, or a salt thereof, represented by the above formula (3') or a sub-formula thereof.

In a fourth embodiment, the present invention provides a compound having a first bioorthogonal functional group and a second bioorthogonal functional group, or a salt thereof, represented by the following formula (4'): 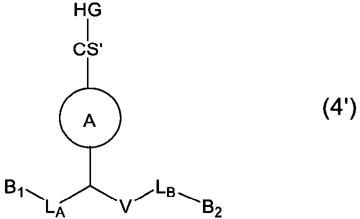 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group.

In a specific embodiment, the structural unit represented by formula (4') may be represented by the following formula (4"): 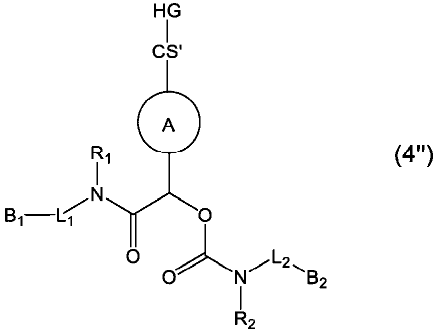 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

The present invention also provides a reagent for derivatizing an antibody or functional substance, the reagent comprising a compound, or a salt thereof, represented by the above formula (4') or a sub-formula thereof.

In a fifth embodiment, the present invention provides a compound, or a salt thereof, of the following (I'), (II'), or (III'):
(I') a compound, or a salt thereof, represented by the following formula (5'): 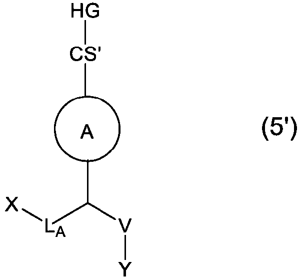 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site),
   V represents an oxygen atom, a sulfur atom, or an amino group (NH),
   L_{A} represents a divalent group, and
   X and Y each independently represent a monovalent group,
(II') a compound, or a salt thereof, represented by the following formula (6'): 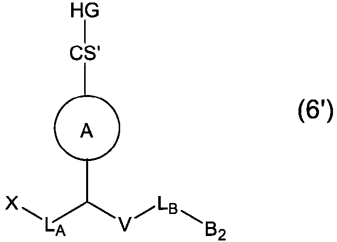 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site),
   V represents an oxygen atom, a sulfur atom, or an amino group (NH),
   L_{A} and L_{B} each independently represent a divalent group,
   X represents a monovalent group, and
   B₂ represents a bioorthogonal functional group, or
(III') a compound, or a salt thereof, represented by the following formula (7'): 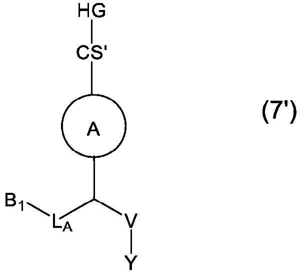 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site),
   V represents an oxygen atom, a sulfur atom, or an amino group (NH),
   L_{A} represents a divalent group,
   B₁ represents a bioorthogonal functional group, and
   Y represents a monovalent group.

In a specific embodiment, the compound of (I'), (II'), or (III') may be a compound of the following (I"), (II"), or (III"), respectively:
(I") a compound, or a salt thereof, represented by the following formula (5"): 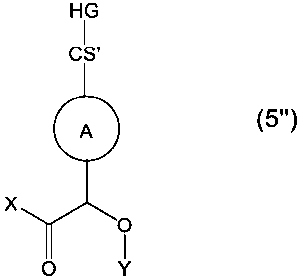 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site), and
   X and Y each independently represent a monovalent group,
(II") a compound, or a salt thereof, represented by the following formula (6"): 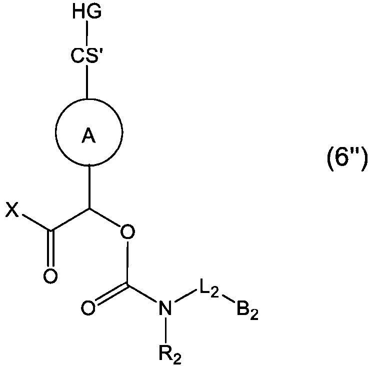 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site),
   R₂ represents a hydrogen atom or a monovalent group,
   L₂ represents a divalent group,
   X represents a monovalent group, and
   B₂ represents a bioorthogonal functional group, or
(III") a compound, or a salt thereof, represented by the following formula (7"): 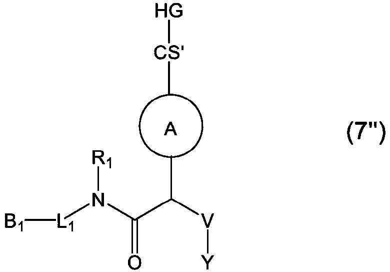 wherein
   HG represents a hydrophilic group or a monovalent group comprising a hydrophilic group,
   CS' represents a divalent group comprising a cleavable site,
   Ring A represents an optionally substituted divalent aromatic ring group (wherein the divalent aromatic ring group forms a pi electron conjugated system with the cleavable site),
   V represents an oxygen atom, a sulfur atom, or an amino group (NH),
   R₁ represents a hydrogen atom or a monovalent group,
   L₁ represents a divalent group,
   B₁ represents a bioorthogonal functional group, and
   Y represents a monovalent group.

In a preferred embodiment, the immunoglobulin unit may be a human immunoglobulin unit.

In a preferred embodiment, the human immunoglobulin unit may be a human IgG antibody.

In a preferred embodiment, the monovalent group comprising a hydrophilic group (HG-) may be a monovalent group represented by the following formula (A):

N(R_{HG1})(R_{HG2})-L_{HG}- (A)

wherein
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more moieties selected from the group consisting of L_{HG}, R_{HG1}, and R_{HG2}.

In a preferred embodiment, the divalent group optionally comprising a hydrophilic group (-L_{HG}-) may be the regioselective conjugate, or a salt thereof, of claim 6, which is a divalent group represented by the following formula (a):

-(C(R_{HG})₂)ₙ₁-(C=O)ₙ₂-(NR_{HG})ₙ₃-(C(R_{HG})₂)ₙ₄- (a)

wherein
the plurality of R_{HGS} each independently represent a hydrogen atom, a hydrophilic group, or a monovalent group optionally comprising a hydrophilic group,
n1 is an integer of 0 to 3,
n2 is an integer of 0 or 1,
n3 is an integer of 0 or 1, and
n4 is an integer of 0 to 3.

In a preferred embodiment, the divalent group represented by formula (a) may be a divalent group represented by the following formula (a1), (a2), or (a3):

(a1) -(C (R_{HG})₂)-;

(a2) -(C(R_{HG})₂)-(C=O)-(NR_{HG})-(C(R_{HG})₂)-; or

(a3) -(C=O)-(C(R_{HG})₂)₂-;

wherein
the plurality of R_{HGS} each independently represent a hydrogen atom, a hydrophilic group, or a C1-6 alkyl group comprising a hydrophilic group.

In a preferred embodiment, the hydrophilic group may be one or more groups selected from the group consisting of a carboxylic acid group, a sulfonic acid group, a sulfamide group, a hydroxyl group, a polyethylene glycol group, a polysarcosine group, and a sugar moiety.

In a preferred embodiment, the hydrophilic group may be one or more groups selected from the group consisting of a sulfonic acid group, a sulfamide group, a hydroxyl group, a polyethylene glycol group, a polysarcosine group, and a sugar moiety.

In a preferred embodiment, the cleavable site may be a site cleavable by an enzyme.

In a preferred embodiment, the enzyme may be one or more enzymes selected from the group consisting of cathepsin B, plasmin, legumain, and caspase.

In a preferred embodiment, the enzyme may be one or more enzymes selected from the group consisting of plasmin, legumain, and caspase.

In a preferred embodiment, the site cleavable by an enzyme may comprise VC, VA, AA, GGFG (SEQ ID NO: 2), FK, VK, AK, GC, VLK, NN, or DDVD (SEQ ID NO: 3).

In a preferred embodiment, the site cleavable by an enzyme may comprise AA, GGFG (SEQ ID NO: 2), FK, VK, AK, GC, VLK, NN, or DDVD (SEQ ID NO: 3).

In a preferred embodiment, the monovalent group comprising a hydrophilic group may comprise one or more amino acid residues comprising a carboxylic acid group in the side chain.

In a preferred embodiment, the structural unit consisted of HG-CS may comprise one or more amino acid residues comprising a carboxylic acid group in the side chain and a peptide moiety comprising a site cleavable by an enzyme.

In a preferred embodiment, the peptide moiety may comprise EVC, EEVC (SEQ ID NO: 4), EEEEVC (SEQ ID NO: 5), DVC, EVA, EAA, EGGFG (SEQ ID NO: 6), EFK, EEFK (SEQ ID NO: 7), EEVK (SEQ ID NO: 8), EEAK (SEQ ID NO: 9), EGC, EEVLK (SEQ ID NO: 10), ENN, EDDVD (SEQ ID NO: 11), β-Ala-VC, EGC, or EEGC (SEQ ID NO: 12).

In a preferred embodiment, the peptide moiety may comprise EAA, EGGFG (SEQ ID NO: 6), EFK, EEFK (SEQ ID NO: 7), EEVK (SEQ ID NO: 8), EEAK (SEQ ID NO: 9), EGC, EEVLK (SEQ ID NO: 10), ENN, EDDVD (SEQ ID NO: 11), β-Ala-VC, or EEGC (SEQ ID NO: 12).

In a preferred embodiment, the cleavable site may be a site cleavable under acidic or reducing conditions.

In a preferred embodiment, the cleavable site may be a disulfide bond.

In a preferred embodiment, L_{A} may be a divalent group having a side chain comprising a hydrophilic group or a functional substance.

In a preferred embodiment, the functional substance may be a pharmaceutical agent, a labeling substance, or a stabilizing agent.

In a preferred embodiment, the bioorthogonal functional group may be a maleimide residue, a thiol residue, a furan residue, a halocarbonyl residue, an alkene residue, an alkyne residue, an azide residue, or a tetrazine residue.

In a preferred embodiment, Ring A and/or Ring A1 may be an optionally substituted phenylene group.

### EFFECTS OF THE INVENTION

The conjugate or a salt thereof of the present invention can have excellent properties such as long in vivo residence time and high monomer ratio (low aggregation rate). The antibody derivatives and compounds, or salts thereof, and reagents of the present invention are useful, for example, as synthetic intermediates in the production of the conjugate.

### BRIEF DESCRIPTION OF THE DRAWINGS

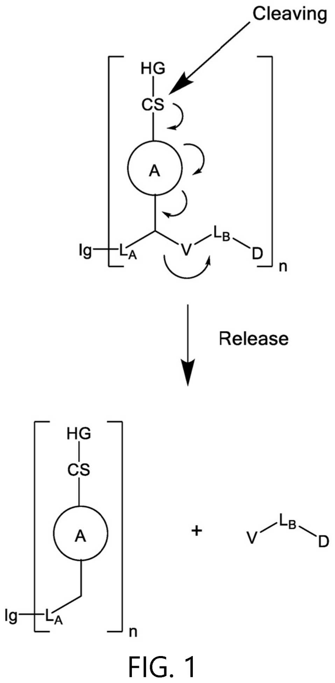

FIG. 1 shows an example of the release of a drug (an active form of the drug in which steric hindrance by the antibody has been eliminated) by the cleavage at a cleavable site and the involvement of a pi electron resonance system. In one embodiment, the conjugate, antibody derivative, and compound of the present invention can be designed to enable such release (see also Background Art as appropriate). The antibody can retain the hydrophilic group even after cleavage. That is, before cleavage, the drug is linked to a hydrophilic group in the form of a conjugate with an antibody, and thus the hydrophobicity of the drug can be inhibited (masking by the hydrophilic group), while after cleavage, the hydrophilic group dissociates from the drug, allowing the drug to exert its original hydrophobicity.

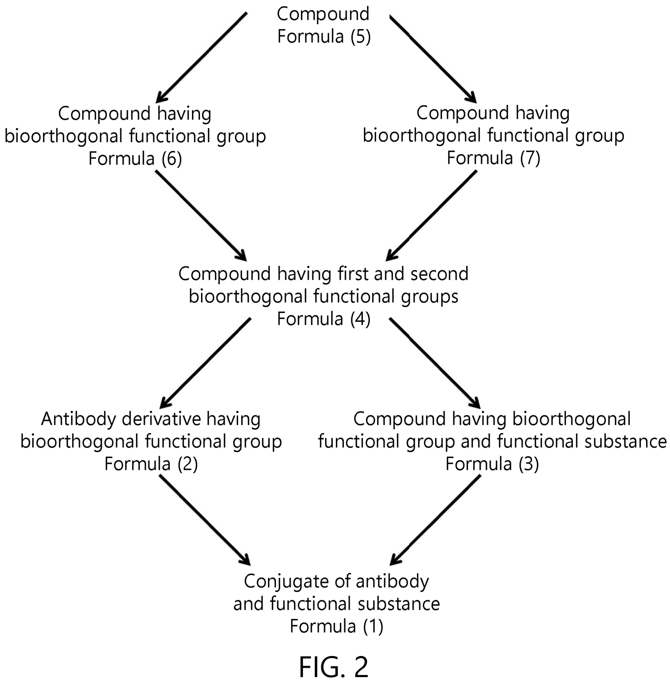

FIG. 2 shows the interrelationship of the conjugate of the present invention represented by formula (1), the antibody derivative of the present invention represented by formula (2), and the compounds of the present invention represented by formulas (3) to (7). These substances share the partial structural unit of the structural unit represented by formula (5), excluding X and Y. In addition, these substances can be synthesized by the scheme shown in FIG. 2. Thus, the present invention provides a series of inventions that are in the relationship of synthetic intermediates and final synthetic products.
FIG. 3 shows a synthesis outline of the conjugate of the present invention represented by formula (1), the antibody derivative of the present invention represented by formula (2), and the compounds of the present invention represented by formulas (3) and (4).
FIG. 4 shows an example of a synthesis outline of the compounds represented by formulas (4) to (7).
FIG. 5 shows an example of a synthesis outline of the compounds represented by formulas (4") to (7"), which are preferred examples of the compounds represented by formulas (4) to (7). DIPEA: N,N-diisopropylethylamine, DMF: N,N-dimethylformamide.

### MODE FOR CARRYING OUT THE INVENTION

### 1. Definitions of General Terms

In the present invention, the term "antibody" is as follows. The term "immunoglobulin unit" corresponds to a divalent monomer unit that is the basic constituent element of such an antibody, and is a unit comprising two heavy chains and two light chains. Therefore, for the immunoglobulin unit, the definitions, examples, and preferred examples of its origin, type (polyclonal or monoclonal, isotype, and full-length antibody or antibody fragment), antigen, and position of cysteine residues are the same as those for the antibody described below.

The origin of the antibody is not particularly limited, and for example, the antibody may be derived from an animal such as a mammal or a bird (e.g., a chicken). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), livestock (e.g., cows, pigs, and goats), and working animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bispecific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment comprising a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include proteins [including oligopeptides and polypeptides, or they may be proteins modified with a biomolecule such as a sugar (e.g., glycoproteins)], sugar chains, nucleic acids, and small compounds. The antibody may be preferably an antibody that recognizes a protein as an antigen. Examples of the protein include cell membrane receptors, cell membrane proteins other than cell membrane receptors (e.g., extracellular matrix proteins), ligands, and soluble receptors.

More specifically, the protein as an antigen of the antibody may be a disease target protein. Examples of the disease target protein include the following.

### (1) Oncology

PD-L1, GD2, PDGFRα (platelet-derived growth factor receptor), CD22, HER2, phosphatidylserine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican-3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil oxidase, DLK-1, TROP-2, α9 Integrin, TAG-72 (CA72-4), and CD70.

### (2) Autoimmune diseases and inflammatory diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 ligands, IL-21, Cadherin-11, CX3CL1, CCL20, IL-36R, IL-10R, CD86, TNF-α, IL-7R, Kv1.3, α9 Integrin, and LIFHT.

### (3) Cranial nerve diseases

CGRP, CD20, β amyloid, β amyloid protofibril, calcitonin gene-related peptide receptor, LINGO (Ig Domain Containing 1), α-synuclein, extracellular tau, CD52, insulin receptor, tau protein, TDP-43, SOD1, TauC3, and JC virus.

### (4) Infectious diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary and rare diseases

Amyloid AL, SEMA4D (CD100), insulin receptor, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic field

Sclerostin, Myostatin, Dickkopf-1, GDF8, RNAKL, HAS, and Siglec-15.

### (8) Hematologic diseases

vWF, Factor IXa, Factor X, IFNγ, C5, BMP-6, Ferroportin, and TFPI.

### (9) Other diseases

BAFF (B cell activating factor), IL-1β, PCSK9, NGF, CD45, TLR-2, GLP-1, TNFR1, C5, CD40, LPA, prolactin receptor, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

An immunoglobulin unit consisting of two heavy chains and two light chains (e.g., a divalent antibody such as IgG) has four disulfide bonds because the heavy-heavy chains and the heavy-light chains are linked by four disulfide bonds. When a reducing agent is allowed to act sufficiently on such an immunoglobulin unit, eight thiol groups are generated from the four disulfide bonds. As the reducing agent, any reducing agent capable of cleaving a disulfide bond to generate a thiol group can be used, and examples thereof include tricarboxyethylphosphine (TCEP), cysteine, dithiothreitol, reduced glutathione, and β-mercaptoethanol. Incidentally, even when all four disulfide bonds are reduced, the heavy and light chains of the antibody do not dissociate. This is because the higher-order structure of the antibody can be maintained by non-covalent bonds between the chains. These non-covalent bonds also maintain the antibody properties (target and binding affinity). For example, trastuzumab deruxtecan, known as Enhertu^{®}, is an ADC with a DAR = 8, in which drugs are conjugated to the thiol groups generated by reducing (cleaving) all four interchain disulfide bonds. Such ADCs are known to act as antigen-specific agents by maintaining their antibody properties.

According to the present invention, specific lysine residues (e.g., lysine residues at positions 246/248, 288/290, or 317) in the heavy chain of the immunoglobulin unit constituting the antibody can be regioselectively modified (see, e.g., WO2018/199337, WO2019/240288, WO2019/240287, and WO2020/090979). In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%.

Furthermore, in the present invention, an immunoglobulin unit comprising two heavy chains and two light chains can be bonded to a modifying group adjacent to Ig (e.g., L_{A}, L₁ described later) via the thiol groups in the side chains of a plurality of cysteine residues (a plurality of cysteine residues in the two heavy chains and two light chains comprised in the immunoglobulin unit) generated by allowing a reducing agent to act on the immunoglobulin unit. The number corresponding to the plurality is, for example, 2 or more (e.g., 2 to 8), preferably 3 or more (e.g., 3 to 8), more preferably 4 or more (e.g., 4 to 8), still more preferably 5 or more (e.g., 5 to 8), and particularly preferably 6 or more (e.g., 6 to 8), 7 or more (e.g., 7 to 8), or 8.

Furthermore, in the present invention, as long as the lysine residues and/or cysteine residues as described above in the immunoglobulin unit or antibody are modified, specific amino acid residues at other positions may be further regioselectively modified. For example, various methods are known for regioselectively modifying a specific amino acid residue at a predetermined position in an immunoglobulin unit or antibody. As such a specific amino acid residue, an amino acid residue having a side chain that is readily modified (e.g., amino group, carboxyl group, amide group, hydroxyl group) (e.g., aspartic acid residue, glutamic acid residue, asparagine residue, glutamine residue, threonine residue, serine residue, tyrosine residue) can be used, but preferably, a tyrosine residue, serine residue, and threonine residue having a side chain comprising a hydroxyl group can be used.

### Halogen atoms

Examples of the halogen atom include a fluorine atom, a chlorine atom, a bromine atom, and an iodine atom.

### Monovalent groups

Examples of the monovalent group include a monovalent hydrocarbon group and a monovalent heterocyclic group.

The monovalent group may be substituted with one or more (e.g., 1 to 10, preferably 1 to 8, more preferably 1 to 6, still more preferably 1 to 5, particularly preferably 1 to 3) substituents as described below.

### Monovalent hydrocarbon groups and related terms

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in the main chain thereof. It should be noted that the chain structure may be linear or branched. Examples of the monovalent chain hydrocarbon group include alkyl, alkenyl, and alkynyl. The alkyl, alkenyl, and alkynyl may be linear or branched.

The alkyl is preferably C1-12 alkyl, more preferably C1-6 alkyl, and still more preferably C1-4 alkyl. When the alkyl has a substituent, the number of carbon atoms does not include the number of carbon atoms of the substituent. Examples of the C1-12 alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C2-12 alkenyl, more preferably C2-6 alkenyl, and still more preferably C2-4 alkenyl. When the alkenyl has a substituent, the number of carbon atoms does not include the number of carbon atoms of the substituent. Examples of the C2-12 alkenyl include vinyl, propenyl, and n-butenyl.

The alkynyl is preferably C2-12 alkynyl, more preferably C2-6 alkynyl, and still more preferably C2-4 alkynyl. When the alkynyl has a substituent, the number of carbon atoms does not include the number of carbon atoms of the substituent. Examples of the C2-12 alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. It should be noted that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably C3-12 cycloalkyl, more preferably C3-6 cycloalkyl, and still more preferably C5-6 cycloalkyl. When the cycloalkyl has a substituent, the number of carbon atoms does not include the number of carbon atoms of the substituent. Examples of the C3-12 cycloalkyl include cyclopropyl, cyclobutyl, cyclopentyl, and cyclohexyl.

The cycloalkenyl is preferably C3-12 cycloalkenyl, more preferably C3-6 cycloalkenyl, and still more preferably C5-6 cycloalkenyl. When the cycloalkenyl has a substituent, the number of carbon atoms does not include the number of carbon atoms of the substituent. Examples of the C3-12 cycloalkenyl include cyclopropenyl, cyclobutenyl, cyclopentenyl, and cyclohexenyl.

The cycloalkynyl is preferably C3-12 cycloalkynyl, more preferably C3-6 cycloalkynyl, and still more preferably C5-6 cycloalkynyl. When the cycloalkynyl has a substituent, the number of carbon atoms does not include the number of carbon atoms of the substituent. Examples of the C3-12 cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic ring structure. It should be noted that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C6-12 aryl, more preferably C6-10 aryl, and still more preferably C6 aryl. When the monovalent aromatic hydrocarbon group has a substituent, the number of carbon atoms does not include the number of carbon atoms of the substituent. Examples of the C6-12 aryl include phenyl and naphthyl.

The monovalent aromatic hydrocarbon group is preferably phenyl.

Among these groups, the monovalent hydrocarbon group is preferably alkyl, cycloalkyl, or aryl.

### Monovalent heterocyclic groups and related terms

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more groups selected from the group consisting of an oxygen atom, a sulfur atom, a nitrogen atom, a phosphorus atom, a boron atom, and a silicon atom, and more preferably comprises one or more groups selected from the group consisting of an oxygen atom, a sulfur atom, and a nitrogen atom as a heteroatom comprised in the heterocyclic group.

The monovalent aromatic heterocyclic group is preferably a C1-15 aromatic heterocyclic group, more preferably a C1-9 aromatic heterocyclic group, and still more preferably a C1-6 aromatic heterocyclic group. When the monovalent aromatic heterocyclic group has a substituent, the number of carbon atoms does not include the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C2-15 nonaromatic heterocyclic group, more preferably a C2-9 nonaromatic heterocyclic group, and still more preferably a C2-6 nonaromatic heterocyclic group. When the monovalent nonaromatic heterocyclic group has a substituent, the number of carbon atoms does not include the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

### Divalent groups

The divalent group is a group having a main chain structure comprising one group or 2 or more (e.g., 2 to 10, preferably 2 to 8, more preferably 2 to 6, still more preferably 2 to 5, and particularly preferably 2 or 3) groups selected from the group consisting of a divalent linear hydrocarbon group, a divalent cyclic hydrocarbon group, a divalent heterocyclic group, -C(=O)-, -C(=S)-, -NR₇-, -C(=O)-NR₇-, -NR₇-C(=O)-, -C(=S)-NR₇-, -NR₇-C(=S)-, -O-, -S-, -(O-R₈)ₘ-, and -(S-R₈)ₘ₁-. R₇ indicates a hydrogen atom or a substituent as described below. R₈ indicates a divalent linear hydrocarbon group, a divalent cyclic hydrocarbon group, or a divalent heterocyclic group. m1 is an integer of 1 to 10, preferably an integer of 1 to 8, more preferably an integer of 1 to 6, still more preferably an integer of 1 to 5, and particularly preferably an integer of 1 to 3.

The divalent linear hydrocarbon group is a linear alkylene, a linear alkenylene, or a linear alkynylene. The linear alkylene is a C1-6 linear alkylene, and is preferably a C1-4 linear alkylene. Examples of the linear alkylene include methylene, ethylene, n-propylene, n-butylene, n-pentylene, and n-hexylene. The linear alkenylene is a C2-6 linear alkenylene, and is preferably a C2-4 linear alkenylene. Examples of the linear alkenylene include ethylenylene, n-propynylene, n-butenylene, n-pentenylene, and n-hexenylene. The linear alkynylene is a C2-6 linear alkynylene, and is preferably a C2-4 linear alkynylene. Examples of the linear alkynylene include ethynylene, n-propynylene, n-butynylene, n-pentynylene, and n-hexynylene. The divalent linear hydrocarbon group is preferably a linear alkylene.

The divalent cyclic hydrocarbon group is an arylene or a divalent nonaromatic cyclic hydrocarbon group. The arylene is preferably a C6-14 arylene, more preferably a C6-10 arylene, and particularly preferably a C6 arylene. Examples of the arylene include phenylene, naphthylene, and anthracenylene. The divalent nonaromatic cyclic hydrocarbon group is preferably a C3-12 monocyclic or polycyclic divalent nonaromatic cyclic hydrocarbon group, more preferably a C4-10 monocyclic or polycyclic divalent nonaromatic cyclic hydrocarbon group, and particularly preferably a C5-8 monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene. The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a heteroatom forming a heterocycle, one or more groups selected from the group consisting of an oxygen atom, a sulfur atom, a nitrogen atom, a phosphorus atom, a boron atom, and a silicon atom, and more preferably comprises one or more groups selected from the group consisting of an oxygen atom, a sulfur atom, and a nitrogen atom.

The divalent aromatic heterocyclic group is preferably a C3-15 divalent aromatic heterocyclic group, more preferably a C3-9 divalent aromatic heterocyclic group, and particularly preferably a C3-6 divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl. The divalent nonaromatic heterocyclic group is preferably a C3-15 nonaromatic heterocyclic group, more preferably a C3-9 nonaromatic heterocyclic group, and particularly preferably a C3-6 nonaromatic heterocyclic group. Examples of the divalent nonaromatic heterocyclic group include pyrrolidionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

Preferably, the divalent group is a divalent group having a main chain structure comprising one group selected from the group consisting of alkylene, arylene, -C(=O)-, -NR₇-, - C(=O)-NR₇-, -NR₇-C(=O)-, -O-, and -(O-R₈)ₘ-, or
a divalent group having a main chain structure comprising two or more groups selected from the group consisting of alkylene, arylene, -C(=O)-, -NR₇-, -C(=O)-NR₇-, -NR₇-C(=O)-, -O-, and -(OR₈)ₘ₁-,
R₇ is a hydrogen atom or alkyl,
R₈ is alkylene or arylene,
m1 may be an integer of 1 to 5 (or 1, 2, 3, 4, or 5).
The alkylene, arylene, and alkyl are as described above.

The main chain structure in the divalent group may be substituted with one or more (e.g., 1 to 10, preferably 1 to 8, more preferably 1 to 6, still more preferably 1 to 5, particularly preferably 1 to 3) substituents as described below.

### Substituents

Examples of the substituents include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O- (Rₐ indicates a hydrogen atom, or a monovalent hydrocarbon group); or
(vi) NR_{b}R_{c}-, NR_{b}R_{c}-C(=O)-, NR_{b}R_{c}-C(=O)-O-, or R_{b}-C(=O)-NR_{c}- (R_{b} and R_{c} are the same or different from each other, and each represents a hydrogen atom or a monovalent hydrocarbon group);
(vii) a nitro group, a sulfate group, a sulfonate group, a cyano group, and a carboxyl group.

The definitions, examples, and preferred examples of the halogen atom, the monovalent hydrocarbon group, and the monovalent heterocyclic group in the substituent are as described above.

The aralkyl refers to arylalkyl. The definitions, examples, and preferred examples of the aryl and the alkyl in the arylalkyl are as described above. The aralkyl is preferably C3-15 aralkyl. Examples of such an aralkyl include benzoyl, phenethyl, naphthylmethyl, and naphthylethyl.

Preferably, the substituent may be:
(i) a halogen atom;
(ii) a C1-12 alkyl, C1-12 phenyl, or C1-12 naphthyl;
(iii) a C3-15 aralkyl;
(iv) a 5- or 6-membered heterocycle;
(v) Rₐ-O-, Rₐ-C(=O)-, Rₐ-O-C(=O)-, or Rₐ-C(=O)-O- (where Rₐ represents a hydrogen atom, or a C1-12 alkyl);
(vi) NR_{b}R_{c}-, NR_{b}R_{c}-C(=O)-, NR_{b}R_{c}-C(=O)-O-, or R_{d}-C(=O)-NRₑ- (where R_{b} and R_{c} are the same as or different from each other, and each represents a hydrogen atom or a C1-12 alkyl); or
(vii) the same groups as listed in the item (vii) above.

More preferably, the substituent may be:
(i) a halogen atom;
(ii) a C1-12 alkyl;
(iii) Rₐ-O-, Rₐ-C(=O)-, Rₐ-O-C(=O)-, or Rₐ-C(=O)-O-, (where Rₐ represents a hydrogen atom or a C1-12 alkyl);
(iv) NR_{b}R_{c}-, NR_{b}R_{c}-C(=O)-, NR_{b}R_{c}-C(=O)-O-, or R_{d}-C(=O)-NR_{c}- (where R_{b} and R_{c} are the same as or different from each other, and each represents a hydrogen atom or a C1-12 alkyl); or
(v) the same groups as listed in the item (vii) above.

Even more preferably, the substituent may be:
(i) a halogen atom;
(ii) a C1-6 alkyl;
(iii) Rₐ-O-, Rₐ-C(=O)-, Rₐ-O-C(=O)-, or Rₐ-C(=O)-O- (where Rₐ represents a hydrogen atom or a C1-6 alkyl);
(iv) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represents a hydrogen atom or a C1-6 alkyl); or
(v) the same groups as listed in the item (vii) above.

Particularly preferably, the substituent may be:
(i) a halogen atom;
(ii) a C1-4 alkyl;
(iii) Rₐ-O-, Rₐ-C(=O)-, Rₐ-O-C(=O)-, or Rₐ-C(=O)-O- (where Rₐ represents a hydrogen atom or a C1-4 alkyl);
(iv) NR_{b}R_{c}-, NR_{b}R_{c}-C(=O)-, NR_{b}R_{c}-C(=O)-O-, or R_{b}-C(=O)-NR_{c}- (where R_{b} and R_{c} are the same as or different from each other, and each represents a hydrogen atom or a C1-4 alkyl); or
(v) the same groups as listed in the item (vii) above.

### Hydrophilic groups

A hydrophilic group is a group that can make the structural unit represented by formulas (1) to (7) or a sub-formula thereof more hydrophilic. By having a hydrophilic group at a predetermined site in the structural unit, the properties of the conjugate can be further improved. Examples of such a hydrophilic group include a carboxylic acid group, a sulfonic acid group, a sulfamide group, a hydroxyl group, a polyethylene glycol group, a polysarcosine group, and a sugar moiety. One or more (e.g., 1, 2, 3, 4, or 5) hydrophilic groups may be included in the conjugate.

A polyethylene glycol (PEG) group is a divalent group represented by -(CH₂-CH₂-O-)ₖ₁-. When the conjugate has a polyethylene glycol group, the conjugate may have a monovalent group in which one bond of the polyethylene glycol group is bonded to a hydrogen atom or a monovalent group (e.g., a monovalent hydrocarbon group). k1 may be, for example, an integer of 3 or more, preferably 4 or more, more preferably 5 or more, and still more preferably 6 or more. k1 may also be an integer of 15 or less, preferably 12 or less, more preferably 10 or less, and still more preferably 9 or less. More specifically, k1 may be an integer from 3 to 15, preferably from 4 to 12, more preferably from 5 to 10, and still more preferably from 4 to 9.

A polysarcosine group is a divalent group represented by -(NCH₃-CH₂-CO-)ₖ₂-. A polysarcosine group can be used as an alternative to PEG. k2 may be, for example, an integer of 3 or more, preferably 4 or more, more preferably 5 or more, and still more preferably 6 or more. k2 may also be an integer of 15 or less, preferably 12 or less, more preferably 10 or less, and still more preferably 9 or less. More specifically, k2 may be an integer from 3 to 15, preferably from 4 to 12, more preferably from 5 to 10, and still more preferably from 4 to 9.

The sugar moiety is a monosaccharide, an oligosaccharide (e.g., disaccharide, trisaccharide, tetrasaccharide, pentasaccharide), or a polysaccharide. The sugar moiety can include an aldose or a ketose, or a combination thereof. The sugar moiety may be a monosaccharide such as ribose, deoxyribose, xylose, arabinose, glucose, mannose, galactose, or fructose, or an amino sugar (e.g., glucosamine), or an oligosaccharide or polysaccharide comprising such a monosaccharide.

In a specific embodiment, the sugar moiety may be a low-molecular-weight hydrophilic group. A low-molecular-weight hydrophilic group refers to a hydrophilic group with a molecular weight of 1500 or less. The molecular weight of the low-molecular-weight hydrophilic group may be preferably 1200 or less, 1000 or less, 800 or less, 700 or less, 600 or less, 500 or less, 400 or less, 300 or less, 200 or less, or 100 or less. Examples of the low-molecular-weight hydrophilic group include a carboxylic acid group, a sulfonic acid group, and a hydroxyl group; and a polyethylene glycol group, a polysarcosine group, and a sugar moiety (e.g., monosaccharide, oligosaccharide) that satisfy the above molecular weight.

### Monovalent groups comprising a hydrophilic group

The monovalent group comprising a hydrophilic group is a monovalent group comprising a hydrophilic group as described above. The monovalent group is as defined above.

Preferably, the monovalent group comprising a hydrophilic group may be a monovalent group represented by the following formula (A):

N(R_{HG1})(R_{HG2})-L_{HG}, (A)

wherein
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more moieties selected from the group consisting of L_{HG}, R_{HG1}, and R_{HG2}. The hyphen (-) placed at the right end in formula (a) indicates a bond.

L_{HG} represents a bond or a divalent group optionally comprising a hydrophilic group. When R_{HG1} and R_{HG2} are each independently a hydrogen atom or a monovalent group not comprising a hydrophilic group, L_{HG} is a divalent group comprising a hydrophilic group. When at least one of R_{HG1} and R_{HG2} is a hydrophilic group or a monovalent group comprising a hydrophilic group, L_{HG} may be a divalent group optionally comprising a hydrophilic group, but is also preferably a divalent group not comprising a hydrophilic group.

R_{HG1} and R_{HG2} each independently represent a hydrogen atom, a hydrophilic group, or a monovalent group optionally comprising a hydrophilic group. The hydrophilic group and the monovalent group are as defined above.

**In** a specific embodiment, the monovalent group optionally comprising a hydrophilic group, represented by R_{HG1} and R_{HG2}, may be a protecting group for an amino group. Also, one of R_{HG1} and R_{HG2} may be a hydrogen atom, and the other may be a protecting group for an amino group. Examples of the protecting group for an amino group include an alkylcarbonyl group (an acyl group) (e.g., an acetyl group, a propoxy group, and a butoxycarbonyl group such as a tertbutoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group).

Alternatively, one of R_{HG1} and R_{HG2} may be a hydrogen atom, and the other may be a monovalent group comprising a hydrophilic group. Examples of the monovalent group comprising a hydrophilic group include an alkyl group comprising a hydrophilic group, a carboxyl group comprising a hydrophilic group, an alkylcarbonyl group comprising a hydrophilic group, an alkyloxycarbonyl group comprising a hydrophilic group, and an oxycarbonyl group comprising a hydrophilic group.

**In** the formula (A), at least one hydrophilic group is comprised in one or more moieties selected from the group consisting of L_{HG}, R_{HG1}, and R_{HG2}. Examples of the moiety comprising at least one hydrophilic group and combinations thereof include the following:
(i) L_{HG} alone;
(ii) R_{HG1} alone;
(iii) R_{HG2} alone;
(iv) a combination of L_{HG} and L_{HG1};
(v) a combination of L_{HG} and L_{HG2};
(vi) a combination of L_{HG1} and L_{HG2}; and
(vii) a combination of L_{HG}, L_{HG1}, and L_{HG2}.

Each of these L_{HG}, R_{HG1}, and R_{HG2} moieties may contain one hydrophilic group, or two or more hydrophilic groups.

### Divalent groups optionally comprising a hydrophilic group

The divalent group optionally comprising a hydrophilic group is a divalent group optionally comprising a hydrophilic group as described above. The divalent group is as described above.

Preferably, the divalent group optionally comprising a hydrophilic group may be a divalent group represented by the following formula (a):

-(C(R_{HG})₂)ₙ₁-(C=O)ₙ₂-(NR_{HG})ₙ₃-(C(R_{HG})₂)ₙ₄- (a)

wherein
the plurality of R_{HG}s each independently represent a hydrogen atom, a hydrophilic group, or a monovalent group optionally comprising a hydrophilic group,
n1 is an integer of 0 to 3,
n2 is an integer of 0 or 1,
n3 is an integer of 0 or 1, and
n4 is an integer of 0 to 3. The hyphens (-) placed at both ends in the formula (a) represent bonds.

In the formula (a), the plurality of R_{HG}s each independently represent a hydrogen atom, a hydrophilic group, or a monovalent group optionally comprising a hydrophilic group. The hydrophilic group and the monovalent group are as described above.

n1 is an integer from 0 to 3, preferably an integer from 0 to 2, and more preferably an integer of 0 or 1.

n2 is an integer of 0 or 1.

n3 is an integer of 0 or 1.

n4 is an integer from 0 to 3, preferably an integer from 0 to 2, and more preferably an integer of 0 or 1.

More preferably, the divalent group optionally comprising a hydrophilic group may be a divalent group represented by the following formula (a1), (a2), or (a3):

(a1) -(C(R_{HG})₂)-;

(a2) -(C(R_{HG})₂)-(C=O)-(NR_{HG})-(C(R_{HG})₂)-; or

(a3) -(C=O)-(C(R_{HG})₂)₂-.

In the formulas (a1), (a2), or (a3), the plurality of R_{HG}s each independently represent a hydrogen atom, a hydrophilic group, or a C1-6 alkyl group comprising a hydrophilic group. The hydrophilic group and the C1-6 alkyl group are as described above.

### Cleavable sites

A cleavable site is a site that can be cleaved in an appropriate environment (e.g., an intracellular or extracellular environment). Examples of cleavable sites include sites cleavable by enzymes (e.g., U.S. Patent No. 6,214,345; Dubowchik et al., Pharm. Therapeutics 83:67-123

(1999); The FEBS Journal 287:1936-1969 (2020)), sites cleavable under acidic conditions (sites cleavable at local acidic locations present in vivo) (e.g., U.S. Patent Nos. 5,622,929, 5,122,368; 5,824,805; The FEBS Journal 287:1936-1969 (2020)), sites cleavable under reducing conditions (e.g., Protein & Cell 9, 33-46 (2018)), and sites cleavable by blood glutathione (e.g., The FEBS Journal 287:1936-1969 (2020)). The cleavable site may be self-immolative (e.g., WO 02/083180, WO 04/043493, WO 05/112919).

The cleavable site may be a cleavable site comprising a peptide. The cleavable site comprising a peptide may be a cleavable site consisting of a cleavable peptide that is cleaved by an enzyme. The cleavable site comprising a peptide may also further contain a peptide not involved in cleavage, in addition to the cleavable peptide cleaved by an enzyme. The cleavable site comprising a peptide, even if it further contains a peptide not involved in cleavage, can be cleaved as long as the cleavable site contains a cleavable peptide that is cleaved by an enzyme.

The amino acid residues constituting the peptide in the cleavable site may be any amino acid residues. Examples of such any amino acid residues include α-amino acid residues, β-amino acid residues, and γ-amino acid residues, with α-amino acid residues being preferred. Examples of α-amino acid residues include alanine, asparagine, cysteine, glutamine, glycine, isoleucine, leucine, methionine, phenylalanine, proline, serine, threonine, tryptophan, tyrosine, valine, aspartic acid, glutamic acid, arginine, histidine, lysine, and citrulline residues. Such any amino acid residues may also be L-amino acid residues or D-amino acid residues, with L-amino acid residues being preferred. Preferably, any amino acid residue may be an L-α-amino acid residue (e.g., the L-form of the specific α-amino acid residue described above) or a glycine residue.

In a specific embodiment, the cleavable site comprising a peptide may contain a cleavable peptide consisting of amino acid residues selected from the group consisting of a valine residue, a phenylalanine residue, a threonine residue, a leucine residue, a citrulline residue, an alanine residue, a glutamic acid residue, a glutamine residue, a lysine residue, an arginine residue, and a methionine residue, or a combination thereof.

The number of amino acid residues constituting the peptide in the cleavable site may be 2 or more, 3 or more, 4 or more, 5 or more, or 6 or more. The number of amino acid residues constituting the peptide in the cleavable site may also be 20 or less, 15 or less, 12 or less, 10 or less, 8 or less, 6 or less, or 4 or less. More specifically, the number of amino acid residues constituting the peptide may be from 2 to 20, from 2 to 15, from 2 to 12, from 2 to 10, from 2 to 8, from 2 to 6, or from 2 to 4; may be from 3 to 20, from 3 to 15, from 3 to 12, from 3 to 10, from 3 to 8, from 3 to 6, or from 3 to 4; or may be from 4 to 20, from 4 to 15, from 4 to 12, from 4 to 10, from 4 to 8, or from 4 to 6.

**In** a specific embodiment, the number of amino acid residues constituting the peptide in the cleavable site may be 2. The number of amino acid residues constituting the peptide in the cleavable site may also be 3 or 4. Alternatively, the number of amino acid residues constituting the cleavable peptide may be 2. The number of amino acid residues constituting the cleavable peptide may also be 3 or 4.

In a preferred embodiment, the cleavable site may be a site cleavable by an enzyme. Examples of sites cleavable by an enzyme include sites cleavable by intracellular proteases (e.g., proteases present in lysosomes or endosomes) or extracellular proteases (e.g., secretory proteases) (typically, a cleavable peptide site). More preferably, the cleavable site is a site cleavable by an intracellular protease (e.g., a protease present in lysosomes or endosomes). Still more preferably, the cleavable site is a site cleavable by a protease present in lysosomes. Particularly preferably, the cleavable site is a site cleavable by cathepsin B, plasmin, legumain, and caspases.

More specifically, the site cleavable by cathepsin B, plasmin, legumain, and caspases may be a cleavable site comprising VC, VA, AA, GGFG (SEQ ID NO: 2), FK, VK, AK, GC, VLK, NN, or DDVD (SEQ ID NO: 3). The peptide sites of VC, VA, AA, GGFG (SEQ ID NO: 2), FK, VK, AK, and GC can be cleaved by cathepsin B. The VLK peptide site is known to be cleaved by interstitial plasmin. The NN peptide site is known to be cleaved by legumain. The DDVD peptide site is known to be cleaved by caspase. Examples of such cleavable sites include EVC, EEVC (SEQ ID NO: 4), EEEEVC (SEQ ID NO: 5), DVC, EVA, EAA, EGGFG (SEQ ID NO: 6), EFK, EEFK (SEQ ID NO: 7), EEVK (SEQ ID NO: 8), EEAK (SEQ ID NO: 9), EGC, EEVLK (SEQ ID NO: 10), ENN, EDDVD (SEQ ID NO: 11), β-Ala-VC, EGC, and EEGC (SEQ ID NO: 12).

In another preferred embodiment, the cleavable site may be a site cleavable under acidic or reducing conditions. Examples of sites cleavable under acidic conditions include alkyloxyarylalkyl residues, tertiary alkyloxycarbamate residues, acetal residues, silane residues, imine residues, vinyl ether residues, β-thiopropionate residues, trityl residues, hydrazone residues, aconityl residues, orthoester residues, carbamoyl residues, and 2-(diphenylphosphino)benzoate residues. Examples of sites cleavable under reducing conditions include disulfide residues, alkoxyalkyl residues, and azo residues.

In a specific embodiment, the divalent group comprising a cleavable site, when adjacent to an optionally substituted divalent aromatic ring group (e.g., Ring A, Ring A1), may satisfy the following conditions (i) to (iii):
(i) the cleavable site is -CO-W- (where the hyphen (-) represents a bond, W is an oxygen atom, a sulfur atom, or an amino group (NH), and is bonded to the above-described divalent aromatic ring group);
(ii) the bond between CO and W is the site that undergoes cleavage; and
(iii) the above-described divalent aromatic ring group constitutes a pi electron conjugated system with W.

When a divalent group comprising a site cleavable by cathepsin B satisfies the above conditions (i) to (iii), the cleavage of the bond between CO and W, in conjunction with the pi electron conjugated system and V in the divalent aromatic ring group in Ring A (wherein the divalent aromatic ring group constitutes the pi electron conjugated system with the cleavable site), can efficiently cleave the bond between the tertiary carbon atom connected to Ring A and V (FIG. 1). W is preferably an oxygen atom or an amino group (NH), and more preferably an amino group (NH). Therefore, the cleavable site is preferably an amide bond site.

In a specific embodiment, the divalent group comprising a cleavable site, represented by CS, may be -L_{CSa}-CSₐ-W_{csa}- [wherein, the hyphen (-) represents a bond, L_{CSa} represents a bond or a divalent group, CSₐ represents a cleavable site, and W_{cs} represents an oxygen atom, a sulfur atom, or an amino group (NH).] The definition, examples, and preferred examples of the divalent group represented by L_{CSa} are the same as for the divalent group described above. The definition, examples, and preferred examples of the cleavable site represented by CSₐ are the same as for the cleavable site described above. W_{csa} reprsents an oxygen atom, a sulfur atom, or an amino group (NH), preferably represents an oxygen atom or an amino group (NH), and more preferably represents an amino group (NH).

In another specific embodiment, the divalent group comprising a cleavable site, represented by CS, may be -L_{CSb}-CS_{b}-W_{csb}- [wherein, the hyphen (-) represents a bond, L_{CSb} represents a bond or a divalent group, CS_{b} represents a cleavable site, and W_{csb} represents a bond or a divalent group.] The definition, examples, and preferred examples of the divalent group represented by L_{CSb} and W_{csb} are the same as for the divalent group described above. The definition, examples, and preferred examples of the cleavable site represented by CS_{b} are the same as for the cleavable site described above. Preferably, in this embodiment, a disulfide bond is preferable as CS_{b}, and an alkylene (e.g., methylene) is preferable as the divalent group of W_{csa}.

### Bioorthogonal functional groups

Bioorthogonal functional groups refer to groups that do not react with biological components (e.g., amino acids, proteins, nucleic acids, lipids, carbohydrates, and phosphates), or react slowly with biological components but selectively with non-biological components. Bioorthogonal functional groups are well known in the art (see, e.g., Sharpless K. B. et al., Angew. Chem. Int. Ed. 40,2004 (2015); Bertozzi C. R. et al., Science 291,2357(2001); Bertozzi C. R. et al., Nature Chemical Biology 1,13 (2005)).

In the present invention, bioorthogonal functional groups for proteins are used as bioorthogonal functional groups. This is because antibodies to be derivatized with the reagent of the present invention are proteins. The bioorthogonal functional groups for proteins are groups that do not react with the side chains of the 20 naturally-occurring amino acid residues constituting proteins, or react slowly with the side chains but react with a target functional group. The 20 naturally-occurring amino acids constituting proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (K). Of these 20 naturally-occurring amino acids, glycine that has no side chain (i.e., the side chain is a hydrogen atom), and alanine, isoleucine, leucine, phenylalanine, and valine with a side chain that is a hydrocarbon group (i.e., the side chain does not contain any heteroatom selected from the group consisting of a sulfur atom, a nitrogen atom, and an oxygen atom) are inactive to common reactions. Thus, the bioorthogonal functional groups for proteins are groups that do not react not only with the side chains of these amino acids wherein the side chains are inactive to common reactions, but also with the side chains of asparagine, glutamine, methionine, proline, serine, threonine, tryptophan, tyrosine, aspartic acid, glutamic acid, arginine, histidine, and lysine, or react slowly with them but react with a target functional group.

Examples of such bioorthogonal functional groups include azide residues, aldehyde residues, thiol residues, alkene residues (in other words, the residues may have a vinylene (ethenylene) moiety, which is a minimum unit having a carbon-carbon double bond; the same applies hereinafter), alkyne residues (in other words, the residues may have an ethynylene moiety, which is a minimum unit having a carbon-carbon triple bond; the same applies hereinafter), halogen residues, tetrazine residues, nitrone residues, hydroxylamine residues, nitrile residues, hydrazine residues, ketone residues, boronic acid residues, cyanobenzothiazole residues, allyl residues, phosphine residues, maleimide residue, disulfide residues, thioester residues, α-halocarbonyl residues (e.g., carbonyl residues having a fluorine atom, a chlorine atom, a bromine atom, or an iodine atom at the alpha position. The same shall apply hereinafter), isonitrile residues, sydnone residues, and selenium residues.

More specifically, the bioorthogonal functional group may correspond to any one chemical structure selected from the group below. 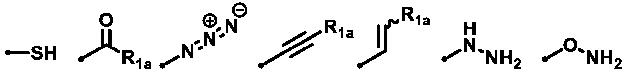 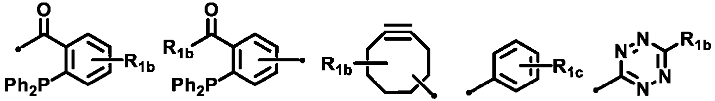 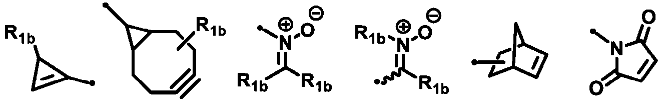 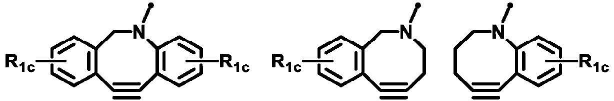 wherein
R₁ₐ, a single or a plurality of R_{1b}s, and a single or a plurality of R_{1c}s are the same or different, and are the above-mentioned substituents or electron-withdrawing groups, and
• is a bond.

Examples of the electron-withdrawing group include a halogen atom, an alkyl substituted with a halogen atom (e.g., trifluoromethyl), a boronic acid residue, mesyl, tosyl, triflate, nitro, cyano, phenyl group, and a keto group (e.g., acyl), with a halogen atom, a boronic acid residue, mesyl, tosyl, and triflate being preferred.

In a specific embodiment, the bioorthogonal functional group may be protected. An optionally protected bioorthogonal functional group refers to an unprotected bioorthogonal functional group or a protected bioorthogonal functional group. The unprotected bioorthogonal functional group corresponds to the bioorthogonal functional group as described above. The protected bioorthogonal functional group is a group that generates a bioorthogonal functional group by cleavage of the protecting group. The cleavage of the protecting group can be performed by a specific treatment under conditions (mild conditions) that cannot cause protein denaturation or degradation (e.g., cleavage of an amide bond). Examples of such a specific treatment include (a) treatment with one or more substance selected from the group consisting of an acidic substance, a basic substance, a reducing agent, an oxidizing agent, and an enzyme, (b) treatment with physicochemical stimulation selected from the group consisting of light, or (c) incubation using a cleavable linker comprising a self-degradable cleavable site. Such protecting groups and the conditions for cleaving them are common technical knowledge in the art (e.g., G. Leriche, L. Chisholm, A. Wagner, Bioorganic & Medicinal Chemistry. 20,571 (2012); Feng P. et al., Journal of the American Chemical Society. 132,1500 (2010).; Bessodes M. et al., Journal of Controlled Release, 99,423 (2004).; DeSimone, J. M., Journal of American Chemical Society. 132,17928 (2010); Thompson, D. H., Journal of Controlled Release, 91,187 (2003); Schoenmarks, R. G., Journal of Controlled Release, 95,291 (2004)).

Examples of the protected bioorthogonal functional group include disulfide residues, ester residues, acetal residues, ketal residues, imine residues, and vicinal diol residues.

More specifically, the protected bioorthogonal functional group may correspond to any one chemical structure selected from the group consisting of the following. 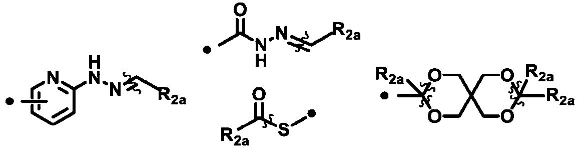 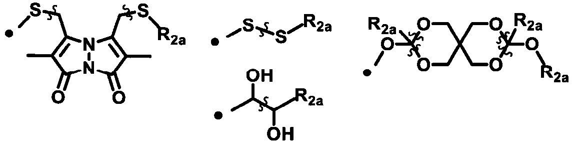 wherein
the wavy line orthogonal to the bond represents a cleavage site,
the single or plurality of R₂ₐs, which are the same or different, are selected from the group consisting of a hydrogen atom and the above-mentioned substituents, and
• is a bond.

Preferably, the optionally protected bioorthogonal functional group is an unprotected bioorthogonal functional group.

### Functional substances

The functional substance is not particularly limited as long as it is a substance that imparts an arbitrary function to the antibody, and examples thereof include drugs, labeling substances, and stabilizers, with drugs or labeling substances being preferred. The functional substance may be a single functional substance or a substance in which two or more functional substances are linked with each other.

The drug may be a drug for any disease. Examples of such a disease include cancer (e.g., lung cancer, stomach cancer, colon cancer, pancreatic cancer, renal cancer, liver cancer, thyroid cancer, prostate cancer, bladder cancer, ovarian cancer, uterine cancer, bone cancer, skin cancer, a brain tumor, and melanoma), autoimmune diseases and inflammatory diseases (e.g., allergic diseases, rheumatoid arthritis, and systemic lupus erythematosus), neurological diseases (e.g., cerebral infarction, Alzheimer's disease, Parkinson disease, and amyotrophic lateral sclerosis), infectious diseases (e.g., microbial infectious diseases and viral infectious diseases), hereditary and rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukemia and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes, and substances comprising them. Examples of chemotherapeutic agents include DNA-damaging agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorus atom (e.g., ³²P), radioisotopes of a sulfur atom (e.g., ³⁵S), radioisotopes of yttrium (e.g., ⁹⁰Y), radioisotopes of technetium (e.g., ^{99m}Tc), radioisotopes of indium (e.g., ¹¹¹In), radioisotopes of an iodine atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracyclines, dmDNA31, tubulysin, and exatecan.

The labeling substance is a substance that makes detection of a target (e.g., a tissue, a cell, or a substance) possible. Examples of the labeling substance include enzymes (e.g., peroxidase, alkaline phosphatase, luciferase, and β-galactosidase), affinity substances (e.g., streptavidin, biotin, digoxigenin, and aptamers), fluorescent substances (e.g., fluorescein, fluorescein isothiocyanate, rhodamine, green fluorescent proteins, and red fluorescent proteins), luminescent substances (e.g., luciferin, aequorin, acridinium esters, tris(2,2'-bipyridyl) Ruthenium, and luminol), and radioisotopes (e.g., those described above) or substances comprising them.

The stabilizer is a substance that enables stabilization of an antibody. Examples of the stabilizer include diols, glycerin, nonionic surfactants, anionic surfactants, natural surfactants, saccharides, and polyols.

The functional substance may also be a peptide, a protein, a nucleic acid, a low molecular weight organic compound, a sugar chain, a lipid, a high-molecular-weight polymer, a metal (e.g., gold), or a chelator. Examples of the peptide include cell membrane permeable peptides, blood-brain barrier permeable peptides, and peptide medicaments. Examples of the protein include enzymes, cytokines, fragment antibodies, lectins, interferons, serum albumin, and antibodies. Examples of the nucleic acid include DNAs, RNAs, and artificial nucleic acids. Examples of the nucleic acid also include RNA interference inducible nucleic acids (e.g., siRNAs), aptamers, and antisense nucleic acids. Examples of the low molecular weight organic compound include proteolysis targeting chimeras, dyes, and photodegradable compounds.

### Salts

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 2. Conjugate or a salt thereof

The present invention provides a conjugate of an antibody and a functional substance, or a salt thereof, comprising a structural unit represented by the following formula (1): 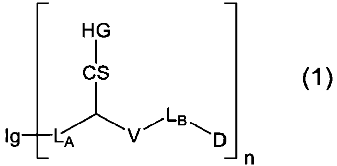 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

In formula (1) and other formulas presented in relation to the present invention, a hyphen (-) indicates that two units present on both sides thereof are covalently bonded to each other. When a unit is not present on one side of a hyphen (-), that hyphen (-) represents a bond.

The antibody comprises an immunoglobulin unit as described above. Examples of such an antibody include IgG antibodies, IgD antibodies, and IgE antibodies, which contain one immunoglobulin unit comprising two heavy chains and two light chains and having disulfide bonds between the heavy chains and between the heavy and light chains; IgA antibodies comprising two such immunoglobulin units; and IgM antibodies comprising four such immunoglobulin units, with IgG antibodies (e.g., IgG1, IgG2, IgG3, IgG4) being preferred. The antibody is preferably a human IgG monoclonal antibody, and more preferably a human IgG full-length monoclonal antibody.

The bond between the antibody and the linker moiety (L_{A}) can be achieved via a functional group in the side chain of a specific amino acid residue in the Ig. Examples of the functional group in the side chain of a specific amino acid residue in the Ig include the amino group in the side chain of a lysine residue in the Ig and the thiol group in the side chain of a cysteine residue in the Ig. When the functional group in the side chain of a specific amino acid residue in the Ig is the amino group in the side chain of a lysine residue, the bond can be achieved by a bond between the amino group in the side chain of the lysine residue and an atom or group capable of bonding thereto (e.g., a carbonyl group). When the functional group in the side chain of a specific amino acid residue in the Ig is the thiol group in the side chain of a cysteine residue, the bond can be achieved by a bond between the thiol group in the side chain of the cysteine residue and an atom or group capable of bonding thereto (e.g., a maleimide residue, a carbonyl group, or a thiol group).

In formula (1), HG represents a hydrophilic group or a monovalent group comprising a hydrophilic group. The hydrophilic group and the monovalent group are as described above. Preferably, HG may represent a monovalent group comprising a hydrophilic group.

CS represents a divalent group comprising a cleavable site. The cleavable site and the divalent group are as described above.

V represents an oxygen atom, a sulfur atom, or an amino group (NH). V is preferably an oxygen atom or a sulfur atom, and more preferably an oxygen atom.

The divalent group represented by L_{A} is a divalent group capable of linking Ig and a carbon atom adjacent to Ring A. The divalent group represented by L_{B} is a divalent group capable of linking D and V.

In a specific embodiment, the divalent groups represented by L_{A} and L_{B} may each contain a moiety generated by the reaction of two mutually reactive bioorthogonal functional groups. Since combinations of two mutually reactive bioorthogonal functional groups are well known, a person skilled in the art can appropriately select such a combination and appropriately set a divalent group comprising a moiety generated by the reaction of the two mutually reactive bioorthogonal functional groups. Examples of combinations of mutually reactive bioorthogonal functional groups include a combination of a thiol residue and a maleimide residue, a combination of a furan residue and a maleimide residue, a combination of a thiol residue and a halocarbonyl residue (halogen is substituted with thiol via substitution reaction), a combination of an alkyne residue (preferably, a cyclic group that may be substituted with a substituent as described above and has a carbon-carbon triple bond) and an azide residue, a combination of a tetrazine residue and an alkene residue, a combination of a tetrazine residue and an alkyne residue, and a combination of a thiol residue and another thiol residue (disulfide bond). Therefore, the moiety may be a group generated by the reaction of a thiol residue and a maleimide residue, a group generated by the reaction of a furan residue and a maleimide residue, a group generated by the reaction of a thiol residue and a halocarbonyl residue, a group generated by the reaction of an alkyne residue and an azide residue, or a group generated by the reaction of a tetrazine residue and an alkene residue, or a disulfide group generated by the combination of a thiol residue and another thiol residue.

In a specific embodiment, the moiety may be a divalent group represented by any one structural formula of the following structural formulas:

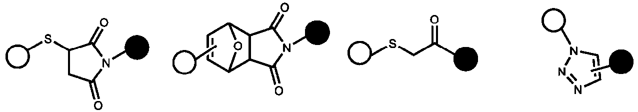

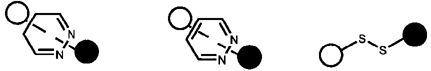

wherein the white circle and the black circle represent bonds.

In L_{A}, if the bond of the white circle is bonded to an atom present on the Ig-binding site side, the bond of the black circle may be bonded to an atom present on the carbon atom side adjacent to Ring A, and if the bond of the white circle is bonded to an atom present on the carbon atom side adjacent to Ring A, the bond of the black circle may be bonded to an atom present on the Ig-binding site side.

In L_{B}, if the bond of the white circle is bonded to an atom present on the V side, the bond of the black circle may be bonded to an atom present on the functional substance (D) binding site side, and if the bond of the white circle is bonded to an atom present on the functional substance (D) binding site side, the bond of the black circle may be bonded to an atom present on the V side.

In a preferred embodiment, the divalent group represented by L_{A} may be -L₁-N(-R₁)-CO-. Here, L₁ represents a divalent group, R₁ represents a hydrogen atom or a monovalent group, and the hyphens present on the left side of L₁ and the right side of CO represent bonds. Preferably, the bond of L₁ is bonded to Ig, and the bond of CO is bonded to a carbon atom adjacent to Ring A.

In another preferred embodiment, the divalent group represented by L_{B} may be -L₂-N(-R₂)-CO-. Here, L₂ represents a divalent group, R₂ represents a hydrogen atom or a monovalent group, and the hyphens present on the left side of L₂ and the right side of CO represent bonds. Preferably, the bond of L₂ is bonded to the functional substance (D), and the bond of CO is bonded to V.

In a specific embodiment, the divalent groups represented by L₁ and L₂ may each contain a moiety generated by the reaction of two mutually reactive bioorthogonal functional groups. The moiety generated by the reaction of two mutually reactive bioorthogonal functional groups, which may be comprised in the divalent groups represented by L₁ and L₂, is the same as the moiety generated by the reaction of two mutually reactive bioorthogonal functional groups as described above, which may be comprised in the divalent groups represented by L_{A} and L_{B}.

R₁ and R₂ each independently represent a hydrogen atom or a monovalent group. The monovalent group is as described above. The monovalent group in R₁ and R₂ is preferably an optionally substituted monovalent hydrocarbon group, more preferably an optionally substituted alkyl group, and still more preferably an alkyl group. As the alkyl group, those mentioned above are preferred.

In a specific embodiment, the monovalent group represented by R₁ and R₂ may be a protecting group for an amino group. Examples of such a protecting group for an amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group).

In a preferred embodiment, R₁ and R₂ each independently represent a hydrogen atom or a protecting group for an amino group. Preferably, R₁ and R₂ may each be a hydrogen atom.

The functional substance represented by D is as described above.

n represents the average number of the bonds per immunoglobulin unit comprising two heavy chains and two light chains, and is 1.5 or more.

In one embodiment, when the functional group in the side chain of a specific amino acid residue in Ig is the amino group in the side chain of a lysine residue, such an average number may be 1.6 or more, preferably 1.7 or more, more preferably 1.8 or more, and still more preferably 1.9 or more. Such an average number may also be 2.4 or less, preferably 2.3 or less, more preferably 2.2 or less, and still more preferably 2.1 or less. Particularly preferably, such an average number may be 2.0.

In another embodiment, when the functional group in the side chain of a specific amino acid residue in Ig is the thiol group in the side chain of a cysteine residue, such an average number may be, for example, 2.0 or more, preferably 4.0 or more, more preferably 6.0 or more, still more preferably 7.0 or more, and particularly preferably 7.5 or more, or 7.8 or more. Such an average number may also be 8.0 or less. More specifically, such an average number may preferably be 2.0 to 8.0, more preferably 4.0 to 8.0, still more preferably 6.0 to 8.0, and particularly preferably 7.0 to 8.0, 7.5 to 8.0, 7.8 to 8.0, or 8.0.

In a specific embodiment, L_{A} may be a divalent group having a side chain comprising a hydrophilic group or a functional substance. The side chain comprising a hydrophilic group or a functional substance may be a side chain comprising a hydrophilic group and a functional substance. The side chain comprising a hydrophilic group and a functional substance may be a side chain comprising (i) a hydrophilic group, (ii) a functional substance, and (iii) a cleavable site between the hydrophilic group and the functional substance. The number of such side chains comprised in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (α): 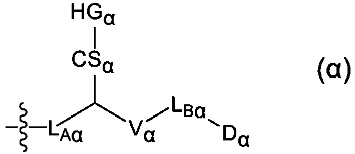 wherein
the hyphen (-) with a wavy line represents a bond to the main chain in L_{A} connecting Ig and a carbon atom in formula (1),
HG_{α} represents a hydrophilic group or a monovalent group comprising a hydrophilic group,
CS_{α} represents a divalent group comprising a cleavable site,
V_{α} represents an oxygen atom, a sulfur atom, or an amino group (NH),
L_{Aα} and L_{Bα} each independently represent a divalent group, and
D_{α} represents a functional substance.

HG_{α} represents a hydrophilic group or a monovalent group comprising a hydrophilic group. The hydrophilic group and the monovalent group are as described above. The hydrophilic group or the monovalent group comprising a hydrophilic group represented by HG_{α} may be the same as or different from the hydrophilic group or the monovalent group comprising a hydrophilic group represented by HG.

CS_{α} represents a divalent group comprising a cleavable site. The cleavable site and the divalent group are as described above. The divalent group comprising a cleavable site represented by CS_{α} may be the same as or different from the divalent group comprising a cleavable site represented by CS.

V_{α} represents an oxygen atom, a sulfur atom, or an amino group (NH). The type of V_{α} may be the same as or different from the type of V. Therefore, when V is an oxygen atom, V_{α} may be a sulfur atom or an amino group (NH). V_{α} is preferably an oxygen atom or a sulfur atom, and more preferably an oxygen atom.

The divalent group represented by L_{Aα} is a divalent group having a bond to the main chain in L_{A} connecting Ig and a carbon atom. The divalent group represented by L_{Aα} may be the same as or different from the divalent group represented by L_{A}. The divalent group represented by L_{Bα} is a divalent group capable of linking D_{α} and V_{α}. The divalent group represented by L_{Bα} may be the same as or different from the divalent group represented by L_{B}. Therefore, in a specific embodiment, the divalent groups represented by L_{Aα} and L_{Bα} may contain a moiety generated by the reaction of two mutually reactive bioorthogonal functional groups, similar to the divalent groups represented by L_{A} and L_{B}, respectively.

D_{α} represents a functional substance. The functional substance is as described above. The functional substance represented by D_{α} may be the same as or different from the functional substance represented by D.

The present invention also provides a conjugate of an antibody and a functional substance, or a salt thereof, comprising a structural unit represented by the following formula (1'): 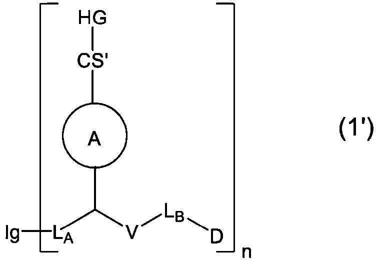 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

In formula (1'), Ig, HG, V, L_{A}, L_{B}, D, and n are each the same as those represented in formula (1).

CS' represents a divalent group comprising a cleavable site. The cleavable site and the divalent group are as described above.

Ring A represents an optionally substituted divalent aromatic ring group (wherein the divalent aromatic ring group forms a pi electron conjugated system with the cleavable site). The divalent aromatic ring group is the arylene or divalent aromatic heterocyclic group described above. The position of the divalent aromatic ring group to which the two adjacent atoms (a carbon atom and an adjacent atom in CS') are bonded is not particularly limited as long as cleavage occurs between V and its adjacent carbon atom due to the conjugation of pi electrons when the cleavable site is cleaved (see FIG. 1). Such a position is well known to those skilled in the art, and can be readily determined by a person skilled in the art according to factors such as the types of cleavable site and divalent aromatic ring group.

Preferably, Ring A may be an optionally substituted divalent monocyclic aromatic ring group. The divalent aromatic ring group is a phenylene group or a divalent monocyclic aromatic heterocyclic group.

More preferably, Ring A may be a divalent 6-membered cyclic aromatic ring group. Examples of the 6-membered cyclic aromatic ring group include the various groups described above. In this case, the position of the divalent 6-membered cyclic aromatic ring group to which the two adjacent atoms are bonded is the ortho position or the para position, preferably the para position.

Still more preferably, Ring A may be an optionally substituted phenylene group. In this case, the position of the phenylene group to which the two adjacent atoms are bonded is the ortho position or the para position, preferably the para position.

The substituent in the optionally substituted divalent aromatic ring group is as described above. Such a substituent may be an electron-withdrawing group as described above.

In a specific embodiment, L_{A} may be a divalent group having a side chain comprising a hydrophilic group or a functional substance. The side chain comprising a hydrophilic group or a functional substance may be a side chain comprising a hydrophilic group and a functional substance. The side chain comprising a hydrophilic group and a functional substance may be a side chain comprising (i) a hydrophilic group, (ii) a functional substance, and (iii) a cleavable site between the hydrophilic group and the functional substance. The number of such side chains comprised in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (α'): 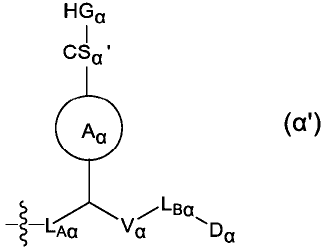 wherein
the hyphen (-) with a wavy line represents a bond to the main chain in L_{A} connecting Ig and a carbon atom in formula (1'),
HG_{α} represents a hydrophilic group or a monovalent group comprising a hydrophilic group,
CS_{α}' represents a divalent group comprising a cleavable site,
Ring A_{α} represents an optionally substituted divalent aromatic ring group (wherein the divalent aromatic ring group forms a pi electron conjugated system with the cleavable site),
V_{α} represents an oxygen atom, a sulfur atom, or an amino group (NH),
L_{Aα} and L_{Bα} each independently represent a divalent group, and
D_{α} represents a functional substance.

In formula (α'), HG_{α}, V_{α}, L_{Aα}, L_{Bα}, and D_{α} are each the same as those represented by formula (α).

CS_{α}' represents a divalent group comprising a cleavable site. The cleavable site and the divalent group are as described above. The divalent group comprising a cleavable site represented by CS_{α}' may be the same as or different from the divalent group comprising a cleavable site represented by CS'.

Ring A_{α} represents an optionally substituted divalent aromatic ring group (wherein the divalent aromatic ring group forms a pi electron conjugated system with the cleavable site). The optionally substituted divalent aromatic ring group is as described above. The optionally substituted divalent aromatic ring group represented by Ring A_{α} may be the same as or different from the optionally substituted divalent aromatic ring group represented by Ring A.

The present invention also provides a conjugate of an antibody and a functional substance, or a salt thereof, comprising a structural unit represented by the following formula (1"): 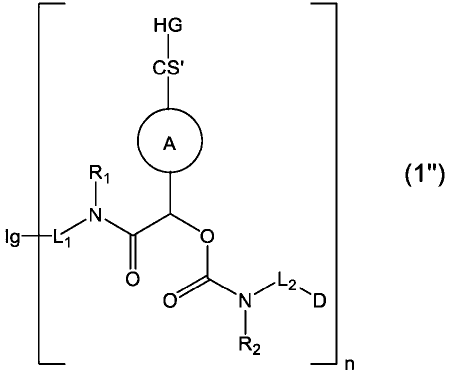 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
D represents a functional substance, and
the average number (n) of said bonds per the immunoglobulin unit is 1.5 or more.

In formula (1"), Ig, HG, D, and n are each the same as those represented in formula (1). In formula (1"), CS' and Ring A are each the same as those represented by formula (1').

R₁ and R₂ each independently represent a hydrogen atom or a monovalent group. The monovalent group is as described above. The monovalent group in R₁ and R₂ is preferably an optionally substituted monovalent hydrocarbon group, more preferably an optionally substituted alkyl group, and still more preferably an alkyl group. As the alkyl group, those mentioned above are preferred.

In a specific embodiment, the monovalent group represented by R₁ and R₂ may be a protecting group for an amino group. Examples of such a protecting group for an amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group).

In a preferred embodiment, R₁ and R₂ each independently represent a hydrogen atom or a protecting group for an amino group. Preferably, R₁ and R₂ may each be a hydrogen atom.

The divalent group represented by L₁ is a divalent group capable of linking Ig and a nitrogen atom. The divalent group represented by L₂ is a divalent group capable of linking D and a nitrogen atom. The divalent groups represented by L₁ and L₂ may each contain a moiety generated by the reaction of two mutually reactive bioorthogonal functional groups. The moiety generated by the reaction of two mutually reactive bioorthogonal functional groups, which may be comprised in the divalent groups represented by L₁ and L₂, is the same as the moiety generated by the reaction of two mutually reactive bioorthogonal functional groups as described above, which may be comprised in the divalent groups represented by L_{A} and L_{B}.

In a specific embodiment, the conjugate of an antibody and a functional substance, or a salt thereof, may be one in which a lysine residue of the antibody is modified. Therefore, the functional group in the side chain of a specific amino acid residue may be the amino group in the side chain of a lysine residue, and n may be from 1.5 to 2.5. In this case, it is preferable that in the above formulas (1), (1'), and (1"), L_{A} has a carbonyl group, and the bond is achieved by an amide bond generated by the bonding between the amino group in the side chain of a lysine residue and the carbonyl group in L_{A}. The lysine residue to be modified is a lysine residue present at position 246/248, 288/290, or 317 according to the Eu numbering, preferably a lysine residue present at position 246/248 or 288/290 according to the Eu numbering.

In the conjugate of an antibody and a functional substance, or a salt thereof, when a lysine residue of the antibody is modified, the structural unit represented by the above formula (1) may further comprise a modified moiety represented by the following formula (I): 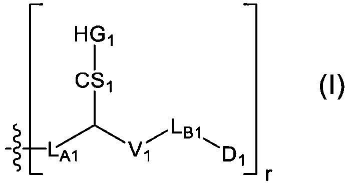 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

In formula (I), the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)). Preferably, the hyphen (-) with a wavy line is bonded to the amino group in the side chain of a lysine residue present at position 246/248 or 288/290 according to Eu numbering (wherein the lysine residue is different from the lysine residue having the amino group to which L_{A} is bonded in formula (1)).

HG₁ represents a hydrophilic group or a monovalent group comprising a hydrophilic group. The hydrophilic group or the monovalent group comprising a hydrophilic group is as described above. The hydrophilic group, or the monovalent group comprising a hydrophilic group, represented by HG₁ may be the same as or different from the hydrophilic group, or the monovalent group comprising a hydrophilic group, represented by HG in the above formula (1).

CS₁ represents a divalent group comprising a cleavable site. The divalent group comprising a cleavable site is as described above. The divalent group comprising a cleavable site, represented by CS₁, may be the same as or different from the divalent group comprising a cleavable site, represented by CS in the above formula (1).

V₁ represents an oxygen atom, a sulfur atom, or an amino group (NH). The type of V₁ may be the same as or different from the type of V. Therefore, when V is an oxygen atom, V₁ may be a sulfur atom or an amino group (NH). V₁ is preferably an oxygen atom or a sulfur atom, and more preferably an oxygen atom.

L_{A1} and L_{B1} each independently represent a divalent group. The divalent groups represented by L_{A1} and L_{B1} may be the same as or different from the divalent groups represented by L_{A} and L_{B}, respectively. Therefore, in a specific embodiment, the divalent groups represented by L_{A1} and L_{B1} may contain a moiety generated by the reaction of two mutually reactive bioorthogonal functional groups, similar to the divalent groups represented by L_{A} and L_{B}, respectively.

D₁ represents a functional substance. The functional substance is as described above. The functional substance represented by D₁ may be the same as or different from the functional substance represented by D in the above formula (1).

The average number r of the bonds per the immunoglobulin unit represents the average number of the bonds per immunoglobulin unit comprising two heavy chains and two light chains, and is 1.5 to 2.5. Such an average number may be 1.6 or more, 1.7 or more, 1.8 or more, or 1.9 or more. Such an average number may also be 2.4 or less, 2.3 or less, 2.2 or less, or 2.1 or less. Particularly preferably, such an average number may be 2.0.

In the conjugate of an antibody and a functional substance, or a salt thereof, when a lysine residue of the antibody is modified, the structural unit represented by the above formula (1') may further comprise a modified moiety represented by the following formula (I'): 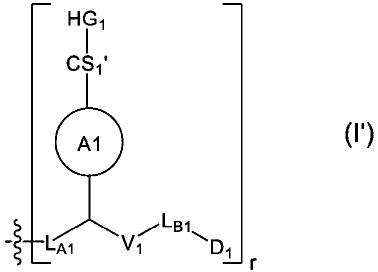 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

In formula (I'), the hyphen (-) with a wavy line, HG₁, V₁, L_{A1}, L_{B1}, D₁, and r are each the same as those represented by formula (I).

CS₁' represents a divalent group comprising a cleavable site. The cleavable site and the divalent group are as described above. The functional substance represented by D₁ may be the same as or different from the functional substance represented by D in the above formula (1').

Ring A1 represents an optionally substituted divalent aromatic ring group (wherein the divalent aromatic ring group forms a pi electron conjugated system with the cleavable site). The optionally substituted divalent aromatic ring group is as described above. The optionally substituted divalent aromatic ring group represented by Ring A1 may be the same as or different from the optionally substituted divalent aromatic ring group represented by Ring A in the above formula (1').

In the conjugate of an antibody and a functional substance or a salt thereof, when a lysine residue of the antibody is modified, the structural unit represented by the above formula (1") may further comprise a modified moiety represented by the following formula (I''): 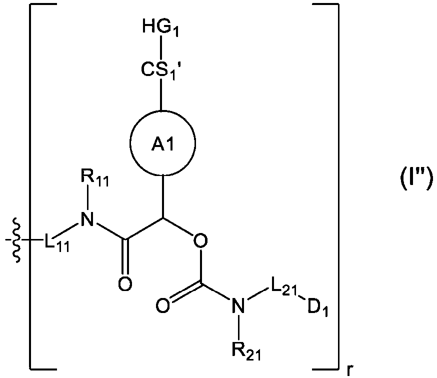 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
D₁ represents a functional substance, and
the average number (r) of said bonds per the immunoglobulin unit is 1.5 to 2.5.

In formula (I''), the hyphen (-) with a wavy line, HG₁, V₁, L_{A1}, L_{B1}, D₁, and r are each the same as those represented by formula (I). In formula (I''), CS₁' and Ring A1 are each the same as those represented by formula (I').

R₁₁ and R₂₁ each independently represent a hydrogen atom or a monovalent group. The monovalent group is as described above. The hydrogen atom or monovalent group represented by R₁₁ and R₂₁ may be the same as or different from those represented by R₁ and R₂ in the above formula (1'').

L₁₁ and L₂₁ each independently represent a divalent group. The divalent group is as described above. The divalent groups represented by L₁₁ and L₂₁ may be the same as or different from those represented by L₁ and L₂ in the above formula (1'').

In a specific embodiment, the conjugate of an antibody and a functional substance, or a salt thereof, may be one in which cysteine residues of the antibody (typically, an immunoglobulin unit contains eight cysteine residues) are modified. Therefore, the functional group in the side chain of the specific amino acid residue may be the thiol group in the side chain of a cysteine residue, and n may be from 2.0 to 8.0. n may preferably be 3.0 or more, 3.5 or more, 4.0 or more, 4.5 or more, 5.0 or more, 5.5 or more, 6.0 or more, 6.5 or more, 7.0 or more, or 7.5 or more.

The conjugate or a salt thereof of the present invention has the desired property of being difficult to aggregate, and thus can be characterized by its aggregation rate. More specifically, the aggregation rate of the conjugate or a salt thereof of the present invention may be 5% or less. This is because, according to the present invention, aggregation of the antibody is readily avoided. The aggregation rate is preferably 4.8% or less, more preferably 4.6% or less, still more preferably 4.4% or less, and particularly preferably 4.2% or less, 4.0% or less, 3.8% or less, 3.6% or less, 3.4% or less, 3.2% or less, 3.0% or less, 2.8% or less, or 2.6% or less. The aggregation rate of the antibody can be measured by size exclusion chromatography (SEC)-HPLC (see Examples and ChemistrySelect, 2020, 5, 8435-8439).

In a preferred embodiment, the conjugate or a salt thereof of the present invention may have an aggregation rate of 2.6% or less. The aggregation rate may also be 2.4% or less, 2.2% or less, 2.0% or less, 1.8% or less, 1.6% or less, 1.4% or less, 1.2% or less, 1.0% or less, or 0.8% or less.

The conjugate or salt thereof of the present invention is useful as pharmaceuticals or as reagents (e.g., diagnostic reagents and reagents for research), for example.

The conjugate or salt thereof of the present invention may be provided in the form of a pharmaceutical composition. Such a pharmaceutical composition may comprise a pharmaceutically acceptable carrier in addition to the conjugate or salt thereof of the present invention. Examples of the pharmaceutically acceptable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogen carbonate, calcium phosphate, and calcium citrate; lubricants such as magnesium stearate, Aerosil, talc, sodium lauryl sulfate; fragrances such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspending agents such as methylcellulose, polyvinylpyrrolidone, and aluminum stearate; dispersing agents such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) for achieving stability.

Examples of preparations suitable for oral administration include solutions in which an effective amount of a ligand is dissolved in diluents such as water, a physiological saline solution; capsules, sachets, and tablets comprising an effective amount of a ligand as a solid or granules; suspensions in which an effective amount of an active ingredient is suspended in an appropriate dispersion medium; and emulsions in which an effective amount of an active ingredient is dissolved and then emulsified in an appropriate dispersion medium.

The pharmaceutical composition is suitable for parenteral administration (e.g., intravenous injection, subcutaneous injection, intramuscular injection, local injection, and intraperitoneal administration). Examples of the pharmaceutical composition suitable for such nonoral administration include aqueous and nonaqueous, isotonic, sterile injectable solutions, which may comprise an antioxidant, a buffer, an antimicrobial agent, a tonicity agent, or the like. Examples thereof also include aqueous and nonaqueous, sterile suspensions, which may comprise a suspending agent, a solubilizing agent, a thickener, a stabilizer, an antiseptic, or the like.

The dose of the pharmaceutical composition, which varies depending on the type and activity of the active ingredient, the severity of the diseases, the species of the animal to be dosed, the drug receptivity, body weight, and age of the subject to be dosed, or the like, can be set as appropriate.

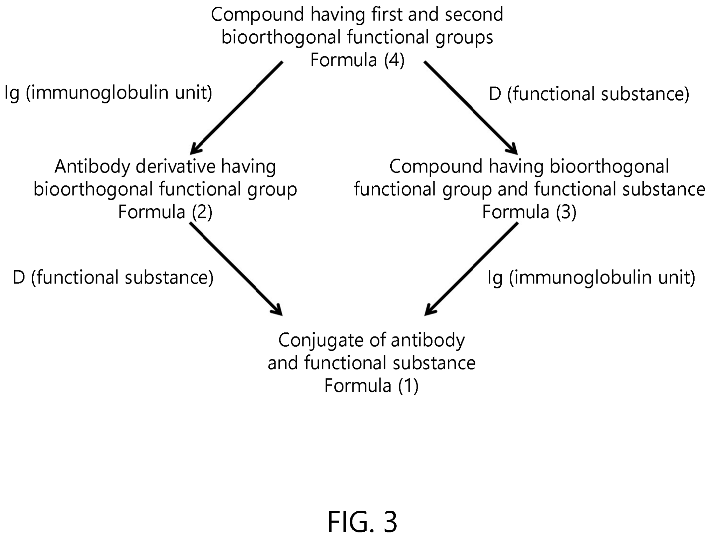

In one embodiment, the conjugate or a salt thereof of the present invention can be produced by reacting an antibody derivative having a bioorthogonal functional group or a salt thereof with a functional substance (FIG. 3). Such a reaction can proceed by the reaction between the bioorthogonal functional group in the antibody derivative and the functional substance.

When the functional substance has a functional group that readily reacts with a bioorthogonal functional group, the functional group of the functional substance can be appropriately reacted with the bioorthogonal functional group in the antibody derivative. The functional group that readily reacts with a bioorthogonal functional group may vary depending on the specific type of the bioorthogonal functional group. A person skilled in the art can appropriately select a suitable functional group as one that readily reacts with a bioorthogonal functional group (e.g., Boutureira et al., Chem. Rev., 2015, 115, 2174-2195). Examples of functional groups that readily react with bioorthogonal functional groups include, but are not limited thereto, an alkyne residue when the bioorthogonal functional group is an azide residue; a maleimide residue and a disulfide residue when the bioorthogonal functional group is a thiol residue; a hydrazine residue when the bioorthogonal functional group is an aldehyde or ketone residue; an azide residue when the bioorthogonal functional group is a norbornene residue; and an alkyne residue when the bioorthogonal functional group is a tetrazine residue. Of course, in the above combinations of bioorthogonal functional groups and functional groups that readily react with them, the combinations can be interchanged. Thus, if the first example of the combinations described above is interchanged, a combination of an alkyne residue as the bioorthogonal functional group and an azide residue as the functional group that readily reacts with the bioorthogonal functional group can be used.

If the functional substance does not have a functional group that readily reacts with the bioorthogonal functional group in the antibody derivative, the drug may be derivatized to have such a functional group. Derivatization is well known to those skilled in the art (e.g., WO2004/010957, US Patent Application Publication No. 2006/0074008, US Patent Application Publication No. 2005/0238649). For example, derivatization may be carried out using any crosslinking agent. Alternatively, derivatization may be carried out using a specific linker having a desired functional group. In the present invention, derivatized functional substances are also merely one type of functional substance and are therefore simply referred to as "functional substances".

The reaction can be appropriately carried out under conditions (mild conditions) that will not cause protein denaturation or degradation (e.g., cleavage of an amide bond). For example, such a reaction can be carried out at room temperature (e.g., about 15 to 30°C) in a suitable reaction system, such as in a buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and still more preferably 30 minutes to 8 hours. For details of such a reaction, see, e.g., G. J. L. Bernardes et al., Chem. Rev., 115, 2174 (2015); G. J. L. Bernardes et al., Chem. Asian. J., 4,630 (2009); B. G. Davies et al., Nat. Commun., 5, 4740 (2014); A. Wagner et al., Bioconjugate. Chem., 25, 825 (2014).

In another embodiment, the conjugate or a salt thereof of the present invention can be produced by reacting a compound comprising a bioorthogonal functional group and a functional substance, or a salt thereof, with a starting antibody comprising Ig (immunoglobulin unit) (FIG. 3). When the conjugate of an antibody and a functional substance, or a salt thereof, is one in which a lysine residue of the antibody is modified, an antibody comprising Ig (immunoglobulin unit) can be used as the starting antibody. When the conjugate of an antibody and a functional substance, or a salt thereof, is one in which a cysteine residue of the antibody is modified, the conjugate can be prepared by contacting, as a starting antibody, an antibody comprising an immunoglobulin unit consisting of two heavy chains and two light chains (comprising four disulfide bonds) with a reducing agent to generate an antibody comprising a thiol group. As the reducing agent, any reducing agent capable of cleaving a disulfide bond to generate a thiol group can be used, and examples thereof include Tris(2-carboxyethyl)phosphine (TCEP), cysteine, dithiothreitol, reduced glutathione, and β-mercaptoethanol. In this embodiment, the bioorthogonal functional group possessed by the compound is preferably a group that can efficiently react with a thiol group (e.g., a maleimide residue, a halocarbonyl group, or a thiol group). The reaction between the compound comprising a bioorthogonal functional group and a functional substance, or a salt thereof, and the above-mentioned starting antibody can be appropriately carried out under the above-mentioned conditions (mild conditions) that will not cause denaturation or degradation of the protein (e.g., cleavage of amide bonds).

The production of the conjugate or a salt thereof can be confirmed, depending on the specific starting materials and the molecular weight of the product, for example, by reversed-phase HPLC under reducing conditions or by mass spectrometry. The conjugate or a salt thereof can be appropriately purified by any purification method such as chromatography (e.g., gel filtration chromatography, ion-exchange chromatography, reversed-phase column chromatography, high-performance liquid chromatography, and affinity chromatography).

### 3. Antibody derivative or a salt thereof

The present invention also provides an antibody derivative having a bioorthogonal functional group, or a salt thereof, the antibody derivative comprising a structural unit represented by the following formula (2): 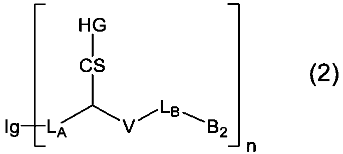 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
B₂ represents a bioorthogonal functional group,
the average number (n) of said bonds per the immunoglobulin unit is 1.5 or more.

In formula (2), Ig, HG, CS, V, L_{A}, L_{B}, and n are each the same as those represented in formula (1). Therefore, the definitions, examples, and preferred examples of these elements and other elements related thereto are the same as those described above.

The bioorthogonal functional group represented by B₂ is as described above.

In a specific embodiment, the bioorthogonal functional group represented by B₂ may be a maleimide residue, a thiol residue, a furan residue, a halocarbonyl residue, an alkene residue, an alkyne residue, an azide residue, or a tetrazine residue. Alternatively, the bioorthogonal functional group represented by B₂ may be a maleimide residue, a thiol residue, a furan residue, a halocarbonyl residue, an alkene residue, an alkyne residue, an azide residue, or a tetrazine residue. These bioorthogonal functional groups are preferable because they have excellent reaction efficiency and high versatility.

In a specific embodiment, L_{A} may be a divalent group having a side chain comprising a hydrophilic group or a functional substance. The side chain comprising a hydrophilic group or a functional substance may be a side chain comprising a hydrophilic group and a functional substance. The side chain comprising a hydrophilic group and a functional substance may be a side chain comprising (i) a hydrophilic group, (ii) a functional substance, and (iii) a cleavable site between the hydrophilic group and the functional substance. The number of such side chains comprised in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (β): 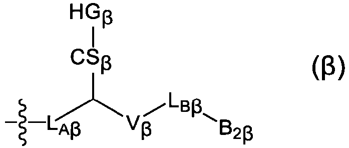 wherein
the hyphen (-) with a wavy line represents a bond to the main chain in L_{A} connecting Ig and a carbon atom in formula (1),
HG_{β} represents a hydrophilic group or a monovalent group comprising a hydrophilic group,
CS_{β} represents a divalent group comprising a cleavable site,
V_{β} represents an oxygen atom, a sulfur atom, or an amino group (NH),
L_{Aβα} and L_{Bβ} each independently represent a divalent group, and
B_{2β} represents a bioorthogonal functional group.

HG_{β} represents a hydrophilic group or a monovalent group comprising a hydrophilic group. The hydrophilic group and the monovalent group are as described above. The hydrophilic group or the monovalent group comprising a hydrophilic group represented by HG_{β} may be the same as or different from the hydrophilic group or the monovalent group comprising a hydrophilic group represented by HG.

CS_{β} represents a divalent group comprising a cleavable site. The cleavable site and the divalent group are as described above. The divalent group comprising a cleavable site represented by CS_{β} may be the same as or different from the divalent group comprising a cleavable site represented by CS.

V_{β} represents an oxygen atom, a sulfur atom, or an amino group (NH). The type of V_{β} may be the same as or different from the type of V. Therefore, when V is an oxygen atom, V_{β} may be a sulfur atom or an amino group (NH). V_{β} is preferably an oxygen atom or a sulfur atom, and more preferably an oxygen atom.

The divalent group represented by L_{Aβ} is a divalent group having a bond to the main chain in L_{A} connecting Ig and a carbon atom. The divalent group represented by L_{Aβ} may be the same as or different from the divalent group represented by L_{A}. The divalent group represented by L_{Bβ} is a divalent group capable of linking D_{β} and V_{β}. The divalent group represented by L_{Bβ} may be the same as or different from the divalent group represented by L_{B}. Therefore, in a specific embodiment, the divalent groups represented by L_{Aβ} and L_{Bα} may contain a moiety generated by the reaction of two mutually reactive bioorthogonal functional groups, similar to the divalent groups represented by L_{A} and L_{B}, respectively.

B_{2β} represents a functional substance. The functional substance is as described above. The functional substance represented by D_{β} may be the same as or different from the functional substance represented by D.

The present invention also provides an antibody derivative having a bioorthogonal functional group, or a salt thereof, the antibody derivative comprising a structural unit represented by the following formula (2'): 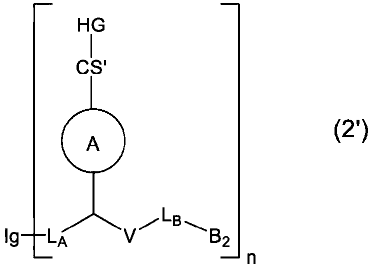 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₂ represents a bioorthogonal functional group, and
the average number (n) of said bonds per the immunoglobulin unit is 1.5 or more.

In formula (2'), Ig, HG, V, L_{A}, L_{B}, B₂, and n are each the same as those represented in formula (1). In formula (2'), CS' and Ring A are each the same as those represented by formula (1'). Therefore, the definitions, examples, and preferred examples of these elements and other elements related thereto are the same as those described above.

In a specific embodiment, L_{A} may be a divalent group having a side chain comprising a hydrophilic group or a functional substance. The side chain comprising a hydrophilic group or a functional substance may be a side chain comprising a hydrophilic group and a functional substance. The side chain comprising a hydrophilic group and a functional substance may be a side chain comprising (i) a hydrophilic group, (ii) a functional substance, and (iii) a cleavable site between the hydrophilic group and the functional substance. The number of such side chains comprised in the divalent group is not particularly limited, and may be, for example, 1 to 4, 1 to 3, 1 or 2, or 1.

In a specific embodiment, the side chain comprising (i) a hydrophilic group, (ii) a functional substance, and (iii) a cleavable site between the hydrophilic group and the functional substance may be represented by the following formula (β'): 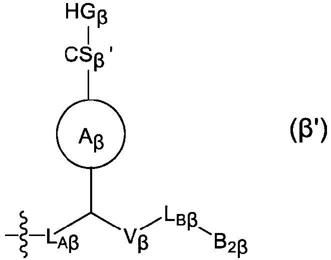 wherein
the hyphen (-) with a wavy line represents a bond to the main chain in L_{A} connecting Ig and a carbon atom in formula (1'),
HG_{β} represents a hydrophilic group or a monovalent group comprising a hydrophilic group,
CS_{β}' represents a divalent group comprising a cleavable site,
Ring A_{β} represents an optionally substituted divalent aromatic ring group (wherein the divalent aromatic ring group forms a pi electron conjugated system with the cleavable site),
V_{β} represents an oxygen atom, a sulfur atom, or an amino group (NH),
L_{Aβ} and L_{Bβ} each independently represent a divalent group, and
B_{2β} represents a bioorthogonal functional group.

In formula (β'), HG_{β}, V_{β}, L_{Aβ}, L_{Bβ}, and B_{2β} are each the same as those represented by formula (β).

CS_{β}' represents a divalent group comprising a cleavable site. The cleavable site and the divalent group are as described above. The divalent group comprising a cleavable site represented by CS_{β}' may be the same as or different from the divalent group comprising a cleavable site represented by CS'.

Ring A_{β} represents an optionally substituted divalent aromatic ring group (wherein the divalent aromatic ring group forms a pi electron conjugated system with the cleavable site). The optionally substituted divalent aromatic ring group is as described above. The optionally substituted divalent aromatic ring group represented by Ring A_{β} may be the same as or different from the optionally substituted divalent aromatic ring group represented by Ring A.

The present invention also provides an antibody derivative having a bioorthogonal functional group, or a salt thereof, the antibody derivative comprising a structural unit represented by the following formula (2"): 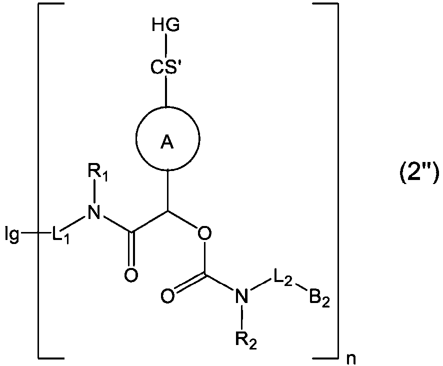 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₂ represents a bioorthogonal functional group, and
the average number (n) of said bonds per the immunoglobulin unit is 1.5 or more.

In formula (2"), Ig, HG, B₂, and n are each the same as those represented in formula (1). In formula (2''), CS' and Ring A are each the same as those represented by formula (1'). In formula (2''), R₁, R₂, L₁, and L₂ are each the same as those represented by formula (1"). Therefore, the definitions, examples, and preferred examples of these elements and other elements related thereto are the same as those described above.

In a specific embodiment, the antibody derivative or a salt thereof may be one in which a lysine residue of the antibody is modified. Therefore, the functional group in the side chain of a specific amino acid residue may be the amino group in the side chain of a lysine residue, and n may be from 1.5 to 2.5. In this case, it is preferable that in the above formulas (2), (2'), and (2"), L_{A} has a carbonyl group, and the bond is achieved by an amide bond generated by the bonding between the amino group in the side chain of a lysine residue and the carbonyl group in L_{A}. The lysine residue to be modified is a lysine residue present at position 246/248, 288/290, or 317 according to the Eu numbering, preferably a lysine residue present at position 246/248 or 288/290 according to the Eu numbering.

In the antibody derivative or a salt thereof, when a lysine residue of the antibody is modified, the structural unit represented by the above formula (2) may further comprise a modified moiety represented by the following formula (II): 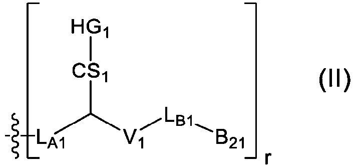 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

In formula (II), the hyphen (-) with a wavy line, HG₁, CS₁, V₁, L_{A1}, L_{B1}, and r are each the same as those represented by formula (I).

B₂₁ represents a bioorthogonal functional group. The bioorthogonal functional group is as described above. The bioorthogonal functional group represented by B₂₁ may be the same as or different from the bioorthogonal functional group represented by B₂₁ in the above formula (2).

In the conjugate of an antibody and a functional substance or a salt thereof, when a lysine residue of the antibody is modified, the structural unit represented by the above formula (2') may further comprise a modified moiety represented by the following formula (II'): 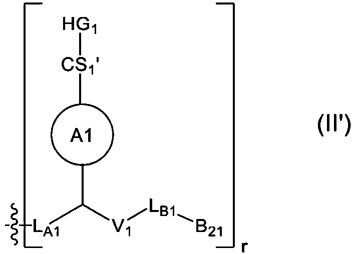 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

In formula (II'), the hyphen (-) with a wavy line, HG₁, CS₁', Ring A1, V₁, L_{A1}, L_{B1}, and r are each the same as those represented by formula (I'). In formula (II'), B₂₁ is the same as that represented in formula (II).

In the conjugate of an antibody and a functional substance or a salt thereof, when a lysine residue of the antibody is modified, the structural unit represented by the above formula (2") may further comprise a modified moiety represented by the following formula (II"): 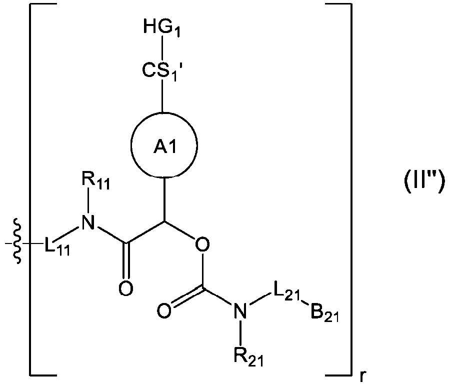 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
B₂₁ represents a bioorthogonal functional group, and
the average number (r) of said bonds per the immunoglobulin unit is 1.5 to 2.5.

In formula (II''), the hyphen (-) with a wavy line, HG₁, CS₁', Ring A1, R₁₁, R₂₁, L₁₁, L₂₁, and r are each the same as those represented by formula (I''). In formula (II'), B₂₁ is the same as that represented in formula (II).

The antibody derivative or a salt thereof of the present invention is useful, for example, as an intermediate for preparation of the conjugate or a salt thereof of the present invention.

The antibody derivative or a salt thereof of the present invention can be produced, for example, by reacting a compound comprising a first bioorthogonal functional group and a second bioorthogonal functional group, or a salt thereof, with a starting antibody comprising Ig (immunoglobulin unit) (FIG. 3). The starting antibody is the same as described above.

The reaction between the compound comprising a first bioorthogonal functional group and a second bioorthogonal functional group, or a salt thereof, and the above-mentioned starting antibody can be appropriately carried out under the above-mentioned conditions (mild conditions) that will not cause denaturation or degradation of the protein (e.g., cleavage of amide bonds).

Confirmation of the generation of the antibody derivative or a salt thereof can be performed in the same manner as the method described for the conjugate of the present invention. The antibody derivative or a salt thereof can be appropriately purified by any purification method as described for the conjugate of the present invention.

### 4. Compound comprising a bioorthogonal functional group and a functional substance, or a salt thereof

The present invention also provides a compound comprising a bioorthogonal functional group and a functional substance, or a salt thereof, represented by the following formula (3): 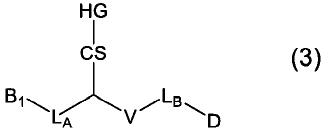 wherein
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
B₁ represents a bioorthogonal functional group, and
D represents a functional substance.

In formula (3), HG, CS, V, L_{A}, L_{B}, and D are as described above in formula (1). Therefore, the definitions, examples, and preferred examples of these elements and other elements related thereto are the same as those described above in formula (1).

B₁ represents a bioorthogonal functional group. The bioorthogonal functional group is as described above.

The present invention also provides a compound having a bioorthogonal functional group and a functional substance, or a salt thereof, represented by the following formula (3'): 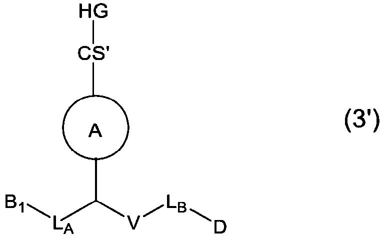 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a bioorthogonal functional group, and
D represents a functional substance.

In formula (3'), HG, CS', Ring A, V, L_{A}, L_{B}, and D are as described above in formula (1'). In formula (3'), B₁ is as described above in formula (3).

The present invention also provides a compound having a bioorthogonal functional group and a functional substance, or a salt thereof, represented by the following formula (3"): 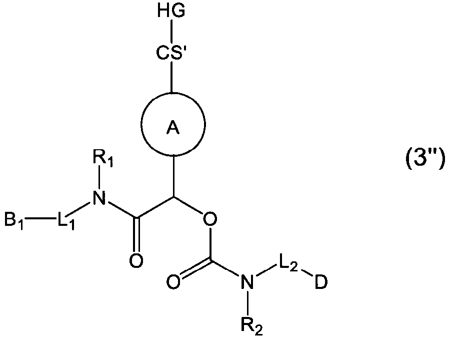 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a bioorthogonal functional group, and
D represents a functional substance.

In formula (3"), HG, CS', Ring A, R₁, R₂, L₁, L₁, and D are as described above in formula (1"). In formula (3"), B₁ is as described above in formula (3).

The compounds represented by formula (3), formula (3'), and formula (3"), or salts thereof, are useful, for example, as intermediates for preparation of the conjugate of the present invention. The compounds represented by formula (3), formula (3'), and formula (3"), or salts thereof are also useful for derivatizing any substance such as biomolecules (e.g., proteins such as antibodies, saccharides, nucleic acids, and lipids).

The compound having a bioorthogonal functional group and a functional substance, or a salt thereof, can be produced, for example, by reacting a compound having a first bioorthogonal functional group and a second bioorthogonal functional group, or a salt thereof, with a functional substance (FIG. 3). The details of the functional substance are as described above.

The reaction of the compound having a first bioorthogonal functional group and a second bioorthogonal functional group, or a salt thereof, with a functional substance can be carried out in a suitable reaction system, for example, in an organic solvent system or an aqueous solution (e.g., buffer) system, at an appropriate temperature (e.g., about 15 to 200°C). The reaction system may contain a suitable catalyst. The reaction time is, for example, 1 minute to 20 hours, preferably 10 minutes to 15 hours, more preferably 20 minutes to 10 hours, and still more preferably 30 minutes to 8 hours. Of course, such a reaction can also be carried out under the mild conditions described above.

The production of the compound having a bioorthogonal functional group and a functional substance, or a salt thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

### 5. Compound having a first bioorthogonal functional group and a second bioorthogonal functional group or a salt thereof

The present invention also provides a compound having a first bioorthogonal functional group and a second bioorthogonal functional group, or a salt thereof, represented by the following formula (4): 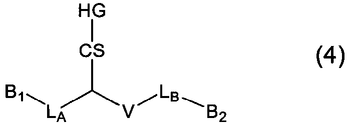 wherein
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group.

In formula (4), HG, CS, V, L_{A}, L_{B}, and B₂ are as described above in formula (2). Therefore, the definitions, examples, and preferred examples of these elements and other elements related thereto are the same as those described above in formula (2).

B₁ represents a first bioorthogonal functional group. The first bioorthogonal functional group is the same as that described above for the bioorthogonal functional group. Preferably, the bioorthogonal functional group is a group that can form an amide bond together with an amino group (i.e., a group that serves as a donor for the carbonyl group moiety in the amide bond), or a group that can efficiently react with a thiol group (e.g., a maleimide residue, a halocarbonyl group, or a thiol group).

B₂ represents a second bioorthogonal functional group. The second bioorthogonal functional group is the same as that described above for the bioorthogonal functional group.

In a specific embodiment, the bioorthogonal functional group represented by B₂ may be a maleimide residue, a thiol residue, a furan residue, a halocarbonyl residue, an alkene residue, an alkyne residue, an azide residue, or a tetrazine residue. Alternatively, the bioorthogonal functional group represented by B₂ may be a maleimide residue, a thiol residue, a furan residue, a halocarbonyl residue, an alkene residue, an alkyne residue, an azide residue, or a tetrazine residue. These bioorthogonal functional groups are preferable because they have excellent reaction efficiency and high versatility.

Preferably, the second bioorthogonal functional group may be a bioorthogonal functional group that does not react with the first bioorthogonal functional group or has low reactivity towards the first bioorthogonal functional group. In this case, intermolecular reactions of the compound represented by formula (4) or a salt thereof can be reduced. Therefore, the first and second bioorthogonal functional groups can be used in combinations that do not react with each other or have low reactivity with each other. Such combinations of bioorthogonal functional groups are well known in the art. For example, for preferred bioorthogonal functional groups such as a maleimide residue, a thiol residue, a furan residue, a halocarbonyl residue, an alkene residue, an alkyne residue, an azide residue, and a tetrazine residue, examples of such combinations are as follows.

**Table A. Examples of combinations of first and second bioorthogonal functional groups that do not react with each other or are less reactive with each other**

| First bioorthogonal functional group | Second bioorthogonal functional group |
|---|---|
| Maleimide residue | Halocarbonyl, alkene, or alkyne residue |
| Thiol residue | Furan, alkene, alkyne, azide, or tetrazine residue |
| Furan residue | Thiol residue, or halocarbonyl residue |
| Halocarbonyl residue | Maleimide, furan, alkene, alkyne, azide, or tetrazine residue |
| Alkene residue | Maleimide, thiol, halocarbonyl, or alkyne residue |
| Alkyne residue | Maleimide, thiol, halocarbonyl, or alkyne residue |
| Azide residue | Thiol residue, or halocarbonyl residue |
| Tetrazine residue | Thiol residue, or halocarbonyl residue |

The present invention also provides a compound having a first bioorthogonal functional group and a second bioorthogonal functional group, or a salt thereof, represented by the following formula (4'): 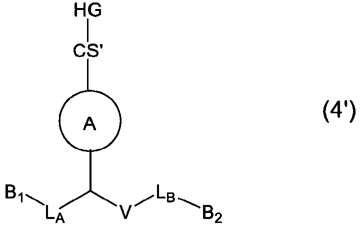 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group.

In formula (4'), HG, CS', Ring A, V, L_{A}, L_{B}, and B₂ are as described above in formula (2'). In formula (4'), B₁ is as described above in formula (4).

The present invention also provides a compound having a first bioorthogonal functional group and a second bioorthogonal functional group, or a salt thereof, represented by the following formula (4"): 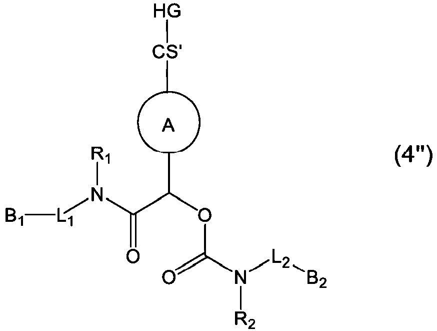 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

In formula (4''), HG, CS', Ring A, R₁, R₂, L_{A1}, L_{B1}, and B₂ are as described above in formula (2"). In formula (4"), B₁ is as described above in formula (4).

The compounds represented by formulas (4), (4'), and (4"), or salts thereof, are useful, for example, as intermediates for preparation of the antibody derivative, and the compounds represented by formulas (3), (3'), and (3") of the present invention. The compounds represented by formulas (4), (4'), and (4"), or salts thereof are also useful for derivatizing any substance such as biomolecules (e.g., proteins such as antibodies, saccharides, nucleic acids, and lipids) and functional substances.

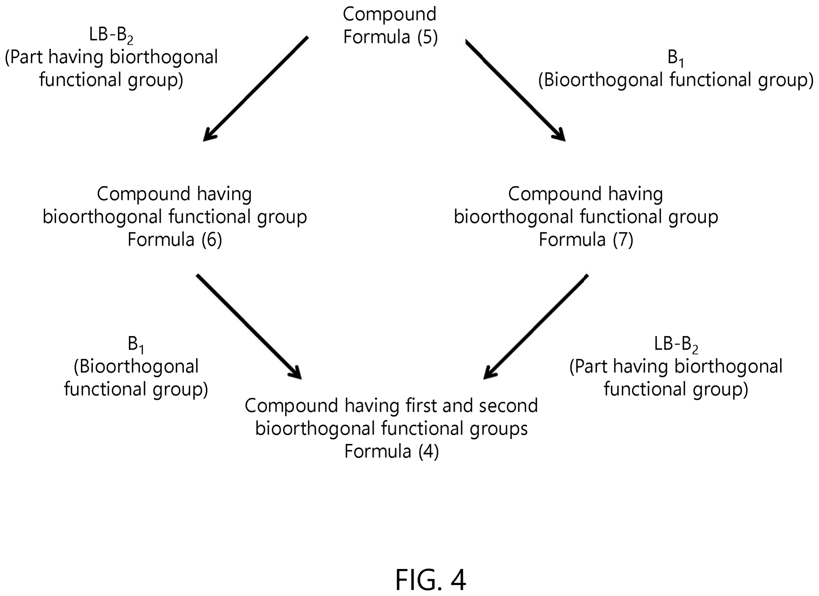

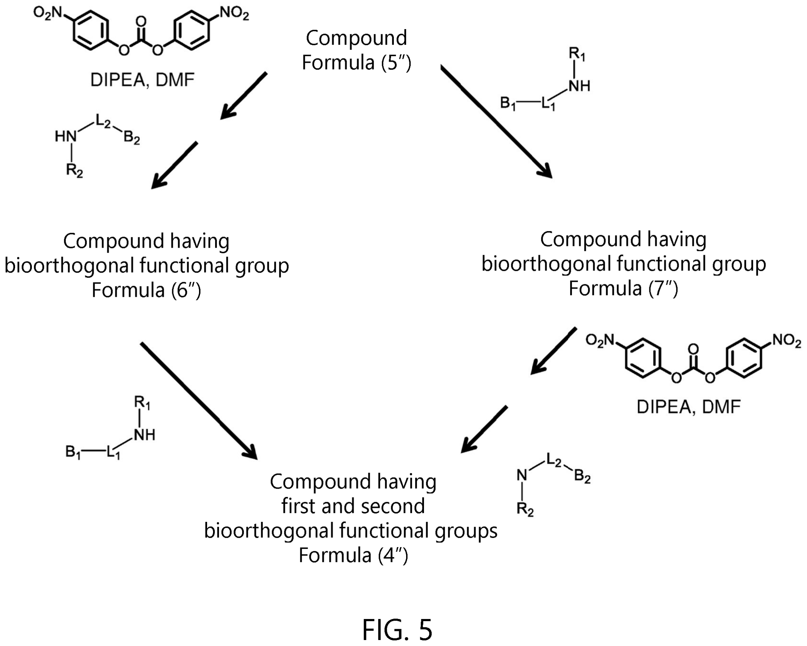

In one embodiment, a compound having a first bioorthogonal functional group and a second bioorthogonal functional group, or a salt thereof, can be produced by reacting a compound having a bioorthogonal functional group represented by formula (6), or a salt thereof, with a suitable compound having B₁ (e.g., a compound represented by B₁-L₁-NH-R₁) (FIGS. 4 and 5). The definitions, examples, and preferred examples of B₁, L₁, and R₁ are as described above.

In another embodiment, the compound having a first bioorthogonal functional group and a second bioorthogonal functional group, or a salt thereof, can be produced by reacting a compound having a bioorthogonal functional group represented by formula (7), or a salt thereof, with a suitable compound having L_{B}-B₂ (e.g., by reaction with bis(4-nitrophenyl) carbonate and N,N-diisopropylethylamine (DIPEA), followed by reaction with a compound of B₂-L₂-NH-R₂) (FIGS. 4 and 5). The definitions, examples, and preferred examples of B₂, L_{B}, and R₂ are as described above.

The reaction can be carried out in a suitable reaction system, for example, in an organic solvent system or an aqueous solution (e.g., buffer) system, at an appropriate temperature (e.g., about 15 to 200°C). The reaction system may contain a suitable catalyst. The reaction time is, for example, 1 minute to 20 hours, preferably 10 minutes to 15 hours, more preferably 20 minutes to 10 hours, and still more preferably 30 minutes to 8 hours. Of course, such a reaction can also be carried out under the mild conditions described above.

The production of the compound having a first bioorthogonal functional group and a second bioorthogonal functional group, or a salt thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

### 6. A series of compounds or salts thereof

### (1) Compound or a salt thereof

The present invention also provides a compound, or a salt thereof, represented by the following formula (5): 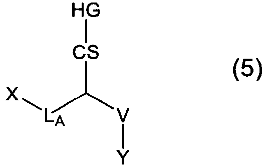 wherein
HG represents a hydrophilic group or a monovalent group comprising a hydrophilic group,
CS represents a divalent group comprising a cleavable site,
V represents an oxygen atom, a sulfur atom, or an amino group (NH),
L_{A} each independently represents a divalent group, and
X and Y each independently represent a monovalent group.

In formula (5), HG, CS, V, and L_{A} are as described above in formula (1). Therefore, the definitions, examples, and preferred examples of these elements and other elements related thereto are the same as those described above in formula (1).

X and Y each independently represent a monovalent group. The monovalent group is as described above.

The present invention also provides a compound, or a salt thereof, represented by the following formula (5'): 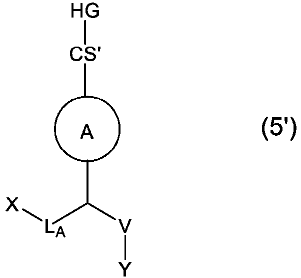 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} each independently represents a divalent group, and
X and Y each independently represent a monovalent group.

In formula (5'), HG, CS', Ring A, V, and L_{A} are as described above in formula (1'). In formula (5'), X and Y are the same as those represented in formula (5).

The present invention also provides a compound, or a salt thereof, represented by the following formula (5"): 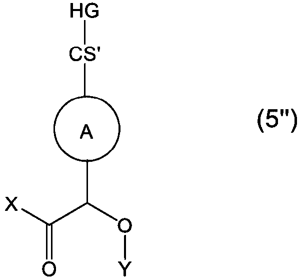 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site), and
X and Y each independently represent a monovalent group.

In formula (5''), HG, CS', and Ring A are as described above in formula (1"). In formula (5"), X and Y are the same as those represented in formula (5).

The compounds represented by formulas (5), (5'), and (5"), or salts thereof, are useful, for example, as synthetic intermediates for the conjugates and antibody derivatives of the present invention, and other compounds of the present invention.

As a synthesis example of the compounds represented by formulas (5), (5'), and (5"), or salts thereof, a synthesis example of the compound represented by formula (5'), or a salt thereof, will be described. The compound represented by formula (5'), or a salt thereof, can be produced, for example, by reacting a compound represented by the following formula (5-1): 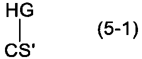 wherein
HG represents a hydrophilic group or a monovalent group comprising a hydrophilic group,
CS' represents a reactive divalent group comprising a cleavable site,
or a salt thereof,
with a compound represented by the following formula (5-2): 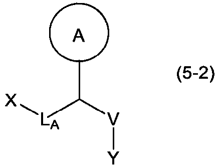 wherein
   Ring A represents an optionally substituted divalent aromatic ring group capable of reacting with CS',
   X and Y each independently represent a monovalent group,
   or a salt thereof.

The definitions, examples, and preferred examples of HG in formula (5-1), the cleavable site and divalent group in CS', and Ring A, L_{A}, V, X, and Y in formula (5-2) are as described above. Such a reaction can be carried out under the same conditions as the reaction conditions described above for the production of a compound having a first bioorthogonal functional group and a second bioorthogonal functional group, or a salt thereof.

Preferably, the compound represented by formula (5-2) may be represented by the following formula (5'-2): 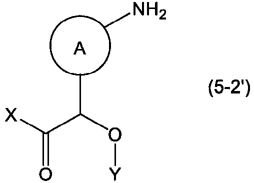 wherein
Ring A represents an optionally substituted divalent aromatic ring group, and
X and Y each independently represent a monovalent group.

### (2) Compound having a bioorthogonal functional group represented by formula (6), or a salt thereof

The present invention also provides a compound having a bioorthogonal functional group, represented by the following formula (6): 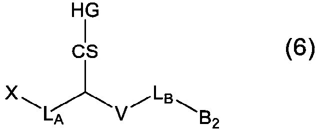 wherein
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
X represents a monovalent group, and
B₂ represents a bioorthogonal functional group,
or a salt thereof.

In formula (6), HG, CS, V, L_{A}, L_{B}, and B₂ are as described above in formula (2). In formula (6), X is the same as that described above in formula (5).

The present invention also provides a compound having a bioorthogonal functional group, represented by the following formula (6'): 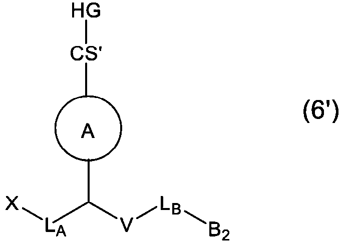 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
X represents a monovalent group, and
B₂ represents a bioorthogonal functional group,
or a salt thereof.

In formula (6'), HG, CS', Ring A, V, L_{A}, L_{B}, and B₂ are as described above in formula (2'). In formula (6'), X is the same as that described above in formula (5).

The present invention also provides a compound having a bioorthogonal functional group, represented by the following formula (6"): 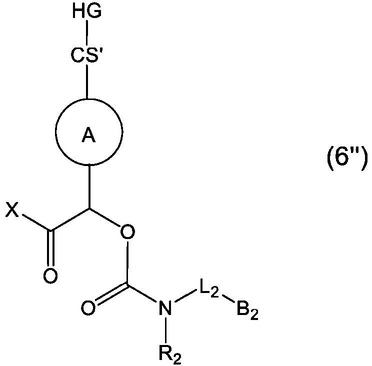 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₂ represents a hydrogen atom or a monovalent group,
L₂ represents a divalent group,
X represents a monovalent group, and
B₂ represents a bioorthogonal functional group,
or a salt thereof.

In formula (6"), HG, CS', Ring A, R₂, L₂, and B₂ are as described above in formula (2''). In formula (6"), X is the same as described in formula (5).

The compounds represented by formulas (6), (6'), and (6"), or salts thereof, are useful, for example, as synthetic intermediates for the conjugates and antibody derivatives of the present invention, and certain compounds of the present invention. Such a compound or a salt thereof is also useful, for example, for derivatizing functional substances.

The compound represented by formulas (6), (6'), or (6"), or a salt thereof, can be produced by reacting a compound represented by formulas (5), (5'), or (5"), or a salt thereof, with a suitable compound having L_{B}-B₂ (e.g., by reaction with bis(4-nitrophenyl) carbonate and N,N-diisopropylethylamine (DIPEA), followed by reaction with a compound of B₂-L₂-NH-R₂) (FIGS. 4 and 5). The definitions, examples, and preferred examples of B₂, L₂, and R₂ are as described above. Such a reaction can be carried out under the same conditions as the reaction conditions described above for the production of a compound having a first bioorthogonal functional group and a second bioorthogonal functional group, or a salt thereof.

### (3) Compound having a bioorthogonal functional group represented by formula (7), or a salt thereof

The present invention also provides a compound having a bioorthogonal functional group, represented by the following formula (7): 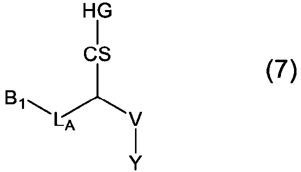 wherein
HG represents a hydrophilic group or a monovalent group comprising a hydrophilic group,
CS represents a divalent group comprising a cleavable site,
V represents an oxygen atom, a sulfur atom, or an amino group (NH),
L_{A} each independently represents a divalent group,
B₁ represents a bioorthogonal functional group, and
Y represents a monovalent group,
or a salt thereof.

In formula (7), HG, CS, V, L_{A}, and B₁ are as described above in formula (3). In formula (7), Y is as described above in formula (5).

The present invention also provides a compound having a bioorthogonal functional group, represented by the following formula (7'): 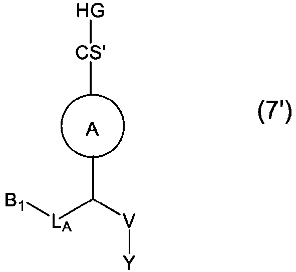 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} represents a divalent group,
B₁ represents a bioorthogonal functional group, and
Y represents a monovalent group,
or a salt thereof.

In formula (7'), HG, CS', Ring A, V, L_{A}, and B₁ are as described above in formula (3'). In formula (7'), Y is the same as that described above in formula (5).

The present invention also provides a compound or a salt thereof having a bioorthogonal functional group, represented by the following formula (7''): 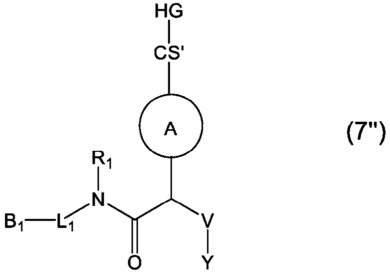 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
R₁ represents a hydrogen atom or a monovalent group,
L₁ represents a divalent group,
B₁ represents a bioorthogonal functional group, and
Y represents a monovalent group.

In formula (7"), HG, CS', Ring A, R₁, L₁, and B₁ are as described above in formula (3''). In formula (7''), Y is the same as described in formula (5).

The compounds represented by formulas (7), (7'), or (7"), or salts thereof, are useful, for example, as synthetic intermediates for the conjugates and antibody derivatives of the present invention, and specific compounds of the present invention. Such compounds or salts thereof are also useful for derivatizing any substance such as biomolecules (e.g., proteins such as antibodies, saccharides, nucleic acids, and lipids).

The compounds represented by formulas (7), (7'), or (7"), or salts thereof, can be produced, for example, by reacting compounds represented by formulas (5), (5'), and (5"), or salts thereof, with suitable compounds having B₁ (e.g., a compound represented by B₁-L₁-NH-R₁) (FIGS. 4 and 5). The definitions, examples, and preferred examples of B₁, L₁, and R₁ are as described above. Such a reaction can be carried out under the same conditions as the reaction conditions described above for the production of a compound having a first bioorthogonal functional group and a second bioorthogonal functional group, or a salt thereof.

The production of the compounds represented by formulas (5), (5'), or (5"), (6), (6'), or (6"), or (7), (7'), or (7"), or salts thereof, can be confirmed, depending on the specific starting materials and the molecular weight of the product, for example, by NMR, HPLC, or mass spectrometry. Such a compound or a salt thereof can be appropriately purified by any purification method such as chromatography (e.g., gel filtration chromatography, ion-exchange chromatography, reversed-phase column chromatography, high-performance liquid chromatography, and affinity chromatography).

### EXAMPLES

Next, the present invention will be described in more detail with reference to Examples, but the present invention is not limited to the following Examples.

The following peptides in Example 1, Ac-Glu (OtBu)-Val-Cit-OH, Z-Glu (OtBu)-Val-Cit-OH, Ac-Glu (OtBu)-Glu (OtBu)-Val-Cit-OH, and SCA (OtBu)-Glu (OtBu)-Val-Cit-OH, were all prepared by similar methods. It should be noted that SCA (OtBu) is an abbreviation for mono-tert-butyl succinate, and SCA is an abbreviation for succinic acid. By solid-phase peptide synthesis using Cl-TCP (Cl) ProTide Resin (CEM Corporation) by the Fmoc method, those capped with an acetyl group at the N-terminus and those capped with succinic acid at the N-terminus were prepared, and then stirred overnight in a 20% HFIP / dichloromethane solution to cleave the peptides from the resin while maintaining the protection of the amino acid side chains. The resin was removed by filtration, and the solution was concentrated, which was then purified by preparative HPLC to obtain peptides as products.

### Ac-Glu (OtBu)-Val-Cit-OH

¹H NMR (400 MHz, DMSO-d₆) δ12.50 (brs, 1H), 8.22 (d, J = 7.2 Hz, 1H), 8.05 (d, J = 8.0 Hz, 1H), 7.69 (d, J = 8.8 Hz, 1H), 5.95-5.93 (m, 1H), 5.38 (brs, 2H), 4.33-4.27 (m, 1H), 4.22-4.18 (m, 1H), 4.15-4.10 (m, 1H), 2.96-2.95 (m, 2H), 2.25-2.19 (m, 2H), 2.00-1.93 (m, 1H), 1.89-1.80 (m, 4H), 1.73-1.66 (m, 2H), 1.61-1.51 (m, 1H), 1.46-1.34 (m, 11H), 0.86 (d, J = 6.8 Hz, 3H), 0.83 (d, J = 6.8 Hz, 3H).
MS (ESI) m/z: 502.30 [M+H]⁺

### Z-Glu (OtBu)-Val-Cit-OH

¹H NMR (400 MHz, DMSO-d₆) δ12.50 (brs, 1H), 7.93-7.37 (m, 8H), 6.05-6.00 (m, 1H), 5.44 (brs, 2H), 5.08 (s, 2H), 4.17-3.81 (m, 3H), 3.00-2.90 (m, 2H), 2.31-2.27 (m, 2H), 2.10-1.34 (m, 16H), 0.89-0.83 (m, 6H).
MS (ESI) m/z: 594.30 [M+H]⁺

### Ac-Glu (OtBu)-Glu (OtBu)-Val-Cit-OH

¹H NMR (400 MHz, DMSO-d₆) δ12.50 (brs, 1H), 8.21 (d, J = 7.2 Hz, 1H), 8.08 (d, J = 8.0 Hz, 1H), 8.04 (d, J = 8.0 Hz, 1H), 7.68 (d, J = 8.0 Hz, 1H), 5.95 (brs, 1H), 5.37 (brs, 2H), 4.32-4.19 (m, 3H), 4.16-4.11 (m, 1H), 2.98-2.94 (m, 2H), 2.27-2.13 (m, 4H), 2.00-1.79 (m, 5H), 1.76-1.52 (m, 5H), 1.42-1.36 (m, 20H), 0.86 (d, J = 6.8 Hz, 3H), 0.82 (d, J = 6.8 Hz, 3H).
MS (ESI) m/z: 687.35 [M+H]⁺

### SCA (OtBu)-Glu (OtBu)-Val-Cit-OH

¹H NMR (400 MHz, DMSO-d₆) δ12.70 (brs, 1H), 8.21 (d, J = 7.2 Hz, 1H), 8.05 (d, J = 8.0 Hz, 1H), 7.68 (d, J = 8.8 Hz, 1H), 5.96 (brs, 1H), 5.25 (brs, 2H), 4.35-4.30 (m, 1H), 4.21-4.18 (m, 1H), 4.15-4.10 (m, 1H), 2.98-2.94 (m, 2H), 2.40-2.28 (m, 4H), 2.24-2.18 (m, 2H), 2.01-1.93 (m, 1H), 1.90-1.81 (m, 1H), 1.73-1.63 (m, 2H), 1.61-1.51 (m, 1H), 1.40-1.31 (m, 20H), 0.86 (d, J = 6.8 Hz, 3H), 0.83 (d, J = 6.8 Hz, 3H).
MS (ESI) m/z: 616.30 [M+H]⁺

### Ac-Glu (OtBu)-Val-Ala-OH

¹H NMR (400 MHz, DMSO-d₆) δ12.50 (brs, 1H), 8.25 (d, J = 6.8Hz, 1H), 8.03 (d, J = 8.0 Hz, 1H), 7.68 (d, J = 9.2 Hz, 1H), 4.33-4.27 (m, 1H), 4.22-4.16 (m, 2H), 2.23-2.18 (m, 2H), 1.99-1.94 (m, 1H), 1.89-1.80 (m, 4H), 1.73-1.65 (m, 1H), 1.39 (s, 9H), 1.27 (d, J = 7.2 Hz, 3H), 0.87 (d, J = 6.8 Hz, 3H), 0.83 (d, J = 6.8 Hz, 3H).
MS (ESI) m/z: 416.20 [M+H]⁺

### Example 1: Synthesis of linker-payload mimic

### (1-1) Synthesis of Linker-payload mimic (1)

Linker-payload mimic (1) was synthesized as follows.

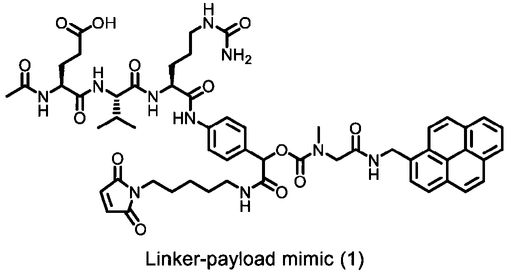

### (1-1-1) Synthesis of Alcohol (2)

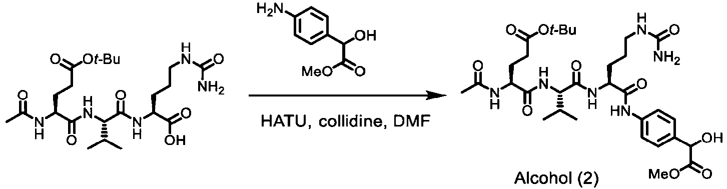

Ac-Glu (OtBu)-Val-Cit-OH (19.9 mg, 39.7 µmol) was dissolved in N,N-dimethylformamide (400 µL), and 1-[bis(dimethylamino)methylene]-1H-1,2,3,triazolo[4,5-b]pyridinium 3-oxide hexafluorophosphate (18.1 mg, 47.6 µmol) and 2,4,6-trimethylpyridine (6.27 µL, 47.6 µmol) were added, followed by stirring at room temperature for 10 minutes. Subsequently, methyl 4-aminomandelate (8.63 mg, 47.6 µmol) was added, and the mixture was stirred at room temperature for 21.5 hours, followed by purification by preparative reversed-phase chromatography. Fractions containing the product were collected, and acetonitrile was removed by concentration under reduced pressure, followed by lyophilization to obtain the above-described Alcohol (2) (28.5 mg, quant).

¹H NMR (400 MHz, DMSO-d₆) δ9.95 (s, 1H), 8.07 (d, J = 7.4 Hz, 1H), 7.99 (d, J = 8.0 Hz, 1H), 7.66 (d, J = 8.4 Hz, 1H), 7.50 (d, J = 8.4 Hz, 2H), 7.25 (d, J = 8.4 Hz, 2H), 5.92 (brs, 1H), 5.36 (brs, 2H), 5.01 (s, 1H), 4.34-4.29 (m, 1H), 4.26-4.20 (m, 1H), 4.14-4.10 (m, 1H), 3.53 (s, 3H), 3.00-2.83 (m, 2H), 2.18-2.13 (m, 2H), 1.94-1.89 (m, 2H), 1.84-1.23 (m, 17H), 0.79 (d, J = 6.8 Hz, 3H), 0.75 (d, J = 6.8 Hz, 3H).
MS (ESI) m/z: 665.30 [M+H]⁺

### (1-1-2) Synthesis of Pyrene (3)

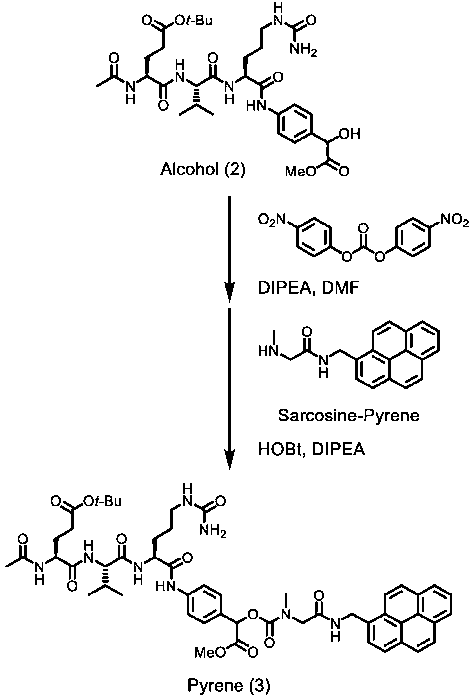

Alcohol (2) (28.5 mg) obtained in (1-1-1) was dissolved in N,N-dimethylformamide (430 µL) and stirred under ice cooling for 5 minutes, then bis(4-nitrophenyl) carbonate (26.6 mg, 85.7 µmol) and N,N-diisopropylethylamine (11.1 µL, 64.4 µmol) were added, and the mixture was stirred at room temperature for 1.5 hours. LC-MS tracking of the reaction revealed the presence of unreacted starting material, and therefore bis(4-nitrophenyl) carbonate (13.3 mg, 42.9 µmol) and N,N-diisopropylethylamine (5.54 µL, 32.2 µmol) were added, and the mixture was stirred at room temperature for 5 hours. Subsequently, the mixture was cooled on ice, then Sarcosine-Pyrene (64.9 mg, 215 µmol), 1-hydroxybenzotriazole (8.7 mg, 64 µmol), and N,N-diisopropylethylamine (57.2 µL, 333 µmol) were added, and the mixture was stirred at room temperature for 16 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (3) (26.1 mg, 26.3 µmol).

¹H NMR (400 MHz, DMSO-d₆) δ10.09-10.07 (m, 1H), 8.63-8.60 (m, 1H), 8.33-7.65 (m, 13H), 7.60-7.57 (m, 2H), 7.37-7.32 (m, 2H), 5.94-5.91 (m, 1H), 5.72-5.70 (m, 1H), 5.37 (brs, 2H), 4.98-4.96 (m, 1H), 4.93-4.00 (m, 4H), 3.85-3.75 (m, 1H), 3.56-3.55 (m, 3H), 2.97-2.87 (m, 5H), 2.17-2.13 (m, 2H), 1.93-1.17 (m, 19H), 0.80-0.73 (m, 6H).
MS (ESI) m/z: 993.40 [M+H]⁺

### (1-1-3) Synthesis of Pyrene (4)

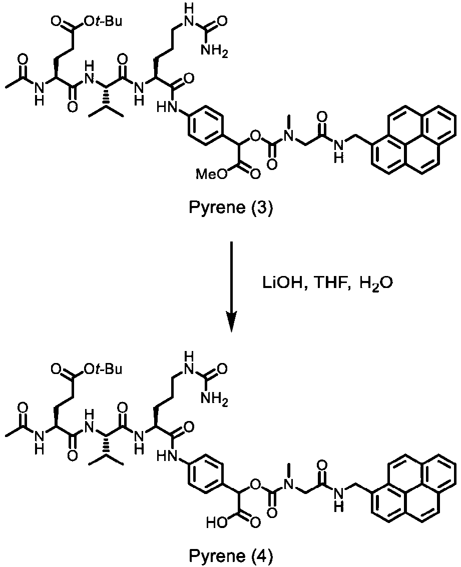

Pyrene (3) (10.8 mg, 10.9 µmol) was dissolved in tetrahydrofuran (700 µL) and water (400 µL) and stirred under ice cooling for 5 minutes, then 1 M aqueous lithium hydroxide solution (109 µL, 109 µmol) was added, and the mixture was stirred at room temperature for 1 hour. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (4) (4.5 mg, 4.6 µmol).

¹H NMR (400 MHz, DMSO-d₆) δ13.05 (brs, 1H), 10.08-10.05 (m, 1H), 8.62-8.59 (m, 1H), 8.33-7.56 (m, 15H), 7.37-7.35 (m, 2H), 5.92 (brs, 1H), 5.61-5.60 (m, 1H), 5.36 (brs, 2H), 4.98-4.03 (m, 5H), 3.88-3.74 (m, 1H), 2.99-2.83 (m, 5H), 2.15-2.13 (m, 2H), 1.93-1.14 (m, 19H), 0.84-0.73 (m, 6H).
MS (ESI) m/z: 979.40 [M+H]⁺

### (1-1-4) Synthesis of Pyrene (5)

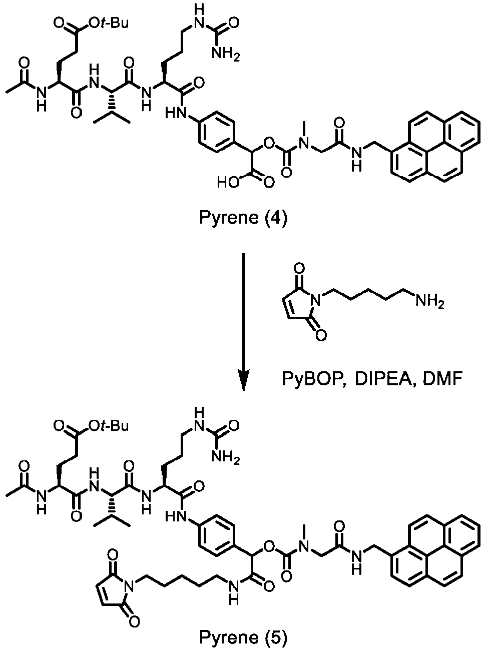

Pyrene (4) (3.7 mg, 3.8 µmol) was dissolved in N,N-dimethylformamide (400 µL) and cooled on ice, then N,N-diisopropylethylamine (1.9 µL, 11 µmol) and 1H-benzotriazol-1-yloxytripyrrolidinophosphonium hexafluorophosphate (2.9 mg, 5.6 µmol) were added. Next, N-(5-aminopentyl)maleimide hydrochloride (1.3 mg, 5.7 µmol) was added, and the mixture was returned to room temperature and stirred for 2 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (5) (1.3 mg, 1.1 µmol).

¹H NMR (400 MHz, DMSO-d₆) δ10.02-9.99 (m, 1H), 8.85-7.88 (m, 14H), 7.67-7.65 (m, 1H), 7.60-7.51 (m, 2H), 7.33-7.27 (m, 2H), 6.91-6.87 (m, 2H), 5.92-5.91 (m, 1H), 5.63-5.62 (m, 1H), 5.36 (brs, 2H), 5.07-4.92 (m, 2H), 4.35-3.76 (m, 5H), 3.18-3.14 (m, 1H), 2.99-2.83 (m, 7H), 2.17-2.13 (m, 2H), 1.95-1.89 (m, 1H), 1.85-1.72 (m, 4H), 1.66-1.45 (m, 3H), 1.40-1.17 (m, 15H), 1.05-1.01 (m, 2H), 0.83-0.73 (m, 6H).
MS (ESI) m/z: 1143.45 [M+H]⁺

### (1-1-5) Synthesis of Linker-payload mimic (1)

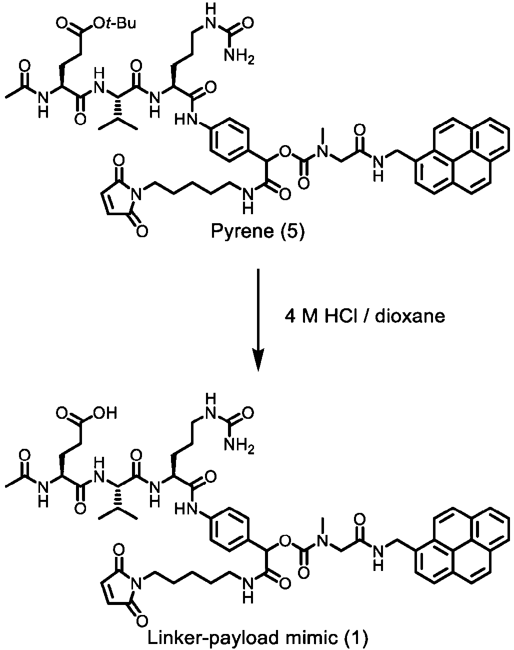

To Pyrene (5) (2.2 mg, 1.9 µmol) were successively added 1,4-dioxane (380 µL) and 4M hydrogen chloride/dioxane solution (95 µL, 380 µmol), and the mixture was stirred at room temperature for 4 hours. After cooling on ice, N,N-diisopropylethylamine (71.8 µL, 418 µmol) was added, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (1) (2.1 mg, 1.9 µmol).

¹H NMR (400 MHz, DMSO-d₆) δ12.06 (brs, 1H), 10.03-10.00 (m, 1H), 8.84-7.88 (m, 14H), 7.67-7.64 (m, 1H), 7.56-7.51 (m, 2H), 7.33-7.27 (m, 2H), 6.90-6.87 (m, 2H), 5.92-5.90 (m, 1H), 5.63-5.61 (m, 1H), 5.36 (brs, 2H), 5.08-4.96 (m, 2H), 4.35-3.76 (m, 5H), 3.18-3.14 (m, 1H), 2.97-2.83 (m, 7H), 2.20-2.16 (m, 2H), 1.93-1.88 (m, 1H), 1.81-1.78 (m, 4H), 1.69-1.53 (m, 3H), 1.35-1.17 (m, 6H), 1.08-1.01 (m, 2H), 0.81-0.73 (m, 6H).
MS (ESI) m/z: 1087.45 [M+H]⁺

### (1-2) Synthesis of Linker-payload mimic (6)

Linker-payload mimic (6) was synthesized as follows.

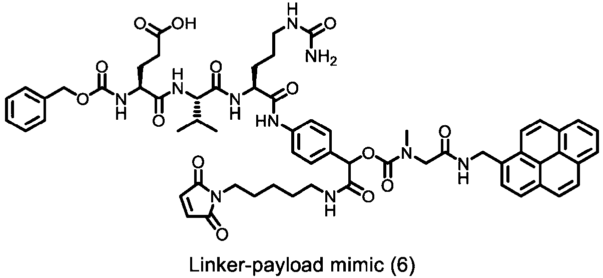

### (1-2-1) Synthesis of Alcohol (7)

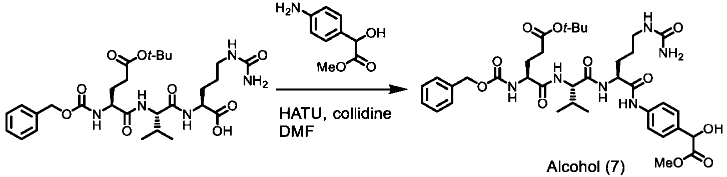

Z-Glu(t-Bu)-Val-Cit-OH (50.0 mg, 84.3 µmol) was dissolved in N,N-dimethylformamide (1.5 mL), and 1-[bis(dimethylamino)methylene]-1H-1,2,3,triazolo[4,5-b]pyridinium 3-oxide hexafluorophosphate (38.4 mg, 101 µmol) and 2,4,6-trimethylpyridine (13.3 µL, 101 µmol) were added, followed by stirring at room temperature for 10 minutes. Subsequently, methyl 4-aminomandelate (18.3 mg, 101 µmol) was added, and the mixture was stirred at room temperature for 16 hours, followed by purification by preparative reversed-phase chromatography. Fractions containing the product were collected, and acetonitrile was removed by concentration under reduced pressure, followed by lyophilization to obtain the above-described Alcohol (7) (49.1 mg, 64.9 µmol).

¹H NMR (400 MHz, DMSO-d₆) δ10.04-9.95 (m, 1H), 8.40-7.28 (m, 12H), 6.00-5.97 (m, 1H), 5.43 (brs, 2H), 5.08-4.97 (m, 3H), 4.43-4.37 (m, 1H), 4.24-4.20 (m, 1H), 4.16-4.05 (m, 1H), 3.60-3.59 (m, 3H), 3.04-2.91 (m, 2H), 2.26-2.20 (m, 2H), 2.03-1.22 (m, 16H), 0.88-0.78 (m, 6H).
MS (ESI) m/z: 757.30 [M+H]⁺

### (1-2-2) Synthesis of Pyrene (8)

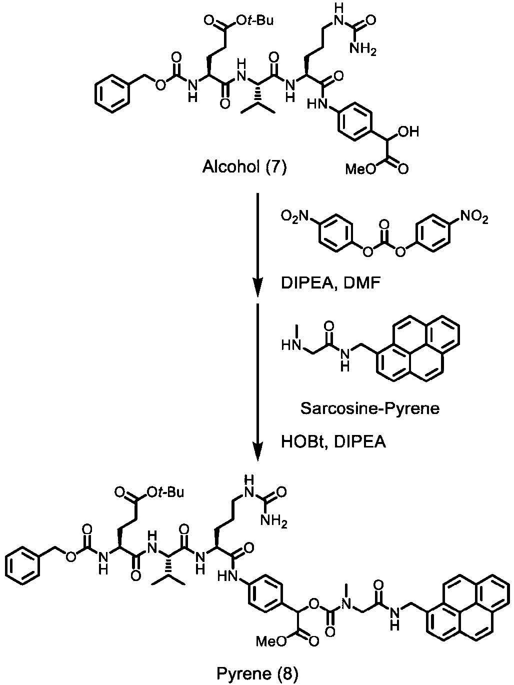

Alcohol (7) (44.6 mg, 58.9 µmol) was dissolved in N,N-dimethylformamide (650 µL) and stirred under ice cooling for 5 minutes, then bis(4-nitrophenyl) carbonate (53.8 mg, 177 µmol) and N, N-diisopropylethylamine (22.5 µL, 133 µmol) were added, and the mixture was stirred at room temperature for 4 hours. Subsequently, the mixture was cooled on ice, then Sarcosine-Pyrene (89.1 mg, 295 µmol), 1-hydroxybenzotriazole (11.9 mg, 88.4 µmol), and N, N-diisopropylethylamine (77.7 µL, 457 µmol) were added, and the mixture was stirred at room temperature for 18 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (8) (49.2 mg, 45.3 µmol).

¹H NMR (400 MHz, DMSO-d6) δ10.11-9.97 (m, 1H), 8.64-8.60 (m, 1H), 8.36-7.95 (m, 11H), 7.82-7.74 (m, 1H), 7.65-7.57 (m, 2H), 7.49-7.19 (m, 8H), 5.91-5.90 (m, 1H), 5.72-5.70 (m, 1H), 5.36 (brs, 2H), 4.98-4.86 (m, 4H), 4.41-4.00 (m, 3H), 3.85-3.75 (m, 1H), 3.56-3.54 (m, 3H), 3.00-2.82 (m, 5H), 2.19-2.12 (m, 2H), 1.92-1.17 (m, 16H), 0.80-0.73 (m, 6H).
MS (ESI) m/z: 1085.45 [M+H]⁺

### (1-2-3) Synthesis of Pyrene (9)

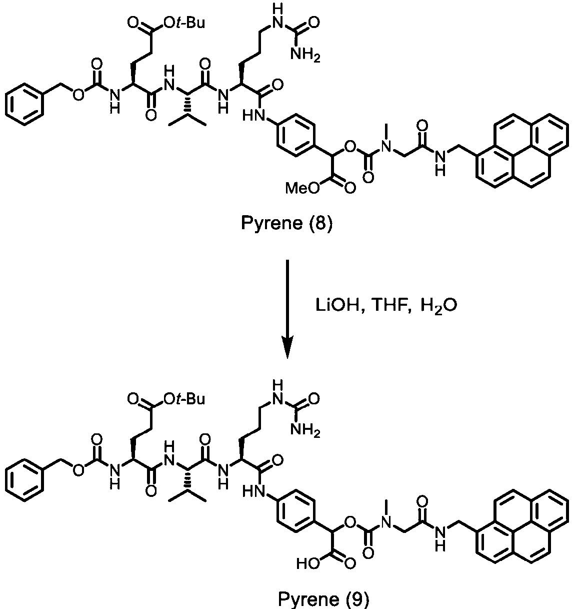

Pyrene (8) (44.8 mg, 41.3 µmol) was dissolved in tetrahydrofuran (3.75 mL) and water (1.25 mL) and stirred under ice cooling for 5 minutes, then lithium hydroxide monohydrate (8.7 mg, 210 µmol) was added, and the mixture was stirred at room temperature for 4 hours. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (9) (18.4 mg, 17.2 µmol).

¹H NMR(400 MHz, DMSO-d₆) δ13.02 (brs, 1H), 10.09-9.97 (m, 1H), 8.62-8.59 (m, 1H), 8.32-7.78 (m, 12H), 7.63-7.44 (m, 3H), 7.37-7.35 (m, 2H), 7.29-7.20 (m, 5H), 5.91 (brs, 1H), 5.61-5.60 (m, 1H), 5.36 (brs, 2H), 4.98-4.86 (m, 4H), 4.45-4.30 (m, 1H), 4.24-4.15 (m, 1H), 4.07-4.02 (m, 1H), 3.84-3.74 (m, 1H), 2.97-2.82 (m, 5H), 2.19-2.12 (m, 2H), 1.93-1.11 (m, 16H), 0.80-0.72 (m, 6H).
MS (ESI) m/z: 1071.45 [M+H]⁺

### (1-2-4) Synthesis of Pyrene (10)

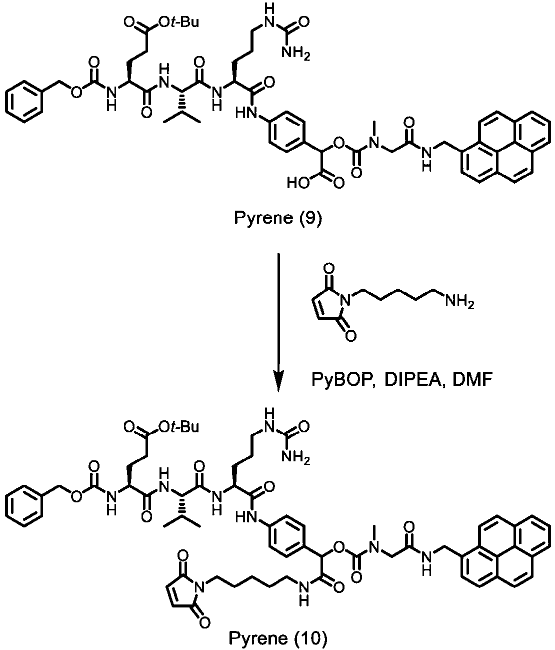

Pyrene (9) (15.6 mg, 14.6 µmol) was dissolved in N,N-dimethylformamide (1.0 mL) and cooled on ice, then N,N-diisopropylethylamine (5.0 µL, 29 µmol) and 1H-benzotriazol-1-yloxytripyrrolidinophosphonium hexafluorophosphate (11.4 mg, 21.9 µmol) were added. Next, N-(5-aminopentyl)maleimide hydrochloride (4.8 mg, 22 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (10) (15.4 mg, 12.5 µmol).

¹H NMR (400 MHz, DMSO-d₆) δ10.11-10.01 (m, 1H), 8.91-8.63 (m, 1H), 8.40-7.96 (m, 12H), 7.72-7.27 (m, 11H), 6.97-6.94 (m, 2H), 5.99 (brs, 1H), 5.71-5.69 (m, 1H), 5.43 (brs, 2H), 5.14-4.96 (m, 4H), 4.49-4.40 (m, 1H), 4.30-4.23 (m, 1H), 4.16-3.83 (m, 3H), 3.25-3.22 (m, 1H), 3.02-2.90 (m, 7H), 2.26-2.22 (m, 2H), 2.01-1.98 (m, 1H), 1.92-1.84 (m, 1H), 1.77-1.66 (m, 2H), 1.65-1.04 (m, 16H), 1.12-1.04 (m, 2H), 0.88-0.80 (m, 6H).
MS (ESI) m/z: 1235.50 [M+H]⁺

### (1-2-5) Synthesis of Linker-payload mimic (6)

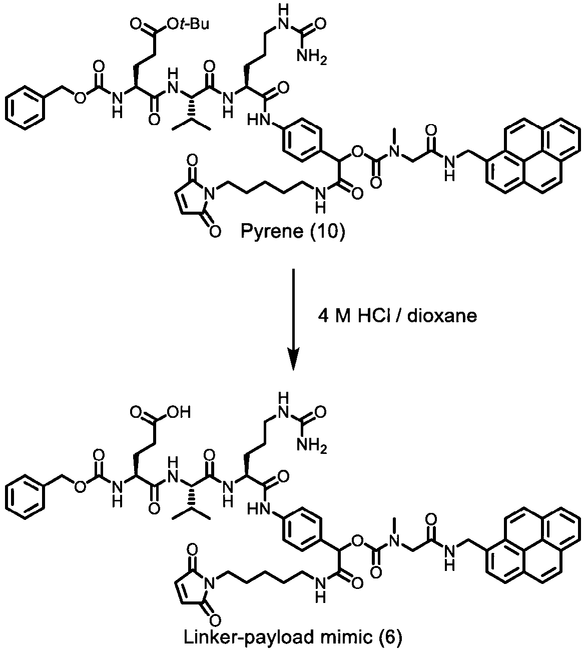

To Pyrene (10) (12.1 mg, 9.79 µmol) were successively added 1,4-dioxane (2.0 mL) and 4M hydrogen chloride/dioxane solution (490 µL, 1.96 mmol), and the mixture was stirred at room temperature for 4 hours. After cooling on ice, N,N-diisopropylethylamine (366 µL, 2.15 mmol) was added, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (6) (6.0 mg, 5.1 µmol).

¹H NMR (400 MHz, DMSO-d₆) δ12.05 (brs, 1H), 10.04-9.94 (m, 1H), 8.84-8.57 (m, 1H), 8.32-7.89 (m, 12H), 7.72-7.20 (m, 11H), 6.90-6.86 (m, 2H), 5.90 (brs, 1H), 5.64-5.62 (m, 1H), 5.36 (brs, 2H), 5.07-4.88 (m, 4H), 4.42-4.29 (m, 1H), 4.21-4.15 (m, 1H), 4.08-3.76 (m, 3H), 3.18-3.14 (m, 1H), 2.95-2.82 (m, 7H), 2.21-2.16 (m, 2H), 1.92-0.97 (m, 13H), 0.82-0.74 (m, 6H).
MS (ESI) m/z: 1179.50 [M+H]⁺

### (1-3) Synthesis of Linker-payload mimic (11)

Linker-payload mimic (11) was synthesized as follows.

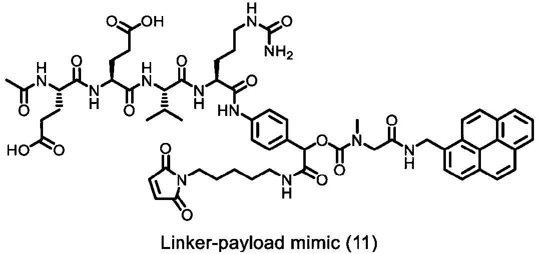

### (1-3-1) Synthesis of Alcohol (12)

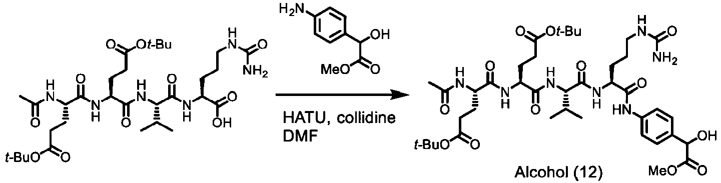

Ac-Glu(t-Bu)-Glu(t-Bu)-Val-Cit-OH (50.0 mg, 72.8 µmol) was dissolved in N,N-dimethylformamide (800 µL), and 1-[bis(dimethylamino)methylene]-1H-1,2,3,triazolo[4,5-b]pyridinium 3-oxide hexafluorophosphate (33.2 mg, 87.4 µmol) and 2,4,6-trimethylpyridine (11.5 µL, 87.4 µmol) were added, followed by stirring at room temperature for 10 minutes. Subsequently, methyl 4-aminomandelate (15.8 mg, 87.4 µmol) was added, and the mixture was stirred at room temperature for 16 hours, followed by purification by preparative reversed-phase chromatography. Fractions containing the product were collected, and acetonitrile was removed by concentration under reduced pressure, followed by lyophilization to obtain the above-described Alcohol (12) (54.0 mg, 63.5 µmol).

¹H NMR (400 MHz, DMSO-d₆) δ10.00 (s, 1H), 8.26-7.88 (m, 3H), 7.68-7.60 (m, 1H), 7.57 (d, J = 8.4 Hz, 2H), 7.32 (d, J = 8.4 Hz, 2H), 6.00-5.97 (m, 1H), 5.43 (brs, 2H), 5.08 (s, 1H), 4.40-4.37 (m, 1H), 4.32-4.19 (m, 3H), 3.60 (s, 3H), 3.09-2.90 (m, 2H), 2.25-2.18 (m, 4H), 2.03-1.53 (m, 10H), 1.46-1.36 (m, 20H), 0.86 (d, J = 6.8 Hz, 3H), 0.82 (d, J = 6.8 Hz, 3H).
MS (ESI) m/z: 850.40 [M+H]⁺

### (1-3-2) Synthesis of Pyrene (13)

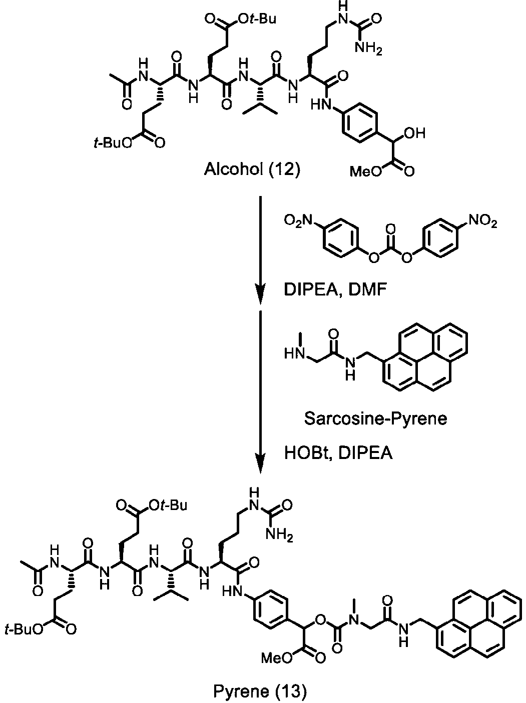

Alcohol (12) (50.3 mg, 59.2 µmol) was dissolved in N,N-dimethylformamide (650 µL) and stirred under ice cooling for 5 minutes, then bis(4-nitrophenyl) carbonate (54.0 mg, 178 µmol) and N,N-diisopropylethylamine (22.7 µL, 133 µmol) were added, and the mixture was stirred at room temperature for 5 hours. Subsequently, the mixture was cooled on ice, then Sarcosine-Pyrene (89.5 mg, 296 µmol), 1-hydroxybenzotriazole (12.0 mg, 88.8 µmol), and N,N-diisopropylethylamine (78.1 µL, 459 µmol) were added, and the mixture was stirred at room temperature for 18 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (13) (34.0 mg, 28.9 µmol).

¹H NMR (400 MHz, DMSO-d₆) δ10.14-10.12 (m, 1H), 8.69-8.67 (m, 1H), 8.40-7.82 (m, 13H), 7.76-7.72 (m, 1H), 7.68-7.65 (m, 2H), 7.44-7.39 (m, 2H), 5.99 (brs, 1H), 5.79 (d, J = 6.4 Hz, 1H), 5.44 (brs, 2H), 5.05-4.97 (m, 2H), 4.44-4.08 (m, 4H), 3.93-3.80 (m, 1H), 3.63-3.62 (m, 3H), 3.05-2.92 (m, 5H), 2.25-2.14 (m, 4H), 2.00-1.28 (m, 30H), 0.87-0.80 (m, 6H).
MS (ESI) m/z: 1178.50 [M+H]⁺

### (1-3-3) Synthesis of Pyrene (14)

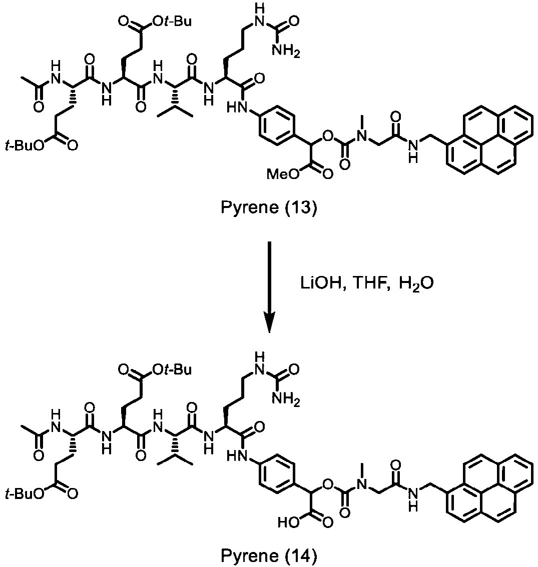

Pyrene (13) (30.7 mg, 26.1 µmol) was dissolved in tetrahydrofuran (2.25 mL) and water (0.75 mL) and stirred under ice cooling for 5 minutes, then lithium hydroxide monohydrate (5.5 mg, 0.13 mmol) was added, and the mixture was stirred at room temperature for 4 hours. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (14) (17.2 mg, 14.8 µmol).

¹H NMR (400 MHz, DMSO-d₆) δ13.06 (brs, 1H), 10.13-10.10 (m, 1H), 8.69-8.66 (m, 1H), 8.40-7.85 (m, 13H), 7.77-7.73 (m, 1H), 7.67-7.64 (m, 2H), 7.44-7.42 (m, 2H), 5.99 (brs, 1H), 5.68-5.67 (m, 1H), 5.44 (brs, 2H), 5.05-4.96 (m, 2H), 4.46-4.10 (m, 4H), 3.92-3.81 (m, 1H), 3.05-2.90 (m, 5H), 2.25-2.14 (m, 4H), 2.03-1.29 (m, 30H), 0.88-0.80 (m, 6H).
MS (ESI) m/z: 1164.55 [M+H]⁺

### (1-3-4) Synthesis of Pyrene (15)

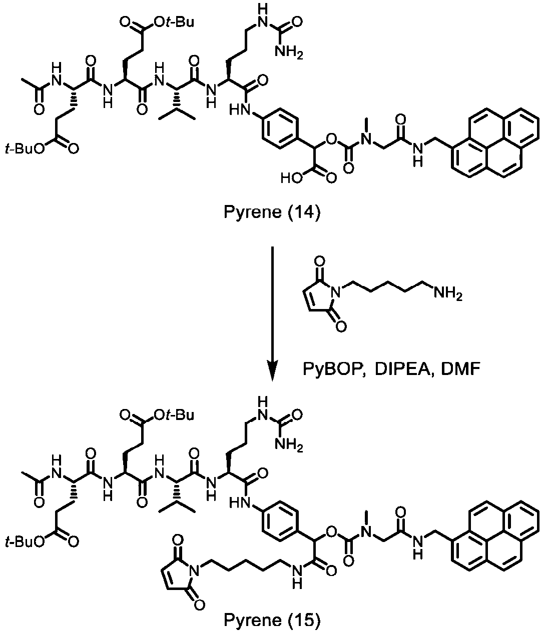

Pyrene (14) (14.7 mg, 12.6 µmol) was dissolved in N,N-dimethylformamide (1.0 mL) and cooled on ice, then N,N-diisopropylethylamine (4.29 µL, 25.2 µmol) and 1H-benzotriazol-1-yloxytripyrrolidinophosphonium hexafluorophosphate (9.8 mg, 19 µmol) were added. Next, N-(5-aminopentyl)maleimide hydrochloride (4.1 mg, 19 µmol) was added, and the mixture was returned to room temperature and stirred for 3.5 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (15) (7.0 mg, 9.2 µmol).

¹H NMR(400 MHz, DMSO-d₆) δ10.08-10.05 (m, 1H), 8.92-7.95 (m, 15H), 7.75-7.73 (m, 1H), 7.63-7.59 (m, 2H), 7.40-7.34 (m, 2H), 6.97-6.94 (m, 2H), 6.00-5.98 (m, 1H), 5.70-5.69 (m, 1H), 5.43 (brs, 2H), 5.14-5.01 (m, 2H), 4.45-4.38 (m, 1H), 4.32-3.83 (m, 5H), 3.25-3.20 (m, 1H), 3.10-2.90 (m, 7H), 2.25-2.18 (m, 4H), 2.08-1.24 (m, 34H), 1.12-1.04 (m, 2H), 0.88-0.81 (m, 6H).
MS (ESI) m/z: 1328.60 [M+H]⁺

### (1-3-5) Synthesis of Linker-payload mimic (11)

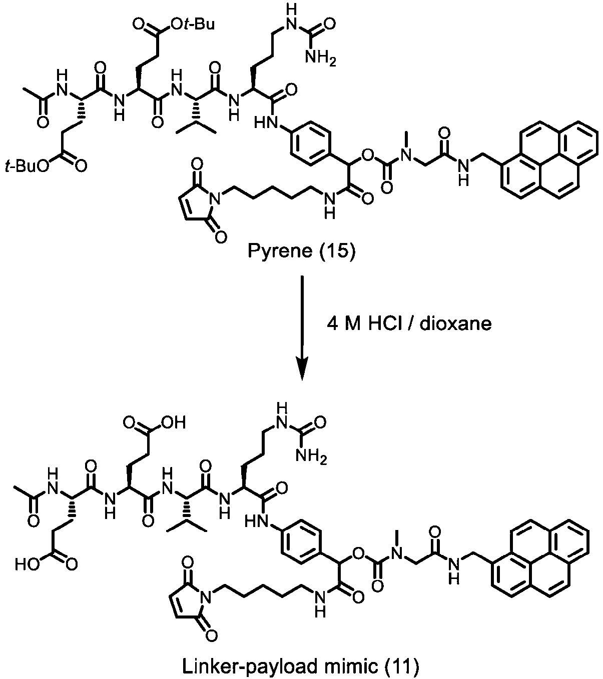

To Pyrene (15) (6.1 mg, 4.6 µmol) were successively added 1,4-dioxane (920 µL) and 4M hydrogen chloride/dioxane solution (230 µL, 918 µmol), and the mixture was stirred at room temperature for 4 hours. After cooling on ice, N,N-diisopropylethylamine (172 µL, 1.10 mmol) was added, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (11) (3.7 mg, 3.0 µmol).

¹H NMR(400 MHz, DMSO-d₆) δ12.04 (brs, 2H), 10.01-9.98 (m, 1H), 8.83-7.89 (m, 15H), 7.71-7.69 (m, 1H), 7.56-7.51 (m, 2H), 7.33-7.26 (m, 2H), 6.90-6.87 (m, 2H), 5.91-5.90 (m, 1H), 5.64-5.62 (m, 1H), 5.36 (brs, 2H), 5.08-4.92 (m, 2H), 4.35-4.32 (m, 1H), 4.25-3.76 (m, 5H), 3.18-3.14 (m, 1H), 2.99-2.82 (m, 7H), 2.21-2.15 (m, 4H), 1.93-1.17 (m, 16H), 1.05-1.01 (m, 2H), 0.82-0.74 (m, 6H).
MS (ESI) m/z: 1216.45 [M+H]⁺

### (1-4) Synthesis of Linker-payload mimic (16)

Linker-payload mimic (16) was synthesized as follows.

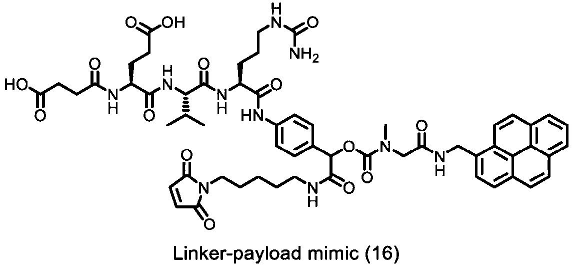

### (1-4-1) Synthesis of Alcohol (17)

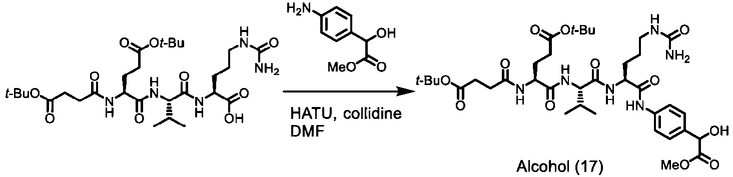

SCA(t-Bu)-Glu(t-Bu)-Val-Cit-OH (50.0 mg, 81.2 µmol) was dissolved in N,N-dimethylformamide (890 µL), and 1-[bis(dimethylamino)methylene]-1H-1,2,3,triazolo[4,5-b]pyridinium 3-oxide hexafluorophosphate (37.0 mg, 97.4 µmol) and 2,4,6-trimethylpyridine (12.8 µL, 97.4 µmol) were added, followed by stirring at room temperature for 10 minutes. Subsequently, methyl 4-aminomandelate (17.6 mg, 97.4 µmol) was added, and the mixture was stirred at room temperature for 16 hours, followed by purification by preparative reversed-phase chromatography. Fractions containing the product were collected, and acetonitrile was removed by concentration under reduced pressure, followed by lyophilization to obtain the above-described Alcohol (17) (60.4 mg, 77.5 µmol).

¹H NMR (400 MHz, DMSO-d₆) δ10.01 (s, 1H), 8.13 (d, J=7.2Hz, 1H), 8.07 (d, J = 8.4 Hz, 1H), 7.73 (d, J = 8.8 Hz, 1H), 7.57 (d, J = 8.4 Hz, 2H), 7.32 (d, J = 8.4 Hz, 2H), 5.99 (brs, 1H), 5.43 (brs, 2H), 5.08 (s, 1H), 4.41-4.30 (m, 2H), 4.21-4.17 (m, 1H), 3.60 (s, 3H), 3.04-2.95 (m, 2H), 2.44-2.15 (m, 6H), 2.03-1.95 (m, 1H), 1.92-1.83 (m, 1H), 1.74-1.58 (m, 3H), 1.46-1.34 (m, 20H), 0.86 (d, J = 6.8 Hz, 3H), 0.83 (d, J = 6.8 Hz, 3H).
MS (ESI) m/z: 779.40 [M+H]⁺

### (1-4-2) Synthesis of Pyrene (18)

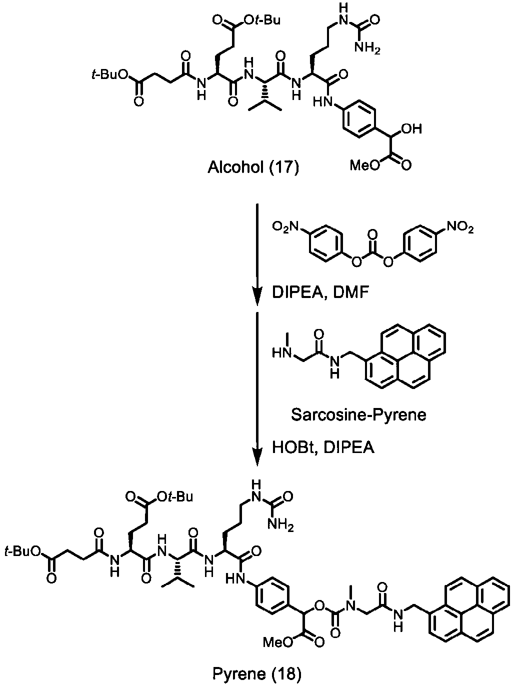

Alcohol (17) (58.0 mg, 74.5 µmol) was dissolved in N,N-dimethylformamide (820 µL) and stirred under ice cooling for 5 minutes, then bis(4-nitrophenyl) carbonate (68.0 mg, 223 µmol) and N,N-diisopropylethylamine (28.5 µL, 168 µmol) were added, and the mixture was stirred at room temperature for 6 hours. Subsequently, the mixture was cooled on ice, then Sarcosine-Pyrene (113 mg, 373 µmol), 1-hydroxybenzotriazole (15.1 mg, 112 µmol), and N,N-diisopropylethylamine (98.3 µL, 578 µmol) were added, and the mixture was stirred at room temperature for 17 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (18) (37.1 mg, 33.5 µmol).

¹H NMR (400 MHz, DMSO-d₆) δ10.15-10.13 (m, 1H), 8.70-8.67 (m, 1H), 8.40-8.03 (m, 11H), 7.90-7.86 (m, 1H), 7.75-7.72 (m, 1H), 7.68-7.64 (m, 2H), 7.44-7.39 (m, 2H), 5.99 (brs, 1H), 5.79 (d, J = 6.8 Hz, 1H), 5.44 (brs, 2H), 5.05-4.98 (m, 2H), 4.40-4.08 (m, 3H), 3.93-3.80 (m, 1H), 3.63-3.62 (m, 3H), 3.05-2.90 (m, 5H), 2.44-2.15 (m, 6H), 2.02-1.98 (m, 1H), 1.91-1.85 (m, 1H), 1.75-1.55 (m, 3H), 1.50-1.30 (m, 20H), 0.88-0.80 (m, 6H).
MS (ESI) m/z: 1107.55 [M+H]⁺

### (1-4-3) Synthesis of Pyrene (19)

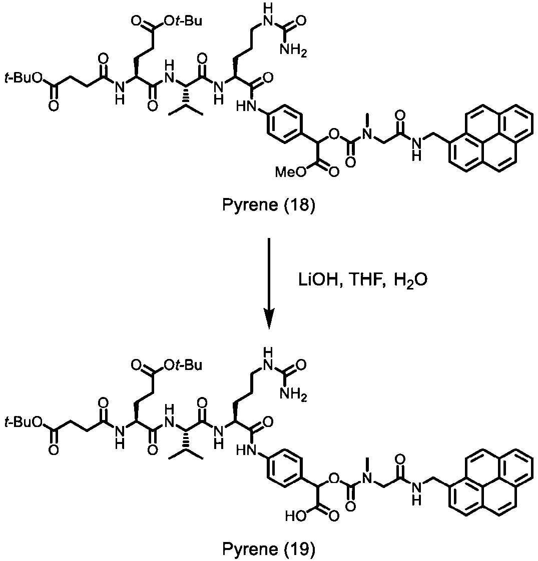

Pyrene (18) (17.4 mg, 15.7 µmol) was dissolved in tetrahydrofuran (675 µL) and water (225 µL) and stirred under ice cooling for 5 minutes, then 1 M aqueous lithium hydroxide solution (86.4 µL, 86.4 µmol) was added, and the mixture was stirred at room temperature for 5 hours. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (19) (17.2 mg, 15.7 µmol).

¹H NMR (400 MHz, DMSO-d₆) δ13.06 (brs, 1H), 10.14-10.11 (m, 1H), 8.68-8.66 (m, 1H), 8.40-8.01 (m, 11H), 7.89-7.85 (m, 1H), 7.75-7.72 (m, 1H), 7.67-7.64 (m, 2H), 7.44-7.41 (m, 2H), 5.99 (brs, 1H), 5.68-5.67 (m, 1H), 5.44 (brs, 2H), 5.05-4.93 (m, 2H), 4.44-4.10 (m, 3H), 3.92-3.81 (m, 1H), 3.05-2.94 (m, 5H), 2.43-2.15 (m, 6H), 2.02-1.97 (m, 1H), 1.88-1.85 (m, 1H), 1.77-1.66 (m, 3H), 1.61-1.30 (m, 20H), 0.88-0.80 (m, 6H).
MS (ESI) m/z: 1093.50 [M+H]⁺

### (1-4-4) Synthesis of Pyrene (20)

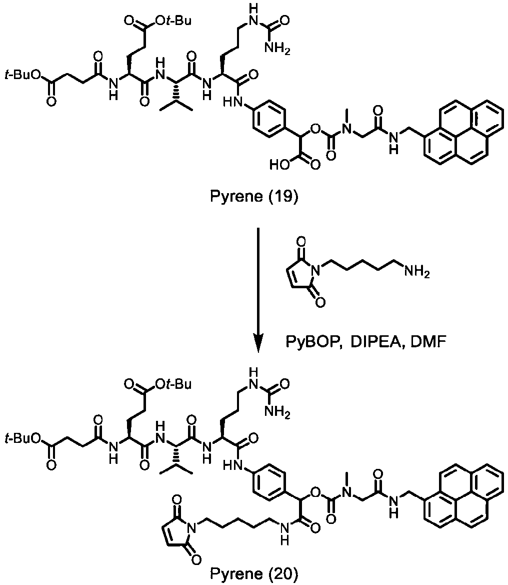

Pyrene (19) (16.3 mg, 14.9 µmol) was dissolved in N,N-dimethylformamide (1.0 mL) and cooled on ice, then N,N-diisopropylethylamine (5.1 µL, 30 µmol) and 1H-benzotriazol-1-yloxytripyrrolidinophosphonium hexafluorophosphate (11.7 mg, 22.4 µmol) were added. Next, N-(5-aminopentyl)maleimide hydrochloride (4.9 mg, 22 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (20) (12.1 mg, 9.62 µmol).

¹H NMR (400 MHz, DMSO-d₆) δ10.01-9.98 (m, 1H), 8.85-7.88 (m, 14H), 7.68-7.65 (m, 1H), 7.56-7.51 (m, 2H), 7.33-7.27 (m, 2H), 6.90-6.87 (m, 2H), 5.92-5.90 (m, 1H), 5.63-5.62 (m, 1H), 5.36 (brs, 2H), 5.08-4.92 (m, 2H), 4.36-3.76 (m, 5H), 3.18-3.14 (m, 1H), 3.00-2.83 (m, 7H), 2.36-2.08 (m, 6H), 1.95-1.89 (m, 1H), 1.84-1.78 (m, 1H), 1.68-1.50 (m, 3H), 1.42-1.17 (m, 24H), 1.07-0.97 (m, 2H), 0.81-0.74 (m, 6H).
MS (ESI) m/z: 1257.55 [M+H]⁺

### (1-4-5) Synthesis of Linker-payload mimic (16)

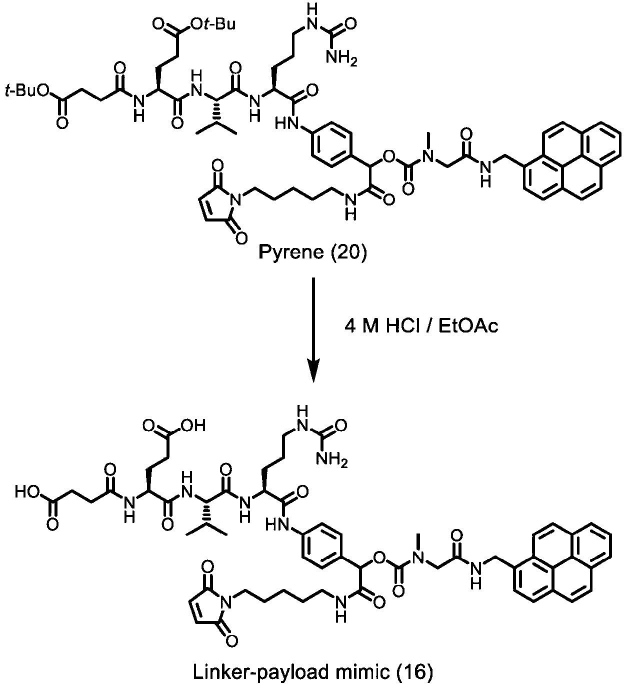

To Pyrene (20) (10.5 mg, 8.35 µmol) were successively added ethyl acetate (1.7 mL) and 4 M hydrogen chloride/ethyl acetate (2.09 mL, 8.36 mmol), and the mixture was stirred at room temperature for 4.5 hours. After cooling on ice, N,N-diisopropylethylamine (781 µL, 4.59 mmol) was added, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (16) (7.5 mg, 6.6 µmol).

¹H NMR(400 MHz, DMSO-d₆) δ12.02 (brs, 2H), 10.04-9.98 (m, 1H), 8.85-7.89 (m, 14H), 7.65-7.62 (m, 1H), 7.56-7.52 (m, 2H), 7.33-7.27 (m, 2H), 6.90-6.87 (m, 2H), 5.92 (brs, 1H), 5.63-5.62 (m, 1H), 5.37 (brs, 2H), 5.08-4.93 (m, 2H), 4.36-3.76 (m, 5H), 3.18-3.14 (m, 1H), 3.02-2.80 (m, 7H), 2.38-2.16 (m, 6H), 1.96-1.77 (m, 2H), 1.70-1.17 (m, 9H), 1.06-0.98 (m, 2H), 0.81-0.74 (m, 6H).
MS (ESI) m/z: 1145.45 [M+H]⁺

### (1-5) Synthesis of Linker-payload mimic (21)

Linker-payload mimic (21) was synthesized as follows.

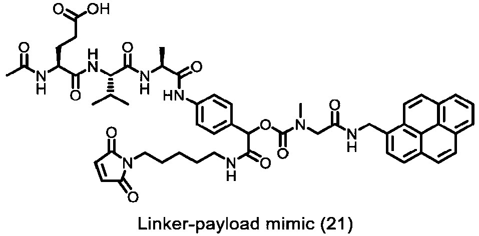

### (1-5-1) Synthesis of Alcohol (22)

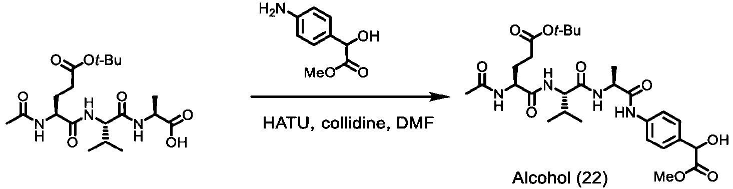

Ac-Glu(OtBu)-Val-Ala-OH (20.9 mg, 50.3 µmol) was dissolved in N,N-dimethylformamide (700 µL), and 1-[bis(dimethylamino)methylene]-1H-1,2,3,triazolo[4,5-b]pyridinium 3-oxide hexafluorophosphate (29.0 mg, 76.3 µmol) and 2,4,6-trimethylpyridine (10.1 µL, 76.7 µmol) were added, followed by stirring at room temperature for 12 minutes. Subsequently, methyl 4-aminomandelate (11.0 mg, 60.7 µmol) was added, and the mixture was stirred at room temperature for 18 hours, followed by purification by preparative reversed-phase chromatography. Fractions containing the product were collected, and acetonitrile was removed by concentration under reduced pressure, followed by lyophilization to obtain the above-described Alcohol (22) (20.3 mg, 35.1 µmol).

¹H NMR (400 MHz, DMSO-d₆) δ9.98-9.89 (m, 1H), 8.32-8.18 (m, 1H), 8.07-7.85 (m, 1H), 7.73-7.59 (m, 1H), 7.55 (d, J = 8.4 Hz, 2H), 7.32 (d, J = 8.4 Hz, 2H), 5.08 (s, 1H), 4.45-4.34 (m, 1H), 4.32-4.27 (m, 1H), 4.20-4.09 (m, 1H), 3.59 (s, 3H), 2.24-2.20 (m, 2H), 2.01-1.96 (m, 1H), 1.90-1.80 (m, 4H), 1.72-1.67 (m, 1H), 1.39-1.36 (m, 9H), 1.30 (d, J = 7.2 Hz, 3H), 0.86 (d, J = 6.8 Hz, 3H), 0.82 (d, J = 6.8 Hz, 3H).
MS (ESI) m/z: 579.30 [M+H]⁺

### (1-5-2) Synthesis of Pyrene (23)

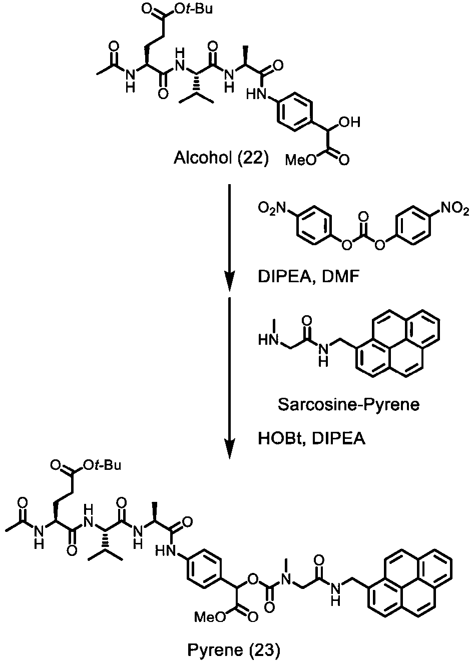

Alcohol (22) (17.5 mg, 30.2 µmol) was dissolved in N,N-dimethylformamide (174 µL) and stirred under ice cooling for 5 minutes, then bis(4-nitrophenyl) carbonate (18.9 mg, 60.8 µmol) and N,N-diisopropylethylamine (7.80 µL, 45.4 µmol) were added, and the mixture was stirred at room temperature for 30 minutes. LC-MS revealed the presence of unreacted starting material, and therefore bis(4-nitrophenyl) carbonate (9.5 mg, 31 µmol) and N,N-diisopropylethylamine (3.90 µL, 22.7 µmol) were added, and the mixture was stirred at room temperature for 1 hour. Subsequently, the mixture was cooled on ice, then Sarcosine-Pyrene (36.2 mg, 120 µmol), 1-hydroxybenzotriazole (6.3 mg, 47 µmol), and N,N-diisopropylethylamine (40.3 µL, 235 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain the above-described Pyrene (23) (12.9 mg, 14.2 µmol).

¹H NMR (400 MHz, DMSO-d₆) δ10.05-10.02 (m, 1H), 8.61-8.60 (m, 1H), 8.30-7.78 (m, 12H), 7.67-7.64 (m, 1H), 7.57 (d, J = 8.8 Hz, 2H), 7.37-7.33 (m, 2H), 5.72-5.70 (m, 1H), 4.98-4.89 (m, 2H), 4.34-4.00 (m, 3H), 3.85-3.75 (m, 1H), 3.56-3.55 (m, 3H), 2.93-2.87 (m, 3H), 2.17-2.13 (m, 2H), 1.94-1.91 (m, 1H), 1.85-1.75 (m, 4H), 1.65-1.61 (m, 1H), 1.31-1.23 (m, 12H), 0.80-0.73 (m, 6H).
MS (ESI) m/z: 907.35 [M+H]⁺

### (1-5-3) Synthesis of Pyrene (24)

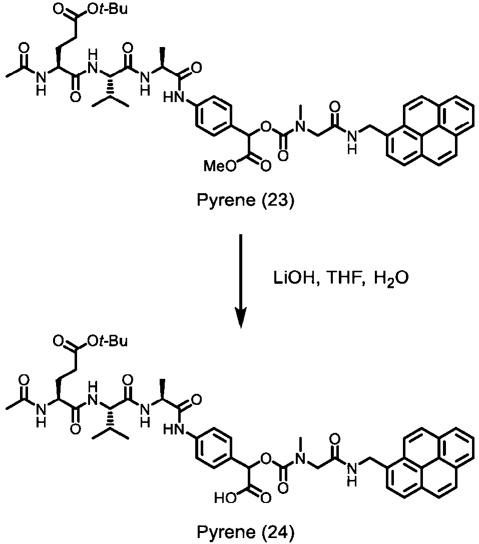

Pyrene (23) (11.8 mg, 13.0 µmol) was dissolved in tetrahydrofuran (800 µL) and water (400 µL) and cooled on ice, then 1 M aqueous lithium hydroxide solution (65 µL, 65 µmol) was added, and the mixture was stirred at room temperature for 30 minutes. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (24) (8.9 mg, 10.0 µmol).

¹H NMR (400 MHz, DMSO-d₆) δ13.05 (brs, 1H), 10.04-9.91 (m, 1H), 8.63-8.60 (m, 1H), 8.33-7.77 (m, 12H), 7.69-7.55 (m, 3H), 7.37-7.35 (m, 2H), 5.61-5.60 (m, 1H), 4.98-4.86 (m, 2H), 4.26-4.03 (m, 3H), 3.84-3.74 (m, 1H), 2.93-2.87 (m, 3H), 2.18-2.13 (m, 2H), 1.95-1.92 (m, 1H), 1.85-1.77 (m, 4H), 1.67-1.62 (m, 1H), 1.31-1.24 (m, 12H), 0.81-0.75 (m, 6H).
MS (ESI) m/z: 893.35 [M+H]⁺

### (1-5-4) Synthesis of Pyrene (25)

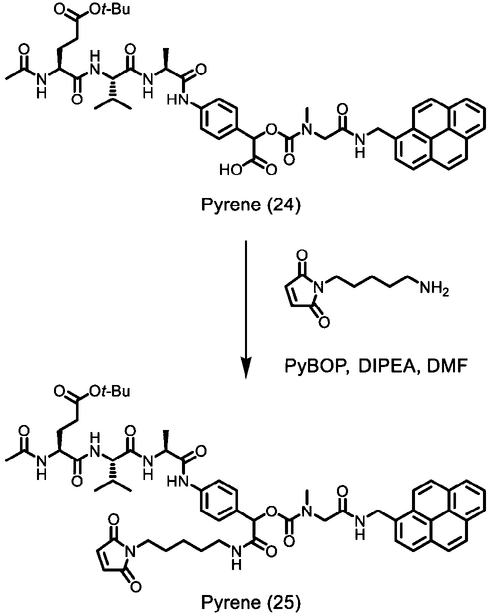

Pyrene (24) (6.7 mg, 7.5 µmol) was dissolved in N,N-dimethylformamide (400 µL) and cooled on ice, then N,N-diisopropylethylamine (3.9 µL, 22 µmol), 1H-benzotriazol-1-yloxytripyrrolidinophosphonium hexafluorophosphate (5.9 mg, 11 µmol) were added. Next, N-(5-aminopentyl)maleimide hydrochloride (2.4 mg, 11 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (25) (4.1 mg, 3.9 µmol).

¹H NMR (400 MHz, DMSO-d₆) δ9.98-9.95 (m, 1H), 8.83-7.77 (m, 14H), 7.67-7.65 (m, 1H), 7.56-7.50 (m, 2H), 7.34-7.27 (m, 2H), 6.89-6.86 (m, 2H), 5.63-5.61 (m, 1H), 5.06-4.93 (m, 2H), 4.37-3.76 (m, 5H), 3.17-3.14 (m, 1H), 2.95-2.83 (m, 5H), 2.17-2.10 (m, 2H), 1.95-1.89 (m, 1H), 1.85-1.75 (m, 4H), 1.65-1.62 (m, 1H), 1.31-1.17 (m, 16H), 1.02-1.00 (m, 2H), 0.81-0.74 (m, 6H).
MS (ESI) m/z: 1057.45 [M+H]⁺

### (1-5-5) Synthesis of Linker-payload mimic (21)

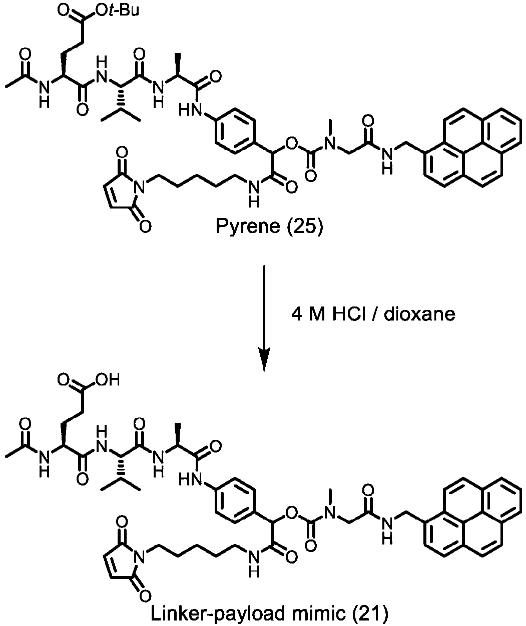

To Pyrene (25) (2.4 mg, 2.3 µmol) were successively added 1,4-dioxane (454 µL) and 4M hydrogen chloride/dioxane solution (568 µL, 2.27 mmol), and the mixture was stirred at room temperature for 4 hours. After adding N,N-dimethylformamide (300 µL), N,N-diisopropylethylamine (214 µL, 1.25 mmol) was added under ice cooling, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (21) (1.1 mg, 1.1 µmol).

¹H NMR (400 MHz, DMSO-d₆) δ11.99 (brs, 1H), 9.98-9.96 (m, 1H), 8.84-7.88 (m, 14H), 7.66-7.63 (m, 1H), 7.54-7.50 (m, 2H), 7.34-7.27 (m, 2H), 6.90-6.87 (m, 2H), 5.63-5.61 (m, 1H), 5.07-4.93 (m, 2H), 4.36-3.76 (m, 5H), 3.17-3.14 (m, 1H), 2.95-2.83 (m, 5H), 2.20-2.16 (m, 2H), 1.95-1.90 (m, 1H), 1.88-1.76 (m, 4H), 1.67-1.62 (m, 1H), 1.32-1.17 (m, 7H), 1.02-1.01 (m, 2H), 0.81-0.74 (m, 6H).
MS (ESI) m/z: 999.35[M-H]⁻

### Comparative Example 1: Synthesis of Linker-payload

### (1-1) Synthesis of Linker-payload (26)

Linker-payload (26) was synthesized as follows.

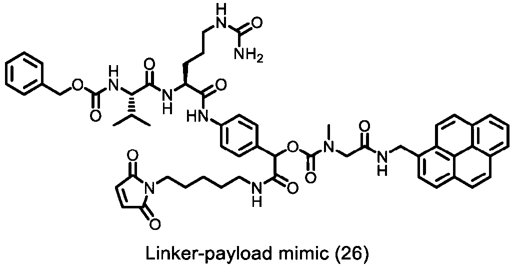

Linker-payload (26) was synthesized according to the following scheme.

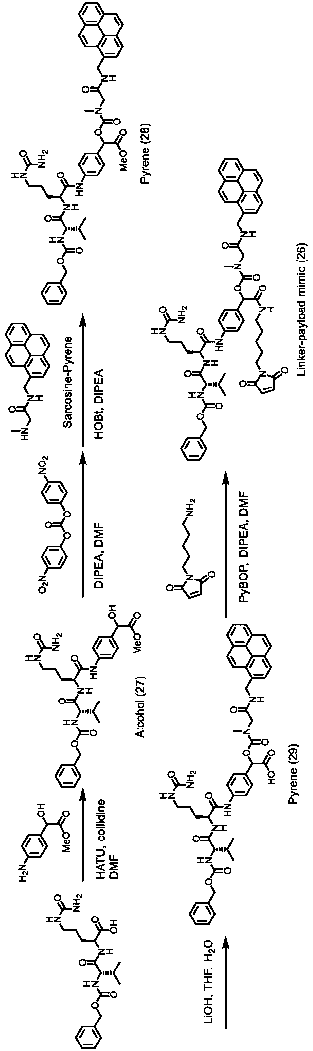

The MS analysis results for Linker-payload (26) were as follows.
MS (ESI) m/z: 1050.55 [M+H]⁺

### Example 2: Synthesis of ADC mimic

### (2-1) Synthesis of ADC mimic

In the following Comparative Examples and Examples, an antibody derivative (thiol-introduced trastuzumab) described in Example 81-7 of WO2019/240287A1 was used as the thiol-introduced antibody. This antibody derivative has the following structure in which a thiol group is regioselectively introduced into trastuzumab (humanized IgG1 antibody) via the amino group in the side chain of the lysine residue at position 246 or 248 of the antibody heavy chain (the position of the lysine residue is according to EU numbering). 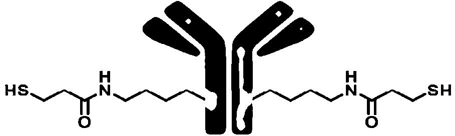 (In the above structure, NH-CH₂-CH₂-CH₂-CH₂- extending from the antibody heavy chain corresponds to the side chain of the lysine residue, and a thiol-containing group, HS-CH₂-CH₂-C (=O), is added to the amino group in the side chain of this lysine residue. Since no modification at other lysine residues was detected by peptide mapping, the antibody is interpreted to have 100% regioselectivity at position 246 or 248 of the antibody heavy chain.)

To a solution of the thiol-introduced antibody (20 µM) in buffer (PBS buffer at pH 7.4), 10 equivalents of a solution of the Linker-payload mimic synthesized in Example 1 (10 mM) in DMF was added. After standing at room temperature for 2 hours, the mixture was purified using NAP-5 Columns (manufactured by GE Healthcare) to obtain an ADC mimic.

### Example 2-1-1: Synthesis of ADC mimic 1

From Linker-payload mimic (1) synthesized in Example 1-1 and the thiol-containing antibody, ADC mimic 1 with the following structure was synthesized. ESI-TOFMS analysis was performed, and a peak was confirmed at 150350 for the reaction product, into which two molecules of Linker-payload mimic (1) were introduced.

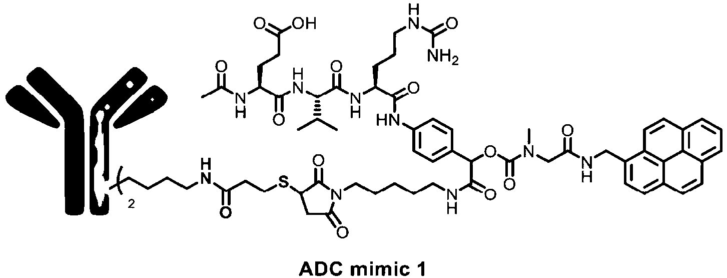

### Example 2-1-2: Synthesis of ADC mimic 2

Similarly, from Linker-payload mimic (6) synthesized in Example 1-2 and the thiol-containing antibody, ADC mimic 2 with the following structure was synthesized. ESI-TOFMS analysis was performed, and a peak was confirmed at 150535 for the reaction product, into which two molecules of Linker-payload mimic (6) were introduced.

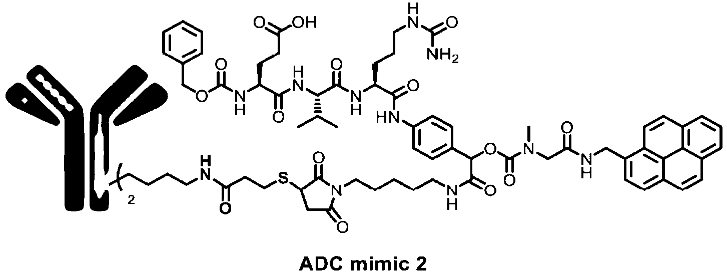

### Example 2-1-3: Synthesis of ADC mimic 3

Similarly, from Linker-payload mimic (11) synthesized in Example 1-3 and the thiol-containing antibody, ADC mimic 3 with the following structure was synthesized. ESI-TOFMS analysis was performed, and a peak was confirmed at 150609 for the reaction product, into which two molecules of Linker-payload mimic (11) were introduced.

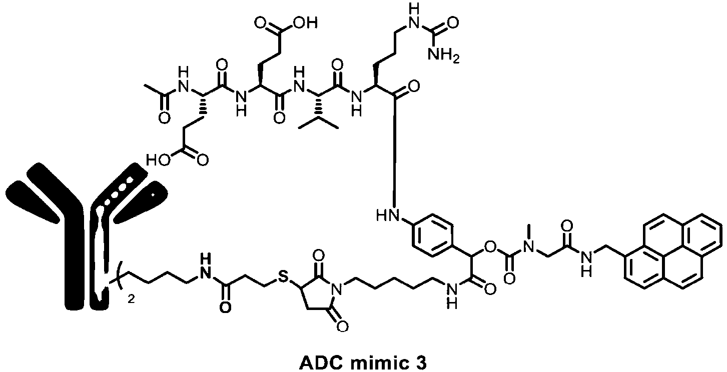

### Example 2-1-4: Synthesis of ADC mimic 4

Similarly, from Linker-payload mimic (16) synthesized in Example 1-4 and the thiol-containing antibody, ADC mimic 4 with the following structure was synthesized. ESI-TOFMS analysis was performed, and a peak was confirmed at 150466 for the reaction product, into which two molecules of Linker-payload mimic (16) were introduced.

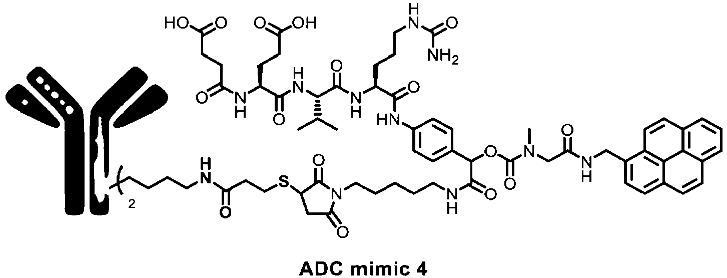

### Comparative Example 2-1-5: Synthesis of ADC mimic 5

Similarly, from Linker-payload mimic (26) synthesized in Comparative Example 1 and the thiol-containing antibody, ADC mimic 5 with the following structure was synthesized. ESI-TOFMS analysis was performed, and a peak was confirmed at 150276 for the reaction product, into which two molecules of Linker-payload mimic (26) were introduced.

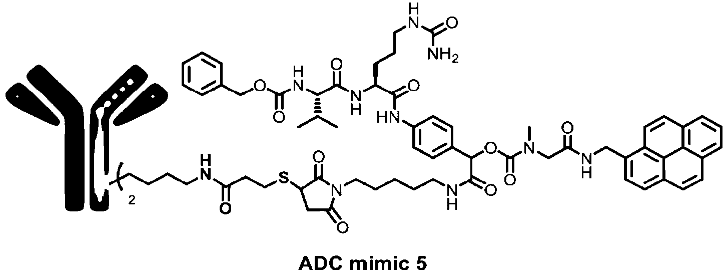

### (2-2) DAR analysis of ADC mimic

ESI-TOFMS analysis of the ADC mimic synthesized in Example 2-1 was performed according to the previous report (WO2019/240287A1), confirming that the DAR was 2.

**Table 1: DAR of ADC mimics**

| | Linker-payload mimic | Example/Comparable Example | DAR |
|---|---|---|---|
| ADC mimic 1 | Linker-payload mimic (1) | Example 2-1-1 | 2 |
| ADC mimic 2 | Linker-payload mimic (6) | Example 2-1-2 | 2 |
| ADC mimic 3 | Linker-payload mimic (11) | Example 2-1-3 | 2 |
| ADC mimic 4 | Linker-payload mimic (16) | Example 2-1-4 | 2 |
| ADC mimic 5 | Linker-payload mimic (26) | Comparable Example 2-1-5 | 2 |

### Example 3: Evaluation of degree of hydrophobicity of ADC and ADC mimic by hydrophobic interaction chromatography (HIC-HPLC)

HIC-HPLC analysis was performed according to the previous report (Anal. Chem., 2019, 91, 20, 12724-12732). The measurement was performed using the following conditions. The degree of hydrophobicity of the ADC can be evaluated based on the retention time of the ADC in the HIC chromatogram.
Measurement system: Chromaster^{®} (Hitachi, Ltd.)
Column: Tosoh Biobuthyl NPR 2.5 µm 4.6 × 35 mm column manufactured by Tosoh Bioscience LLC
Gradient: Linear gradient of eluent A/B
Flow rate: 0.8 mL/min
Eluent A: 1.1 M (NH₄)₂SO₄, 25 mM Na₂HPO₄/NaH₂PO₄ (pH6.0)
Eluent B: 25 mM Na₂HPO₄/NaH₂PO₄ (pH6.0, 25 v/v% isopropanol added)
Detector: UV (280 nm)

**Table 2: Evaluation of degree of hydrophobicity of ADCs and ADC mimics using HIC-HPLC**

| | Linker-payload mimic or Linker-payload | Example/Comparable Example | Retention Time |
|---|---|---|---|
| ADC mimic 1 | Linker-payload mimic (1) | Example 2-1-1 | 11.0 min |
| ADC mimic 2 | Linker-payload mimic (6) | Example 2-1-2 | 12.9 min |
| ADC mimic 3 | Linker-payload mimic (11) | Example 2-1-3 | 10.5 min |
| ADC mimic 4 | Linker-payload mimic (16) | Example 2-1-4 | 11.0 min |
| ADC mimic 5 | Linker-payload mimic (26) | Comparable Example 2-1-5 | 13.6 min |

As a result, it was confirmed that the ADC mimics synthesized in Examples 2-1-1 to 2-1-4 tended to have a short retention time, indicating high degree of hydrophilicity. Therefore, it was confirmed that the ADC mimics synthesized in Examples 2-1-1 to 2-1-4 are preferable ADCs because they are considered to have slow plasma clearance and a long residence time in the body.

### Example 4: Evaluation of aggregation rate of ADC and ADC mimic by size exclusion chromatography (SEC-HPLC)

SEC-HPLC analysis was performed according to the previous report (ChemistrySelect, 2020, 5, 8435-8439). The measurement was performed using the following conditions.
Measurement system: 1260 HPLC system (Agilent Technologies, Inc.)
Column: AdvanceBio SEC 300Å 2.7 µm, 4.6 mm × 150 mm manufactured by Agilent Technologies, Inc.
Flow rate: 0.25 mL/min
Eluent: 100 mM sodium dihydrogen phosphate/disodium hydrogen phosphate, 250 mM aqueous sodium chloride solution (pH 6.8), 10%v/v isopropanol
Detector: UV (280 nm)

**Table 3: Evaluation of aggregation rate of ADCs and ADC mimics using SEC-HPLC**

| | Linker-payload mimic or Linker-payload | Example/Comparable Example | Aggregation Rate |
|---|---|---|---|
| ADC mimic 1 | Linker-payload mimic (1) | Example 2-1-1 | 1.6 % |
| ADC mimic 2 | Linker-payload mimic (6) | Example 2-1-2 | 2.7 % |
| ADC mimic 3 | Linker-payload mimic (11) | Example 2-1-3 | 1.5 % |
| ADC mimic 4 | Linker-payload mimic (16) | Example 2-1-4 | 2.4 % |
| ADC mimic 5 | Linker-payload mimic (26) | Comparable Example 2-1-5 | 2.7 % |

### Example 5: Evaluation of ADC mimic using cathepsin B enzyme

The cleavability of various ADC mimics by cathepsin B was evaluated by analyzing the amounts of fluorescent molecules detached from the ADC mimic as described below.

### (5-1) Cathepsin B cleavability test

The test was performed as follows according to the previous report (Nature Communications 2018, 9, 2512). To 180 µL of MES buffer (10 mM MES, 40 µM DTT, pH 5.0), an ADC mimic was added to a concentration of 1 mg/mL, and then 30 µL was dispensed into each of six Eppendorf tubes. Of the six samples, three were immediately treated with 100 µL of acetonitrile at 0°C, vortexed, and then centrifuged to obtain a precipitate. The resulting supernatant was collected and subjected to HPLC analysis. The remaining three tubes were incubated at 37°C for 6 hours. To each sample, 100 µL of acetonitrile was added, vortexed, and then centrifuged to obtain a precipitate. The resulting supernatant was collected and subjected to HPLC analysis.

### (5-2) Quantitation of detached fluorescent molecules using HPLC analysis

The quantitation was performed by measuring the amount of the fluorescent molecules detached from the ADC mimic using a liquid chromatography/fluorescence detection method. The three samples to which acetonitrile was immediately added at 0°C in Example 7-1 were designated as the 0-hour samples, and the three samples incubated at 37°C for 6 hours as described in Example 7-1 were designated as the 6-hour samples, and the difference in fluorescence intensity between the 6-hour samples and the 0-hour samples was analyzed.

Separately, using Pyrene, the correlation between the area of fluorescence intensity by HPLC and the concentration was calculated. Using that calculation formula, the difference in fluorescence intensity of each of the above ADC mimics was converted to concentration. The ratio of the aforementioned difference in fluorescence intensity, with the concentration at 0 hour as 100%, was calculated as the detachment rate.

**Table 4: Evaluation of cleavability of ADC mimics by cathepsin B**

| | Linker-payload mimic | Example/Comparable Example | Detachment rate of fluorescent molecules at 6h |
|---|---|---|---|
| ADC mimic 1 | Linker-payload mimic (1) | Example 2-1-1 | 200 % |
| ADC mimic 2 | Linker-payload mimic (6) | Example 2-1-2 | 200 % |
| ADC mimic 3 | Linker-payload mimic (11) | Example 2-1-3 | 200 % |
| ADC mimic 4 | Linker-payload mimic (16) | Example 2-1-4 | 79 % |
| ADC mimic 5 | Linker-payload mimic (26) | Comparable Example 2-1-5 | 200 % |

As shown in Table 4, it was found that the synthesized ADC mimics have sufficient cleavability by cathepsin B.

### Example 6: Evaluation of ADC mimic using mouse plasma

### (6-1) Plasma stability test of ADC mimic

To 500 µL of mouse plasma (Charles River Laboratories), the ADC mimic was added to a concentration of 0.1 mg/mL, followed by sterile filtration. This solution was dispensed in 50 µL aliquots into six Eppendorf tubes. Three of the six samples were stored in an incubator set at 37°C for 4 days. The remaining three were similarly stored in a freezer at -80°C for 4 days. To each sample, 100 µL of acetonitrile was added, vortexed, and then centrifuged to obtain a precipitate. The resulting supernatant was collected and subjected to HPLC analysis.

### (6-2) Quantitation of detached fluorescent molecules using HPLC analysis

The quantitation was performed by measuring the amount of the fluorescent molecules detached from the ADC mimic using a liquid chromatography/fluorescence detection method. The three samples stored in the freezer in Example 9-1 were designated as Day = 0 samples, and the three samples stored at 37°C in Example 9-1 were designated as Day = 4 samples, and the difference in fluorescence intensity between Day = 4 and Day = 0 was analyzed.

The detachment rate of the fluorescent molecules was calculated according to Example 5-2. The results were as shown in the table below, in which the detachment rate of fluorescent molecules was evaluated.

**Table 5: Results of plasma stability test using ADCs mimics**

| | Linker-payload mimic | Example/Comparable Example | Detachment rate of payload at Day 4 |
|---|---|---|---|
| ADC mimic 1 | Linker-payload mimic (1) | Example 2-1-1 | 7 % |
| ADC mimic 2 | Linker-payload mimic (6) | Example 2-1-2 | 15 % |
| ADC mimic 3 | Linker-payload mimic (11) | Example 2-1-3 | 5 % |
| ADC mimic 4 | Linker-payload mimic (16) | Example 2-1-4 | 3 % |
| ADC mimic 5 | Linker-payload mimic (26) | Comparable Example 2-1-5 | 52 % |

As a result, compared to the ADC mimic synthesized in Comparative Example 1, the ADC mimics synthesized in Examples 2-1-1 and 2-1-2 showed 3-fold or higher stability, and the ADC mimics synthesized in Examples 2-1-3 and 2-1-4 showed 10-fold or higher stability.

### Example 7: Synthesis of ADC mimic

### Example 7-1: Synthesis of ADC mimic 6

To a solution of 3 mg/mL of an anti-HER2 IgG antibody trastuzumab (Chugai Pharmaceutical Co., Ltd.) in PBSE buffer (50 mM phosphate buffered saline (PBS), 10 mM EDTA, pH = 7.4), 8 equivalents of a solution (2 mM) of a reducing agent (TCEP) in PBSE relative to the antibody were added, and the mixture was incubated at 37°C for 1 hour. To the resulting reaction mixture, dimethylacetamide (DMA) (6%v/v) and 10 mM solutions of various Linker-payload mimics (1) in DMA (20 equivalents relative to the antibody) were successively added, and the mixture was gently stirred at 20°C for 1 hour. An excess amount of N-acetylcysteine was added to the reaction mixture, which was stirred at 25°C for 20 minutes. The final mixture was purified using a NAP-5 desalting column (GE Healthcare), eluted with formulation buffer (20 mM histidine, with 5 wt% trehalose added, pH 5.2), to obtain ADC mimic 6.

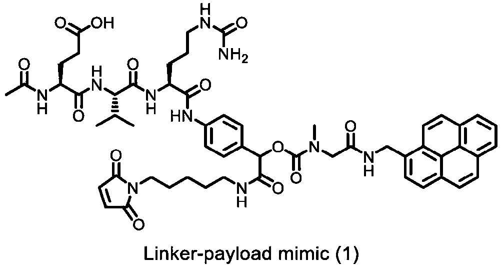

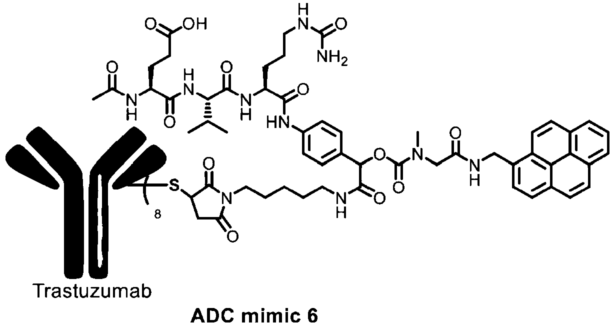

### Example 7-2: Synthesis of ADC mimic 7

Following Example 7-1, ADC mimic 7 was obtained from Linker-payload mimic (11).

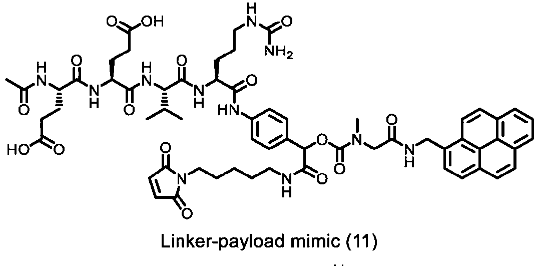

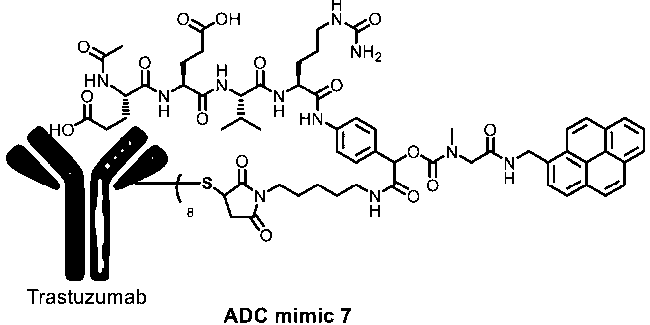

### Example 7-3: Synthesis of ADC mimic 8

Following Example 7-1, ADC mimic 8 was obtained from Linker-payload mimic (21).

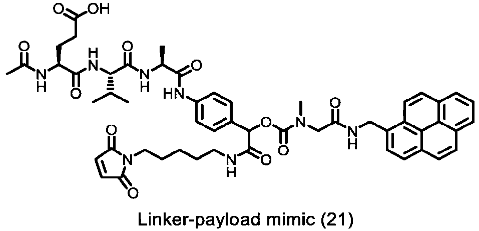

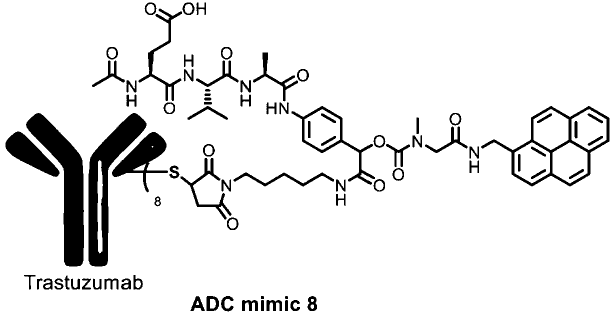

### Example 7-4: Synthesis of ADC mimic 9

Following Example 7-1, ADC mimic 9 was obtained by reacting Linker-payload mimic (1) with the anti-CD20 IgG antibody rituximab (Roche).

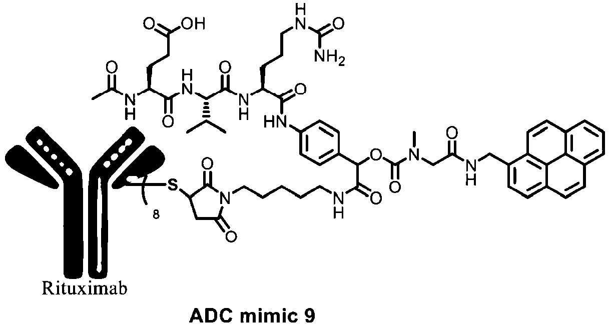

### Example 7-5: Synthesis of ADC mimic 10

Following Example 7-1, ADC mimic 10 was obtained by reacting Linker-payload mimic (11) with the anti-CD20 IgG antibody rituximab (Roche).

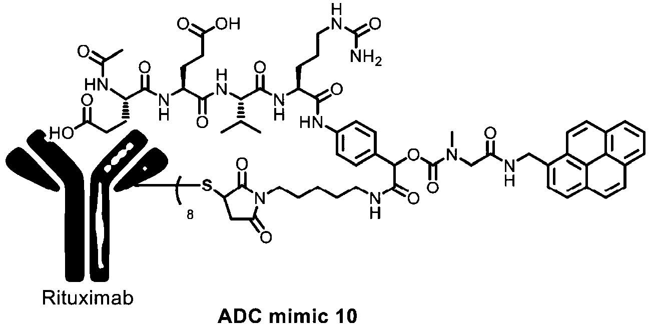

### Example 7-6: Synthesis of ADC mimic 11

Following Example 7-1, ADC mimic 11 was obtained by reacting Linker-payload mimic (21) with the anti-CD20 IgG antibody rituximab (Roche).

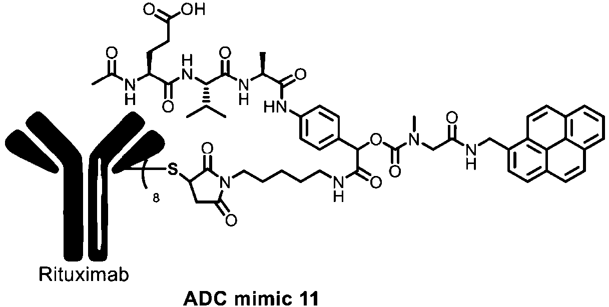

### Example 7-7: Synthesis of ADC mimic 12

Following Example 7-1, ADC mimic 12 was obtained by reacting Linker-payload mimic (1) with the anti-TNF-α IgG antibody infliximab (Centocor).

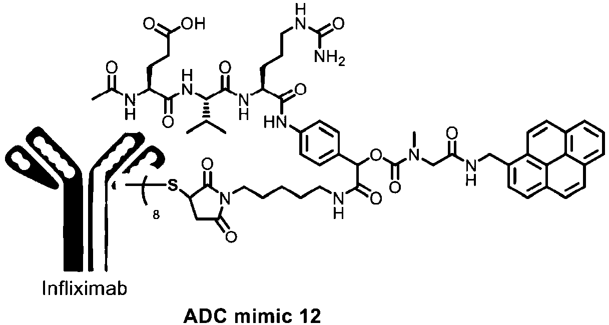

### Example 7-8: Synthesis of ADC mimic 13

Following Example 7-1, ADC mimic 13 was obtained by reacting Linker-payload mimic (11) with the anti-TNF-α IgG antibody infliximab (Centocor).

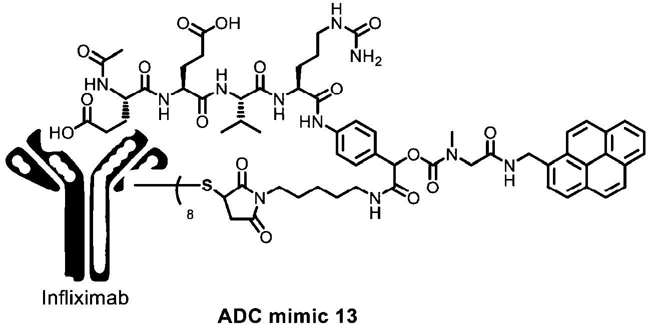

### Example 7-9: Synthesis of ADC mimic 14

Following Example 7-1, ADC mimic 14 was obtained by reacting Linker-payload mimic (21) with the anti-TNF-α IgG antibody infliximab (Centocor).

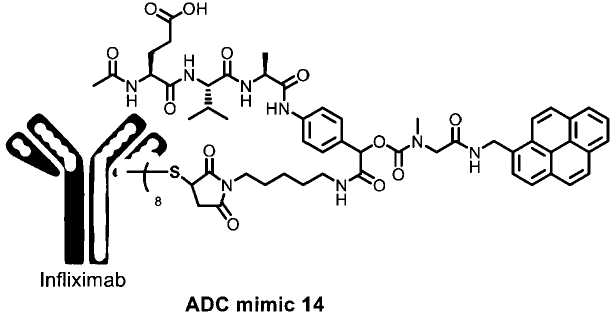

### Comparative Example 2: Synthesis of ADC mimic

### Comparative Example 2-1: Synthesis of Linker-payload mimic (30)

Linker-payload mimic (30) was synthesized as follows. Linker-payload mimic (30) was synthesized in one step from commercially available MC-VC-PAB-PNP (CAS No: 159857-81-5) and known Sarcosine-Pyrene (WO2018218004A1).

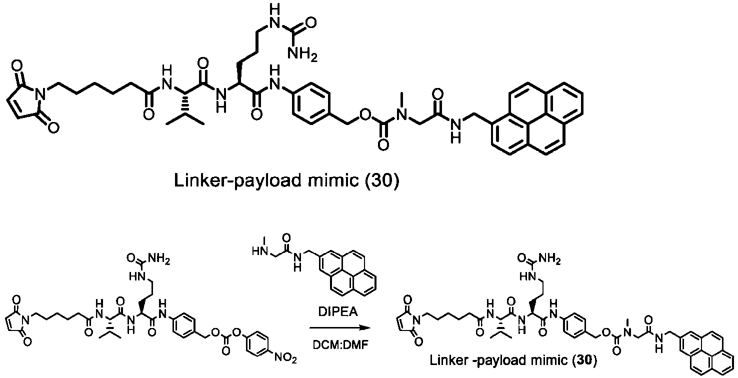

Commercially available MC-VC-PAB-PNP (CAS No: 159857-81-5) (15.5 mg, 0.021 mmol) was dissolved in dichloromethane (1 mL), and a solution of N,N-diisopropylethylamine (0.025 mL, 0.142 mmol) and known Sarcosine-Pyrene (WO2018218004A1) (7.6 mg, 0.025 mmol) in dimethylformamide (0.5 mL) was added, followed by stirring for 17 hours. After purification by preparative reversed-phase chromatography, fractions containing the product were collected, and acetonitrile was removed by concentration under reduced pressure, followed by lyophilization to obtain Linker-payload mimic (30) (7.3 mg, 0.008 mmol).

¹H NMR (400 MHz, DMSO-d6) δ9.98 (s, 1H), 8.34 (d, J = 9.2 Hz, 2H), 8.32-8.23 (m, 4H), 8.16 (s, 2H), 8.10-8.00 (m, 4H), 7.80 (d, J = 8.8 Hz, 1H), 7.59 (d, J = 8.4 Hz, 2H), 7.31 (d, J = 8.0 Hz, 2H), 6.99 (s, 2H), 5.96 (m, 1H), 5.40 (s, 2H), 5.01 (s, 2H), 4.95 (d, J = 6.0 Hz, 2H), 4.38 (m, 1H), 4.19 (m, 1H), 3.03-2.92 (m, 3H), 2.67 (m, 1H), 2.33 (m, 1H), 2.20-2.07 (m, 2H), 1.97 (m, 1H), 1.67 (m, 1H), 1.59 (m, 1H), 1.51-1.45 (m, 6H), 1.26-1.15 (m, 3H), 0.83 (dd, J = 12.8, 6.8 Hz, 6H).
MS (ESI) m/z: 901.45 [M+H]⁺

### Comparative Example 2-2: Synthesis of ADC mimics 15 to 17

Following Example 7-1, Linker-payload mimic (30) was reacted to obtain ADC mimic 15 from trastuzumab, ADC mimic 16 from rituximab, and ADC mimic 17 from infliximab.

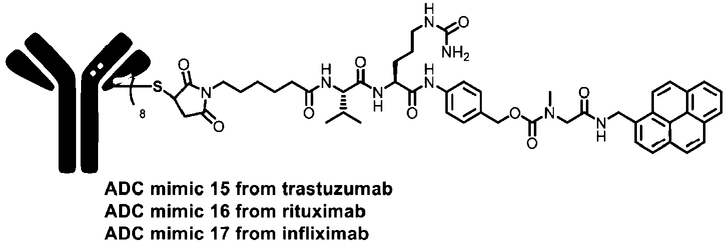

### Comparative Example 2-3: Synthesis of Linker-payload mimic (31)

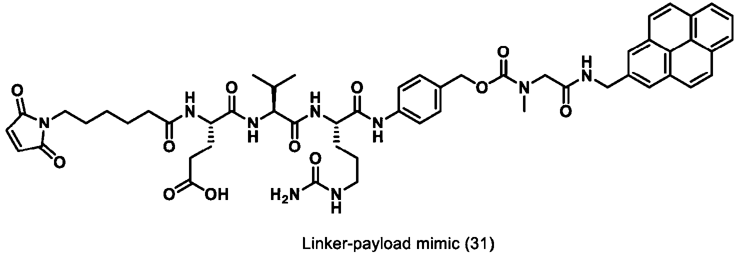

### Comparative Example 2-3-1: Synthesis of Pyrene (32)

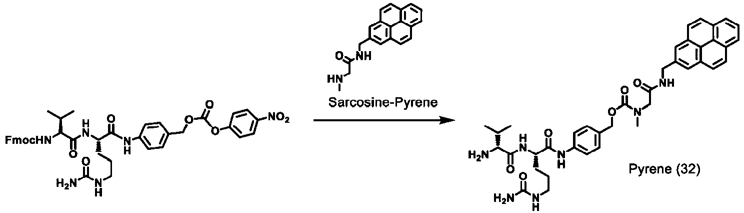

Fmoc-Val-Cit-PAB-PNP (CAS No: 863971-53-2, 121.2 mg, 0.15 mmol) was dissolved in N,N-dimethylformamide (5 mL), to which known (described in WO2018218004A1) sarcosine-Pyrene (59.2 mg, 0.196 mmol), N,N-diisopropylethylamine (39 µL, 0.227 mmol), and 4-dimethylaminopyridine (3.7 mg, 0.03 mmol) were added, and the mixture was stirred at room temperature for 2 hours. Subsequently diethylamine (2 mL, 18.95 mmol) was added and the mixture was stirred at room temperature for 1.5 hours. After concentration under reduced pressure, the reaction mixture was purified by preparative reversed-phase chromatography. Fractions containing the product were collected, and acetonitrile was removed by concentration under reduced pressure, followed by lyophilization to obtain the above-described Pyrene (32) (73.7 mg, 0.104 mmol).

¹H NMR (400 MHz, DMSO-d6) δ10.21 (s, 1H), 8.68 (s, 1H), 8.43-7.93 (m, 12H), 7.60 (t, J = 7.1 Hz, 2H), 7.31 (m, 2H), 6.04 (s, 1H), 5.48 (s, 2H), 5.02 (d, J = 16.1 Hz, 4H), 4.54 (s, 1H), 3.96 (s, 2H), 3.66 (s, 2H), 2.92 (d, J = 6.1 Hz, 3H), 2.08 (q, J = 6.6 Hz, 1H), 1.80-1.56 (m, 2H), 1.46 (s, 2H), 1.21-1.13 (m, 1H), 0.94 (dt, J = 6.8, 3.0 Hz, 6H).
MS (ESI) m/z: 708.80 [M+H]⁺

### Comparative Example 2-3-2: Synthesis of Pyrene (33)

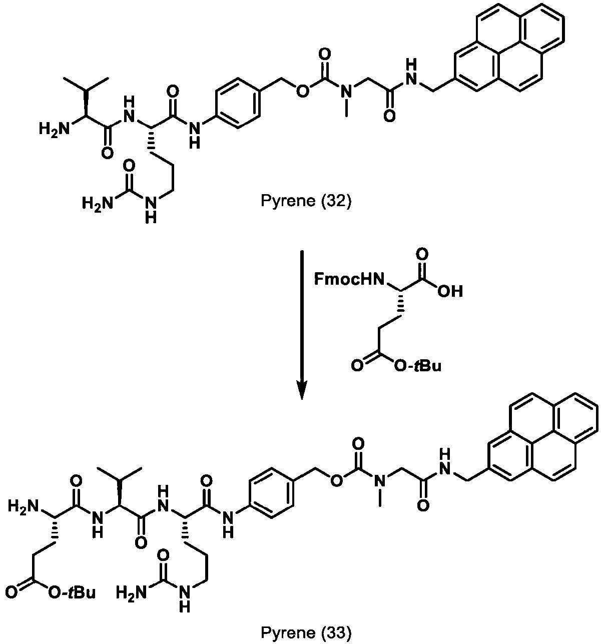

Fmoc-Glu(OtBu)-OH•H₂O (11.1 mg, 0.025 mmol) was dissolved in dimethylformamide (1 mL), to which Pyrene (32) (17.3 mg, 0.024 mmol), 1-hydroxy-7-azabenzotriazole (5.1 mg, 0.037 mmol), 1-(3-dimethylaminopropyl)-3-ethylcarbodiimide hydrochloride (7.3 mg, 0.038 mmol), and triethylamine (7.1 µL, 0.51 mmol) were added, and the mixture was stirred at room temperature for 2.5 hours. Subsequently, diethylamine (0.2 mL, 1.91 mmol) was added and the mixture was stirred at room temperature for 1.5 hours. After concentration under reduced pressure, the reaction mixture was purified by preparative reversed-phase chromatography. Fractions containing the product were collected, and acetonitrile was removed by concentration under reduced pressure, followed by lyophilization to obtain Pyrene (33) (15.6 mg, 0.017 mmol).

¹H NMR (400 MHz, Chloroform-d) δ10.87 (s, 1H), 8.68 (m, 1H), 8.41-7.97 (m, 13H), 7.60-7.58 (m, 2H), 7.30 (m, 2H), 6.00 (t, J = 6.2 Hz, 1H), 5.45 (s, 2H), 5.05-4.99 (m, 4H), 4.46 (m, 1H), 4.26 (m, 1H), 3.96 (s, 2H), 3.88 (m, 1H), 3.07 (m, 1H), 2.96 (m, 1H), 2.93 (d, J = 5.6 Hz, 3H), 2.68 (t, J = 1.8 Hz, 1H), 2.34-2.30 (m, 3H), 2.03 (m, 1H), 1.93-1.90 (m, 2H), 1.30 (s, 9H), 1.15 (s, 1H), 0.92-0.86 (m, 6H).
MS (ESI) m/z: 893.45 [M+H]⁺

### Comparative Example 2-3-3: Synthesis of Pyrene (34)

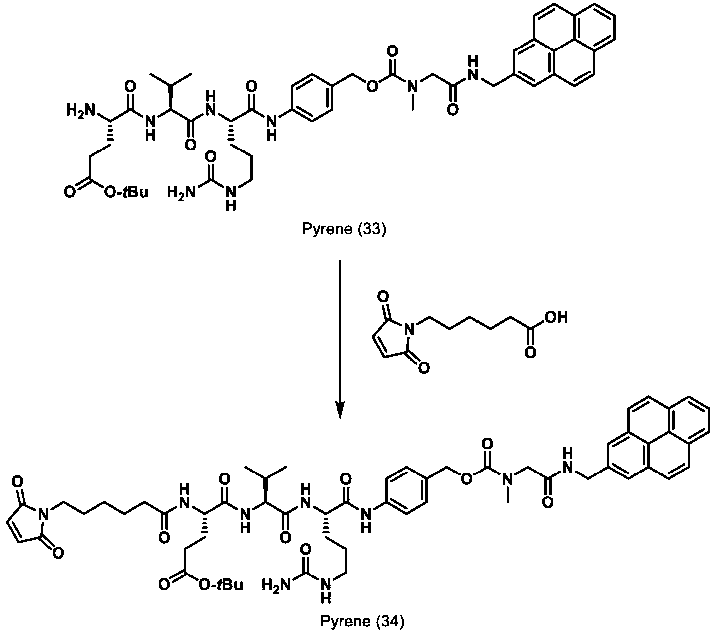

Pyrene (33) (15.6 mg, 0.017 mmol) was dissolved in dimethylformamide (1.5 mL), and 6-maleimide hexanoic acid (3.7 mg, 0.018 mmol), 1-hydroxy-7-azabenzotriazole (3.5 mg, 0.025 mmol), 1-(3-dimethylaminopropyl)-3-ethylcarbodiimide hydrochloride (4.8 mg, 0.025 mmol), and triethylamine (4.8 µL, 0.34 mmol) were added, and the mixture was stirred at room temperature for 3 hours. Subsequently, 6-maleimide hexanoic acid (1.8 mg, 0.009 mmol), 1-(3-dimethylaminopropyl)-3-ethylcarbodiimide hydrochloride (2.3 mg, 0.012 mmol), and triethylamine (2.4 µL, 0.17 mmol) were added, and the mixture was stirred at room temperature for 1.5 hours. Subsequently, 4-dimethylaminopyridine (0.5 mg, 0.004 mmol) was added, and the mixture was further stirred for 2.5 hours. After concentration under reduced pressure, the reaction mixture was purified by column chromatography (dichloromethane: methanol = 9:1). Fractions containing the product were collected and concentrated under reduced pressure to obtain the above-described Pyrene (34) (8.0 mg, 0.007 mmol).

¹H NMR (400 MHz, DMSO-d6) δ10.02 (s, 1H), 8.65 (m, 1H), 8.40-7.96 (m, 9H), 7.69-7.53 (m, 3H), 7.28 (m, 2H), 7.00-6.98 (m, 2H), 5.97 (m, 1H), 5.41 (s, 2H), 5.04-4.98 (m, 4H), 4.56 (t, 5.4Hz, 1H), 4.41-4.27 (m, 2H), 4.21-4.15 (m, 2H), 3.95 (s, 2H), 2.91 (d, J = 5.6 Hz, 3H), 2.22-2.15 (m, 2H), 2.10-2.07 (m, 2H), 1.69 (m, 1H), 1.59 (m, 1H), 1.50-1.44 (m, 6H), 1.46 (d, J = 2.0 Hz, 9H), 1.35-1.15 (m, 4H), 0.82 (dd, J = 14.8, 6.8 Hz, 6H).
MS (ESI) m/z: 1086.60 [M+H]⁺

### Comparative Example 2-3-4: Synthesis of Linker-payload mimic (31)

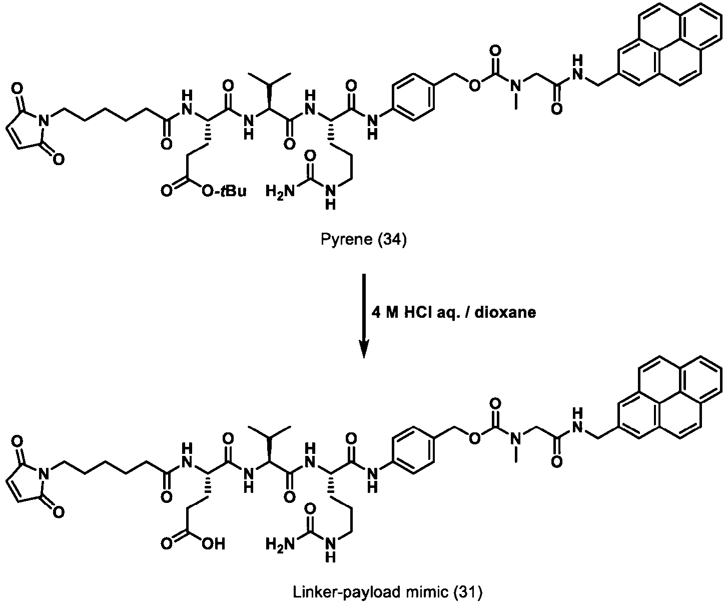

Pyrene (4) (9.7 mg, 0.0089 mmol) was dissolved in 1,4-dioxane (1 mL) and a solution of hydrogen chloride in 1,4-dioxane (1 mL), and the mixture was stirred in an ice-water bath for 2 hours. Dimethylformamide (1 mL) was added, and the mixture was allowed to warm to room temperature and then concentrated under reduced pressure. Subsequently, the reaction mixture was purified by preparative reversed-phase chromatography. Fractions containing the product were collected, and acetonitrile was removed by concentration under reduced pressure, followed by lyophilization to obtain Linker-payload mimic (31) (2.0 mg, 0.002 mmol).

¹H NMR (400 MHz, DMSO-d6) δ10.03 (s, 1H), 8.67 (m, 1H), 8.40-7.96 (m, 9H), 7.69-7.57 (m, 3H), 7.28 (m, 2H), 6.98 (s, 2H), 5.98 (m, 1H), 5.87 (m, 1H), 5.42 (s, 2H), 5.05-4.97 (m, 4H), 4.4 (m, 1H), 4, 29 (m, 1H), 4.19 (m, 1H), 3.95 (s, 2H), 2.90 (d, J = 5.2 Hz, 3H), 2.22-2.17 (m, 2H), 2.11-2.09 (m, 2H), 1.71-1.59 (m, 3H), 1.49-1.44 (m, 6H), 1.30-1.15 (m, 4H), 0.82 (dd, J = 15.0, 6.6 Hz, 6H).
MS (ESI) m/z: 1030.50 [M+H]⁺

### Comparative Examples 2-4: Synthesis of ADC mimics 18 to 20

Following Example 7-1, Linker-payload mimic (31) was reacted to obtain ADC mimic 18 from trastuzumab, ADC mimic 19 from rituximab, and ADC mimic 20 from infliximab.

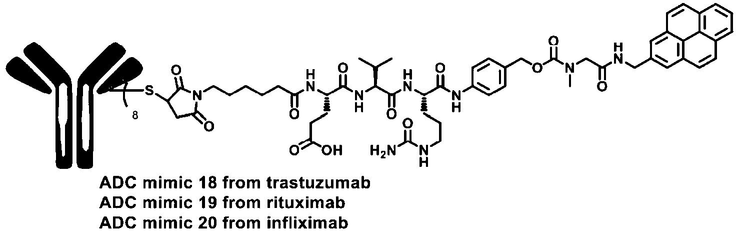

### Example 8: DAR analysis of ADC mimic

For DAR analysis of ADC mimics synthesized in Example 7 and Comparative Example 2, HIC-HPLC analysis was performed according to the previous report (Anal. Chem., 2019, 91, 20, 12724-12732). The measurement was performed using the following conditions.

### Measurement system: Chromaster^{®} (Hitachi, Ltd.)

Column: Tosoh Biobuthyl NPR 2.5 µm 4.6 × 35 mm column manufactured by Tosoh Bioscience LLC
Gradient: Linear gradient of eluent A/B
Flow rate: 0.8 mL/min
Eluent A: 1.1 M (NH₄)₂SO₄, 25 mM Na₂HPO₄/NaH₂PO₄ (pH6.0)
Eluent B: 25 mM Na₂HPO₄/NaH₂PO₄ (pH6.0, 25 v/v% isopropanol added)
Detector: UV (280 nm)

**Table 6: DAR of ADC mimics**

| | Linker-payload mimic | Example/Comparable Example | DAR |
|---|---|---|---|
| ADC mimic 6 | Linker-payload mimic (1) | Example 7-1 | 8 |
| ADC mimic 7 | Linker-payload mimic (11) | Example 7-2 | 8 |
| ADC mimic 8 | Linker-payload mimic (21) | Example 7-3 | 8 |
| ADC mimic 9 | Linker-payload mimic (1) | Example 7-4 | 8 |
| ADC mimic 10 | Linker-payload mimic (11) | Example 7-5 | 8 |
| ADC mimic 11 | Linker-payload mimic (21) | Example 7-6 | 8 |
| ADC mimic 12 | Linker-payload mimic (1) | Example 7-7 | 8 |
| ADC mimic 13 | Linker-payload mimic (11) | Example 7-8 | 8 |
| ADC mimic 14 | Linker-payload mimic (21) | Example 7-9 | 8 |
| ADC mimic 15 | Linker-payload mimic (30) | Comparable Example 2-2 | 8 |
| ADC mimic 16 | Linker-payload mimic (30) | Comparable Example 2-2 | 8 |
| ADC mimic 17 | Linker-payload mimic (30) | Comparable Example 2-2 | 8 |
| ADC mimic 18 | Linker-payload mimic (31) | Comparable Example 2-4 | 8 |
| ADC mimic 19 | Linker-payload mimic (31) | Comparable Example 2-4 | 8 |
| ADC mimic 20 | Linker-payload mimic (31) | Comparable Example 2-4 | 8 |

### Example 9: Evaluation of degree of hydrophobicity of ADC mimic by hydrophobic interaction chromatography (HIC-HPLC)

Using the conditions of Example 8, HIC-HPLC analysis was performed. The measurement was performed using the following conditions. The degree of hydrophobicity of the ADC mimic can be evaluated based on the retention time of the ADC in the HIC chromatogram.

**Table 7: Evaluation of degree of hydrophobicity of trastuzumab-based ADC mimics using HIC-HPLC**

| | Linker-payload mimic | Example/Comparable Example | Retention Time |
|---|---|---|---|
| ADC mimic 6 | Linker-payload mimic (1) | Example 7-1 | 11.6 min |
| ADC mimic 7 | Linker-payload mimic (11) | Example 7-2 | 10.9 min |
| ADC mimic 8 | Linker-payload mimic (21) | Example 7-3 | 11.9 min |
| ADC mimic 15 | Linker-payload mimic (30) | Comparable Example 2-1 | 16.5 min |
| ADC mimic 18 | Linker-payload mimic (31) | Comparable Example 2-4 | 16.2 min |

Compared to the linear ADCs, ADC mimics 15 and 18, the exo-type ADCs, ADC mimics 6, 7, and 8, have shorter retention times in the HIC chromatogram, indicating that ADC mimics 6, 7, and 8 are more hydrophilic ADC mimics.

**Table 8: Evaluation of degree of hydrophobicity of rituximab-based ADC mimics using HIC-HPLC**

| | Linker-payload mimic | Example/Comparable Example | Retention Time |
|---|---|---|---|
| ADC mimic 6 | Linker-payload mimic (1) | Example 7-4 | 11.6 min |
| ADC mimic 7 | Linker-payload mimic (11) | Example 7-5 | 11.3 min |
| ADC mimic 8 | Linker-payload mimic (21) | Example 7-6 | 11.6 min |
| ADC mimic 15 | Linker-payload mimic (30) | Comparable Example 2-1 | 16.4 min |
| ADC mimic 18 | Linker-payload mimic (31) | Comparable Example 2-4 | 16.0 min |

Compared to the linear ADCs, ADC mimics 16 and 19, the exo-type ADCs, ADC mimics 9, 10, and 11, have shorter retention times in the HIC chromatogram, indicating that ADC mimics 9, 10, and 11 are more hydrophilic ADC mimics.

**Table 9: Evaluation of degree of hydrophobicity of infliximab-based ADC mimics using HIC-HPLC**

| | Linker-payload mimic | Example/Comparable Example | Retention Time |
|---|---|---|---|
| ADC mimic 12 | Linker-payload mimic (1) | Example 7-7 | 11.9 min |
| ADC mimic 13 | Linker-payload mimic (11) | Example 7-8 | 11.3 min |
| ADC mimic 14 | Linker-payload mimic (21) | Example 7-9 | 11.8 min |
| ADC mimic 17 | Linker-payload mimic (30) | Comparable Example 2-1 | 16.4 min |
| ADC mimic 20 | Linker-payload mimic (31) | Comparable Example 2-4 | 16.2 min |

Compared to the linear ADCs, ADC mimics 17 and 20, the exo-type ADCs, ADC mimics 12, 13, and 14, have shorter retention times in the HIC chromatogram, indicating that ADC mimics 12, 13, and 14 are more hydrophilic ADC mimics.

As a result, it was confirmed that the ADC mimics synthesized in Examples 7-1, 7-2, 7-3, 7-4, 7-5, 7-6, 7-7, 7-8, and 7-9 tended to have a short retention time, indicating a high degree of hydrophilicity. Therefore, it was confirmed that the ADC mimics synthesized in Examples 7-1, 7-2, 7-3, 7-4, 7-5, 7-6, 7-7, 7-8, and 7-9 are preferable ADCs because they are considered to have slow plasma clearance and a long residence time in the body.

### Example 10: Evaluation of aggregation rate of ADC and ADC mimic by size exclusion chromatography (SEC-HPLC)

SEC-HPLC analysis was performed according to the previous report (ChemistrySelect, 2020, 5, 8435-8439). The measurement was performed using the following conditions.
Measurement system: 1260 HPLC system (Agilent Technologies, Inc.)
Column: AdvanceBio SEC 300Å 2.7 µm, 4.6 mm × 150 mm manufactured by Agilent Technologies, Inc.
Flow rate: 0.25 mL/min
Eluent: 100 mM sodium dihydrogen phosphate/disodium hydrogen phosphate, 250 mM aqueous sodium chloride solution (pH 6.8), 10%v/v isopropanol
Detector: UV (280 nm)

**Table 10: Evaluation of aggregation rate of trastuzumab-based ADC mimics using SEC-HPLC**

| | Linker-payload mimic | Example/Comparable Example | Aggregation Rate |
|---|---|---|---|
| ADC mimic 6 | Linker-payload mimic (1) | Example 7-1 | 0.7 % |
| ADC mimic 7 | Linker-payload mimic (11) | Example 7-2 | 0.8 % |
| ADC mimic 8 | Linker-payload mimic (21) | Example 7-3 | 0.4 % |
| ADC mimic 15 | Linker-payload mimic (30) | Comparable Example 2-1 | 100 % |
| ADC mimic 18 | Linker-payload mimic (31) | Comparable Example 2-4 | 20 % |

**Table 11: Evaluation of aggregation rate of rituximab-based ADC mimics using SEC-HPLC**

| | Linker-payload mimic | Example/Comparable Example | Aggregation Rate |
|---|---|---|---|
| ADC mimic 9 | Linker-payload mimic (1) | Example 7-4 | 0.6 % |
| ADC mimic 10 | Linker-payload mimic (11) | Example 7-5 | 1.1 % |
| ADC mimic 11 | Linker-payload mimic (21) | Example 7-6 | 1.4 % |
| ADC mimic 16 | Linker-payload mimic (30) | Comparable Example 2-1 | 100 % |
| ADC mimic 19 | Linker-payload mimic (31) | Comparable Example 2-4 | 12 % |

**Table 12: Evaluation of aggregation rate of infliximab-based ADC mimics using SEC-HPLC**

| | Linker-payload mimic | Example/Comparable Example | Aggregation Rate |
|---|---|---|---|
| ADC mimic 12 | Linker-payload mimic (1) | Example 7-7 | 2.1 % |
| ADC mimic 13 | Linker-payload mimic (11) | Example 7-8 | 1.9 % |
| ADC mimic 14 | Linker-payload mimic (21) | Example 7-9 | 0.3 % |
| ADC mimic 17 | Linker-payload mimic (30) | Comparable Example 2-1 | 100 % |
| ADC mimic 20 | Linker-payload mimic (31) | Comparable Example 2-4 | 16 % |

As a result, it was confirmed that the ADC mimics synthesized in Examples 7-1, 7-2, 7-3, 7-4, 7-5, 7-6, 7-7, 7-8, and 7-9 tended to have a low aggregation rate, indicating higher stability. Therefore, it was confirmed that the ADC mimics synthesized in Examples 7-1, 7-2, 7-3, 7-4, 7-5, 7-6, 7-7, 7-8, and 7-9 are preferable ADCs.

### Example 11: Evaluation of ADC mimic using cathepsin B enzyme

The cleavability of various ADC mimics by cathepsin B was evaluated by analyzing the amounts of fluorescent molecules detached from the ADC mimic as described below.

### Example 11-1: Cathepsin B cleavability test

The test was performed as follows according to the previous report (Nature Communications 2018, 9, 2512). To 180 µL of MES buffer (10 mM MES, 40 µM DTT, pH 5.0), ADC mimic 7 was added to a concentration of 1 mg/mL, and then 30 µL was dispensed into each of six Eppendorf tubes. Of the six samples, three were immediately treated with 100 µL of acetonitrile at 0°C, vortexed, and then centrifuged to obtain a precipitate. The resulting supernatant was collected and subjected to HPLC analysis. The remaining three tubes were incubated at 37°C for 6 hours. To each sample, 100 µL of acetonitrile was added, vortexed, and then centrifuged to obtain a precipitate. The resulting supernatant was collected and subjected to HPLC analysis.

### Example 11-2: Quantitation of detached fluorescent molecules using HPLC analysis

The quantitation was performed by measuring the amount of the fluorescent molecules detached from ADC mimic 7 using a liquid chromatography/fluorescence detection method. The three samples to which acetonitrile was immediately added at 0°C in Example 11-1 were designated as the 0-hour samples, and the three samples incubated at 37°C for 6 hours as described in Example 11-1 were designated as the 6-hour samples, and the difference in fluorescence intensity between the 6-hour samples and the 0-hour samples was analyzed.

Separately, using Pyrene, the correlation between the area of fluorescence intensity by HPLC and the concentration was calculated. Using that calculation formula, the differences in fluorescence intensity of the samples of above ADC mimic were converted to concentration. The ratio of the aforementioned difference in fluorescence intensity, normalized to 100% at 0 hours, was calculated as the detachment rate. The detachment rate of fluorescent molecules from ADC mimic 7 at 6 hours was 400%, indicating that ADC mimic 7 has sufficient cleavability by cathepsin B.

### Example 12: Synthesis of Linker-payload

### (12-1) Synthesis of Linker-payload (35)

Linker-payload (35) was synthesized as follows.

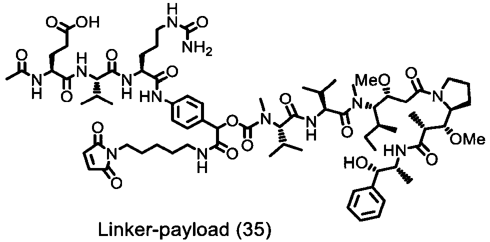

### (12-1-1) Synthesis of Carbonate (36)

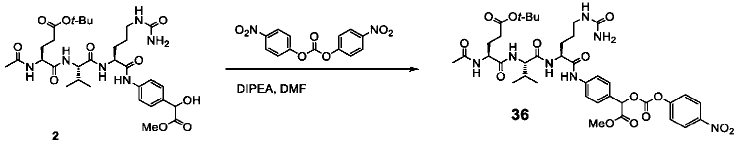

Alcohol (2) (105 mg, 0.158 mmol) obtained in (1-1-1) was dissolved in N,N-dimethylformamide (2 mL) and stirred under ice cooling for 5 minutes, then bis(4-nitrophenyl) carbonate (100 mg, 0.329 mmol) and N,N-diisopropylethylamine (83 µL, 0.48 mmol) were added, and the mixture was stirred at room temperature for 19.5 hours under a nitrogen atmosphere. After N,N-dimethylformamide was removed with an evaporator, the resulting crude product was dissolved by adding ethyl acetate (3 mL), followed by addition of diethyl ether (3 mL). After removing the residue from the resulting solution by filtration, the organic solvent was removed by a vacuum pump to obtain Carbonate (36) (102 mg, 0.123 mmol).
MS (ESI) m/z: 830.1 [M+H]⁺, 852.1 [M+Na]⁺

### (12-1-2) Synthesis of Compound (37)

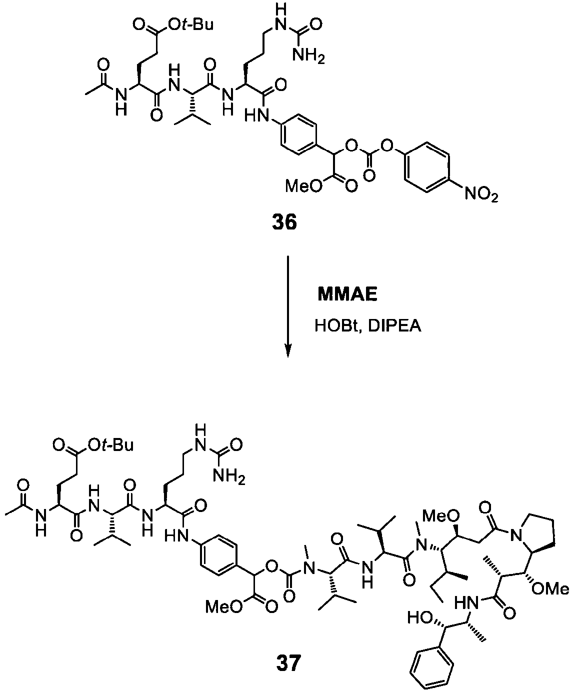

Compound (36) (69 mg, 0.083 mmol) obtained in (12-1-1) was dissolved in N,N-dimethylformamide (3.5 mL), and 1-hydroxybenzotriazole (16 mg, 0.10 mmol) and commercially available monomethylauristatin E (MMAE, 61 mg, 0.085 mmol) were added at room temperature. Subsequently, diisopropylethylamine (29 µL, 0.17 mmol) was added, and the mixture was stirred at room temperature for 22.5 hours under a nitrogen atmosphere. After the organic solvent was removed with an evaporator, a solution of acetonitrile: water = 1:1 was added, and the mixture was purified by preparative reversed-phase chromatography. Fractions containing the product were collected, and acetonitrile was removed by concentration under reduced pressure, followed by lyophilization to obtain Compound 37 (79 mg, 0.056 mmol).
MS (ESI) m/z: 1408.9 [M+H]⁺

### (12-1-3) Synthesis of Compound (38)

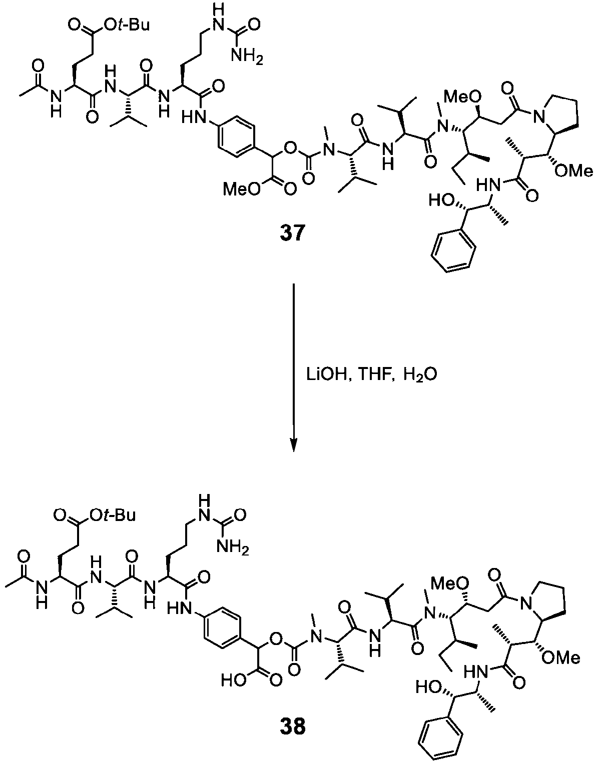

Compound (37) obtained in (12-1-2) (97 mg, 0.069 mmol) was dissolved in tetrahydrofuran (7 mL) and water (2 mL), and lithium hydroxide (1.0 M, 1.4 mL, 1.4 mmol) was added under ice cooling, and directly the mixture was stirred for 1 hour. After adjusting the pH to 6 by adding hydrochloric acid to the reaction solution, acetonitrile: water = 1:1 was added, and the mixture was purified by preparative reversed-phase chromatography. Fractions containing the product were collected, and acetonitrile was removed by concentration under reduced pressure, followed by lyophilization to obtain Compound 38 (70 mg, 0.050 mmol).
MS (ESI) m/z: 1394.7 [M+H]⁺

### (12-1-4) Synthesis of Compound (39)

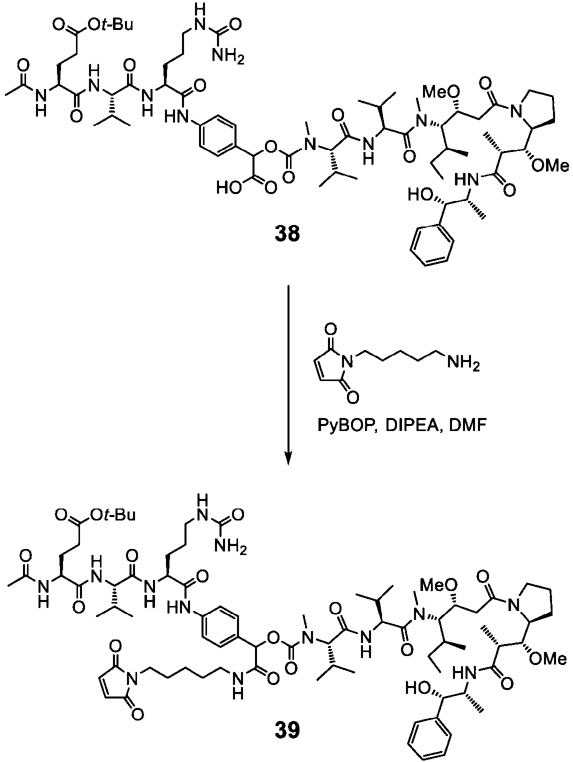

Compound (38) obtained in (12-1-3) (34 mg, 0.024 mmol) was dissolved in N,N-dimethylformamide (3 mL) and cooled on ice, then N,N-diisopropylethylamine (25 µL, 0.14 mmol) and 1H-benzotriazol-1-yloxytripyrrolidinophosphonium hexafluorophosphate (20 mg, 0.038 mmol) were added. Next, N-(5-aminopentyl)maleimide hydrochloride (8.7 mg, 0.040 mmol) was added, and the mixture was returned to room temperature and stirred for 20 hours. After the reaction was completed, the reaction mixture was purified by preparative reversed-phase chromatography. Fractions containing the product were collected, and acetonitrile was removed by concentration under reduced pressure, followed by lyophilization to obtain Compound (39) (29 mg, 0.019 mmol).
MS (ESI) m/z: 1558.9 [M+H]⁺

### (12-1-5) Synthesis of Linker-payload (35)

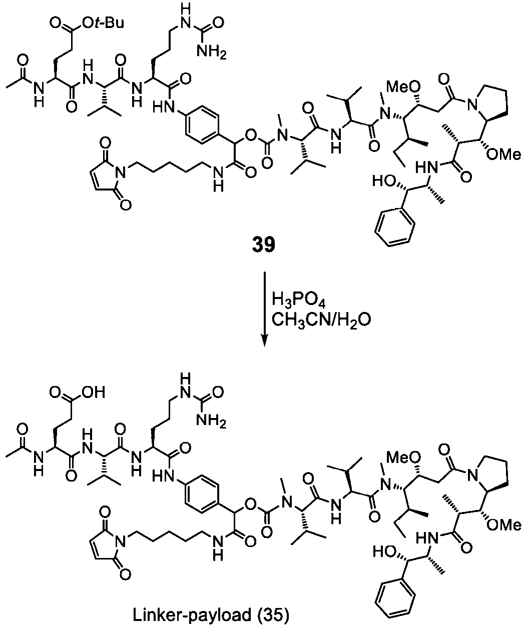

To Compound (39) (29 mg, 0.019 mmol) obtained in (12-1-4) were sequentially added acetonitrile (500 µL) and 85 wt% aqueous phosphoric acid solution (0.50 mL, 7.3 mmol), and the mixture was stirred at room temperature for 3 hours. After the reaction was completed, water (2 mL) was added, and the reaction solution was purified by preparative reversed-phase chromatography, and fractions containing the product were collected, and acetonitrile was removed by concentration under reduced pressure, followed by lyophilization to obtain Linker-payload (35) (18 mg, 0.012 mmol).
MS (ESI) m/z: 1502.9 [M+H]⁺

### (12-2) Synthesis of Linker-payload (40)

Linker-payload (40) was synthesized as follows.

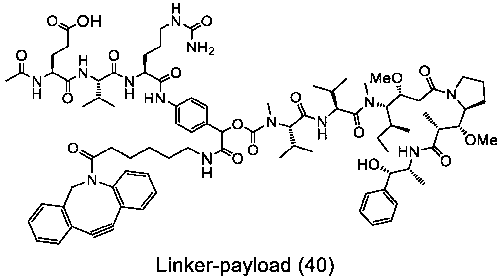

### (12-2-1) Synthesis of Compound (41)

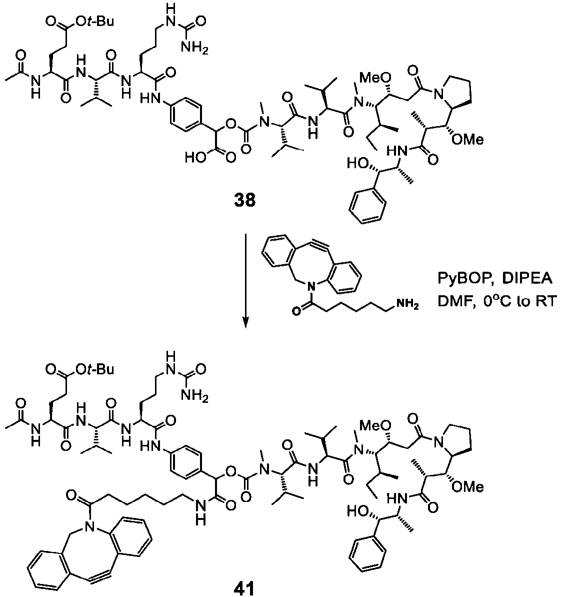

Compound (38) (10 mg, 0.0072 mmol) obtained in (12-1-3) was dissolved in N,N-dimethylformamide (1 mL) and cooled on ice, then N,N-diisopropylethylamine (10 µL, 0.057 mmol) and 1H-benzotriazol-1-yloxytripyrrolidinophosphonium hexafluorophosphate (7.0 mg, 0.013 mmol) were added. Then, a solution of DBCO-hexylamine (4.7 mg, 0.015 mmol) in N,N-dimethylformamide (0.5 mL) was added, and the mixture was returned to room temperature and stirred for 20 hours. After the reaction was completed, the reaction mixture was purified by preparative reversed-phase chromatography. Fractions containing the product were collected, and acetonitrile was removed by concentration under reduced pressure, followed by lyophilization to obtain Compound (41) (5.8 mg, 0.0034 mmol).
MS (ESI) m/z: 1696.0 [M+H]⁺

### (12-2-2) Synthesis of Linker-payload (40)

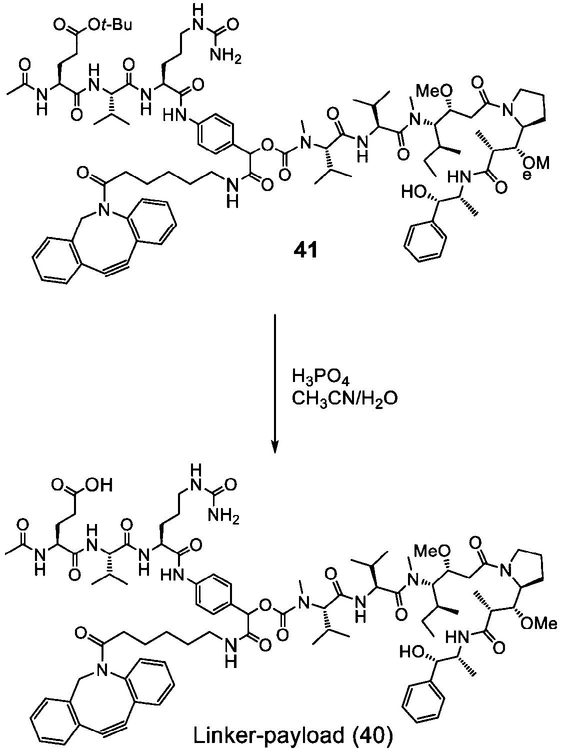

Acetonitrile (500 µL) and 85 wt% aqueous phosphoric acid solution (0.50 mL, 7.3 mmol) were sequentially added to Compound (41) (13 mg, 0.0077 mmol) obtained in (12-2-1), and the mixture was stirred at room temperature for 4 hours. After the reaction was completed, water (2 mL) was added, and the reaction solution was purified by preparative reversed-phase chromatography. Subsequently, fractions containing the product were collected, and acetonitrile was removed by concentration under reduced pressure, followed by lyophilization to obtain Linker-payload (40) (7.4 mg, 0.0045 mmol).
MS (ESI) m/z: 1638.9 [M+H]⁺

### (12-3) Synthesis of Linker-payload (42)

Linker-payload (42) was synthesized as follows.

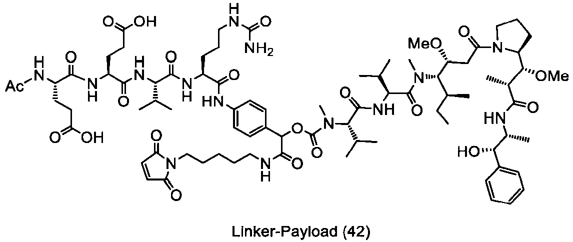

### (12-3-1) Synthesis of Carbonate (43)

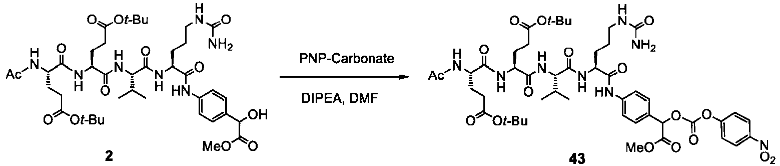

Alcohol (2) (140 mg, 0.165 mmol) obtained in (1-1-1) was dissolved in N,N-dimethylformamide (4 mL) and stirred under ice cooling for 5 minutes, then bis(4-nitrophenyl) carbonate (108 mg, 0.355 mmol) and N,N-diisopropylethylamine (100 µL, 0.574 mmol) were added, and the mixture was stirred at room temperature for 18 hours under a nitrogen atmosphere. After the organic solvent was removed with an evaporator, a solution of acetonitrile : water = 1:1 was added, and the mixture was purified by preparative reversed-phase chromatography. Fractions containing the product were collected, and acetonitrile was removed by concentration under reduced pressure, followed by lyophilization to obtain Compound 43 (130 mg, 0.128 mmol).
MS (ESI) m/z: 1015.6 [M+H]⁺

### (12-3-2) Synthesis of Compound (44)

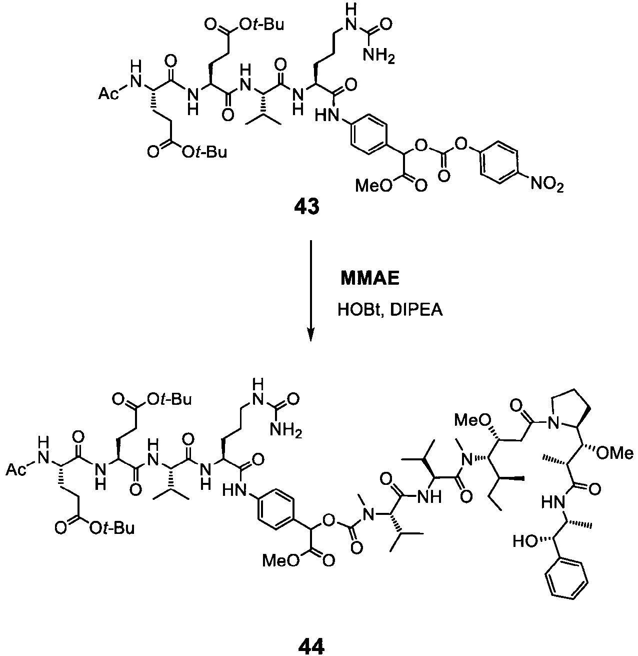

Compound (43) (85 mg, 0.084 mmol) obtained in (12-3-1) was dissolved in N,N-dimethylformamide (2 mL), and 1-hydroxybenzotriazole (20 mg, 0.13 mmol) and commercially available monomethylauristatin E (MMAE, 63 mg, 0.088 mmol) were added at room temperature. Subsequently, diisopropylethylamine (75 µL, 0.43 mmol) was added, and the mixture was stirred at room temperature for 23 hours under a nitrogen atmosphere. After the organic solvent was removed with an evaporator, a solution of acetonitrile : water = 1:1 was added, and the mixture was purified by preparative reversed-phase chromatography. Fractions containing the product were collected, and acetonitrile was removed by concentration under reduced pressure, followed by lyophilization to obtain Compound 44 (94 mg, 0.059 mmol).
MS (ESI) m/z: 1593.6 [M+H]⁺

### (12-3-3) Synthesis of Compound (45)

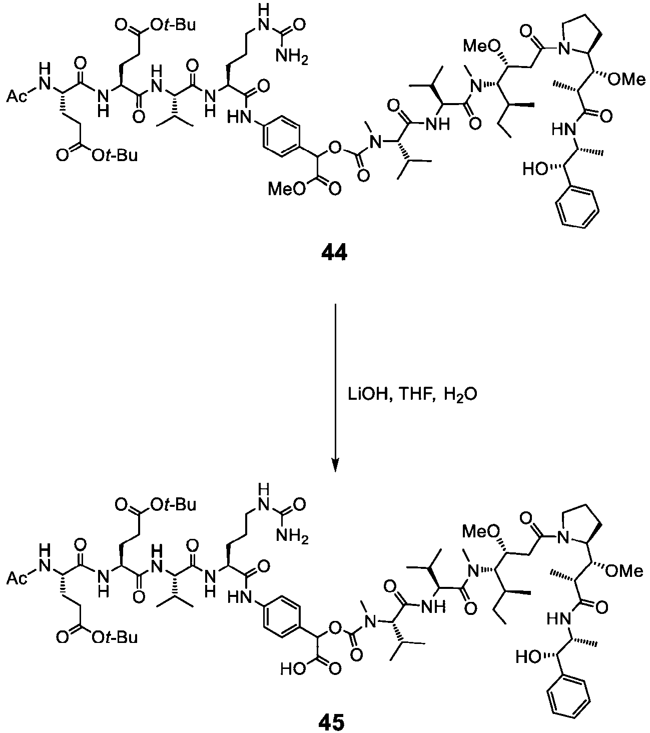

Compound (44) obtained in (12-3-2) (94 mg, 0.059 mmol) was dissolved in tetrahydrofuran (5 mL) and water (2 mL), and lithium hydroxide (1.0 M, 0.6 mL, 0.6 mmol) was added under ice cooling, and the mixture was stirred as is for 1 hour. After adjusting the pH to 5 by adding hydrochloric acid to the reaction solution, acetonitrile: water = 1:1 was added, and the mixture was purified by preparative reversed-phase chromatography. Fractions containing the product were collected, and acetonitrile was removed by concentration under reduced pressure, followed by lyophilization to obtain Compound 45 (77 mg, 0.049 mmol).
MS (ESI) m/z: 1579.7 [M+H]⁺

### (12-3-4) Synthesis of Compound (46)

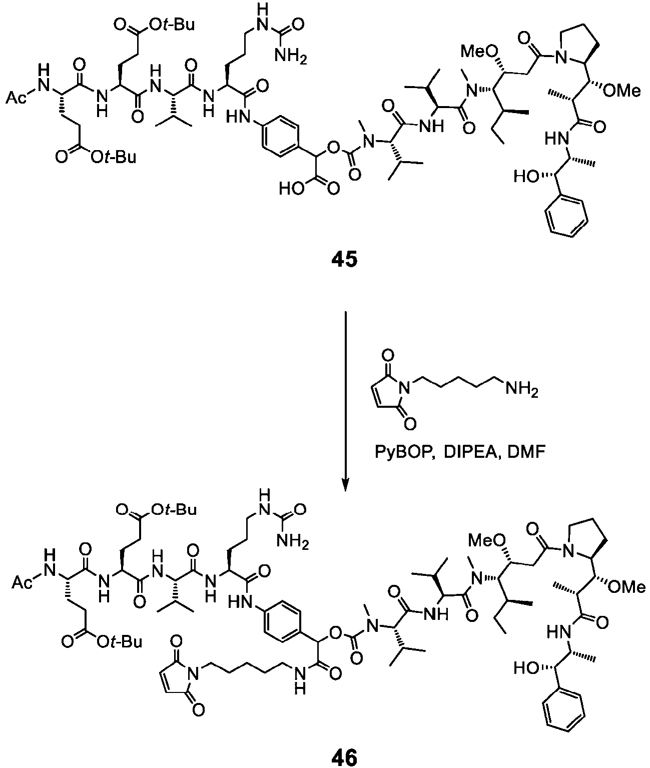

Compound (45) (77 mg, 0.049 mmol) obtained in (12-3-3) was dissolved in N,N-dimethylformamide (3 mL) and cooled on ice, then N,N-diisopropylethylamine (50 µL, 0.29 mmol) and 1H-benzotriazol-1-yloxytripyrrolidinophosphonium hexafluorophosphate (87 mg, 0.17 mmol) were added. Next, N-(5-aminopentyl)maleimide hydrochloride (35 mg, 0.16 mmol) was added, and the mixture was returned to room temperature and stirred for 20 hours. After the reaction was completed, the reaction mixture was purified by preparative reversed-phase chromatography. Fractions containing the product were collected, and acetonitrile was removed by concentration under reduced pressure, followed by lyophilization to obtain Compound (46) (65 mg, 0.037 mmol).
MS (ESI) m/z: 1744.7 [M+H]⁺

### (12-3-5) Synthesis of Linker-payload (42)

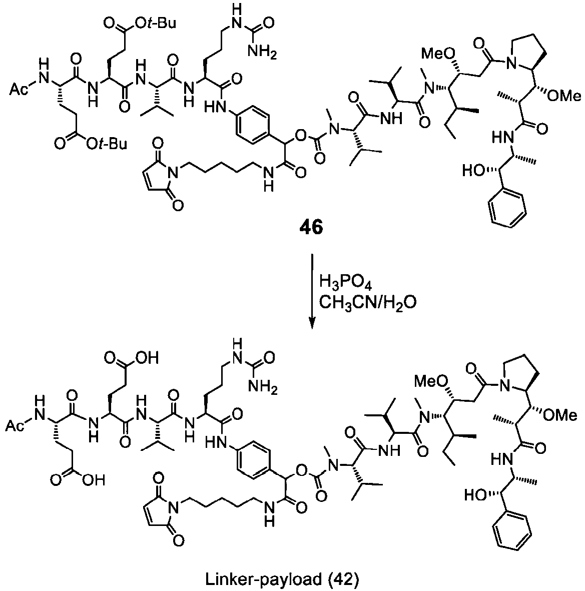

Acetonitrile (2 mL) and 85 wt% aqueous phosphoric acid solution (1.00 mL, 14.6 mmol) were sequentially added to Compound (46) (65 mg, 0.037 mmol) obtained in (12-3-4), and the mixture was stirred at room temperature for 6 hours. After the reaction was completed, water (2 mL) was added, and the reaction solution was purified by preparative reversed-phase chromatography. Subsequently, fractions containing the product were collected, and acetonitrile was removed by concentration under reduced pressure, followed by lyophilization to obtain Linker-payload (42) (49 mg, 0.030 mmol).
MS (ESI) m/z: 1631.6 [M+H]⁺

### (12-4) Synthesis of Linker-payload (47)

Linker-payload (47) was synthesized as follows.

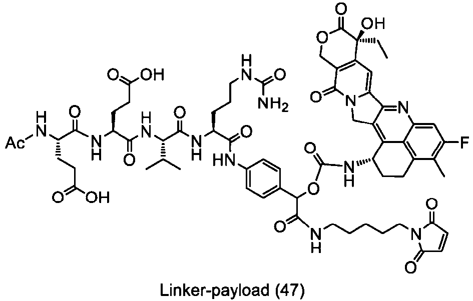

### (12-4-1) Synthesis of Compound (48)

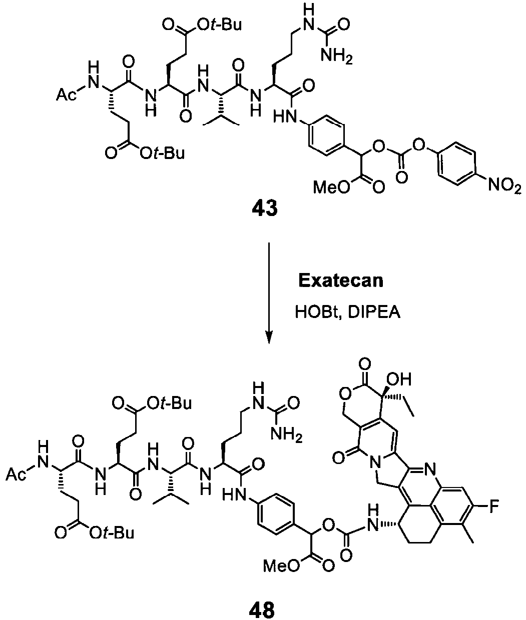

Compound (43) (67 mg, 0.066 mmol) obtained in (12-3-1) was dissolved in N,N-dimethylformamide (2 mL), and 1-hydroxybenzotriazole (17 mg, 0.11 mmol) and commercially available Exatecan mesylate (CAS: 169869-90-3, 35 mg, 0.066 mmol) were added at room temperature. Subsequently, diisopropylethylamine (50 µL, 0.29 mmol) was added, and the mixture was stirred at room temperature for 4 hours under a nitrogen atmosphere. After the organic solvent was removed with an evaporator, a solution of acetonitrile : water = 1:1 was added, and the mixture was purified by preparative reversed-phase chromatography. Fractions containing the product were collected, and acetonitrile was removed by concentration under reduced pressure, followed by lyophilization to obtain Compound 48 (57 mg, 0.043 mmol).
MS (ESI) m/z: 1311.7 [M+H]⁺

### (12-4-2) Synthesis of Compound (49)

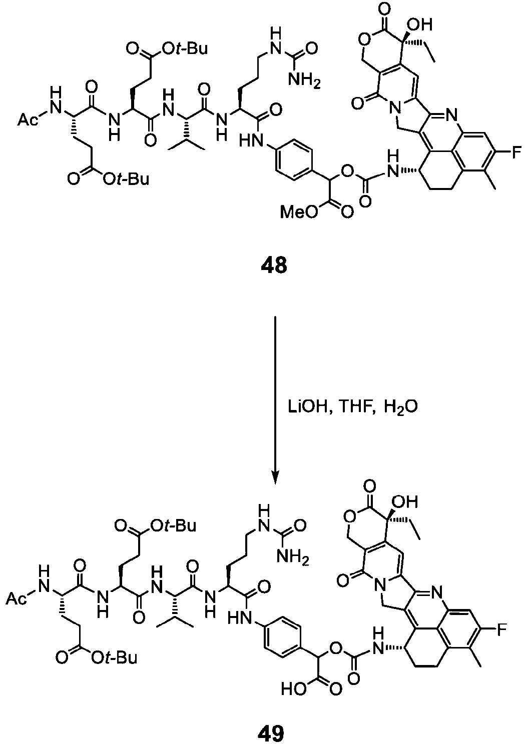

Compound (48) (57 mg, 0.043 mmol) obtained in (12-4-1) was dissolved in tetrahydrofuran (3 mL) and water (1.5 mL), and lithium hydroxide (1.0 M, 0.5 mL, 0.5 mmol) was added under ice cooling, and the mixture was stirred as is for 1 hour. After adjusting the pH to 5 by adding hydrochloric acid to the reaction solution, acetonitrile: water = 1:1 was added, and the mixture was purified by preparative reversed-phase chromatography. Fractions containing the product were collected, and acetonitrile was removed by concentration under reduced pressure, followed by lyophilization to obtain Compound 49 (45 mg, 0.035 mmol).
MS (ESI) m/z: 1297.6 [M+H]⁺

### (12-4-3) Synthesis of Compound (50)

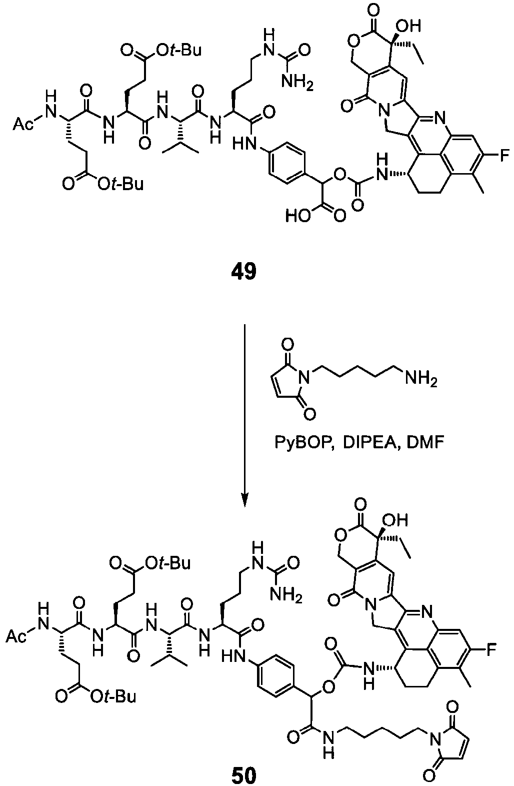

Compound (49) (45 mg, 0.035 mmol) obtained in (12-4-2) was dissolved in N,N-dimethylformamide (3 mL) and cooled on ice, then N,N-diisopropylethylamine (30 µL, 0.17 mmol) and 1H-benzotriazol-1-yloxytripyrrolidinophosphonium hexafluorophosphate (55 mg, 0.11 mmol) were added. Next, N-(5-aminopentyl)maleimide hydrochloride (22 mg, 0.10 mmol) was added, and the mixture was returned to room temperature and stirred for 18 hours. After the reaction was completed, the reaction mixture was purified by preparative reversed-phase chromatography. Fractions containing the product were collected, and acetonitrile was removed by concentration under reduced pressure, followed by lyophilization to obtain Compound (50) (22 mg, 0.015 mmol).
MS (ESI) m/z: 1462.7 [M+H]⁺

### (12-3-5) Synthesis of Linker-payload (47)

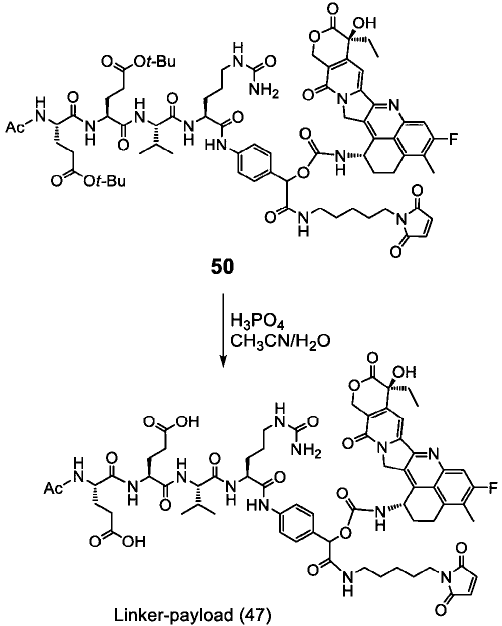

Acetonitrile (1 mL) and 85 wt% aqueous phosphoric acid solution (1.0 mL, 14.6 mmol) were sequentially added to Compound (50) (22 mg, 0.015 mmol) obtained in (12-4-3), and the mixture was stirred at room temperature for 1.5 hours. After the reaction was completed, water (1 mL) was added, and the reaction solution was purified by preparative reversed-phase chromatography, Subsequently, and fractions containing the product were collected, and acetonitrile was removed by concentration under reduced pressure, followed by lyophilization to obtain Linker-payload (47) (18.8 mg, 0.0139 mmol).

¹H NMR (300 MHz; DMSO-d₆) δ10.04 (s, 1H), 8.10-8.05 (m, 4H), 7.80-7.77 (m, 2H), 7.59-7.55 (m, 2H), 7.35-7.30 (m, 3H), 6.99 (d, J = 8.8 Hz, 2H), 6.54-6.53 (m, 1H), 6.01 (brs, 1H), 5.74 (d, J = 9.0 Hz, 1H), 5.43 (brs, 4H), 5.33-5.28 (m, 2H), 4.29-4.15 (m, 4H), 3.19-2.98 (m, 5H), 2.43-2.38 (m, 5H), 2.27-2.20 (m, 7H), 1.95-1.83 (m, 8H), 1.73-1.62 (m, 4H), 1.42-1.30 (m, 7H), 1.23-1.13 (m, 3H), 0.84 (m, 9H).
MS (ESI) m/z: 1349.2 [M+H]⁺

### (12-5) Synthesis of Linker-payload (125)

Linker-payload (125) shown below was synthesized in the same manner as for the synthesis of Linker-payload mimic (120), except that MMAE was used instead of sarcosine-pyrene.

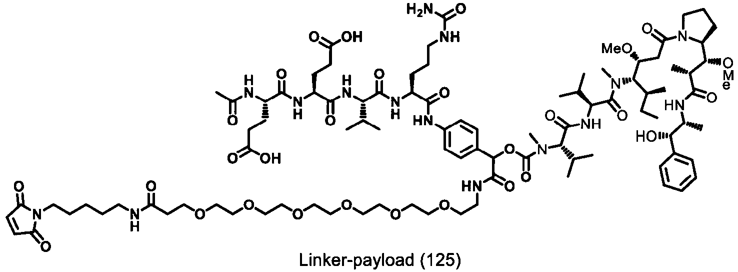

MS (ESI) m/z: 1968.14 [M+H]⁺

### (12-6) Synthesis of Linker-payload (126)

Linker-payload (126) shown below was synthesized in the same manner as for the synthesis of Linker-payload mimic (35), except that Exatecan mesylate was used instead of MMAE.

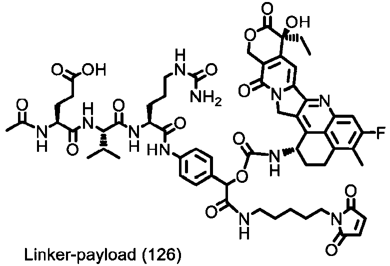

MS (ESI) m/z: 1220.50 [M+H]⁺

### Example 13: Synthesis of ADC

### Example 13-1: Synthesis of ADC 1

To a solution of a 3 mg/mL of anti-HER2 IgG antibody trastuzumab (Chugai Pharmaceutical Co., Ltd.) in PBSE buffer (50 mM phosphate buffered saline (PBS), 10 mM EDTA, pH = 7.4), 8 equivalents of a solution (2 mM) of a reducing agent (TCEP) in PBSE relative to the antibody were added, and the mixture was incubated at 37°C for 1 hour. To the resulting reaction mixture, dimethylacetamide (DMA) (6%v/v) and 10 mM solutions of various Linker-payload (35) in DMA (20 equivalents relative to the antibody) were successively added, and the mixture was gently stirred at 20°C for 1 hour. An excess amount of N-acetylcysteine was added to the reaction mixture, which was stirred at 25°C for 20 minutes. The final mixture was purified using a NAP-5 desalting column (GE Healthcare), eluted with formulation buffer (20 mM histidine, with 5 wt% trehalose added, pH 5.2), to obtain ADC mimic 1.

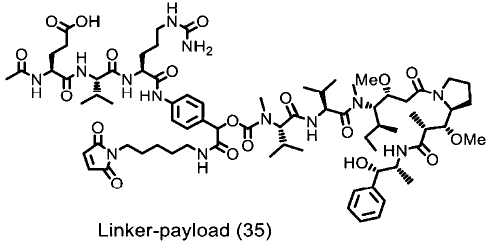

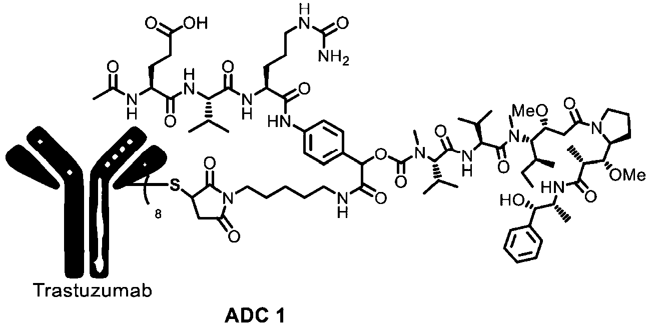

### Example 13-2: Synthesis of ADC 2

Following Example 13-1, ADC 2 was obtained from Linker-payload (42).

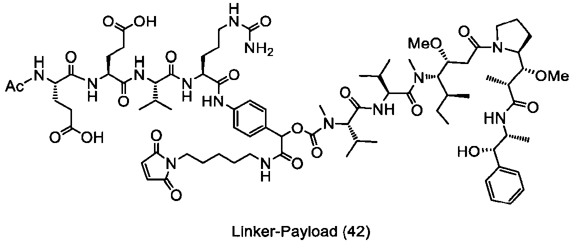

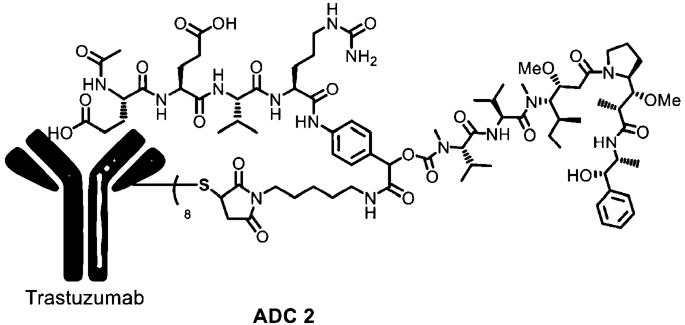

### Example 13-3: Synthesis of ADC 3

Following Example 13-1, ADC 3 was obtained from Linker-payload (47).

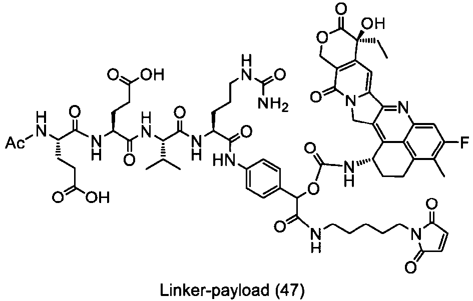

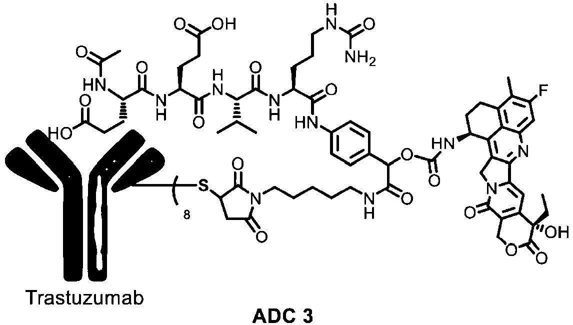

### Example 13-4: Synthesis of ADC 9

Following Example 13-1, ADC 9 was obtained from Linker-payload (125).

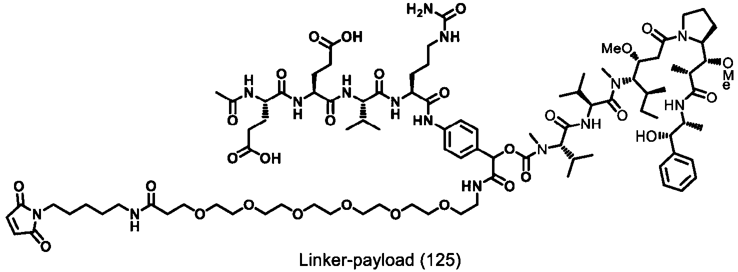

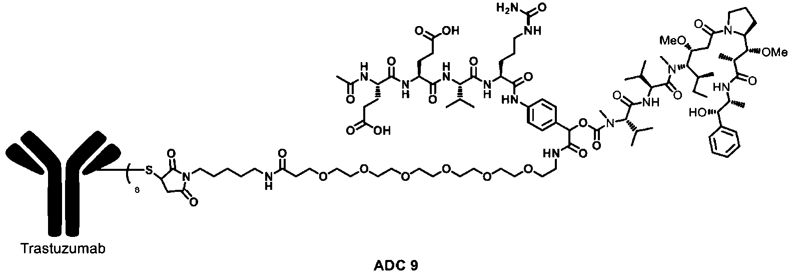

### Example 13-5: Synthesis of ADC 10

Following Example 13-1, ADC 10 was obtained from Linker-payload (126).

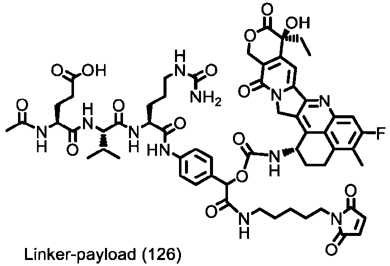

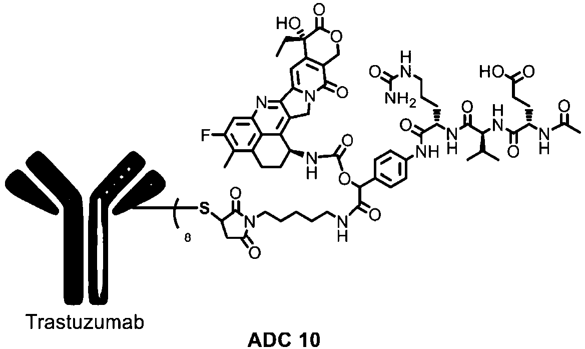

### Example 13-6: Synthesis of ADC 17

Following Example 13-1, ADC 17 was obtained from Linker-payload (146).

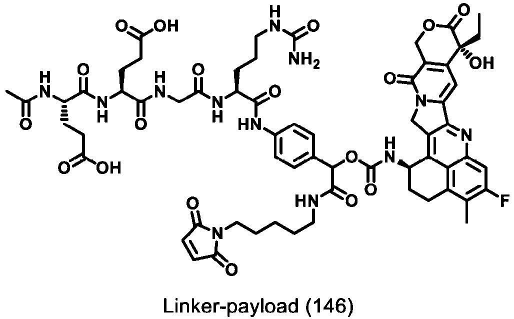

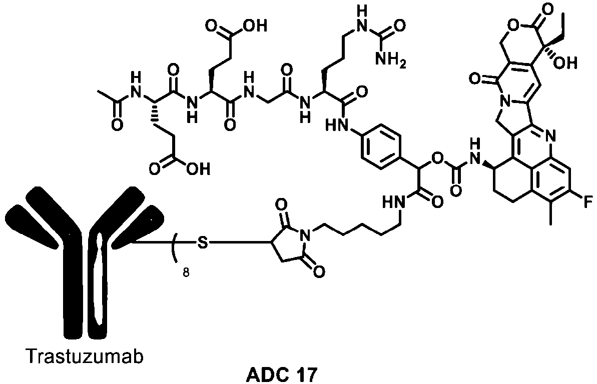

### Example 14: HIC-HPLC analysis of ADC

Using the conditions of Example 8, HIC-HPLC analysis was performed.

**Table 13: DAR of ADCs**

| | Linker-payload | Example | DAR |
|---|---|---|---|
| ADC 1 | Linker-payload (35) | Example 13-1 | 8 |
| ADC 2 | Linker-payload (42) | Example 13-2 | 8 |
| ADC 3 | Linker-payload (47) | Example 13-3 | 8 |
| ADC 9 | Linker-payload (125) | Example 13-4 | 8 |
| ADC 10 | Linker-payload (126) | Example 13-5 | 8 |
| ADC 17 | Linker-payload (146) | Example 13-6 | 8 |

Subsequently, the hydrophobicity of the ADC was evaluated using HIC-HPLC. The measurement was performed according to Example 9. The degree of hydrophobicity of the ADC can be evaluated based on the retention time of the ADC in the HIC chromatogram. Trastuzumab, which is the starting antibody, was used as a comparison.

**Table 14: Evaluation of degree of hydrophobicity of trastuzumab-based ADCs using HIC-HPLC**

| | Linker-payload | Example | Retention Time |
|---|---|---|---|
| ADC 1 | Linker-payload (35) | Example 13-1 | 12.3 min |
| ADC 2 | Linker-payload (42) | Example 13-2 | 11.1 min |
| ADC 3 | Linker-payload (47) | Example 13-3 | 9.1 min |
| ADC 10 | Linker-payload (126) | Example 13-5 | 9.3 min |
| ADC 17 | Linker-payload (146) | Example 13-6 | 9.0 min |
| Trastuzumab | None | None | 8.2 min |

It can be seen that ADCs 1, 2, and 3, which are exo-type ADCs, have retention times in the HIC chromatogram comparable to the starting antibody and are more hydrophilic ADCs.

### Example 15: Evaluation of aggregation rate of ADC by size exclusion chromatography (SEC-HPLC)

SEC-HPLC analysis was performed according to Example 10.

**Table 15: Evaluation of aggregation rate of trastuzumab-based ADCs using SEC-HPLC**

| | Linker-payload | Example | Aggregation Rate |
|---|---|---|---|
| ADC 1 | Linker-payload (35) | Example 13-1 | 0.8 % |
| ADC 2 | Linker-payload (42) | Example 13-2 | 0.6 % |
| ADC 3 | Linker-payload (47) | Example 13-3 | 0.5 % |
| ADC 10 | Linker-payload (126) | Example 13-5 | 0.1 % |
| ADC 17 | Linker-payload (146) | Example 13-6 | 0.4 % |
| Trastuzumab | None | None | 0.5 % |

As a result, it was confirmed that the ADCs synthesized in Examples 13-1, 13-2, and 13-3 tended to have a low aggregation rate, indicating higher stability. Therefore, it was confirmed that the ADCs synthesized in Examples 13-1, 13-2, and 13-3 are preferable ADCs.

### Example 16: Synthesis of ADC

### (16-1) Synthesis of ADC 4

In the following Comparative Examples and Examples, an antibody derivative (thiol-introduced trastuzumab) described in Example 81-7 of WO2019/240287A1 was used as the thiol-introduced antibody. This antibody derivative has the following structure in which a thiol group is regioselectively introduced into trastuzumab (humanized IgG1 antibody) via the amino group in the side chain of the lysine residue at position 246 or 248 of the antibody heavy chain (the position of the lysine residue is according to EU numbering). 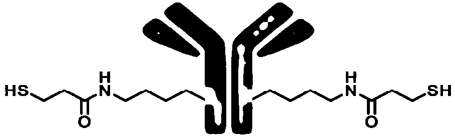 (In the above structure, NH-CH₂-CH₂-CH₂-CH₂- extending from the antibody heavy chain corresponds to the side chain of the lysine residue, and a thiol-containing group, HS-CH₂-CH₂-C (=O), is added to the amino group in the side chain of this lysine residue. Since no modification at other lysine residues was detected by peptide mapping, the antibody is interpreted to have 100% regioselectivity at position 246 or 248 of the antibody heavy chain.)

To a solution of the thiol-introduced antibody (20 µM) in buffer (PBS buffer at pH 7.4), 10 equivalents of a solution of tLinker-payload (35) synthesized in Example 13-1 (10 mM) in DMF were added. After standing at room temperature for 2 hours, the mixture was purified using NAP-5 column (manufactured by GE Healthcare) to obtain ADC 4. ESI-TOFMS analysis was performed, and a peak was confirmed at 151414 for the reaction product, into which two molecules of Linker-payload (35) were introduced.

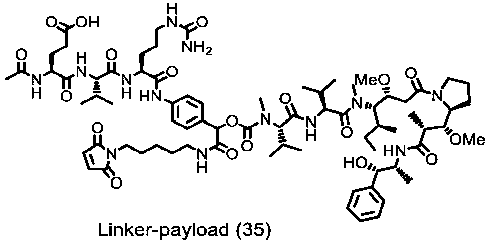

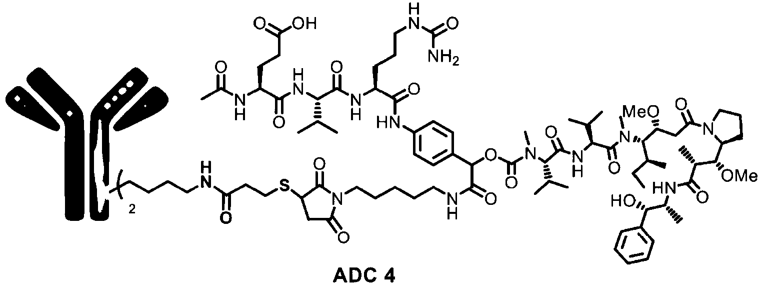

### Example 16-2: Synthesis of ADC 5

Following Example 16-1, ADC 5 was obtained from Linker-payload (42). ESI-TOFMS analysis was performed, and a peak was confirmed at 151673 for the reaction product, into which two molecules of Linker-payload (42) were introduced.

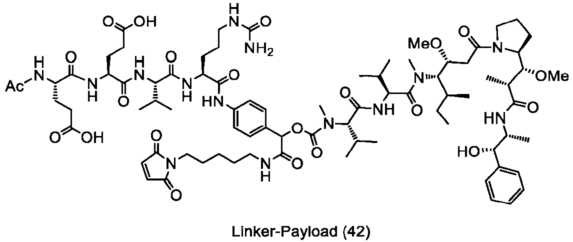

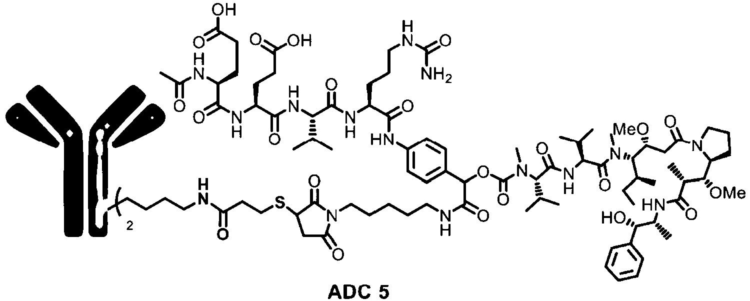

### Example 16-3: Synthesis of ADC 6

Following Example 16-1, ADC 6 was obtained from Linker-payload (47). ESI-TOFMS analysis was performed, and a peak was confirmed at 151109 for the reaction product, into which two molecules of Linker-payload (47) were introduced.

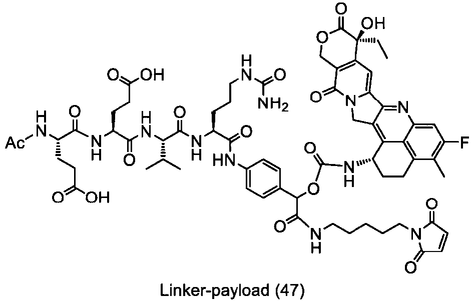

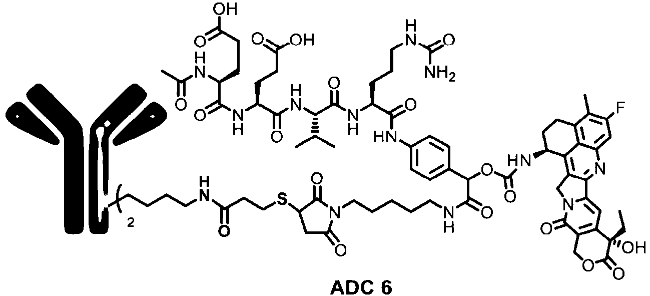

### Example 16-4: Synthesis of ADC 6

Following Example 16-1, ADC 8 was obtained from Linker-payload (125). ESI-TOFMS analysis was performed, and a peak was confirmed at 150524 for the reaction product, into which two molecules of Linker-payload (125) were introduced.

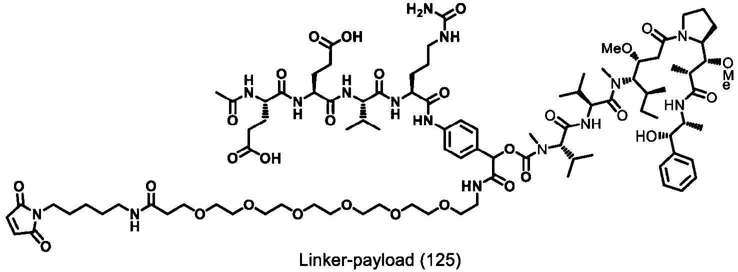

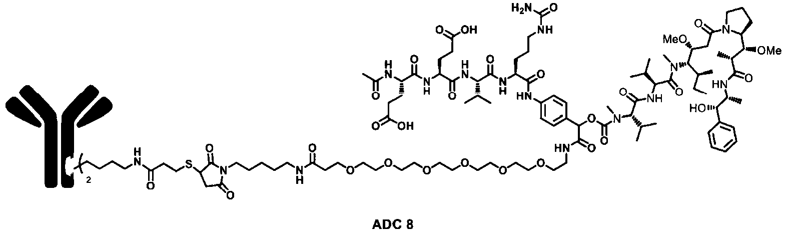

### Example 16-5: Synthesis of ADC 11

Following Example 16-1, ADC 11 was obtained from Linker-payload (126). ESI-TOFMS analysis was performed, and a peak was confirmed at 150615 for the reaction product, into which two molecules of Linker-payload (126) were introduced.

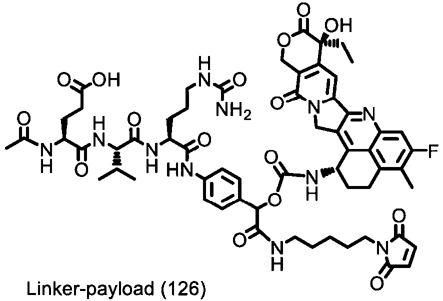

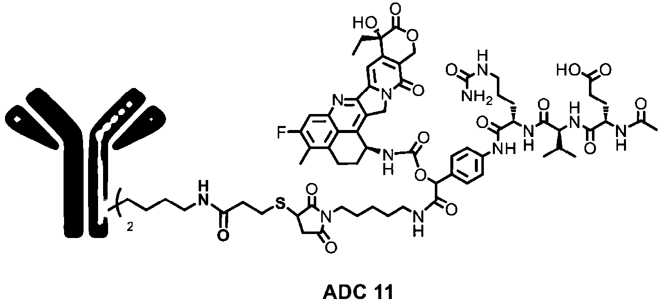

### Example 16-6: Synthesis of ADC 12

Following Example 16-1, ADC 12 was obtained from Linker-payload (130). ESI-TOFMS analysis was performed, and a peak was confirmed at 152861 for the reaction product, into which two molecules of Linker-payload (130) were introduced.

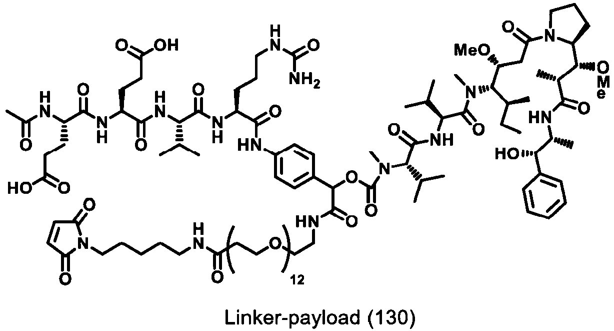

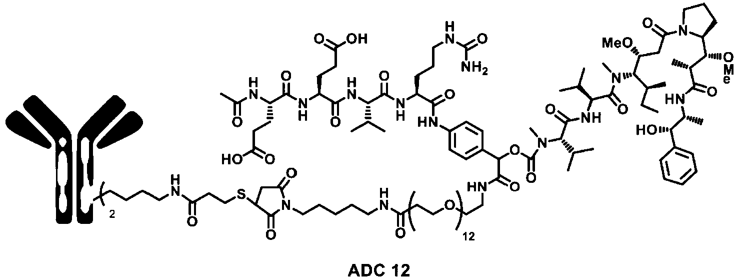

### Example 16-7: Synthesis of ADC 13

Following Example 16-1, ADC 13 was obtained from Linker-payload (132). ESI-TOFMS analysis was performed, and a peak was confirmed at 151632 for the reaction product, into which two molecules of Linker-payload (132) were introduced.

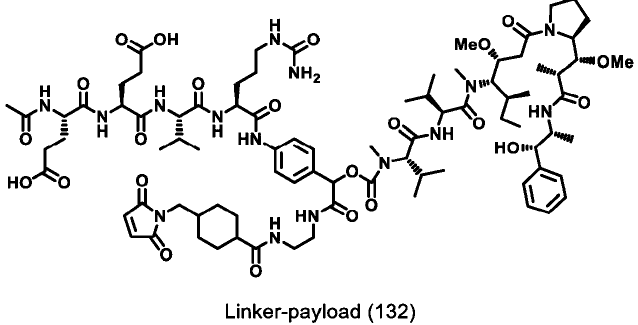

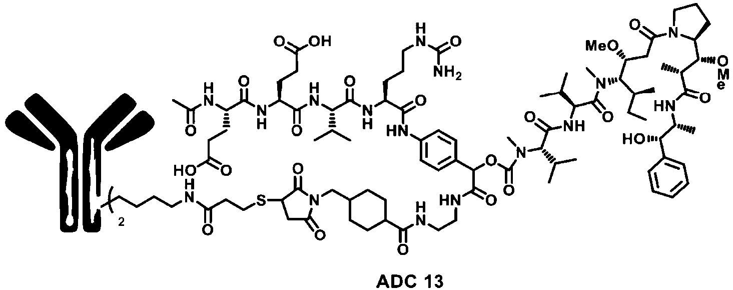

### Example 16-8: Synthesis of ADC 14

Following Example 16-1, ADC 14 was obtained from Linker-payload (134). ESI-TOFMS analysis was performed, and a peak was confirmed at 151409 for the reaction product, into which two molecules of Linker-payload (134) were introduced.

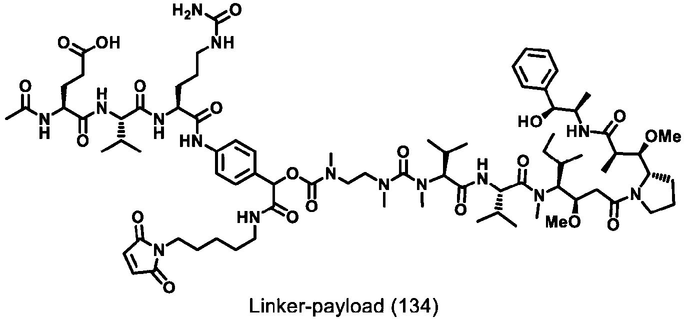

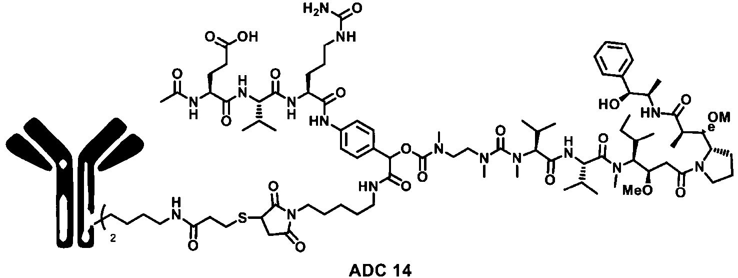

### Example 16-9: Synthesis of ADC 15

Following Example 16-1, ADC 15 was obtained from Linker-payload (141). ESI-TOFMS analysis was performed, and a peak was confirmed at 151095 for the reaction product, into which two molecules of Linker-payload (141) were introduced.

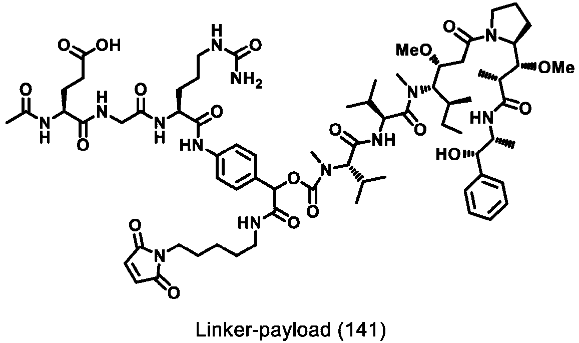

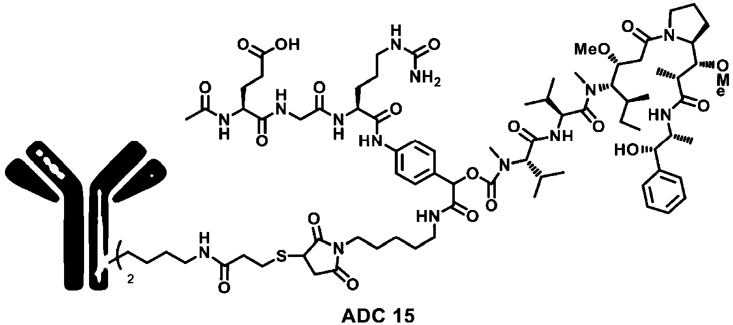

### Example 16-10: Synthesis of ADC 16

Following Example 16-1, ADC 16 was obtained from Linker-payload (146). ESI-TOFMS analysis was performed, and a peak was confirmed at 150789 for the reaction product, into which two molecules of Linker-payload (146) were introduced.

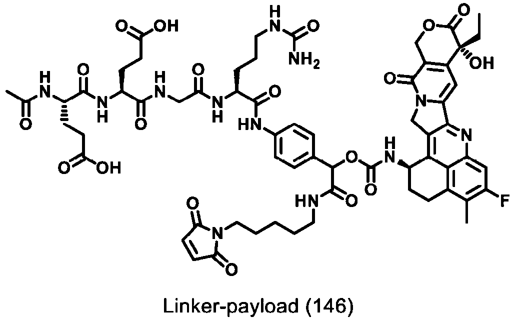

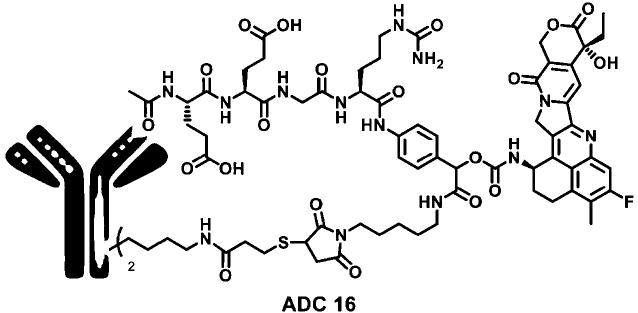

### Example 17: HIC-HPLC analysis of ADC

Using the conditions of Example 8, HIC-HPLC analysis was performed.

**Table 16: DAR of ADCs**

| | Linker-payload | Example | DAR |
|---|---|---|---|
| ADC 4 | Linker-payload (35) | Example 16-1 | 2 |
| ADC 5 | Linker-payload (42) | Example 16-2 | 2 |
| ADC 6 | Linker-payload (47) | Example 16-3 | 2 |
| ADC 12 | Linker-payload (130) | Example 16-6 | 2 |
| ADC 13 | Linker-payload (132) | Example 16-7 | 2 |
| ADC 14 | Linker-payload (134) | Example 16-8 | 2 |
| ADC 15 | Linker-payload (141) | Example 16-9 | 2 |
| ADC 16 | Linker-payload (146) | Example 16-10 | 2 |

Subsequently, the hydrophobicity of the ADC was evaluated using HIC-HPLC. The measurement was performed according to Example 9. The degree of hydrophobicity of the ADC can be evaluated based on the retention time of the ADC in the HIC chromatogram. Trastuzumab, which is the starting antibody, was used as a comparison.

**Table 17: Evaluation of degree of hydrophobicity of trastuzumab-based ADCs using HIC-HPLC**

| | Linker-payload | Example | Retention Time |
|---|---|---|---|
| ADC 4 | Linker-payload (35) | Example 16-1 | 11.2 min |
| ADC 5 | Linker-payload (42) | Example 16-2 | 10.7 min |
| ADC 6 | Linker-payload (47) | Example 16-3 | 10.4 min |
| ADC 12 | Linker-payload (130) | Example 16-6 | 9.8 min |
| ADC 13 | Linker-payload (132) | Example 16-7 | 9.6 min |
| ADC 14 | Linker-payload (134) | Example 16-8 | 9.7 min |
| ADC 15 | Linker-payload (141) | Example 16-9 | 10.9 min |
| ADC 16 | Linker-payload (146) | Example 16-10 | 10.4 min |
| Trastuzumab | None | None | 8.2 min |

It can be seen that ADCs 4, 5, 6, 12, 13, 14, 15, and 16, which are exo-type ADCs, have retention times in the HIC chromatogram comparable to the starting antibody and are more hydrophilic ADCs.

### Example 18: Evaluation of aggregation rate of ADC by size exclusion chromatography (SEC-HPLC)

SEC-HPLC analysis was performed according to Example 10.

**Table 18: Evaluation of aggregation rate of trastuzumab-based ADCs using SEC-HPLC**

| | Linker-payload | Example | Aggregation Rate |
|---|---|---|---|
| ADC 4 | Linker-payload (35) | Example 16-1 | 0.6 % |
| ADC 5 | Linker-payload (42) | Example 16-2 | 0.8 % |
| ADC 6 | Linker-payload (47) | Example 16-3 | 0.5 % |
| ADC 12 | Linker-payload (130) | Example 16-6 | 1.3 % |
| ADC 13 | Linker-payload (132) | Example 16-7 | 1.2 % |
| ADC 14 | Linker-payload (134) | Example 16-8 | 1.3 % |
| ADC 15 | Linker-payload (141) | Example 16-9 | 1.5 % |
| ADC 16 | Linker-payload (146) | Example 16-10 | 1.5 % |
| ADC 18 | Linker-payload (42) | Example 26-5 | 1.4 % |
| ADC 19 | Linker-payload (42) | Example 26-6-3 | 1.0 % |
| Trastuzumab | None | None | 0.5 % |

As a result, it was confirmed that the ADCs synthesized in Examples 16-1, 16-2, 16-3, 16-6, 16-7, 16-8, 16-9, and 16-10, and Examples 26-5 and 26-6-3 tended to have a low aggregation rate, indicating higher stability. Therefore, it was confirmed that the ADCs synthesized in Examples 16-1, 16-2, 16-3, 16-6, 16-7, 16-8, 16-9, and 16-10, and Examples 26-5 and 26-6-3 are preferable ADCs.

### Example 19: Synthesis of Linker-payload mimic

### (19-1) Synthesis of Linker-payload mimic (51)

Linker-payload mimic (51) was synthesized as follows.

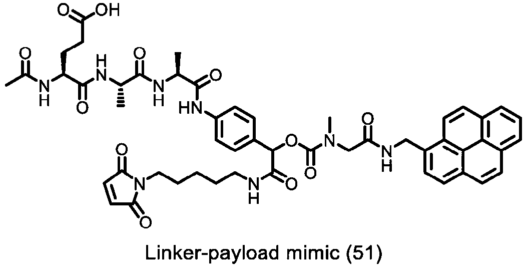

### (19-1-1) Synthesis of Alcohol (52)

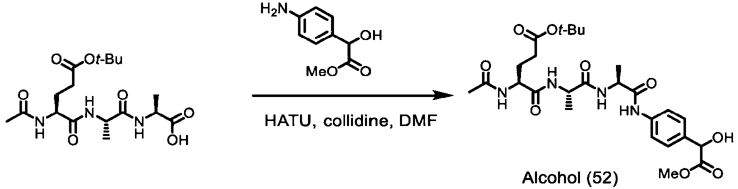

Ac-Glu(OtBu)-Ala-Ala-OH (51.0 mg, 132 µmol) was dissolved in N,N-dimethylformamide (660 µL), and 1-[bis(dimethylamino)methylene]-1H-1,2,3,triazolo[4,5-b]pyridinium 3-oxide hexafluorophosphate (60.1 mg, 158 µmol), 2,4,6-trimethylpyridine (20.8 µL, 158 µmol) were added, followed by stirring at room temperature for 10 minutes. Subsequently, methyl 4-aminomandelate (28.6 mg, 158 µmol) was added, and the mixture was stirred at room temperature for 18.5 hours, followed by purification by preparative reversed-phase chromatography. Fractions containing the product were collected, and acetonitrile was removed by concentration under reduced pressure, followed by lyophilization to obtain the above-described Alcohol (52) (62.8 mg, 114 µmol).

¹H NMR (400 MHz, DMSO-d₆) δ9.91 (s, 1H), 8.08-8.02 (m, 3H), 7.56 (d, J = 8.4 Hz, 2H), 7.32 (d, J = 8.4 Hz, 1H), 5.08 (s, 1H), 4.39-4.32 (m, 1H), 4.28-4.20 (m, 2H), 3.59 (s, 3H), 2.23 (t, J = 8.0 Hz, 2H), 1.90-1.81 (m, 4H), 1.74-1.67 (m, 1H), 1.38 (s, 9H), 1.30 (d, J = 7.2 Hz, 3H), 1.22 (d, J = 7.2 Hz, 3H).
MS (ESI) m/z: 551.25 [M+H]⁺

### (19-1-2) Synthesis of Pyrene (53)

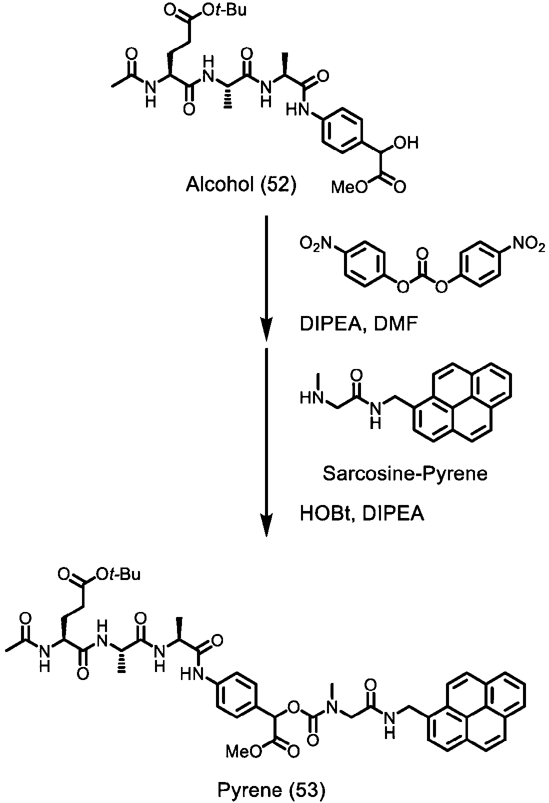

Alcohol (52) (62.8 mg, 114 µmol) obtained in 19-1-1 was dissolved in N,N-dimethylformamide (570 µL) and stirred under ice cooling for 5 minutes, then bis(4-nitrophenyl) carbonate (104 mg, 342 µmol) and N,N-diisopropylethylamine (43.6 µL, 256 µmol) were added, and the mixture was stirred at room temperature for 4 hours. Subsequently, the mixture was cooled on ice, then Sarcosine-Pyrene (172 mg, 570 µmol), 1-hydroxybenzotriazole (23.1 mg, 171 µmol), and N,N-diisopropylethylamine (150 µL, 883 µmol) were added, and the mixture was stirred at room temperature for 20 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (53) (80.2 mg, 91.2 µmol).

¹H NMR (400 MHz, DMSO-d₆) δ10.04-10.01 (m, 1H), 8.69-8.64 (m, 1H), 8.39-8.24 (m, 4H), 8.18-8.01 (m, 8H), 7.88-7.85 (m, 1H), 7.66-7.64 (m, 2H), 7.44-7.40 (m, 2H), 5.79-5.77 (m, 1H), 5.04-4.97 (m, 2H), 4.43-4.23 (m, 3H), 4.11-4.07 (m, 1H), 3.91-3.82 (m, 1H), 3.62-3.61 (m, 3H), 2.99-2.93 (m, 3H), 2.23 (t, J = 8.0 Hz, 2H), 1.88-1.82 (m, 4H), 1.75-1.65 (m, 1H), 1.37-1.30 (m, 12H), 1.25-1.21 (m, 3H).
MS (ESI) m/z: 879.35 [M+H]⁺

### (19-1-3) Synthesis of Pyrene (54)

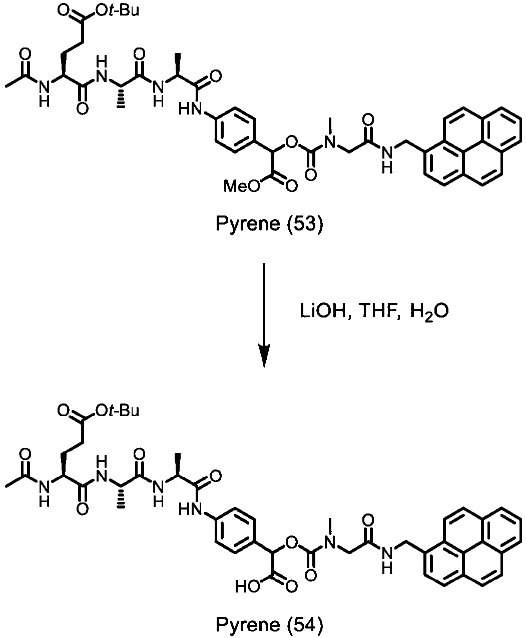

Pyrene (53) (28.5 mg, 32.4 µmol) was dissolved in tetrahydrofuran (2.55 mL) and water (0.85 mL) and stirred under ice cooling for 5 minutes, then lithium hydroxide monohydrate (6.8 mg, 162 µmol) was added, and the mixture was stirred at room temperature for 1 hour. After the reaction was completed, the pH was adjusted to approximately 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (54) (15.2 mg, 17.6 µmol).

¹H NMR (400 MHz, DMSO-d₆) δ13.05 (brs, 1H), 10.04-10.00 (m, 1H), 8.68-8.65 (m, 1H), 8.39-8.22 (m, 4H), 8.19-8.00 (m, 8H), 7.87-7.84 (m, 1H), 7.67-7.63 (m, 2H), 7.44-7.42 (m, 2H), 5.67 (s, 1H), 5.04-4.96 (m, 2H), 4.44-4.20 (m, 3H), 4.13-4.09 (m, 1H), 3.90-3.80 (m, 1H), 2.99-2.92 (m, 3H), 2.24 (t, J = 8.0 Hz, 2H), 1.88-1.82 (m, 4H), 1.75-1.65 (m, 1H), 1.37-1.30 (m, 12H), 1.25-1.22 (m, 3H).
MS (ESI) m/z: 865.30 [M+H]⁺

### (19-1-4) Synthesis of Pyrene (55)

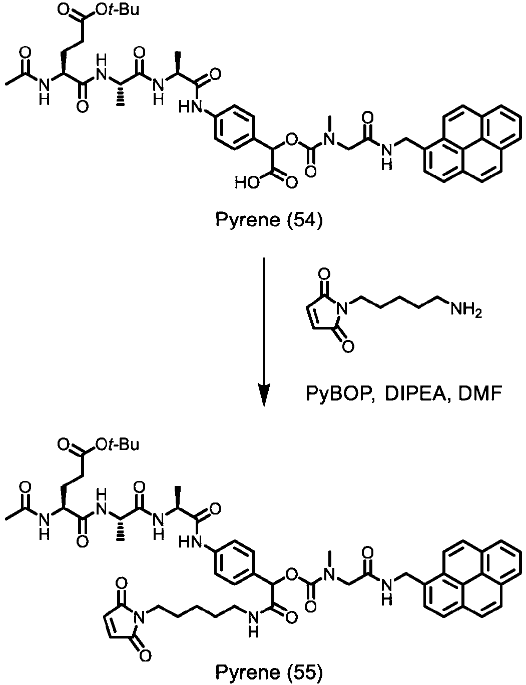

Pyrene (54) (14.2 mg, 16.4 µmol) was dissolved in N,N-dimethylformamide (330 µL) and cooled on ice, then N,N-diisopropylethylamine (5.6 µL, 32.8 µmol) and 1H-benzotriazol-1-yloxytripyrrolidinophosphonium hexafluorophosphate (12.8 mg, 24.6 µmol) were added. Next, N-(5-aminopentyl)maleimide hydrochloride (5.4 mg, 24.6 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (55) (11.7 mg, 11.4 µmol).

¹H NMR (400 MHz, DMSO-d₆) δ9.99-9.95 (m, 1H), 8.89-7.7.94 (m, 14H), 7.62-7.58 (m, 2H), 7.40-7.34 (m, 2H), 6.99-6.94 (m, 2H), 5.70-5.68 (m, 1H), 5.10-4.99 (m, 2H), 4.44-4.23 (m, 2H), 4.14-3.82 (m, 2H), 3.30-3.21 (m, 1H), 3.01-2.88 (m, 5H), 2.23 (t, J = 8.0 Hz, 2H), 1.88-1.67 (m, 5H), 1.14-1.08 (m, 22H).
MS (ESI) m/z: 1029.45 [M+H]⁺

### (19-1-5) Synthesis of Linker-payload mimic (51)

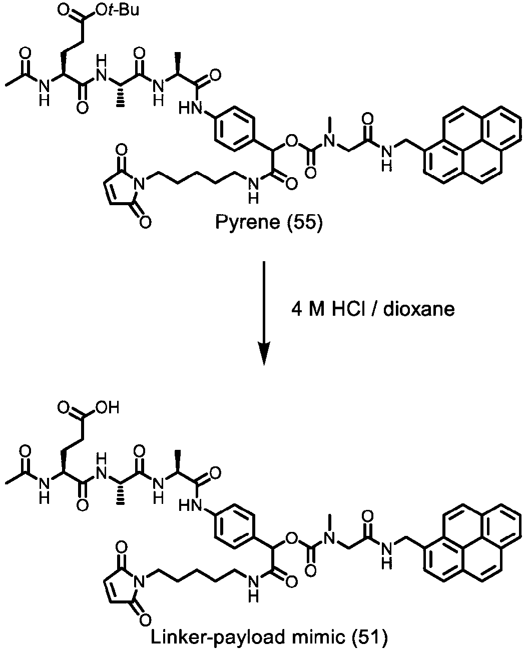

To Pyrene (55) (9.69 mg, 9.42 µmol) were successively added 1,4-dioxane (1.90 mL) and 4 M hydrochloric acid/dioxane solution (2.36 mL, 9.42 mmol), and the mixture was stirred at room temperature for 5 hours. After cooling on ice, N,N-diisopropylethylamine (1.76 mL, 10.4 mmol) was added, and the mixture was stirred at room temperature for 10 minutes. The reaction solution was purified by preparative reversed-phase chromatography, and fractions containing the product were collected, and acetonitrile was removed by concentration under reduced pressure, followed by lyophilization to obtain Linker-payload mimic (51) (4.03 mg, 4.14 µmol).

¹H NMR (400 MHz, DMSO-d₆) δ12.07 (brs, 1H), 9.99-9.96 (m, 1H), 8.89-7.7.94 (m, 14H), 7.62-7.58 (m, 2H), 7.40-7.34 (m, 2H), 6.96-6.94 (m, 2H), 5.70-5.68 (m, 1H), 5.14-4.99 (m, 2H), 4.42-4.23 (m, 2H), 4.14-3.82 (m, 2H), 3.31-3.21 (m, 1H), 3.01-2.89 (m, 5H), 2.28-2.24 (m, 2H), 1.89-1.69 (m, 5H), 1.41-1.03 (m, 13H).
MS (ESI) m/z: 973.40 [M+H]⁺

### (19-2) Synthesis of Linker-payload mimic (56)

Linker-payload mimic (56) was synthesized as follows.

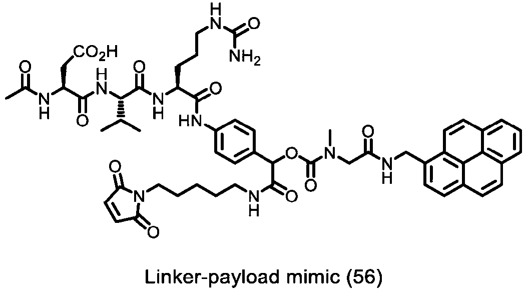

### (19-2-1) Synthesis of Alcohol (57)

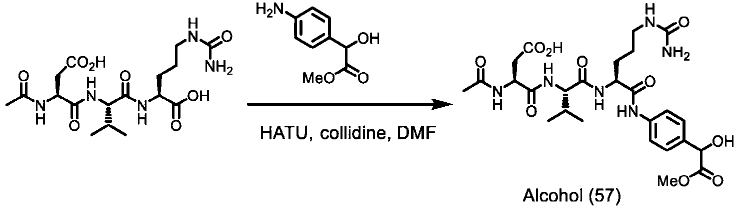

Ac-Asp(OtBu)-Val-Cit-OH (51.7 mg, 103 µmol) was dissolved in N,N-dimethylformamide (520 µL), and 1-[bis(dimethylamino)methylene]-1H-1,2,3,triazolo[4,5-b]pyridinium 3-oxide hexafluorophosphate (46.9 mg, 123 µmol) and 2,4,6-trimethylpyridine (15.9 µL, 123 µmol) were added, followed by stirring at room temperature for 10 minutes. Subsequently, methyl 4-aminomandelate (22.3 mg, 123 µmol) was added, and the mixture was stirred at room temperature for 19 hours, followed by purification by preparative reversed-phase chromatography. Fractions containing the product were collected, and acetonitrile was removed by concentration under reduced pressure, followed by lyophilization to obtain the above-described Alcohol (57) (56.3 mg, 86.5 µmol).

¹H NMR (400 MHz, DMSO-d₆) δ9.99 (s, 1H), 8.27 (d, J = 8.4 Hz, 1H), 8.17 (d, J = 8.4 Hz, 1H), 7.59 (d, J = 8.8 Hz, 1H), 7.56 (d, J = 8.8 Hz, 2H), 7.31 (d, J = 8.8 Hz, 2H), 5.98 (brs, 1H), 5.41 (brs, 2H), 5.08 (s, 1H), 4.64-4.59 (m, 1H), 4.39-4.34 (m, 1H), 4.22-4.18 (m, 1H), 3.59 (s, 3H), 3.01-2.94 (m, 2H), 2.69-2.63 (m, 1H), 2.44-2.38 (m, 1H), 2.00-1.95 (m, 1H), 1.83 (s, 3H), 1.69-1.66 (m, 1H), 1.62-1.53 (m, 1H), 1.43-1.34 (m, 11H), 0.84 (d, J = 6.8 Hz, 3H), 0.79 (d, J = 6.8 Hz, 3H).
MS (ESI) m/z: 651.35 [M+H]⁺

### (19-2-2) Synthesis of Pyrene (58)

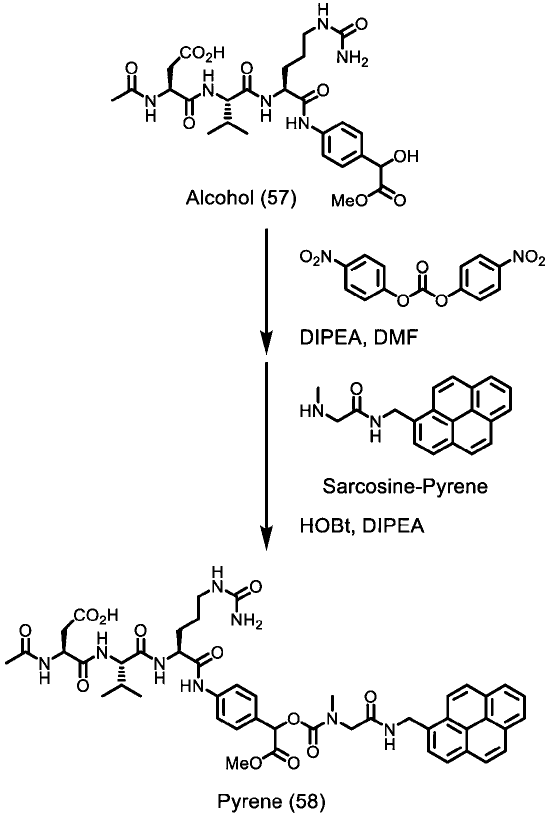

Alcohol (57) (55.0 mg, 84.5 µmol) was dissolved in N,N-dimethylformamide (423 µL) and stirred under ice cooling for 5 minutes, then bis(4-nitrophenyl) carbonate (77.1 mg, 254 µmol) and N,N-diisopropylethylamine (32.3 µL, 190 µmol) were added, and the mixture was stirred at room temperature for 2 hours. Subsequently, the mixture was cooled on ice, then Sarcosine-Pyrene (76.7 mg, 254 µmol), 1-hydroxybenzotriazole (17.1 mg, 127 µmol), and N,N-diisopropylethylamine (53.9 µL, 317 µmol) were added, and the mixture was stirred at room temperature for 1.5 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (58) (60.9 mg, 62.2 µmol).

¹H NMR (400 MHz, DMSO-d₆) δ10.14-10.11 (m, 1H), 8.68-8.66 (m, 1H), 8.39-8.01 (m, 10H), 7.89-7.85 (m, 1H), 7.67-7.58 (m, 3H), 7.44-7.39 (m, 2H), 5.98 (brs, 1H), 5.79-5.77 (m, 1H), 5.42 (brs, 2H), 5.04-4.97 (m, 2H), 4.65-4.59 (m, 1H), 4.39-4.36 (m, 1H), 4.28-4.07 (m, 2H), 3.92-3.82 (m, 1H), 3.62-3.61 (m, 3H), 2.99-2.91 (m, 5H), 2.69-2.63 (m, 1H), 2.44-2.38 (m, 1H), 2.01-1.97 (m, 1H), 1.83 (s, 3H), 1.70-1.60 (m, 2H), 1.44-1.30 (m, 11H), 0.86-0.77 (m, 6H).
MS (ESI) m/z: 979.45 [M+H]⁺

### (19-2-3) Synthesis of Pyrene (59)

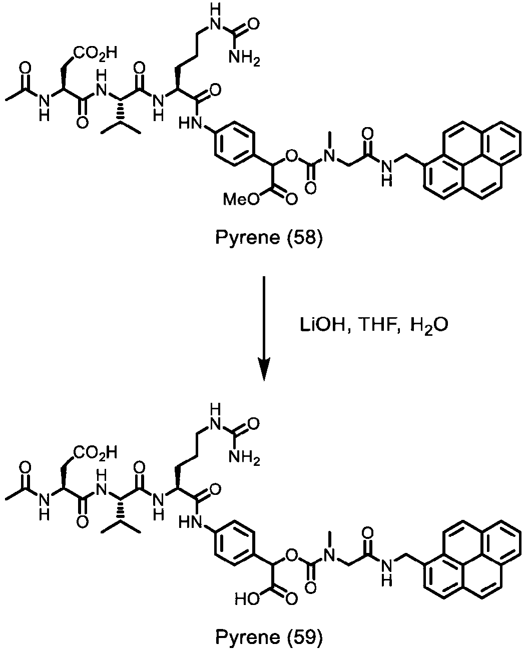

Pyrene (58) (24.9 mg, 25.4 µmol) was dissolved in tetrahydrofuran (1.88 mL) and water (625 µL) and stirred under ice cooling for 5 minutes, then 1 M aqueous lithium hydroxide solution (30.5 µL, 30.5 µmol) was added, and the mixture was stirred at room temperature for 50 minutes. After the reaction was completed, the pH was adjusted to about 6 with 0.1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (59) (8.3 mg, 8.6 µmol).

¹H NMR (400 MHz, DMSO-d₆) δ13.06 (brs, 1H), 10.13-10.09 (m, 1H), 8.68-8.65 (m, 1H), 8.39-8.00 (m, 10H), 7.88-7.84 (m, 1H), 7.67-7.59 (m, 3H), 7.44-7.41 (m, 2H), 5.98 (brs, 1H), 5.68-5.67 (m, 1H), 5.42 (brs, 2H), 5.04-4.93 (m, 2H), 4.64-4.61 (m, 1H), 4.41-4.35 (m, 1H), 4.32-4.09 (m, 2H), 3.91-3.80 (m, 1H), 2.99-2.91 (m, 5H), 2.70-2.64 (m, 1H), 2.44-2.38 (m, 1H), 2.01-1.98 (m, 1H), 1.83 (s, 3H), 1.70-1.57 (m, 2H), 1.45-1.30 (m, 11H), 0.86-0.77 (m, 6H).
MS (ESI) m/z: 965.45 [M+H]⁺

### (19-2-4) Synthesis of Pyrene (60)

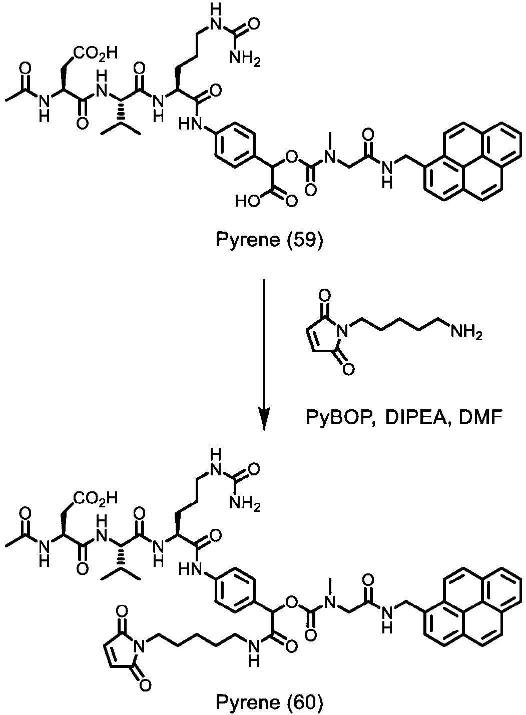

Pyrene (59) (7.2 mg, 7.5 µmol) was dissolved in N,N-dimethylformamide (150 µL) and cooled on ice, then N,N-diisopropylethylamine (2.5 µL, 14.9 µmol) and 1H-benzotriazol-1-yloxytripyrrolidinophosphonium hexafluorophosphate (5.8 mg, 11.2 µmol) were added. Next, N-(5-aminopentyl)maleimide hydrochloride (2.4 mg, 11.2 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (60) (5.7 mg, 5.1 µmol).

¹H NMR (400 MHz, DMSO-d₆) δ10.07-10.04 (m, 1H), 8.91-8.88 (m, 1H), 8.65-7.95 (m, 12H), 7.62-7.58 (m, 3H), 7.40-7.36 (m, 2H), 6.96-6.94 (m, 2H), 5.97 (brs, 1H), 5.70-5.68 (m, 1H), 5.41 (brs, 2H), 5.14-5.00 (m, 2H), 4.65-4.61 (m, 1H), 4.42-4.37 (m, 1H), 4.25-4.19 (m, 1H), 4.15-3.82 (m, 2H), 3.30-3.21 (m, 2H), 3.04-2.89 (m, 7H), 2.70-2.64 (m, 1H), 2.44-2.38 (m, 1H), 2.01-1.97 (m, 1H), 1.83 (s, 3H), 1.75-1.60 (m, 2H), 1.48-1.24 (m, 15H), 1.12-1.03 (m, 2H), 0.86-0.78 (m, 6H).
MS (ESI) m/z: 1129.50 [M+H]⁺

### (19-2-5) Synthesis of Linker-payload mimic (56)

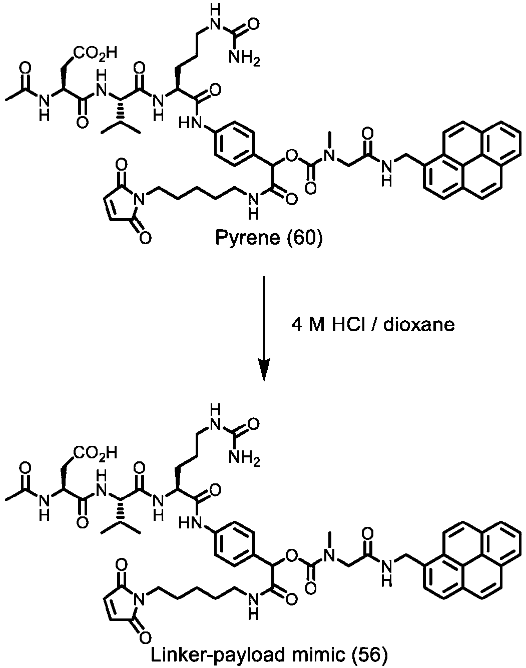

To Pyrene (60) (4.7 mg, 4.2 µmol) was added acetonitrile (208 µL), and 85% phosphoric acid solution (72.4 µL, 1.25 mmol) was added under ice cooling, and the mixture was stirred at room temperature for 3 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (56) (3.1 mg, 2.9 µmol).

¹H NMR (400 MHz, DMSO-d₆) δ12.38 (brs, 1H), 10.05-10.01 (m, 1H), 8.91-8.88 (m, 1H), 8.65-7.94 (m, 12H), 7.63-7.58 (m, 3H), 7.40-7.34 (m, 2H), 6.96-6.93 (m, 2H), 6.00 (brs, 1H), 5.70-5.68 (m, 1H), 5.44 (brs, 2H), 5.15-4.99 (m, 2H), 4.64-4.60 (m, 1H), 4.43-4.37 (m, 1H), 4.25-4.22 (m, 1H), 4.15-3.82 (m, 2H), 3.30-3.21 (m, 2H), 3.04-2.89 (m, 7H), 2.73-2.69 (m, 1H), 2.46-2.44 (m, 1H), 2.04-1.96 (m, 1H), 1.84-1.83 (m, 3H), 1.77-1.59 (m, 2H), 1.44-1.04 (m, 8H), 0.86-0.77 (m, 6H).
MS (ESI) m/z: 1073.50 [M+H]⁺

### (19-3) Synthesis of Linker-payload mimic (61)

Linker-payload mimic (1) was synthesized as follows.

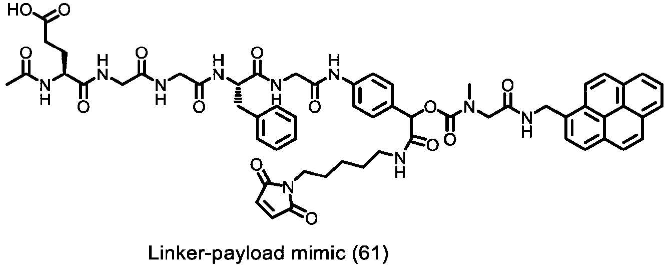

### (19-3-1) Synthesis of Alcohol (62)

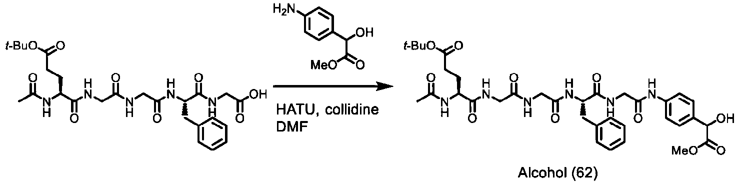

Ac-Glu(OtBu)-Gly-Gly-Phe-Gly-OH (52.6 mg, 88.7 µmol) was dissolved in N,N-dimethylformamide (650 µL), and 1-[bis(dimethylamino)methylene]-1H-1,2,3,triazolo[4,5-b]pyridinium 3-oxide hexafluorophosphate (40.5 mg, 106 µmol) and 2,4,6-trimethylpyridine (14.0 µL, 106 µmol) were added, followed by stirring at room temperature for 10 minutes. Subsequently, methyl 4-aminomandelate (19.3 mg, 106 µmol) was added, and the mixture was stirred at room temperature for 20 hours, followed by purification by preparative reversed-phase chromatography. Fractions containing the product were collected, and acetonitrile was removed by concentration under reduced pressure, followed by lyophilization to obtain the above-described Alcohol (62) (36.1 mg, 49.7 µmol).

¹H NMR (400 MHz, DMSO-d₆)δ 9.85 (s, 1H), 8.42-8.39 (m, 1H), 8.19-8.14 (m, 2H), 8.07-8.02 (m, 2H), 7.57 (d, J = 8.8 Hz, 2H), 7.33 (d, J = 8.8 Hz, 2H), 7.27 (d, J = 8.0 Hz, 4H), 7.21-7.16 (m, 1H), 5.08 (s, 1H), 4.54-4.48 (m, 1H), 4.25-4.20 (m, 1H), 3.94-3.82 (m, 1H), 3.79-3.61 (m, 4H), 3.60 (s, 3H), 3.09-3.04 (m, 1H), 2.85-2.79 (m, 1H), 2.23 (t, J = 8.0 Hz, 2H), 1.92-1.83 (m, 4H), 1.75-1.68 (m, 1H), 1.38 (s, 9H).
MS (ESI) m/z: 727.30 [M+H]⁺

### (19-3-2) Synthesis of Pyrene (63)

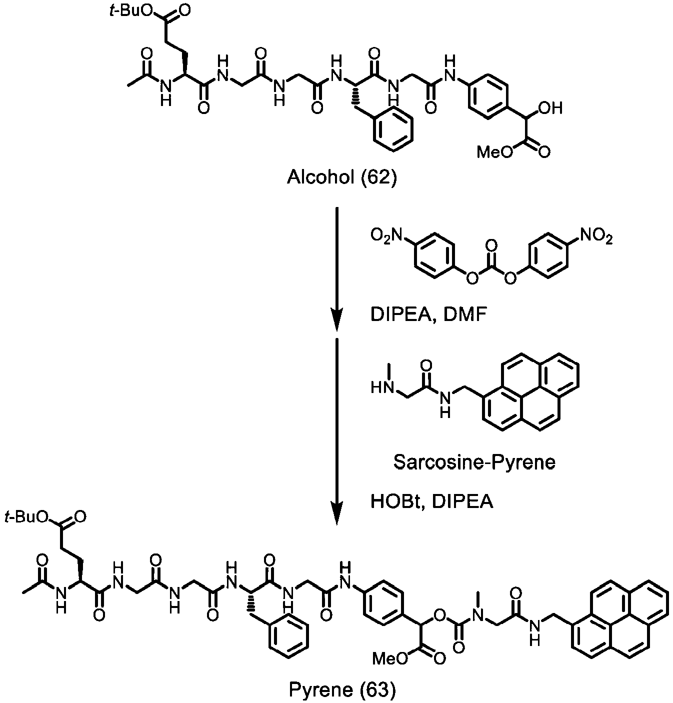

Alcohol (62) (35.0 mg, 48.2 µmol) obtained in 1-1-1 was dissolved in N,N-dimethylformamide (360 µL) and stirred under ice cooling for 5 minutes, then bis(4-nitrophenyl) carbonate (44.0 mg, 144 µmol) and N,N-diisopropylethylamine (18.4 µL, 108 µmol) were added, and the mixture was stirred at room temperature for 2 hours. Subsequently, the mixture was cooled on ice, then Sarcosine-Pyrene (43.7 mg, 144 µmol), 1-hydroxybenzotriazole (9.8 mg, 72.3 µmol), and N,N-diisopropylethylamine (30.7 µL, 181 µmol) were added, and the mixture was stirred at room temperature for 2 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (63) (34.9 mg, 33.0 µmol).

¹H NMR (400 MHz, DMSO-d₆) δ9.99-9.96 (m, 1H), 8.67-8.66 (m, 1H), 8.45-7.87 (m, 14H), 7.67-7.63 (m, 2H), 7.45-7.41 (m, 2H), 7.26-7.23 (m, 4H), 7.20-7.17 (m, 1H), 5.80-5.78 (m, 1H), 5.04-4.97 (m, 2H), 4.55-4.52 (m, 1H), 4.28-4.07 (m, 2H), 3.94-3.62 (m, 10H), 3.12-3.04 (m, 1H), 3.00-2.93 (m, 3H), 2.87-2.79 (m, 1H), 2.24-2.20 (m, 2H), 1.92-1.84 (m, 4H), 1.75-1.66 (m, 1H), 1.37-1.36 (m, 9H).
MS (ESI) m/z: 1055.40 [M+H]⁺

### (19-3-3) Synthesis of Pyrene (64)

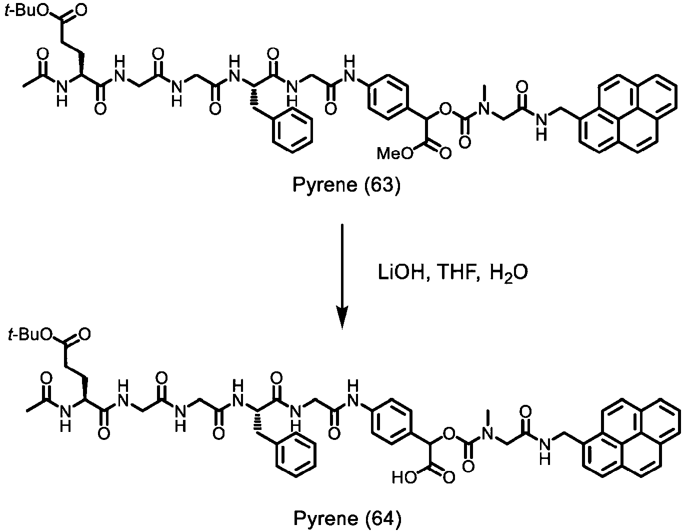

Pyrene (63) (18.2 mg, 17.2 µmol) was dissolved in tetrahydrofuran (1.27 mL) and water (0.43 mL) and stirred under ice cooling for 5 minutes, then 1 M aqueous lithium hydroxide solution (24.1 µL, 24.1 µmol) was added, and the mixture was stirred at room temperature for 2.5 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (64) (14.2 mg, 13.6 µmol).

¹H NMR (400 MHz, DMSO-d₆) δ13.04 (brs, 1H), 9.99-9.96 (m, 1H), 8.68-8.66 (m, 1H), 8.47-7.86 (m, 14H), 7.66-7.64 (m, 2H), 7.45-7.42 (m, 2H), 7.26-7.23 (m, 4H), 7.19-7.17 (m, 1H), 5.69-5.68 (m, 1H), 5.04-4.96 (m, 2H), 4.54-4.52 (m, 1H), 4.32-4.09 (m, 2H), 3.94-3.58 (m, 7H), 3.12-3.05 (m, 1H), 3.00-2.93 (m, 3H), 2.87-2.79 (m, 1H), 2.24-2.20 (m, 2H), 1.92-1.84 (m, 4H), 1.78-1.66 (m, 1H), 1.37-1.36 (m, 9H).
MS (ESI) m/z: 1041.45 [M+H]⁺

### (19-3-4) Synthesis of Pyrene (65)

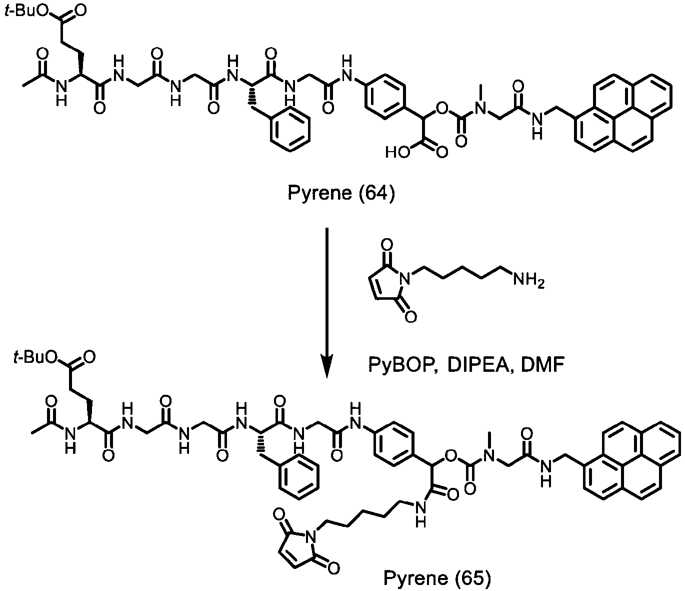

Pyrene (64) (12.4 mg, 11.9 µmol) was dissolved in N,N-dimethylformamide (240 µL) and cooled on ice, then N,N-diisopropylethylamine (4.0 µL, 23.8 µmol) and 1H-benzotriazol-1-yloxytripyrrolidinophosphonium hexafluorophosphate (9.3 mg, 17.9 µmol) were added. Next, N-(5-aminopentyl)maleimide hydrochloride (3.9 mg, 17.9 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (65) (8.4 mg, 7.0 µmol).

¹H NMR (400 MHz, DMSO-d₆) δ9.94-9.91 (m, 1H), 8.91-8.88 (m, 1H), 8.63-7.95 (m, 15H), 7.62-7.59 (m, 2H), 7.42-7.35 (m, 2H), 7.27-7.23 (m, 4H), 7.20-7.16 (m, 1H), 6.96-6.93 (m, 2H), 5.71-5.69 (m, 1H), 5.14-4.99 (m, 2H), 4.58-4.52 (m, 1H), 4.25-3.60 (m, 9H), 3.30-3.20 (m, 2H), 3.12-2.79 (m, 7H), 2.24-2.20 (m, 2H), 1.92-1.84 (m, 4H), 1.75-1.66 (m, 1H), 1.41-1.24 (m, 13H), 1.11-1.03 (m, 2H).
MS (ESI) m/z: 1205.50 [M+H]⁺

### (19-3-5) Synthesis of Linker-payload mimic (61)

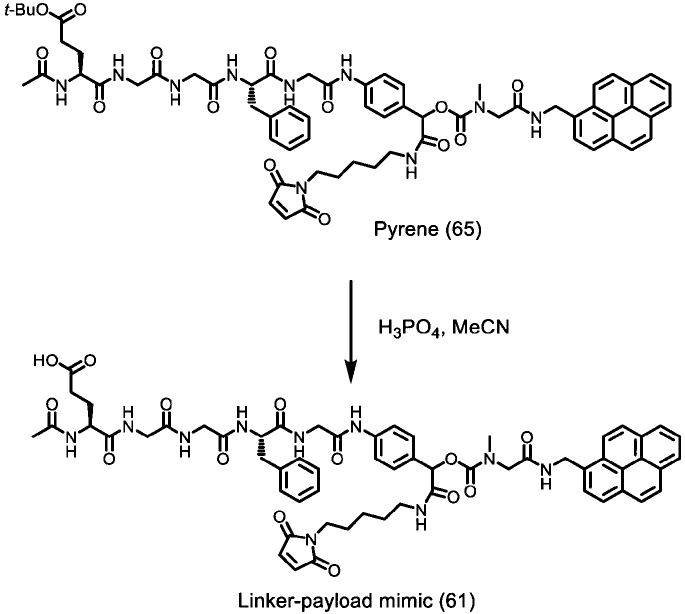

To Pyrene (65) (8.3 mg, 6.89 µmol) was added acetonitrile (345 µL), and 85% phosphoric acid solution (120 µL, 2.07 mmol) was added under ice cooling, and the mixture was stirred at room temperature for 3 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (61) (4.0 mg, 3.48 µmol).

¹H NMR (400 MHz, DMSO-d₆) δ12.12 (brs, 1H), 9.94-9.91 (m, 1H), 8.91-8.88 (m, 1H), 8.65-7.96 (m, 15H), 7.62-7.59 (m, 2H), 7.42-7.35 (m, 2H), 7.27-7.23 (m, 4H), 7.20-7.16 (m, 1H), 6.96-6.93 (m, 2H), 5.71-5.69 (m, 1H), 5.14-4.99 (m, 2H), 4.58-4.52 (m, 1H), 4.26-3.61 (m, 9H), 3.30-3.20 (m, 2H), 3.12-2.79 (m, 7H), 2.27-2.23 (m, 2H), 1.94-1.84 (m, 4H), 1.77-1.67 (m, 1H), 1.41-1.30 (m, 4H), 1.11-1.03 (m, 2H).
MS (ESI) m/z: 1149.45 [M+H]⁺

### (19-4) Synthesis of Linker-payload mimic (66)

Linker-payload mimic (66) was synthesized as follows.

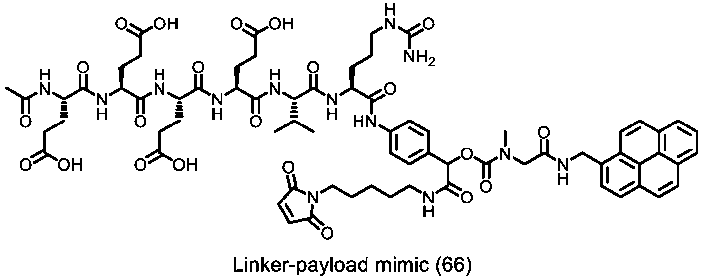

### (19-4-1) Synthesis of Alcohol (67)

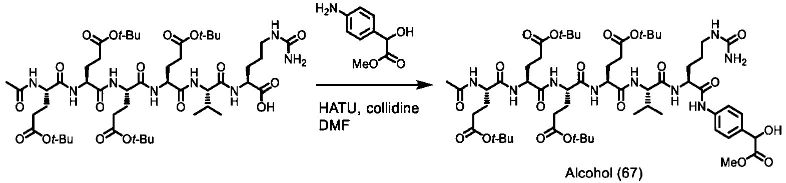

Ac-Glu(OtBu)-Glu(OtBu)-Glu(OtBu)-Phe-Glu(OtBu)-OH (50.2 mg, 47.3 µmol) was dissolved in N,N-dimethylformamide (237 µL), and 1-[bis(dimethylamino)methylene]-1H-1,2,3,triazolo[4,5-b]pyridinium 3-oxide hexafluorophosphate (21.6 mg, 56.8 µmol) and 2,4,6-trimethylpyridine (7.48 µL, 56.8 µmol) were added, followed by stirring at room temperature for 10 minutes. Subsequently, methyl 4-aminomandelate (10.3 mg, 56.8 µmol) was added, and the mixture was stirred at room temperature for 20 hours, followed by purification by preparative reversed-phase chromatography. Fractions containing the product were collected, and acetonitrile was removed by concentration under reduced pressure, followed by lyophilization to obtain the above-described Alcohol (67) (47.7 mg, 39.1 µmol).
MS (ESI) m/z: 1220.65 [M+H]⁺

### (19-4-2) Synthesis of Pyrene (68)

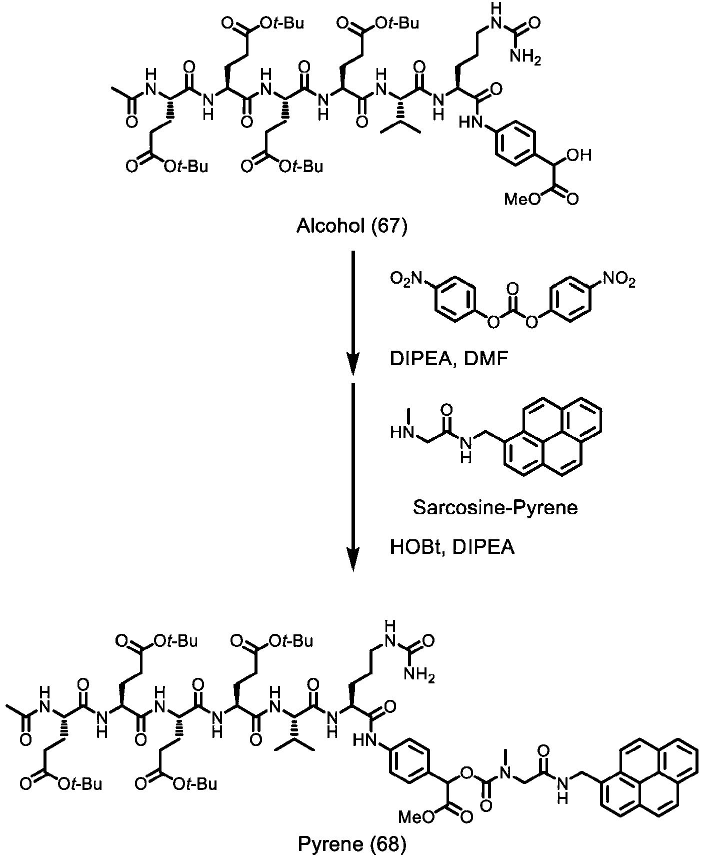

Alcohol (67) (46.3 mg, 37.9 µmol) obtained in 1-1-1 was dissolved in N,N-dimethylformamide (380 µL) and stirred under ice cooling for 5 minutes, then bis(4-nitrophenyl) carbonate (34.6 mg, 114 µmol) and N,N-diisopropylethylamine (14.5 µL, 85.3 µmol) were added, and the mixture was stirred at room temperature for 2 hours. Subsequently, the mixture was cooled on ice, then Sarcosine-Pyrene (34.5 mg, 114 µmol), 1-hydroxybenzotriazole (7.7 mg, 56.9 µmol), and N,N-diisopropylethylamine (24.2 µL, 142 µmol) were added, and the mixture was stirred at room temperature for 2 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (68) (37.1 mg, 24.0 µmol).
MS (ESI) m/z: 775.30 [M+H]⁺

### (19-4-3) Synthesis of Pyrene (69)

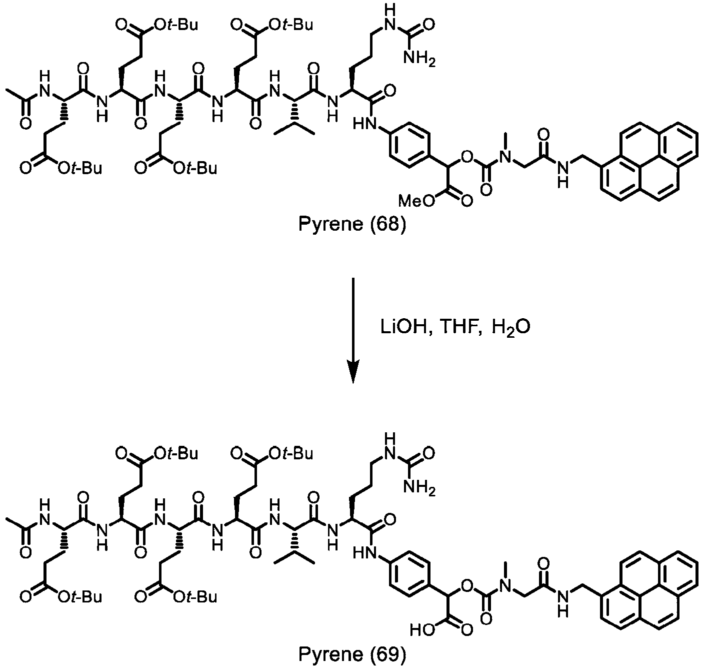

Pyrene (68) (20.3 mg, 13.1 µmol) was dissolved in tetrahydrofuran (983 µL) and water (327 µL) and stirred under ice cooling for 5 minutes, then 1 M aqueous lithium hydroxide solution (31.4 µL, 31.4 µmol) was added, and the mixture was stirred at room temperature for 2.5 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (69) (16.7 mg, 10.9 µmol).
MS (ESI) m/z: 1534.85 [M+H]⁺

### (19-4-4) Synthesis of Pyrene (70)

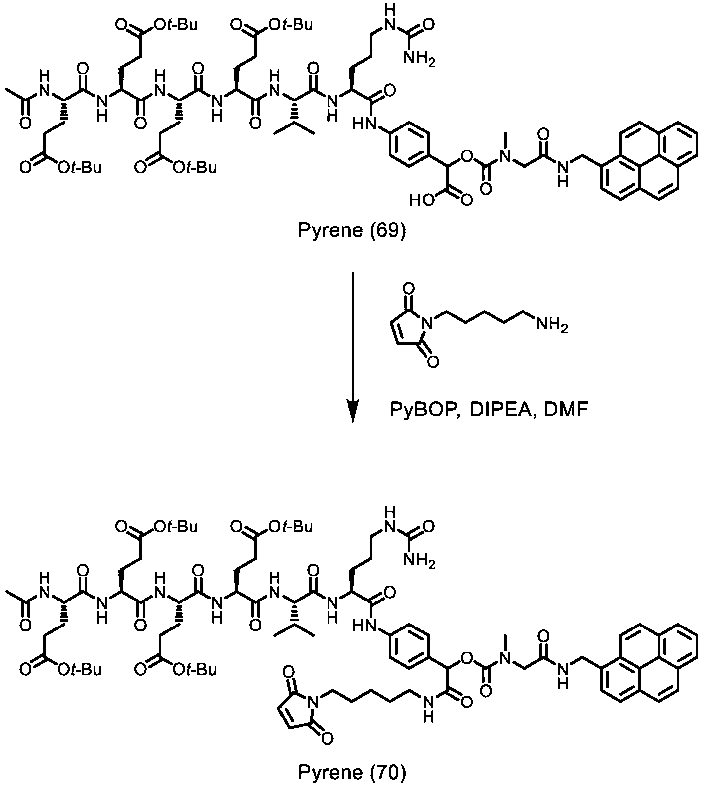

Pyrene (69) (14.9 mg, 9.71 µmol) was dissolved in N,N-dimethylformamide (486 µL) and cooled on ice, then N,N-diisopropylethylamine (3.3 µL, 19.4 µmol) and 1H-benzotriazol-1-yloxytripyrrolidinophosphonium hexafluorophosphate (7.6 mg, 14.6 µmol) were added. Next, N-(5-aminopentyl)maleimide hydrochloride (3.2 mg, 14.6 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (70) (13.1 mg, 7.71 µmol).
MS (ESI) m/z: 1698.90 [M+H]⁺

### (19-4-5) Synthesis of Linker-payload mimic (66)

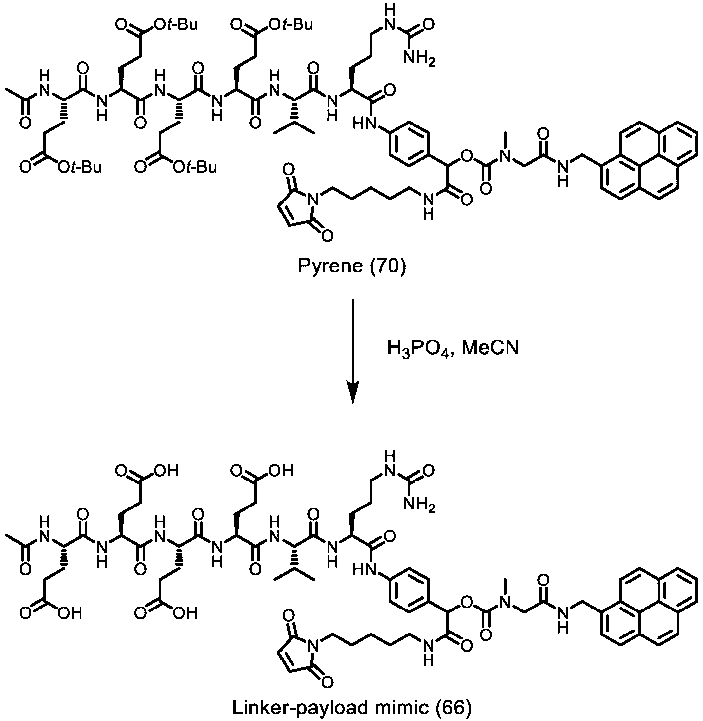

To Pyrene (70) (5.25 mg, 3.09 µmol) was added acetonitrile (309 µL), and 85% phosphoric acid solution (53.8 µL, µ927mol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (66) (3.7 mg, 2.51 µmol).
MS (ESI) m/z: 1474.00 [M+H]⁺

### (19-5) Synthesis of Linker-payload mimic (71)

Linker-payload mimic (71) was synthesized as follows.

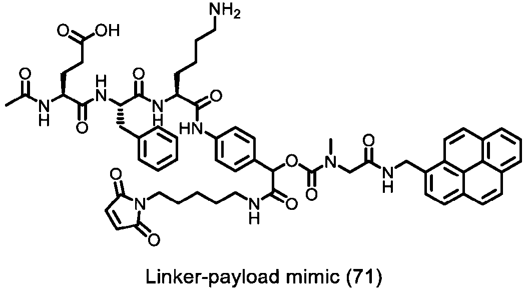

### (19-5-1) Synthesis of Alcohol (72)

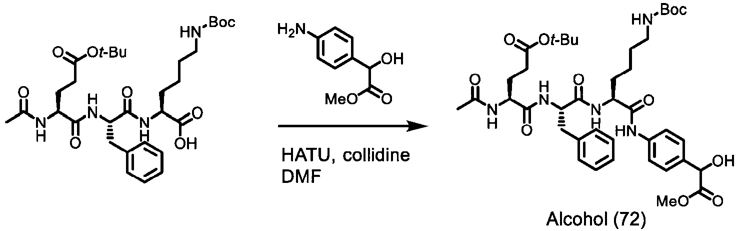

Ac-Glu(OtBu)-Phe-Lys-OH (200 mg, 322 µmol) was dissolved in N,N-dimethylformamide (1.61 mL), and 1-[bis(dimethylamino)methylene]-1H-1,2,3,triazolo[4,5-b]pyridinium 3-oxide hexafluorophosphate (147 mg, 387 µmol) and 2,4,6-trimethylpyridine (51.0 µL, 387 µmol) were added, followed by stirring at room temperature for 10 minutes. Subsequently, methyl 4-aminomandelate (70.1 mg, 387 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (72) (196 mg, 250 µmol).
MS (ESI) m/z: 784.35 [M+H]⁺

### (19-5-2) Synthesis of Pyrene (73)

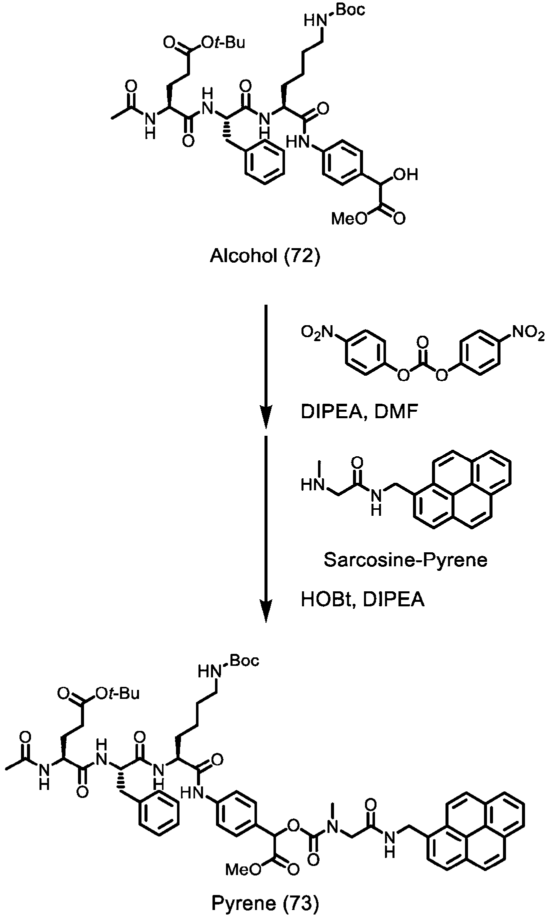

Alcohol (72) (40.1 mg, 51.2 µmol) was dissolved in N,N-dimethylformamide (512 µL) and stirred under ice cooling for 5 minutes, then bis(4-nitrophenyl) carbonate (46.7 mg, 153 µmol) and N,N-diisopropylethylamine (22.0 µL, 115 µmol) were added, and the mixture was stirred at room temperature for 2 hours and 20 minutes. Subsequently, the mixture was cooled on ice, then Sarcosine-Pyrene (46.4 mg, 153 µmol), 1-hydroxybenzotriazole (10.4 mg, 76.7 µmol), and N,N-diisopropylethylamine (33.0 µL, 192 µmol) were added, and the mixture was stirred at room temperature for 3 hours. After the reaction, the reaction mixture was purified by normal-phase preparative chromatography. Fractions containing the product were collected, and dichloromethane and methanol were removed by concentration under reduced pressure to obtain the above-described Pyrene (73) (52.1 mg, 46.8 µmol).
MS (ESI) m/z: 1112.55 [M+H]⁺

### (19-5-3) Synthesis of Pyrene (74)

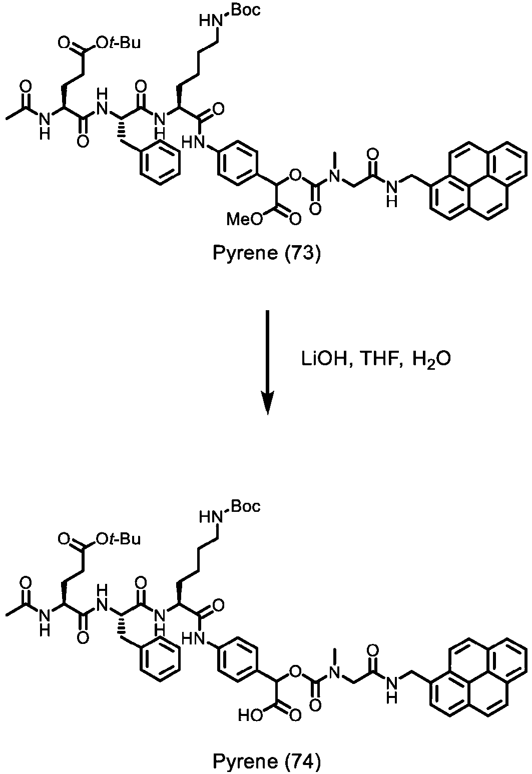

Pyrene (73) (51.0 mg, 45.9 µmol) was dissolved in tetrahydrofuran (3.45 mL) and water (1.15 mL) and stirred under ice cooling for 5 minutes, then 1 M aqueous lithium hydroxide solution (110 µL, 110 µmol) was added, and the mixture was stirred at room temperature for 2 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (74) (30.4 mg, 27.7 µmol).
MS (ESI) m/z: 1098.55 [M+H]⁺

### (19-5-4) Synthesis of Pyrene (75)

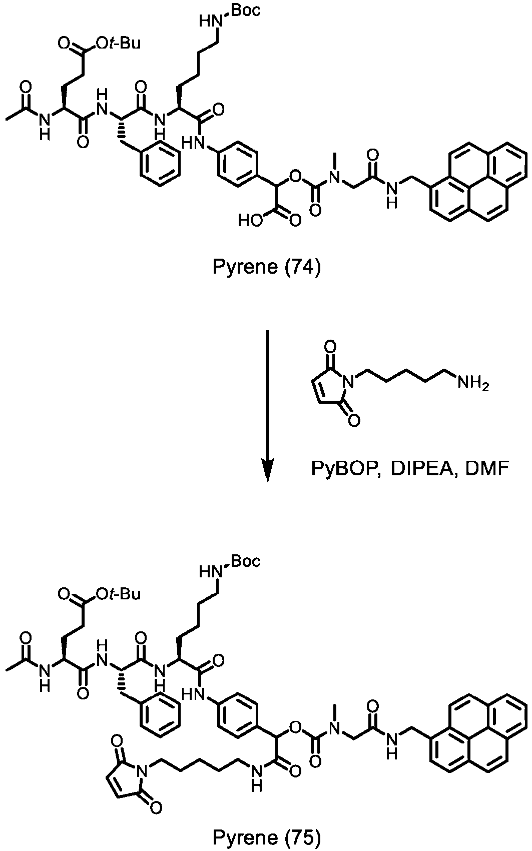

Pyrene (74) (23.0 mg, 20.9 µmol) was dissolved in N,N-dimethylformamide (1.05 mL) and cooled on ice, then N,N-diisopropylethylamine (7.1 µL, 41.9 µmol) and 1H-benzotriazol-1-yloxytripyrrolidinophosphonium hexafluorophosphate (16.3 mg, 31.4 µmol) were added. Next, N-(5-aminopentyl)maleimide hydrochloride (6.87 mg, 31.4 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (75) (1.4 mg, 1.11 µmol).
MS (ESI) m/z: 1265.80 [M+H]⁺

### (19-5-5) Synthesis of Linker-payload mimic (71)

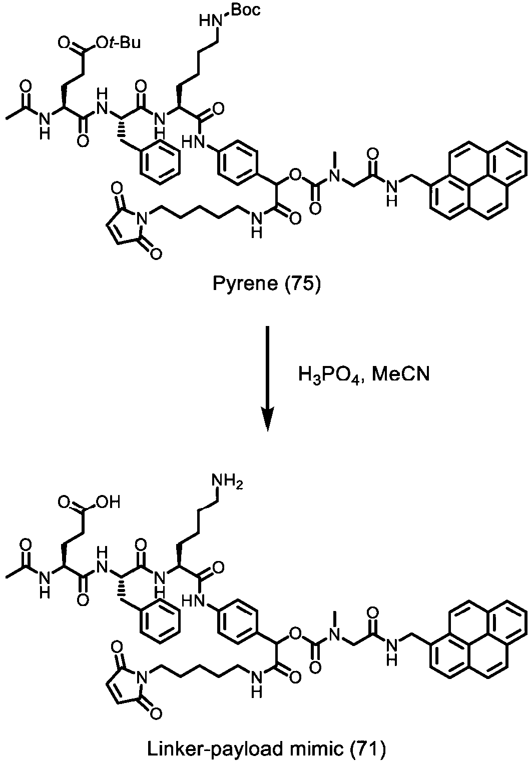

To Pyrene (75) (1.40 mg, 1.11 µmol) was added acetonitrile (55.4 µL), and 85% phosphoric acid solution (333 µL, 22.7 µmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (71) (0.6 mg, 0.54 µmol).
MS (ESI) m/z: 1106.60 [M+H]⁺

### (19-6) Synthesis of Linker-payload mimic (76)

Linker-payload mimic (76) was synthesized as follows.

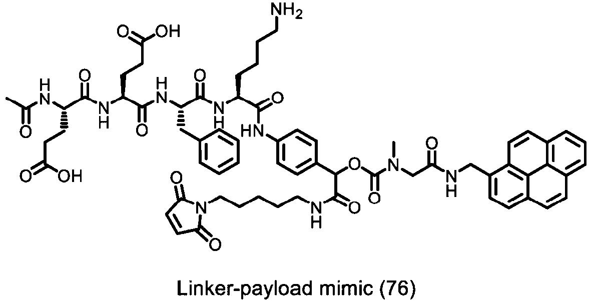

### (19-6-1) Synthesis of Alcohol (77)

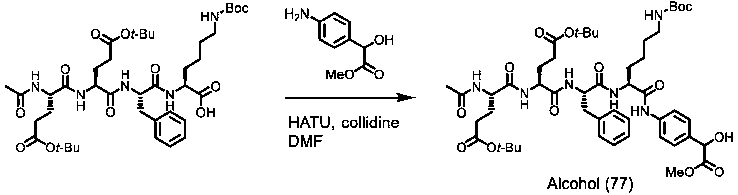

Ac-Glu(OtBu)-Glu(OtBu)-Phe-Lys-OH (52.1 mg, 64.6 µmol) was dissolved in N,N-dimethylformamide (323 µL), and 1-[bis(dimethylamino)methylene]-1H-1,2,3,triazolo[4,5-b]pyridinium 3-oxide hexafluorophosphate (29.5 mg, 77.6 µmol) and 2,4,6-trimethylpyridine (10.2 µL, 77.6 µmol) were added, followed by stirring at room temperature for 10 minutes. Subsequently, methyl 4-aminomandelate (13.9 mg, 77.6 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (77) (53.6 mg, 55.3 µmol).
MS (ESI) m/z: 969.55 [M+H]⁺

### (19-6-2) Synthesis of Pyrene (78)

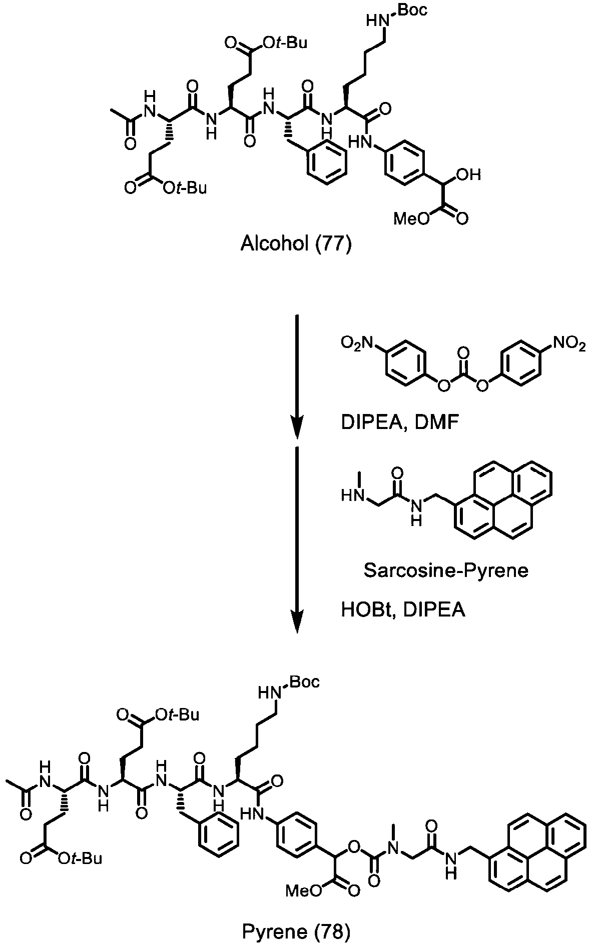

Alcohol (77) (52.3 mg, 54.0 µmol) was dissolved in N,N-dimethylformamide (540 µL) and stirred under ice cooling for 5 minutes, then bis(4-nitrophenyl) carbonate (49.2 mg, 162 µmol) and N,N-diisopropylethylamine (20.7 µL, 122 µmol) were added, and the mixture was stirred at room temperature for 3 hours. Subsequently, the mixture was cooled on ice, then Sarcosine-Pyrene (98.0 mg, 324 µmol), 1-hydroxybenzotriazole (10.9 mg, 81.0 µmol), and N,N-diisopropylethylamine (89.5 µL, 524 µmol) were added, and the mixture was stirred at room temperature for 1.5 hours. After the reaction, the reaction mixture was purified by preparative reversed-phase chromatography. Fractions containing the product were collected, and acetonitrile was removed by concentration under reduced pressure, followed by lyophilization to obtain the above-described Pyrene (78) (51.3 mg, 39.5 µmol).
MS (ESI) m/z: 1297.65 [M+H]⁺

### (19-6-3) Synthesis of Pyrene (79)

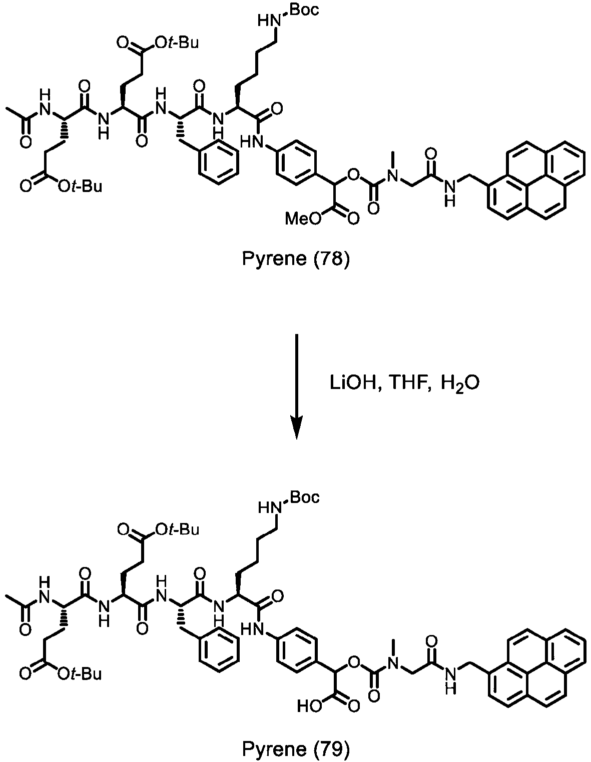

Pyrene (78) (28.5 mg, 22.0 µmol) was dissolved in tetrahydrofuran (1.65 mL) and water (550 µL) and stirred under ice cooling for 5 minutes, then 1 M aqueous lithium hydroxide solution (52.8 µL, 52.8 µmol) was added, and the mixture was stirred at room temperature for 4 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (79) (22.6 mg, 17.6 µmol).
MS (ESI) m/z: 1283.60 [M+H]⁺

### (19-6-4) Synthesis of Pyrene (80)

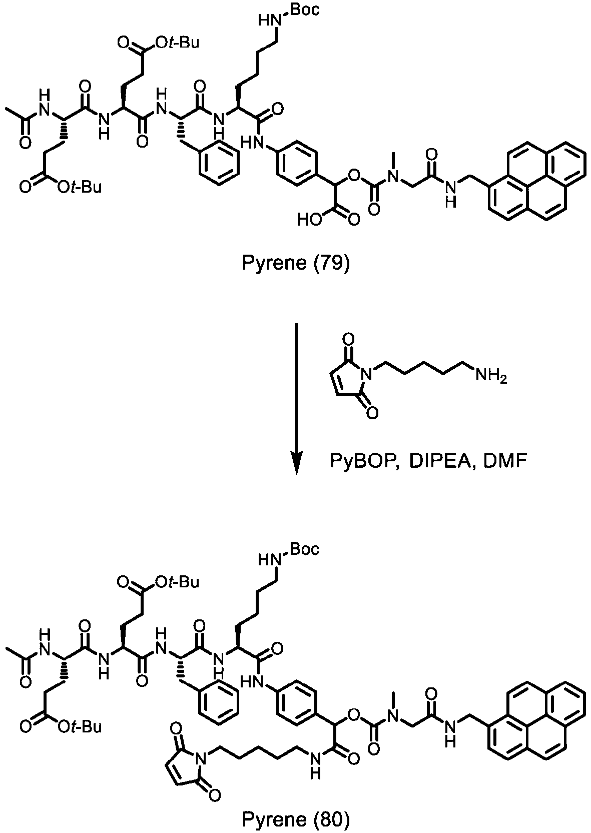

Pyrene (79) (12.4 mg, 9.66 µmol) was dissolved in N,N-dimethylformamide (326 µL) and cooled on ice, then 2,4,6-trimethylpyridine (4.0 µL, 30.3 µmol) and 1-[bis(dimethylamino)methylene]-1H-1,2,3,triazolo[4,5-b]pyridinium 3-oxide hexafluorophosphate (5.5 mg, 14.5 µmol) were added. Next, N-(5-aminopentyl)maleimide hydrochloride (3.1 mg, 14.1 µmol) was added, and the mixture was returned to room temperature and stirred for 2 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (80) (6.3 mg, 4.35 µmol).
MS (ESI) m/z: 1448.65 [M+H]⁺

### (19-6-5) Synthesis of Linker-payload mimic (76)

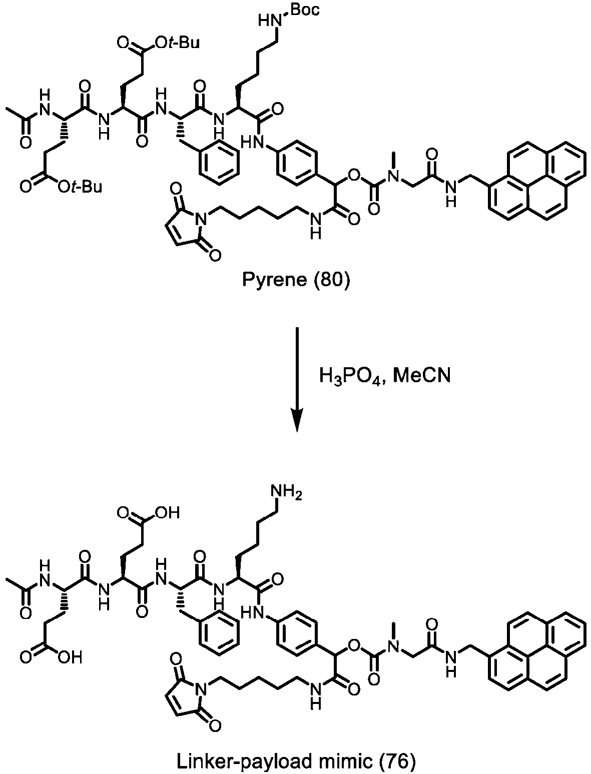

To Pyrene (80) (5.3 mg, 3.60 µmol) was added acetonitrile (370 µL), and 85% phosphoric acid solution (125 µL, 180 µmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (76) (4.2 mg, 3.39 µmol).
MS (ESI) m/z: 1235.55 [M+H]⁺

### (19-7) Synthesis of Linker-payload mimic (81)

Linker-payload mimic (81) was synthesized as follows.

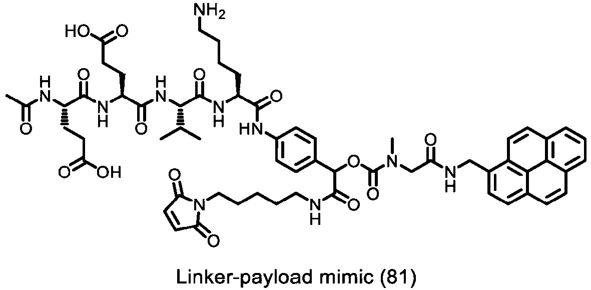

### (19-6-1) Synthesis of Alcohol (82)

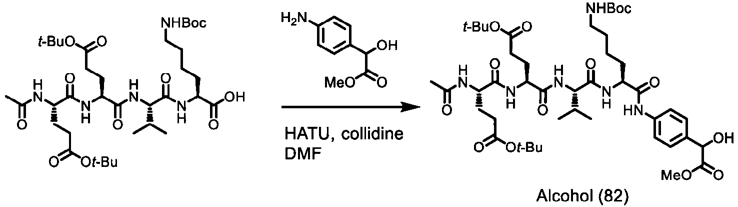

Ac-Glu(OtBu)-Glu(OtBu)-Val-Lys-OH (41.0 mg, 54.1 µmol) was dissolved in N,N-dimethylformamide (270 µL), and 1-[bis(dimethylamino)methylene]-1H-1,2,3,triazolo[4,5-b]pyridinium 3-oxide hexafluorophosphate (24.7 mg, 64.9 µmol) and 2,4,6-trimethylpyridine (8.5 µL, 64.9 µmol) were added, followed by stirring at room temperature for 10 minutes. Subsequently, methyl 4-aminomandelate (11.8 mg, 64.9 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (82) (43.9 mg, 47.7 µmol).
MS (ESI) m/z: 921.55 [M+H]⁺

### (19-7-2) Synthesis of Pyrene (83)

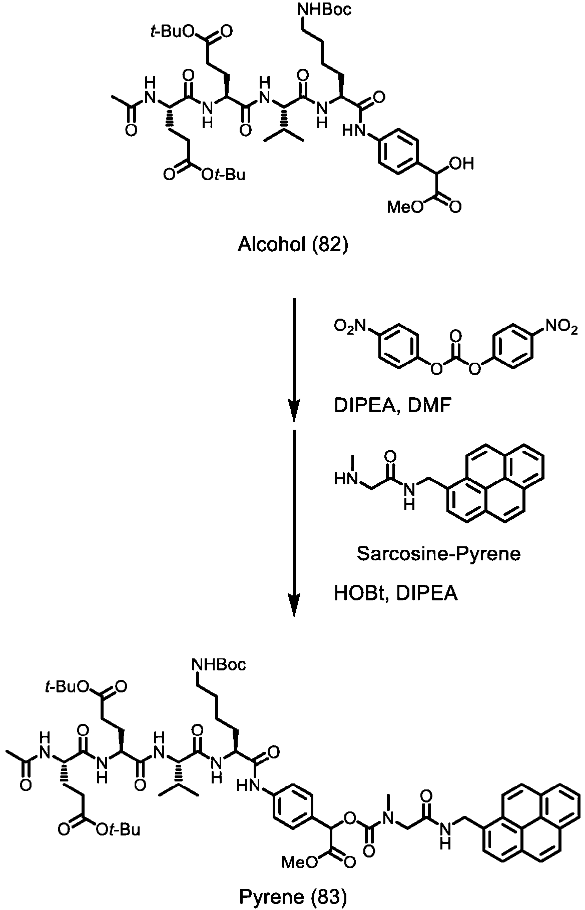

Alcohol (82) (42.1 mg, 45.7 µmol) was dissolved in N,N-dimethylformamide (457 µL) and stirred under ice cooling for 5 minutes, then bis(4-nitrophenyl) carbonate (41.7 mg, 137 µmol) and N,N-diisopropylethylamine (17.5 µL, 103 µmol) were added, and the mixture was stirred at room temperature for 1 hour and 10 minutes. Subsequently, the mixture was cooled on ice, then Sarcosine-Pyrene (41.5 mg, 137 µmol), 1-hydroxybenzotriazole (9.3 mg, 68.6 µmol), and N,N-diisopropylethylamine (29.1 µL, 171 µmol) were added, and the mixture was stirred at room temperature for 2 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (83) (54.8 mg, 43.9 µmol).
MS (ESI) m/z: 1249.65 [M+H]⁺

### (19-7-3) Synthesis of Pyrene (84)

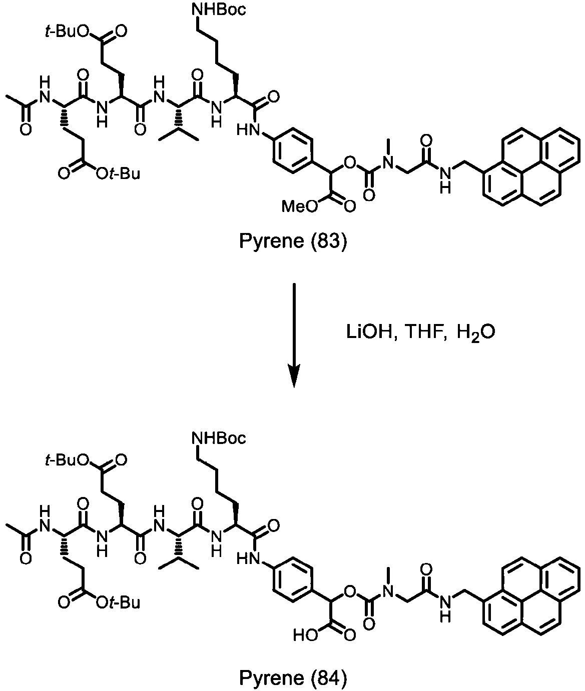

Pyrene (83) (30.0 mg, 24.0 µmol) was dissolved in tetrahydrofuran (1.8 mL) and water (0.6 mL) and stirred under ice cooling for 5 minutes, then 1 M aqueous lithium hydroxide solution (57.6 µL, 57.6 µmol) was added, and the mixture was stirred at room temperature for 2 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (84) (21.4 mg, 17.3 µmol).
MS (ESI) m/z: 1235.65 [M+H]⁺

### (19-7-4) Synthesis of Pyrene (95)

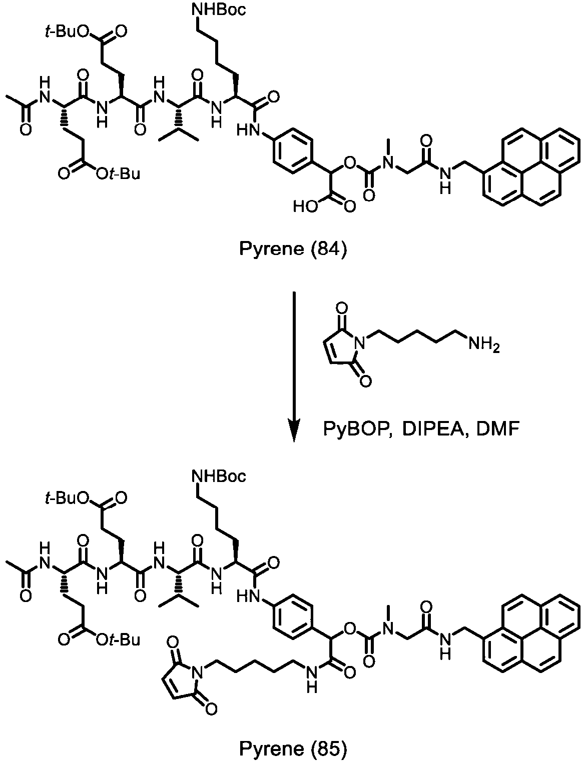

Pyrene (94) (20.6 mg, 16.7 µmol) was dissolved in N,N-dimethylformamide (835 µL) and cooled on ice, then N,N-diisopropylethylamine (5.7 µL, 33.4 µmol) and 1H-benzotriazol-1-yloxytripyrrolidinophosphonium hexafluorophosphate (13.0 mg, 25.0 µmol) were added. Next, N-(5-aminopentyl)maleimide hydrochloride (5.47 mg, 25.0 µmol) was added, and the mixture was returned to room temperature and stirred for 1.5 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (95) (16.7 mg, 1.19 µmol).
MS (ESI) m/z: 1399.80 [M+H]⁺

### (19-7-5) Synthesis of Linker-payload mimic (81)

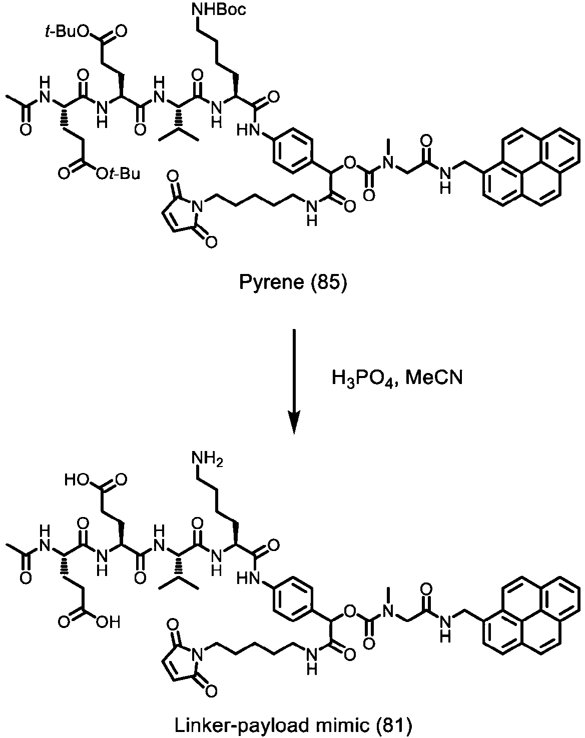

To Pyrene (85) (6.9 mg, 4.93 µmol) was added acetonitrile (490 µL), and 85% phosphoric acid solution (168 µL, 2.90 mmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (81) (4.1 mg, 3.45 µmol).
MS (ESI) m/z: 1187.55 [M+H]⁺

### (19-8) Synthesis of Linker-payload mimic (86)

Linker-payload mimic (86) was synthesized as follows.

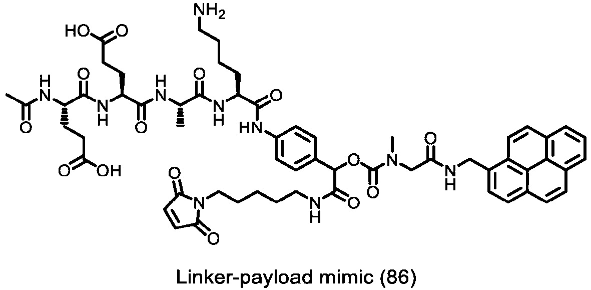

### (19-8-1) Synthesis of Alcohol (87)

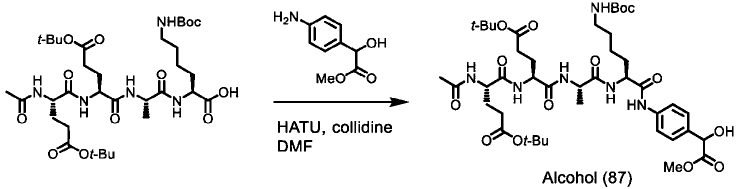

Ac-Glu(OtBu)-Glu(OtBu)-Ala-Lys-OH (40.7 mg, 55.8 µmol) was dissolved in N,N-dimethylformamide (280 µL), and 1-[bis(dimethylamino)methylene]-1H-1,2,3,triazolo[4,5-b]pyridinium 3-oxide hexafluorophosphate (25.4 mg, 66.9 µmol) and 2,4,6-trimethylpyridine (8.8 µL, 66.9 µmol) were added, followed by stirring at room temperature for 10 minutes. Subsequently, methyl 4-aminomandelate (12.1 mg, 66.9 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (87) (41.3 mg, 46.2 µmol).
MS (ESI) m/z: 893.50 [M+H]⁺

### (19-8-2) Synthesis of Pyrene (88)

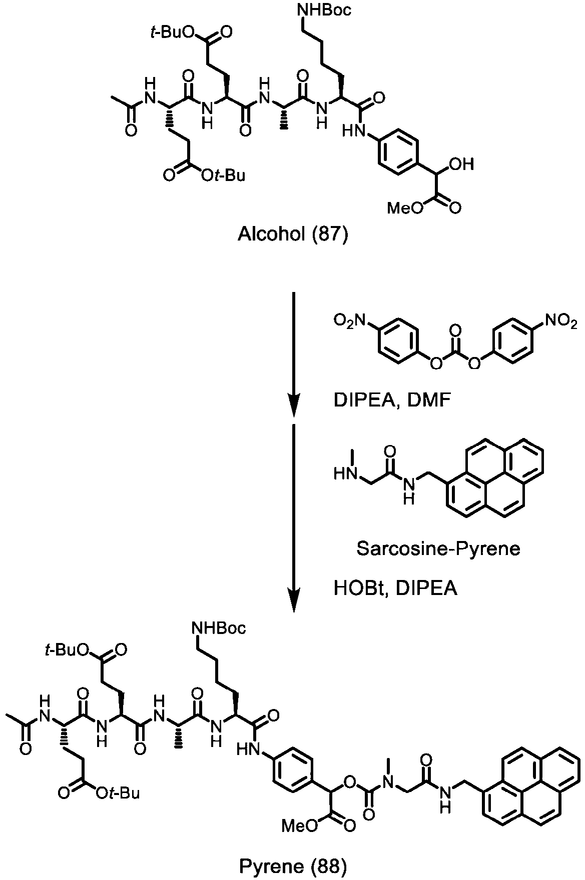

Alcohol (87) (40.3 mg, 45.1 µmol) was dissolved in N,N-dimethylformamide (451 µL) and stirred under ice cooling for 5 minutes, then bis(4-nitrophenyl) acid ester (41.2 mg, 135 µmol), N,N-diisopropylethylamine (17.3 µL, 101 µmol) were added, and the mixture was stirred at room temperature for 2 hours and 10 minutes. Subsequently, the mixture was cooled on ice, then Sarcosine-Pyrene (40.9 mg, 135 µmol), 1-hydroxybenzotriazole (9.1 mg, 67.7 µmol) and N,N-diisopropylethylamine (28.8 µL, 169 µmol) were added, and the mixture was stirred at room temperature for 2 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (88) (51.7 mg, 42.3 µmol).
MS (ESI) m/z: 1221.60 [M+H]⁺

### (19-8-3) Synthesis of Pyrene (89)

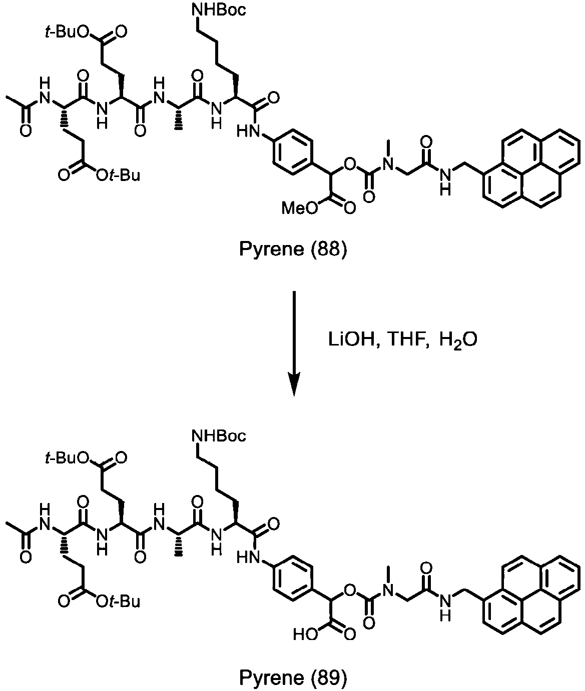

Pyrene (88) (26.1 mg, 21.4 µmol) was dissolved in tetrahydrofuran (1.60 mL) and water (0.53 mL) and stirred under ice cooling for 5 minutes, then 1 M aqueous lithium hydroxide solution (57.6 µL, 57.6 µmol) was added, and the mixture was stirred at room temperature for 4 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (89) (18.1 mg, 15.0 µmol).
MS (ESI) m/z: 1207.60 [M+H]⁺

### (19-8-4) Synthesis of Pyrene (90)

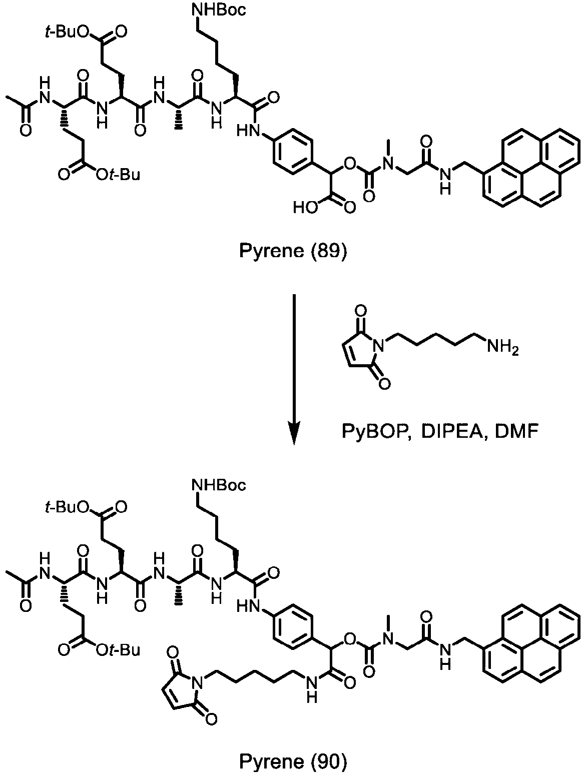

Pyrene (89) (17.3 mg, 14.3 µmol) was dissolved in N,N-dimethylformamide (715 µL) and cooled on ice, then N,N-diisopropylethylamine (4.9 µL, 28.6 µmol) and 1H-benzotriazol-1-yloxytripyrrolidinophosphonium hexafluorophosphate (11.2 mg, 21.5 µmol) were added. Next, N-(5-aminopentyl)maleimide hydrochloride (4.7 mg, 21.5 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (90) (8.4 mg, 6.12 µmol).
MS (ESI) m/z: 1371.70 [M+H]⁺

### (19-8-5) Synthesis of Linker-payload mimic (86)

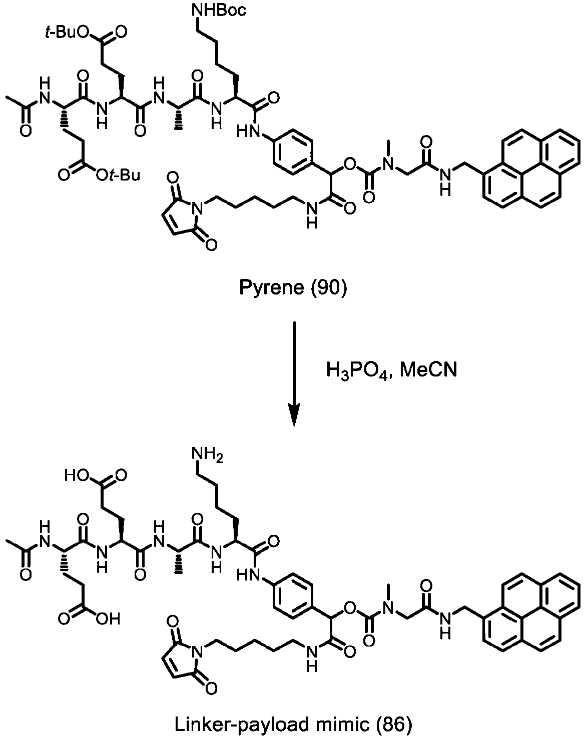

To Pyrene (90) (5.7 mg, 4.16 µmol) was added acetonitrile (420 µL), and 85% phosphoric acid solution (142 µL, 2.45 mmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (86) (3.2 mg, 2.76 µmol).
MS (ESI) m/z: 1159.55 [M+H]⁺

### (19-9) Synthesis of Linker-payload mimic (91)

Linker-payload mimic (91) was synthesized as follows.

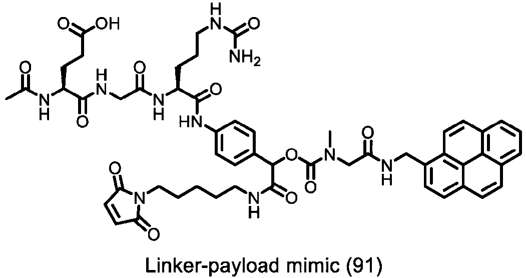

### (19-9-1) Synthesis of Alcohol (92)

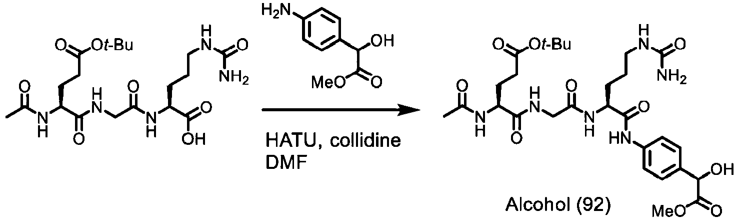

Ac-Glu(OtBu)-Gly-Cit-OH (50.4 mg, 110 µmol) was dissolved in N,N-dimethylformamide (548 µL), and 1-[bis(dimethylamino)methylene]-1H-1,2,3,triazolo[4,5-b]pyridinium 3-oxide hexafluorophosphate (50.0 mg, 132 µmol) and 2,4,6-trimethylpyridine (17.0 µL, 132 µmol) were added, followed by stirring at room temperature for 10 minutes. Subsequently, methyl 4-aminomandelate (23.8 mg, 132 µmol) was added, and the mixture was stirred at room temperature for 21 hours, followed by purification by preparative reversed-phase chromatography. Fractions containing the product were collected, and acetonitrile was removed by concentration under reduced pressure, followed by lyophilization to obtain the above-described Alcohol (92) (44.0 mg, 70.7 µmol).
MS (ESI) m/z: 623.35 [M+H]⁺

### (19-9-2) Synthesis of Pyrene (93)

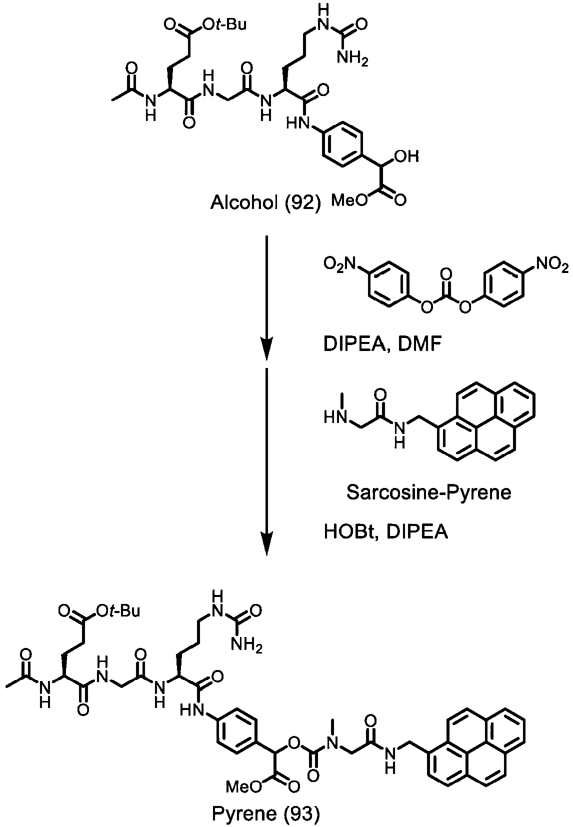

Alcohol (92) (15.3 mg, 24.6 µmol) was dissolved in N,N-dimethylformamide (246 µL) and stirred under ice cooling for 5 minutes, then bis(4-nitrophenyl) carbonate (22.4 mg, 73.7 µmol) and N,N-diisopropylethylamine (9.4 µL, 55.3 µmol) were added, and the mixture was stirred at room temperature for 2 hours and 20 minutes. Subsequently, the mixture was cooled on ice, then Sarcosine-Pyrene (22.3 mg, 73.7 µmol), 1-hydroxybenzotriazole (5.02 mg, 36.9 µmol), and N,N-diisopropylethylamine (16.0 µL, 92.1 µmol) were added, and the mixture was stirred at room temperature for 4 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (93) (17.5 mg, 18.4 µmol).
MS (ESI) m/z: 951.45 [M+H]⁺

### (19-9-3) Synthesis of Pyrene (94)

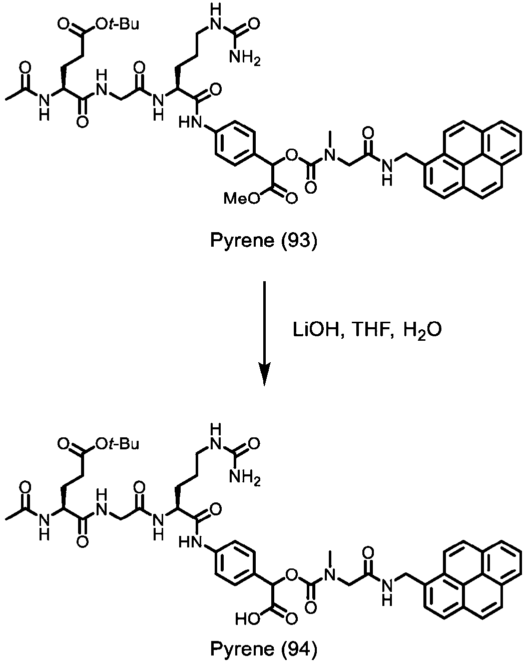

Pyrene (93) (21.5 mg, 22.6 µmol) was dissolved in tetrahydrofuran (1.70 mL) and water (565 µL) and stirred under ice cooling for 5 minutes, then 1 M aqueous lithium hydroxide solution (54.3 µL, 54.3 µmol) was added, and the mixture was stirred at room temperature for 2 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (94) (11.5 mg, 12.3 µmol).
MS (ESI) m/z: 937.40 [M+H]⁺

### (19-9-4) Synthesis of Pyrene (95)

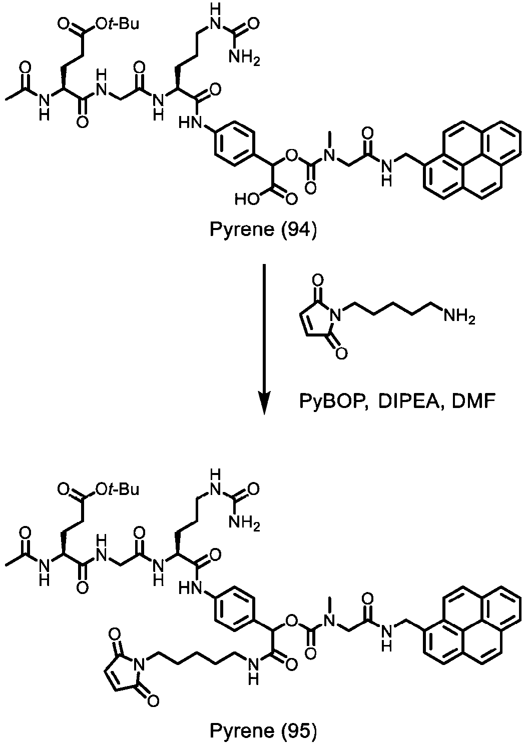

Pyrene (94) (11.5 mg, 12.3 µmol) was dissolved in N,N-dimethylformamide (1.05 mL) and cooled on ice, then N,N-diisopropylethylamine (4.2 µL, 24.5 µmol) and 1H-benzotriazol-1-yloxytripyrrolidinophosphonium hexafluorophosphate (9.58 mg, 18.4 µmol) were added. Next, N-(5-aminopentyl)maleimide hydrochloride (4.03 mg, 18.4 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (95) (9.00 mg, 8.20 µmol).
MS (ESI) m/z: 1101.50 [M+H]⁺

### (19-9-5) Synthesis of Linker-payload mimic (91)

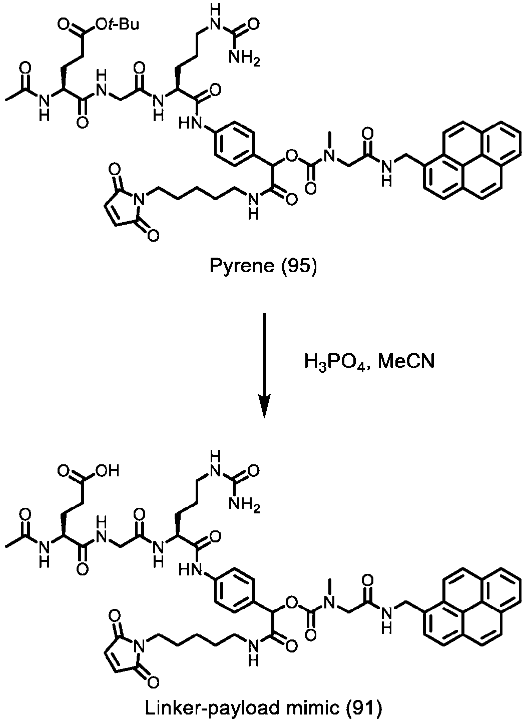

To Pyrene (95) (8.00 mg, 7.26 µmol) was added acetonitrile (363 µL), and 85% phosphoric acid solution (149 µL, 2.18 µmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (91) (5.60 mg, 53.6 µmol).
¹H NMR (400 MHz, DMSO-d₆)
MS (ESI) m/z: 1045.50 [M+H]⁺

### (19-10) Synthesis of Linker-payload mimic (11)

Linker-payload mimic (11) was also synthesized as described below by a different route from (1-3).

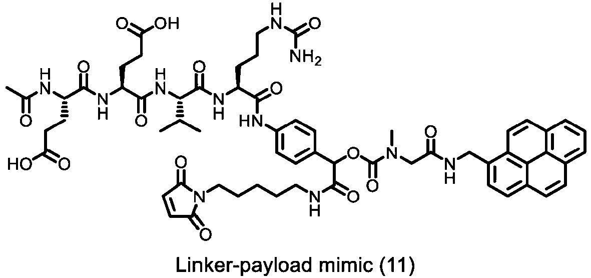

### (19-10-1) Synthesis of Alcohol (96)

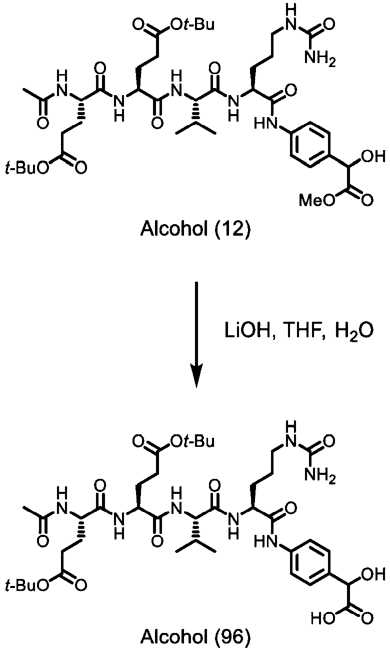

Alcohol (12) (80.0 mg, 94.1 µmol) obtained in Example (1-3-1) was dissolved in tetrahydrofuran (7.0 mL) and water (2.35 mL) and stirred under ice cooling for 5 minutes, then 1 M aqueous lithium hydroxide solution (226 µL, 226 µmol) was added, and the mixture was stirred at room temperature for 2 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Alcohol (96) (61.5 mg, 73.6 µmol).
MS (ESI) m/z: 836.40 [M+H]⁺

### (19-10-2) Synthesis of Alcohol (97)

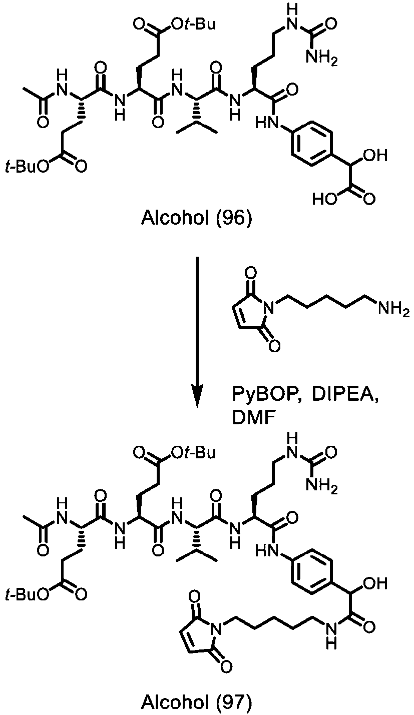

Alcohol (96) (60.5 mg, 72.4 µmol) was dissolved in N,N-dimethylformamide (3.6 mL) and cooled on ice, then N,N-diisopropylethylamine (25 µL, 145 µmol) and 1H-benzotriazol-1-yloxytripyrrolidinophosphonium hexafluorophosphate (56.5 mg, 109 µmol) were added. Next, N-(5-aminopentyl)maleimide hydrochloride (23.7 mg, 109 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Alcohol (97) (68.0 mg, 72.4 µmol).
MS (ESI) m/z: 1000.50 [M+H]⁺

### (19-10-3) Synthesis of Compound (98)

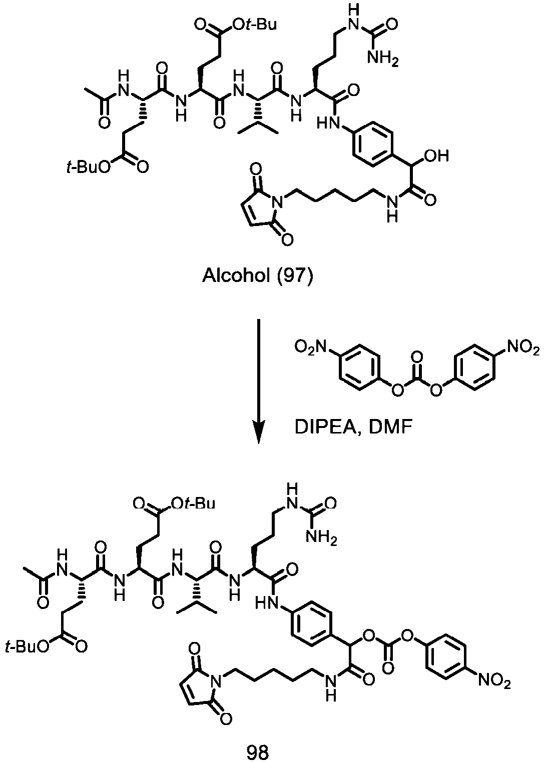

Alcohol (97) (10.0 mg, 10.0 µmol) was dissolved in N,N-dimethylformamide (0.1 mL) and cooled on ice, then bis(4-nitrophenyl) carbonate (30.4 mg, 100 µmol) and N,N-diisopropylethylamine (3.8 µL, 22.5 µmol) were added, and the mixture was stirred at room temperature for 3 hours. After the reaction, the reaction mixture was purified by normal-phase preparative chromatography. Fractions containing the product were collected, and acetonitrile was removed by concentration under reduced pressure, followed by lyophilization to obtain the above-described Compound (98) (5.6 mg, 4.8 µmol).
MS (ESI) m/z: 1163.50 [M+H]⁺

### (19-10-4) Synthesis of Pyrene (15)

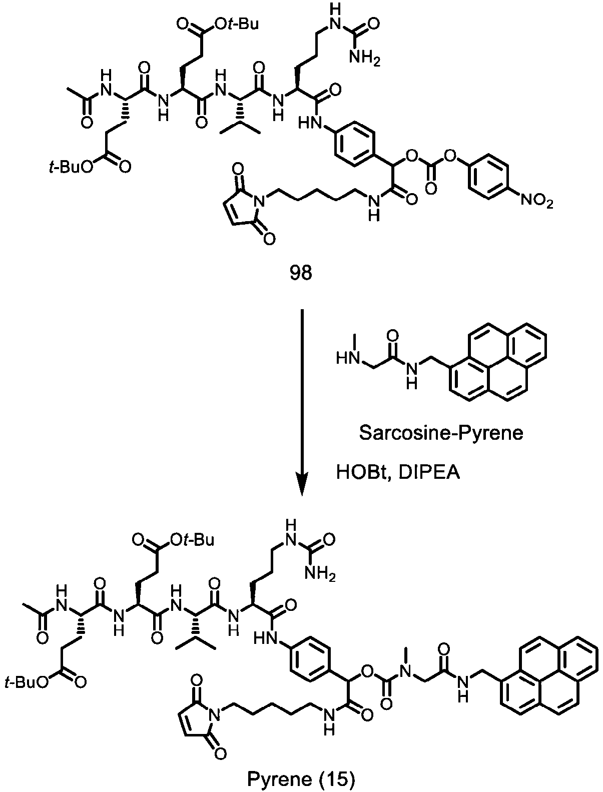

Compound (98) (10.0 mg, 8.6 µmol) was dissolved in N,N-dimethylformamide (86 µL) and cooled on ice, then Sarcosine-Pyrene (2.2 mg, 7.2 µmol), 1-hydroxybenzotriazole (1.5 mg, 11 µmol), and N,N-diisopropylethylamine (1.8 µL, 11 µmol) were added, and the mixture was stirred at room temperature for 4 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (15) (3.0 mg, 2.3 µmol).
MS (ESI) m/z: 1328.60 [M+H]⁺

### (19-10-5) Synthesis of Linker-payload mimic (15)

Linker-payload mimic (15) was synthesized in the same manner as in Example (1-3-5).

### (19-11) Synthesis of Linker-payload mimic (99)

Linker-payload mimic (99) was synthesized as follows.

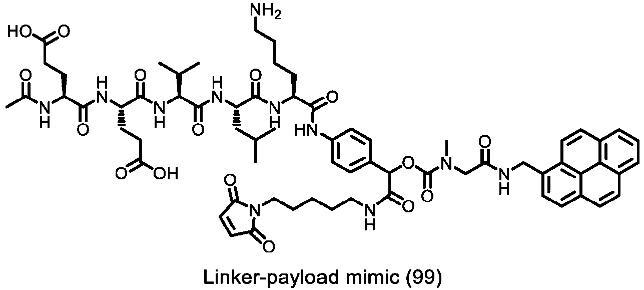

### (19-11-1) Synthesis of Alcohol (100)

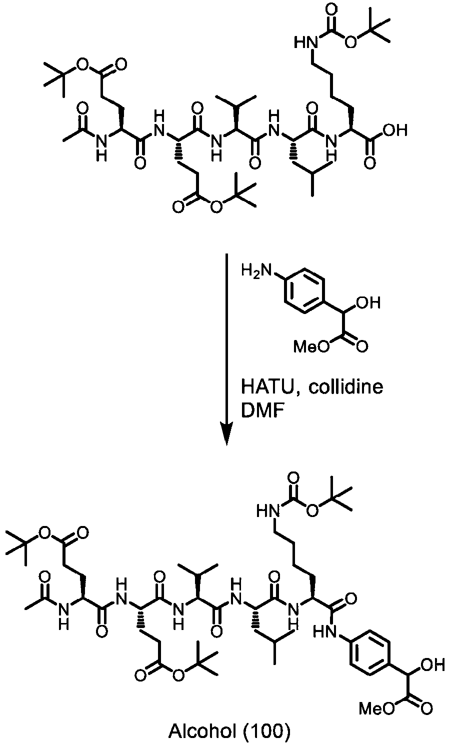

Ac-Glu(OtBu)-Glu(OtBu)-Val-Leu-Lys-OH (47.2 mg, 54.2 µmol) was dissolved in N,N-dimethylformamide (0.54 mL), and 1-[bis(dimethylamino)methylene]-1H-1,2,3,triazolo[4,5-b]pyridinium 3-oxide hexafluorophosphate (31.3 mg, 82.3 µmol) and 2,4,6-trimethylpyridine (10.9 µL, 81.8 µmol) were added, followed by stirring at room temperature for 10 minutes. Subsequently, methyl 4-aminomandelate (11.8 mg, 65.1 µmol) was added, and the mixture was stirred at room temperature for 68 hours, followed by purification by preparative reversed-phase chromatography. Fractions containing the product were collected, and acetonitrile was removed by concentration under reduced pressure, followed by lyophilization to obtain the above-described Alcohol (100) (31.9 mg, 30.8 µmol).
MS (ESI) m/z: 1034.60 [M+H]⁺

### (19-11-2) Synthesis of Pyrene (101)

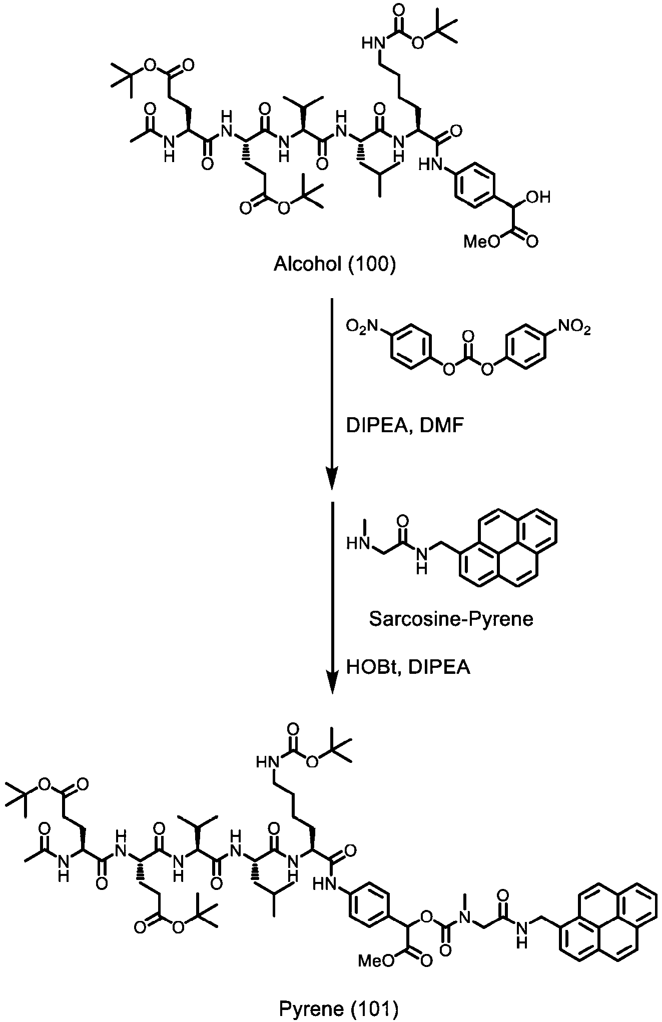

Alcohol (100) (30.5 mg, 29.5 µmol) was dissolved in N,N-dimethylformamide (169 µL) and stirred under ice cooling for 5 minutes, then bis(4-nitrophenyl) carbonate (27.4 mg, 90.1 µmol) and N,N-diisopropylethylamine (11.4 µL, 66.3 µmol) were added, and the mixture was stirred at room temperature for 2 hours. Subsequently, the mixture was cooled on ice, then Sarcosine-Pyrene (17.7 mg, 51.6 µmol), 1-hydroxybenzotriazole (6.0 mg, 44.2 µmol), and N,N-diisopropylethylamine (39.3 µL, 228 µmol) were added, and the mixture was stirred at room temperature for 21 hours. After the reaction, the reaction mixture was purified with silica gel column chromatography. Fractions containing the product were collected, and dichloromethane and methanol were removed by concentration under reduced pressure to obtain the above-described Pyrene (101) (20.2 mg, 14.8 µmol).
MS (ESI) m/z: 1362.75 [M+H]⁺

### (19-11-3) Synthesis of Pyrene (102)

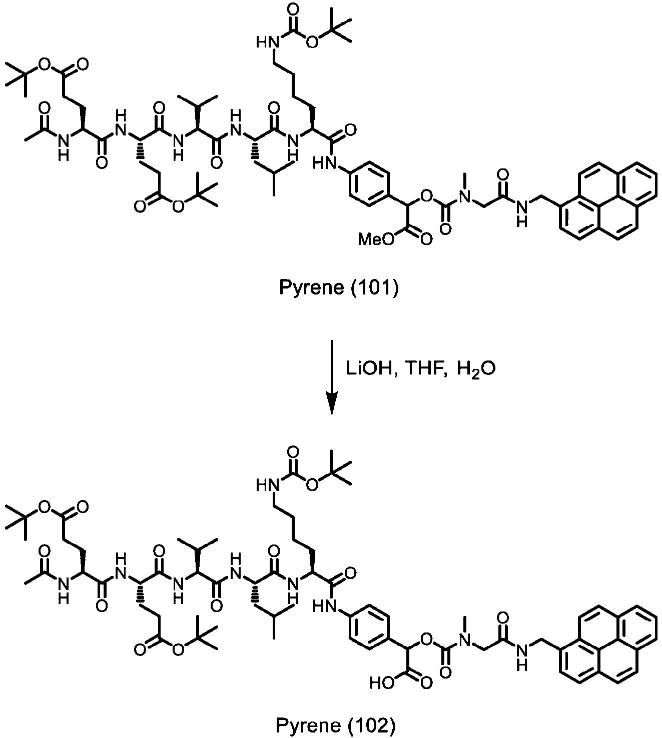

Pyrene (101) (20.5 mg, 15.0 µmol) was dissolved in tetrahydrofuran (1.0 mL) and water (0.5 mL) and stirred under ice cooling for 5 minutes, then 1 M aqueous lithium hydroxide solution (75.0 µL, 75.0 µmol) was added, and the mixture was stirred at room temperature for 5 hours. After the reaction was completed, the reaction mixture was subjected to liquid-liquid extraction and concentrated under reduced pressure to obtain the above-described Pyrene (102) (18.8 mg, 13.9 µmol).
MS (ESI) m/z: 1348.90 [M+H]⁺

### (19-11-4) Synthesis of Pyrene (103)

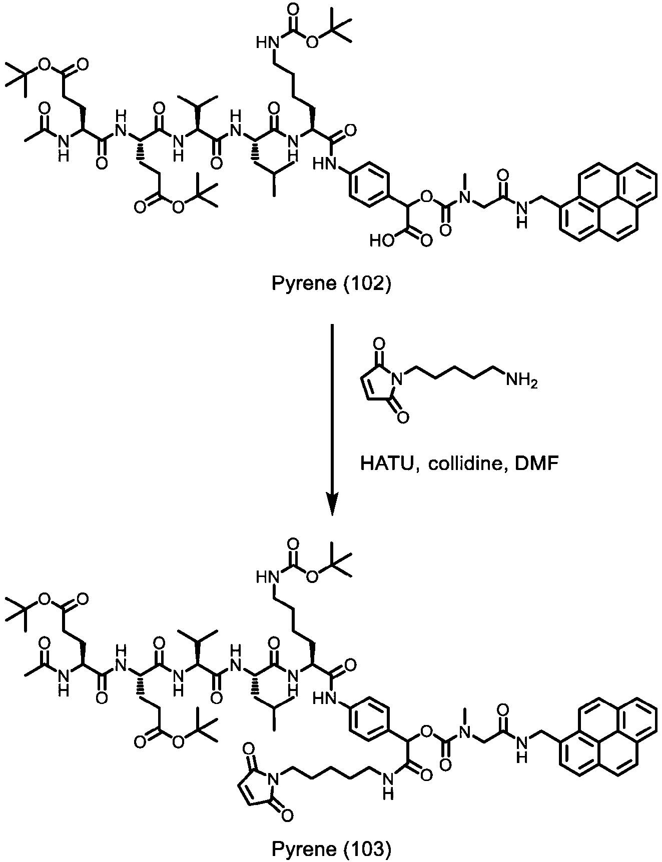

Pyrene (102) (11.9 mg, 8.82 µmol) was dissolved in N,N-dimethylformamide (441 µL) and cooled on ice, then 2,4,6-trimethylpyridine (3.6 µL, 26.8 µmol) and 1-[bis(dimethylamino)methylene]-1H-1,2,3,triazolo[4,5-b]pyridinium 3-oxide hexafluorophosphate (5.0 mg, 13.2 µmol) were added. Next, N-(5-aminopentyl)maleimide hydrochloride (2.8 mg, 12.3 µmol) was added, and the mixture was returned to room temperature and stirred for 2.5 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (103) (5.2 mg, 3.43 µmol).
MS (ESI) m/z: 1513.75 [M+H]⁺

### (19-11-5) Synthesis of Linker-payload mimic (99)

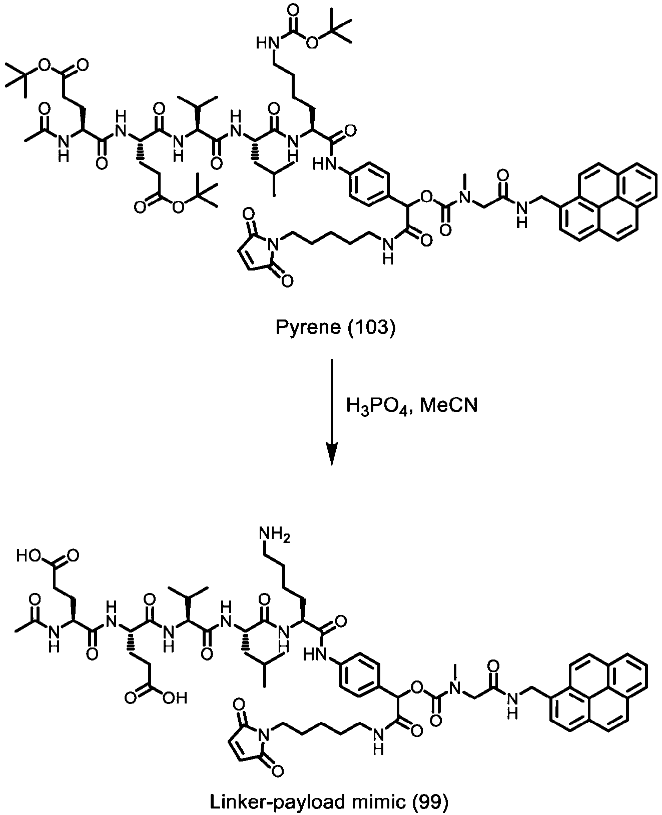

To Pyrene (103) (4.5 mg, 2.97 µmol) was added acetonitrile (300 µL), and 85% phosphoric acid solution (102 µL, 1.48 mmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (99) (2.1 mg, 1.61 µmol).
MS (ESI) m/z: 1300.60 [M+H]⁺

### (19-12) Synthesis of Linker-payload mimic (104)

Linker-payload mimic (104) was synthesized as follows.

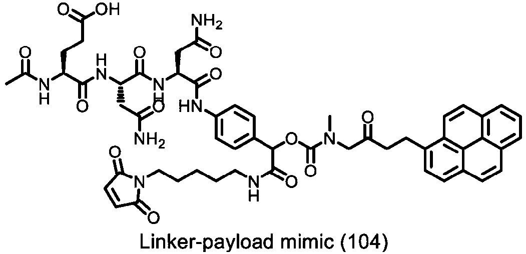

### (19-12-1) Synthesis of Alcohol (105)

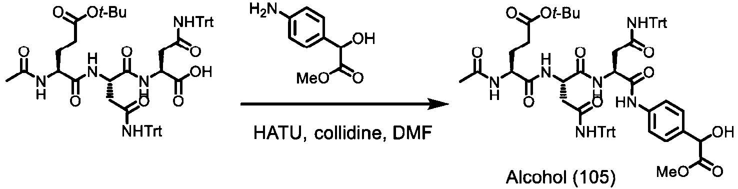

Ac-Glu(OtBu)-Asn(NHTrt)-Asn(NHTrt)-OH (100 mg, 104 µmol) was dissolved in N,N-dimethylformamide (520 µL), and 1-[bis(dimethylamino)methylene]-1H-1,2,3,triazolo[4,5-b]pyridinium 3-oxide hexafluorophosphate (47.5 mg, 125 µmol) and 2,4,6-trimethylpyridine (16.5 µL, 125 µmol) were added, followed by stirring at room temperature for 10 minutes. Subsequently, methyl 4-aminomandelate (22.6 mg, 125 µmol) was added, and the mixture was stirred at room temperature for 16.5 hours, followed by purification by preparative reversed-phase chromatography. Fractions containing the product were collected, and acetonitrile was removed by concentration under reduced pressure, followed by lyophilization to obtain the above-described Alcohol (105) (110 mg, 98.5 µmol).
MS (ESI) m/z: 1121.50 [M+H]⁺

### (19-12-2) Synthesis of Pyrene (106)

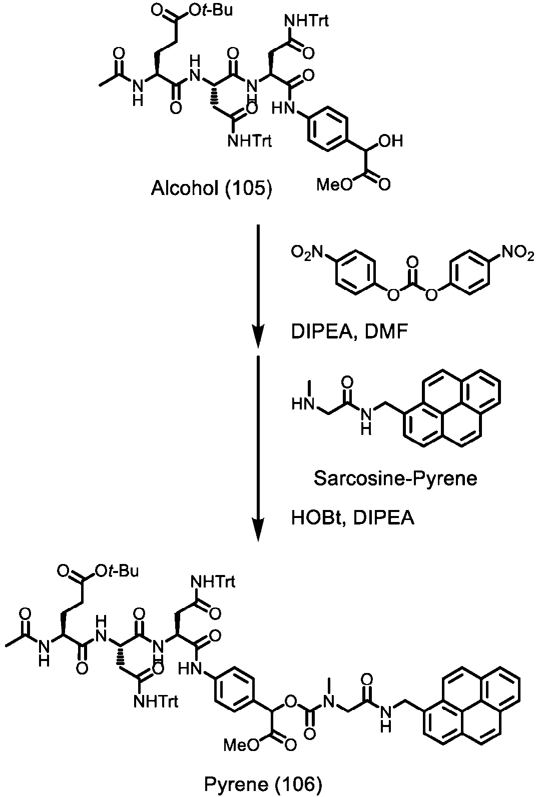

Alcohol (105) (92.6 mg, 82.6 µmol) was dissolved in N,N-dimethylformamide (413 µL) and stirred under ice cooling for 5 minutes, then bis(4-nitrophenyl) carbonate (75.4 mg, 248 µmol) and N,N-diisopropylethylamine (31.6 µL, 186 µmol) were added, and the mixture was stirred at room temperature for 1 hour. Subsequently, the mixture was cooled on ice, then Sarcosine-Pyrene (74.9 mg, 248 µmol), 1-hydroxybenzotriazole (16.7 mg, 124 µmol), and N,N-diisopropylethylamine (52.7 µL, 310 µmol) were added, and the mixture was stirred at room temperature for 2 hours. After the reaction, the reaction mixture was purified by normal-phase preparative chromatography (dichloromethane: methanol). Fractions containing the product were collected and concentrated under reduced pressure to obtain the above-described Pyrene (106) (58.0 mg, 40.0 µmol).
MS (ESI) m/z: 1449.65 [M+H]⁺

### (19-12-3) Synthesis of Pyrene (107)

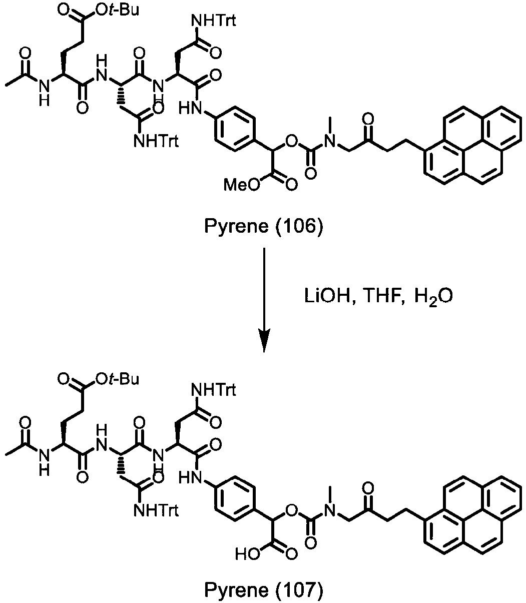

Pyrene (106) (30.0 mg, 20.7 µmol) was dissolved in tetrahydrofuran (1.58 mL) and water (525 µL) and stirred under ice cooling for 5 minutes, then 1 M aqueous lithium hydroxide solution (49.7 µL, 49.7 µmol) was added, and the mixture was stirred at room temperature for 2.5 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (107) (15.3 mg, 10.7 µmol).
MS (ESI) m/z: 1435.60 [M+H]⁺

### (19-12-4) Synthesis of Pyrene (108)

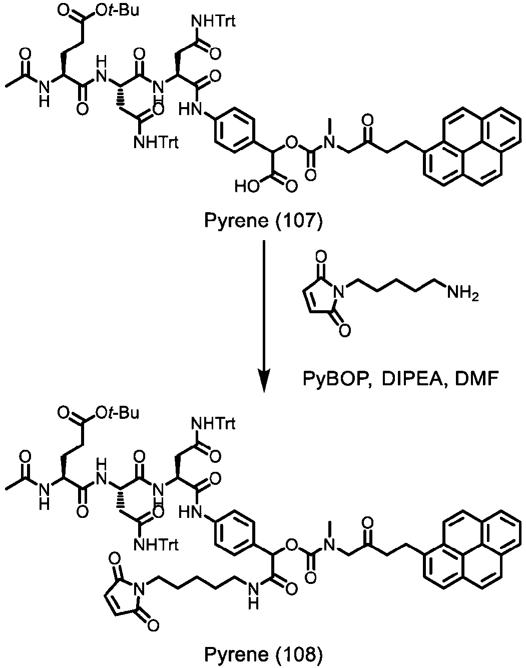

Pyrene (107) (14.2 mg, 9.9 µmol) was dissolved in N,N-dimethylformamide (400 µL) and cooled on ice, then N,N-diisopropylethylamine (3.4 µL, 19.8 µmol) and 1H-benzotriazol-1-yloxytripyrrolidinophosphonium hexafluorophosphate (7.7 mg, 14.8 µmol) were added. Next, N-(5-aminopentyl)maleimide hydrochloride (3.2 mg, 14.8 µmol) was added, and the mixture was returned to room temperature and stirred for 1 hour. After the reaction was completed, the reaction mixture was purified by normal-phase preparative chromatography (dichloromethane: methanol). Fractions containing the product were collected and concentrated under reduced pressure to obtain Pyrene (108) (11.7 mg, 7.3 µmol).
MS (ESI) m/z: 1599.80 [M+H]⁺

### (19-12-5) Synthesis of Linker-payload mimic (104)

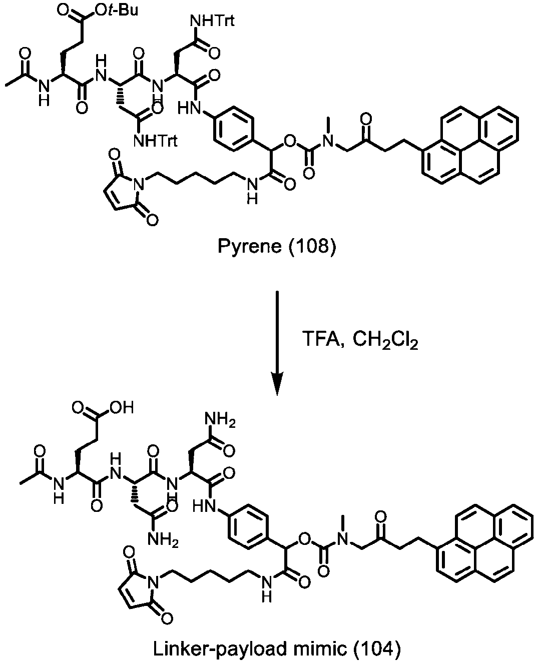

To Pyrene (108) (5.0 mg, 3.1 µmol) was dissolved in dichloromethane (282 µL) and cooled on ice, then trifluoroacetic acid (31.3 µL, 409 µmol) was added, and the mixture was stirred at room temperature for 2.5 hours. The reaction solution was purified by preparative reversed-phase chromatography, and fractions containing the product were collected, and acetonitrile was removed by concentration under reduced pressure, followed by lyophilization to obtain Linker-payload mimic (104) (2.3 mg, 2.2 µmol).
MS (ESI) m/z: 1059.50 [M+H]⁺

### (19-13) Synthesis of Linker-payload mimic (109)

Linker-payload mimic (109) was synthesized as follows.

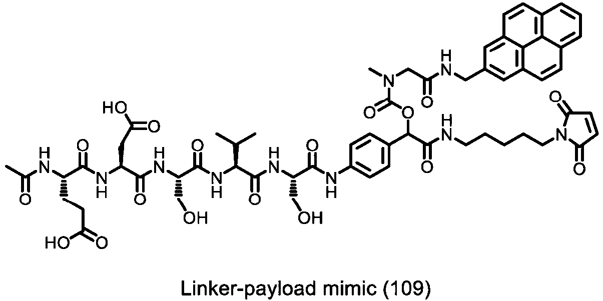

### (19-13-1) Synthesis of Alcohol (111)

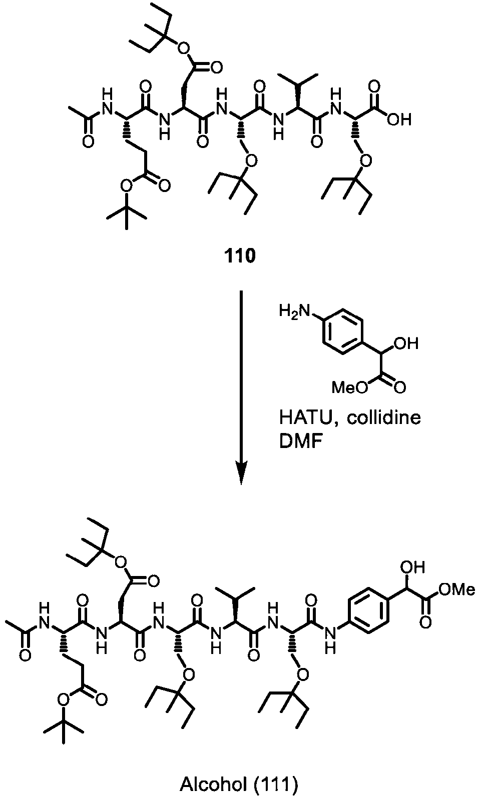

Compound (110) (50.7 mg, 53.3 µmol) was dissolved in N,N-dimethylformamide (266 µL), and 1-[bis(dimethylamino)methylene]-1H-1,2,3,triazolo[4,5-b]pyridinium 3-oxide hexafluorophosphate (24.3 mg, 63.9 µmol) and 2,4,6-trimethylpyridine (8.4 µL, 63.9 µmol) were added, followed by stirring at room temperature for 10 minutes. Subsequently, methyl 4-aminomandelate (11.6 mg, 63.9 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (111) (48.4 mg, 43.8 µmol).
MS (ESI) m/z: 1105.65 [M+H]⁺

### (19-13-2) Synthesis of Pyrene (112)

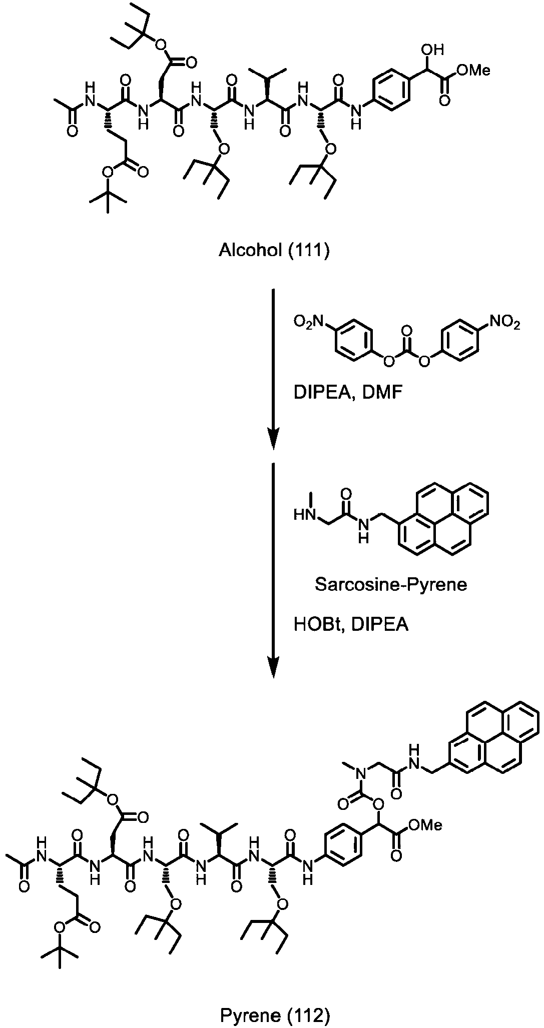

Alcohol (111) (48.4 mg, 43.8 µmol) was dissolved in N,N-dimethylformamide (156 µL) and stirred under ice cooling for 5 minutes, then bis(4-nitrophenyl) carbonate (21.6 mg, 69.6 µmol) and N,N-diisopropylethylamine (8.8 µL, 51.2 µmol) were added, and the mixture was stirred at room temperature for 2 hours and 10 minutes. Subsequently, the mixture was cooled on ice, then Sarcosine-Pyrene (16.3 mg, 48.0 µmol), 1-hydroxybenzotriazole (55.0 mg, 41.0 µmol), and N,N-diisopropylethylamine (18.8 µL, 109 µmol) were added, and the mixture was stirred at room temperature for 2 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (112) (22.1 mg, 15.4 µmol).
MS (ESI) m/z: 1433.95 [M+H]⁺

### (19-13-3) Synthesis of Pyrene (113)

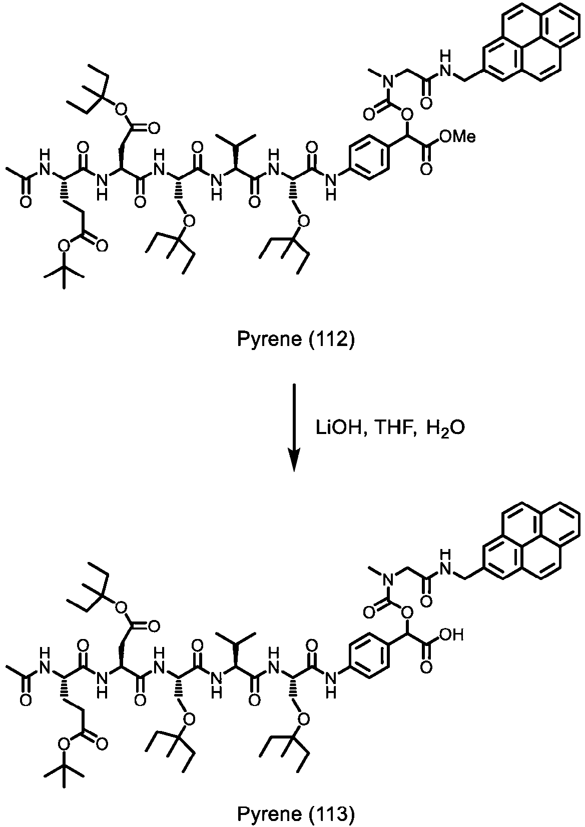

Pyrene (112) (20.8 mg, 14.5 µmol) was dissolved in tetrahydrofuran (1.08 mL) and water (0.36 mL) and stirred under ice cooling for 5 minutes, then 1 M aqueous lithium hydroxide solution (34.8 µL, 34.8 µmol) was added, and the mixture was stirred at room temperature for 1 hour. After the reaction was completed, the pH was adjusted to about 6 with acetic acid, and the reaction mixture was purified by preparative reversed-phase chromatography. Fractions containing the product were collected, and acetonitrile was removed by concentration under reduced pressure, followed by lyophilization to obtain the above-described Pyrene (113) (2.3 mg, 1.6 µmol).
MS (ESI) m/z: 1420.80 [M+H]⁺

### (19-13-4) Synthesis of Pyrene (114)

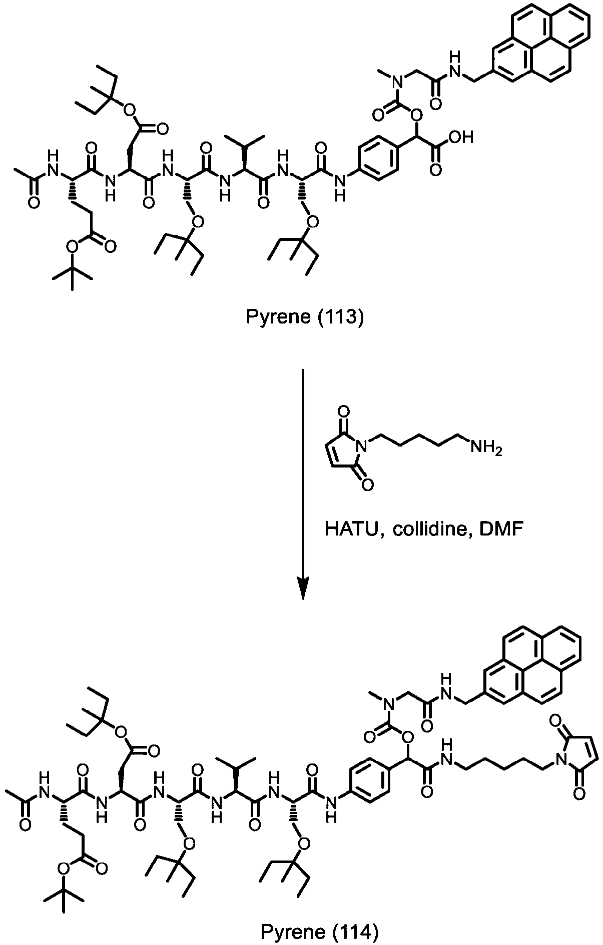

Pyrene (113) (2.3 mg, 1.6 µmol) was dissolved in 2,4,6-trimethylpyridine (200 µL) and cooled on ice, then N,N-diisopropylethylamine (0.56 µL, 3.3 µmol) and 1-[bis(dimethylamino)methylene]-1H-1,2,3,triazolo[4,5-b]pyridinium 3-oxide hexafluorophosphate (1.19 mg, 3.13 µmol) were added. Next, N-(5-aminopentyl)maleimide hydrochloride (0.58 mg, 2.5 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (114) (2.9 mg, 1.8 µmol).
¹H NMR (400 MHz, DMSO-d₆)
MS (ESI) m/z: 1606.15 [M+Na]⁺

### (19-13-5) Synthesis of Linker-payload mimic (109)

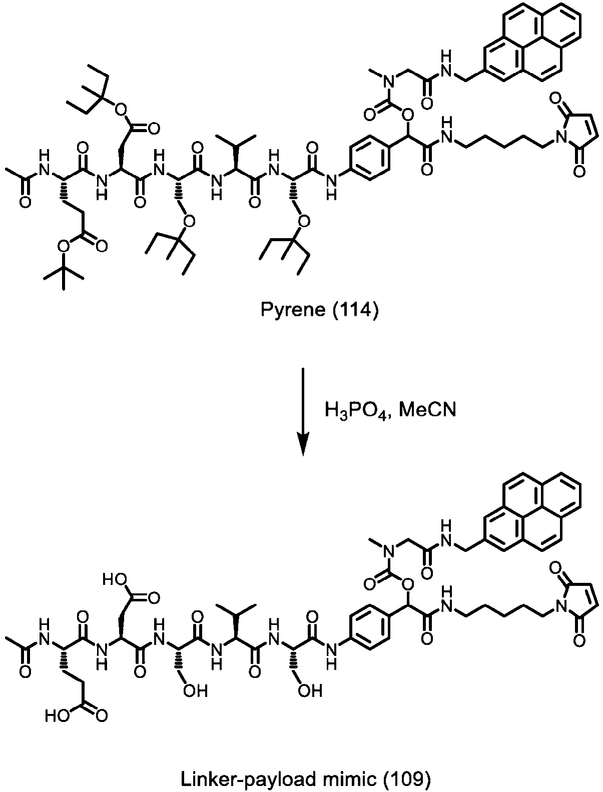

To Pyrene (114) (2.2 mg, 1.4 µmol) was added acetonitrile (278 µL), and 85% phosphoric acid solution (71.3 µL, 1.04 mmol) was added under ice cooling, and the mixture was stirred at room temperature for 23 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (109) (0.7 mg, 0.5 µmol).
MS (ESI) m/z: 1275.70 [M+H]⁺

### (19-14) Synthesis of Linker-payload mimic (115)

Linker-payload mimic (115) was synthesized as follows.

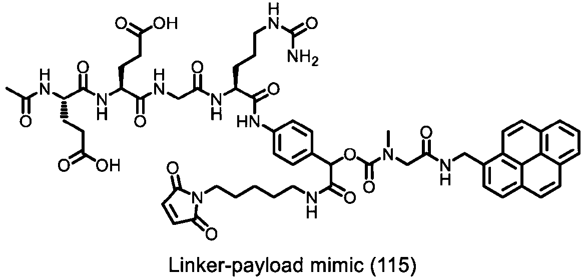

### (19-14-1) Synthesis of Alcohol (116)

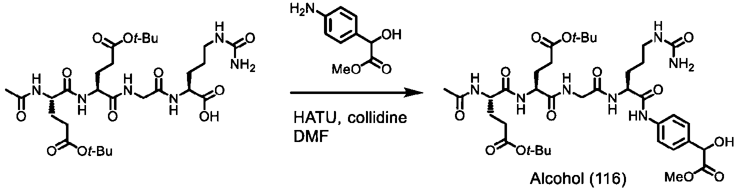

Ac-Glu(OtBu)-Glu(OtBu)-Gly-Cit-OH (50.0 mg, 77.6 µmol) was dissolved in N,N-dimethylformamide (388 µL), and 1-[bis(dimethylamino)methylene]-1H-1,2,3,triazolo[4,5-b]pyridinium 3-oxide hexafluorophosphate (35.3 mg, 93.1 µmol) and 2,4,6-trimethylpyridine (12.0 µL, 93.1 µmol) were added, followed by stirring at room temperature for 10 minutes. Subsequently, methyl 4-aminomandelate (16.8 mg, 93.1 µmol) was added, and the mixture was stirred at room temperature for 21 hours, followed by purification by preparative reversed-phase chromatography. Fractions containing the product were collected, and acetonitrile was removed by concentration under reduced pressure, followed by lyophilization to obtain the above-described Alcohol (116) (53.3 mg, 66.0 µmol).
MS (ESI) m/z: 808.50 [M+H]⁺

### (19-14-2) Synthesis of Pyrene (117)

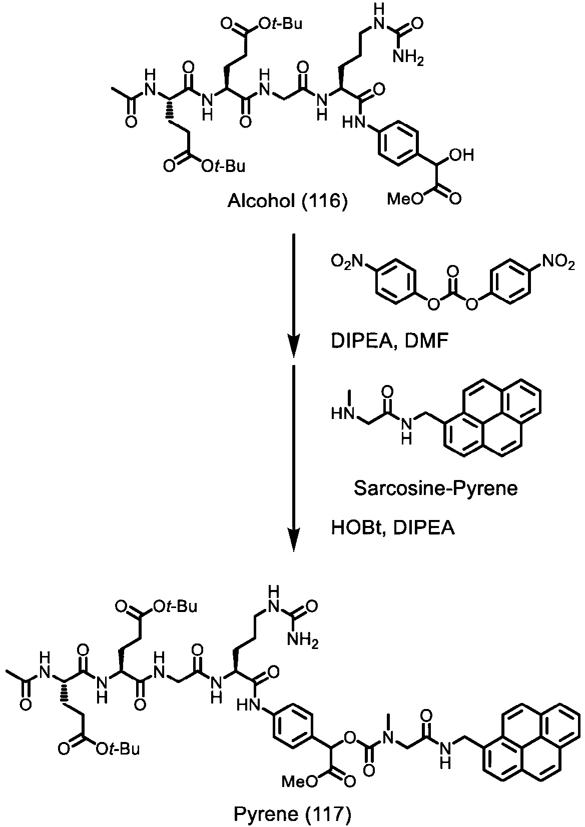

Alcohol (116) (25.0 mg, 30.9 µmol) was dissolved in N,N-dimethylformamide (309 µL) and stirred under ice cooling for 5 minutes, then bis(4-nitrophenyl) carbonate (28.2 mg, 92.7 µmol) and N,N-diisopropylethylamine (11.8 µL, 69.5 µmol) were added, and the mixture was stirred at room temperature for 2 hours. Subsequently, the mixture was cooled on ice, then Sarcosine-Pyrene (28.0 mg, 92.7 µmol), 1-hydroxybenzotriazole (6.3 mg, 46.4 µmol), and N,N-diisopropylethylamine (20.1 µL, 116 µmol) were added, and the mixture was stirred at room temperature for 4 hours. After the reaction, the reaction mixture was purified by normal-phase preparative chromatography. Fractions containing the product were collected, and acetonitrile was removed by concentration under reduced pressure, followed by lyophilization to obtain the above-described Pyrene (117) (25.3 mg, 22.2 µmol).
MS (ESI) m/z: 1136.50 [M+H]⁺

### (19-14-3) Synthesis of Pyrene (118)

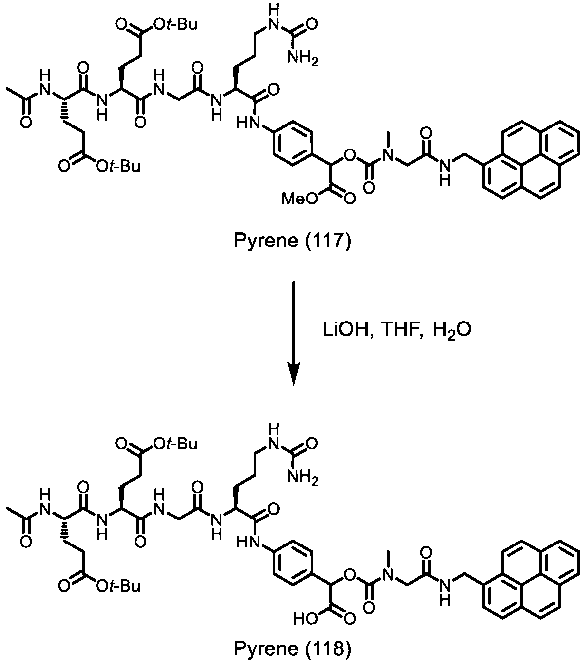

Pyrene (117) (15.0 mg, 13.2 µmol) was dissolved in tetrahydrofuran (1.32 mL) and water (330 µL) and stirred under ice cooling for 5 minutes, then 1 M aqueous lithium hydroxide solution (31.7 µL, 31.7 µmol) was added, and the mixture was stirred at room temperature for 3 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (118) (9.3 mg, 8.32 µmol).
MS (ESI) m/z: 1122.50 [M+H]⁺

### (19-14-4) Synthesis of Pyrene (119)

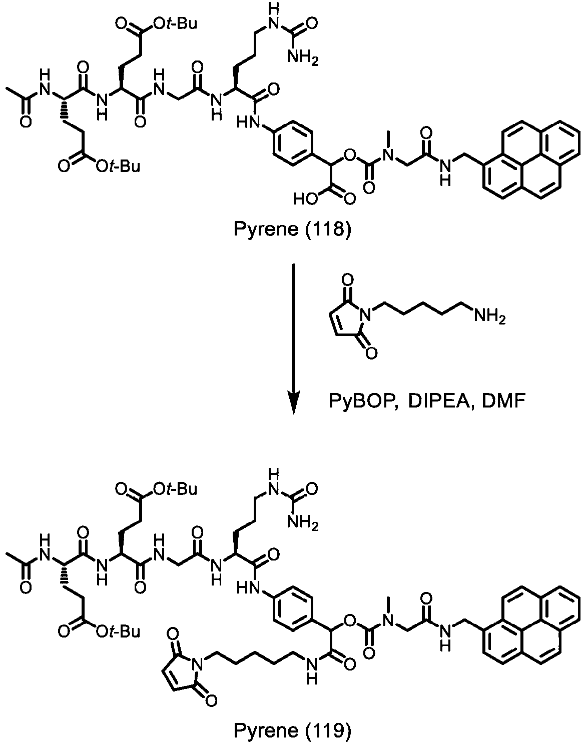

Pyrene (118) (8.8 mg, 7.8 µmol) was dissolved in N,N-dimethylformamide (784 µL) and cooled on ice, then N,N-diisopropylethylamine (2.67 µL, 15.6 µmol) and 1H-benzotriazol-1-yloxytripyrrolidinophosphonium hexafluorophosphate (6.10 mg, 11.7 µmol) were added. Next, N-(5-aminopentyl)maleimide hydrochloride (2.6 mg, 12 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (119) (8.1 mg, 6.3 µmol).
MS (ESI) m/z: 1286.80 [M+H]⁺

### (19-14-5) Synthesis of Linker-payload mimic (115)

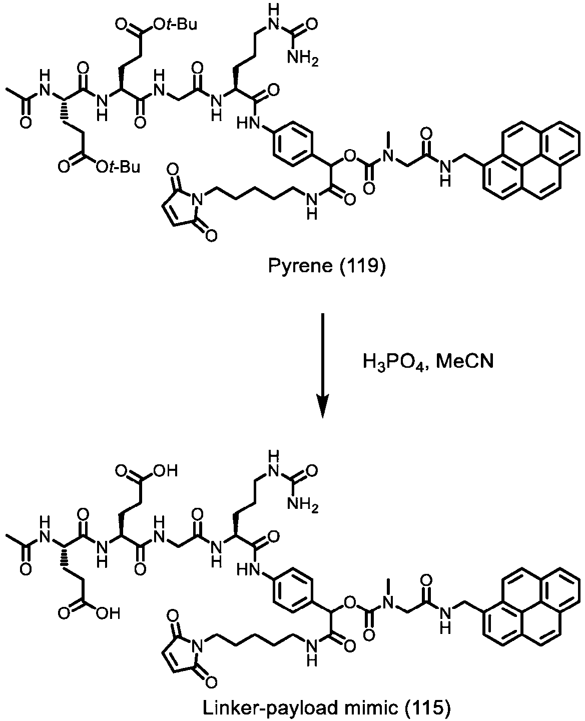

To Pyrene (119) (7.3 mg, 5.7 µmol) was added acetonitrile (284 µL), and 85% phosphoric acid solution (117 µL, 1.72 mmol) was added under ice cooling, and the mixture was stirred at room temperature for 17 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (115) (5.4 mg, 4.6 µmol).
MS (ESI) m/z: 1174.50 [M+H]⁺

### (19-15) Synthesis of Linker-payload mimic (120)

Linker-payload mimic (120) was synthesized as follows.

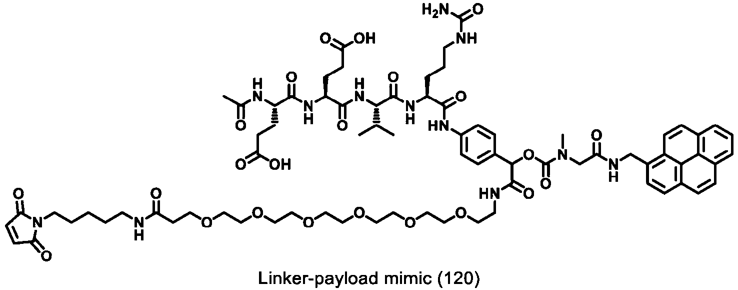

### (19-15-1) Synthesis of Compound (122)

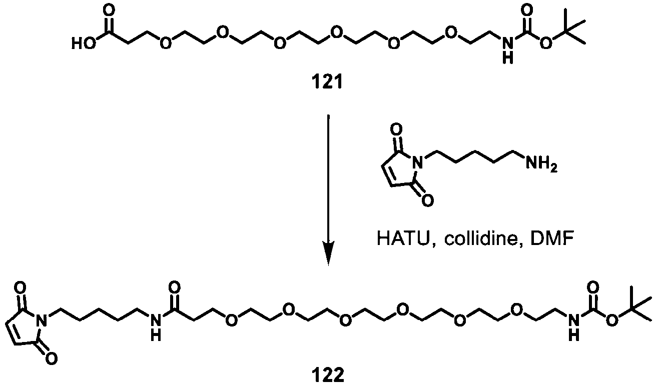

21-[(tert-butoxycarbonyl)amino]-4,7,10,13,16,19-hexaoxaheneicosanoic acid (121) (70.0 mg, 154 µmol) was dissolved in N,N-dimethylformamide (7.72 mL) and cooled on ice, then N,N-diisopropylethylamine (52.0 µL, 309 µmol) and 1H-benzotriazol-1-yloxytripyrrolidinophosphonium hexafluorophosphate (120 mg, 232 µmol) were added. Next, N-(5-aminopentyl)maleimide hydrochloride (50.6 mg, 232 µmol) was added, and the mixture was returned to room temperature and stirred for 4 hours. After the reaction was completed, the reaction mixture was purified by preparative reversed-phase chromatography. Fractions containing the product were collected, and acetonitrile was removed by concentration under reduced pressure, followed by lyophilization to obtain Compound (122) (83.4 mg, 135 µmol).
MS (ESI) m/z: 618.50 [M+H]⁺

### (19-15-2) Synthesis of Compound (123)

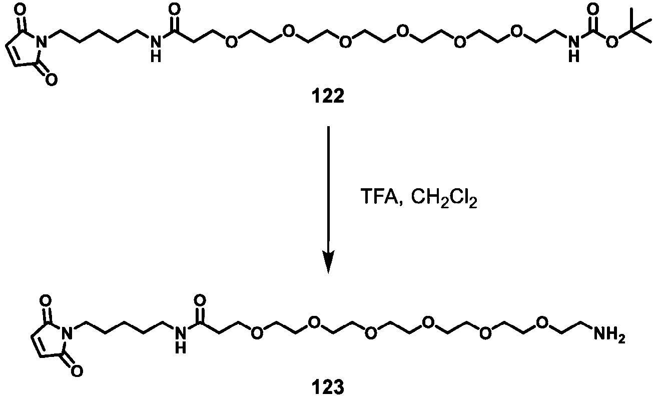

Compound (122) (82.0 mg, 133 µmol) was dissolved in dichloromethane (13.3 mL) and trifluoroacetic acid (6.64 mL), and the mixture was stirred at room temperature for 30 minutes. After the reaction was completed, dichloromethane and trifluoroacetic acid were removed by concentration under reduced pressure to obtain the above-described Compound (123) (71.8 mg, quant).
MS (ESI) m/z: 518.40 [M+H]⁺

### (19-15-3) Synthesis of Pyrene (124)

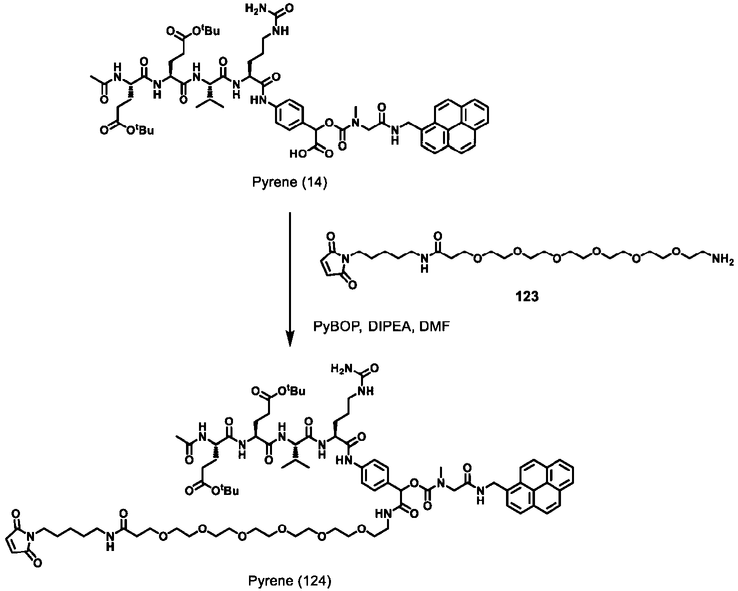

Pyrene (14) (16.0 mg, 14.0 µmol) was dissolved in N,N-dimethylformamide (690 µL) and cooled on ice, then N,N-diisopropylethylamine (9.3 µL, 55.0 µmol) and 1H-benzotriazol-1-yloxytripyrrolidinophosphonium hexafluorophosphate (11 mg, 21.0 µmol) were added. Then, PEG6 (11.0 mg, 21.0 µmol) was added, and the mixture was returned to room temperature and stirred for 2.5 hours. After the reaction was completed, the reaction mixture was purified by preparative reversed-phase chromatography. Fractions containing the product were collected, and acetonitrile was removed by concentration under reduced pressure, followed by lyophilization to obtain Pyrene (124) (11.2 mg, 6.73 µmol).
MS (ESI) m/z: 1664.80 [M+H]⁺

### (19-15-4) Synthesis of Linker-payload mimic (120)

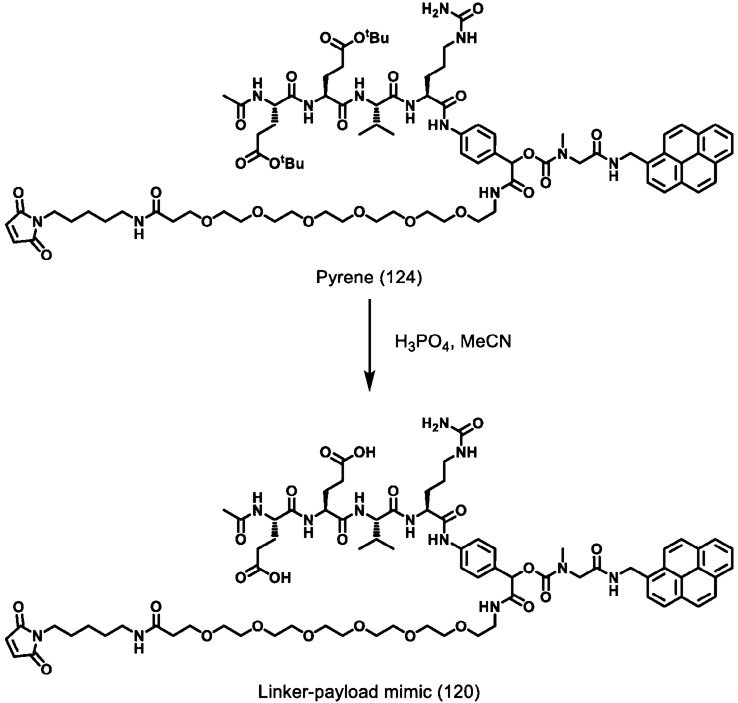

To Pyrene (124) (5.0 mg, 3.0 µmol) was added acetonitrile (200 µL), and 85% phosphoric acid solution (60.0 µL, 880 µmol) was added under ice cooling, and the mixture was stirred at room temperature for 25 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (120) (2.4 mg, 1.5 µmol).
MS (ESI) m/z: 1552.65 [M+H]⁺

### (19-16) Synthesis of Linker-payload mimic (124)

Linker-payload mimic (124) was synthesized as follows.

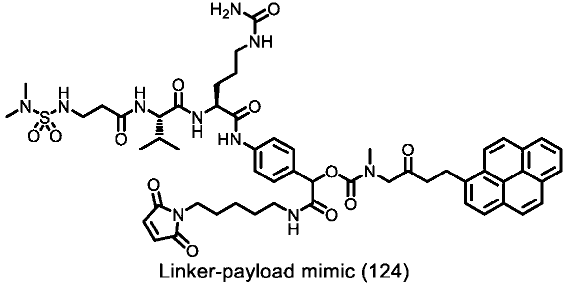

### (19-16-1) Synthesis of Alcohol (190)

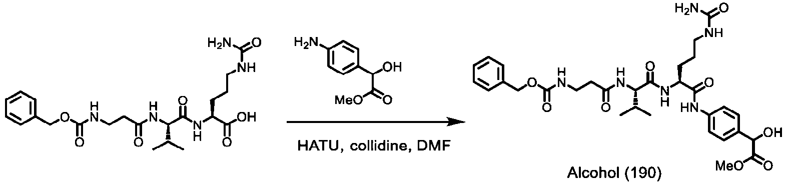

Cbz-βAla-Val-Cit-OH (30.8 mg, 64.2 µmol) was dissolved in N,N-dimethylformamide (313 µL), and 1-[bis(dimethylamino)methylene]-1H-1,2,3,triazolo[4,5-b]pyridinium 3-oxide hexafluorophosphate (29.3 mg, 77.1 µmol) and 2,4,6-trimethylpyridine (10.2 µL, 77.1 µmol) were added, followed by stirring at room temperature for 10 minutes. Subsequently, methyl 4-aminomandelate (14.0 mg, 77.1 µmol) was added, and the mixture was stirred at room temperature for 17.5 hours, followed by purification by preparative reversed-phase chromatography. Fractions containing the product were collected, and acetonitrile was removed by concentration under reduced pressure, followed by lyophilization to obtain the above-described Alcohol (190) (32.0 mg, 49.8 µmol).
MS (ESI) m/z: 643.30 [M+H]⁺

### (19-16-2) Synthesis of Alcohol (191)

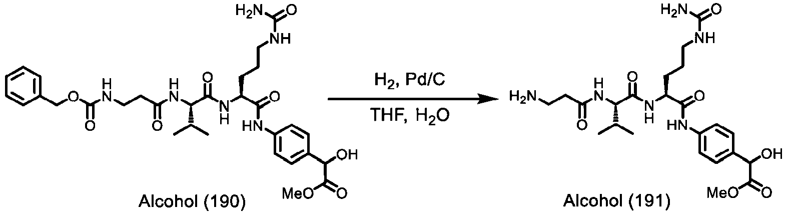

Alcohol (190) (135.7 mg, 211 µmol) was dissolved in THF (5.3 mL) and water (5.3 mL), 5% palladium on carbon (9.6 mg, 2.1 µmol) was added, and after replacing the atmosphere with hydrogen, the mixture was stirred at room temperature for 5 hours. After the reaction, the solid was removed by filtration, the resulting product was washed with water and lyophilized to obtain alcohol (191) (105.5 mg, 207 µmol).
MS (ESI) m/z: 509.25 [M+H]⁺

### (19-16-3) Synthesis of Alcohol (127)

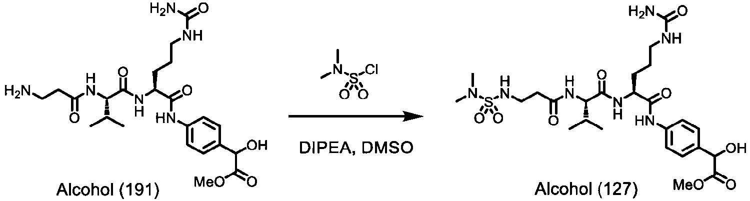

Alcohol (191) (104.3 mg, 205 µmol) was dissolved in dimethyl sulfoxide (2.05 mL), and N,N-diisopropylethylamine (105 µL, 615 µmol) and dimethylsulfamoyl chloride (32.7 µL, 308 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain Alcohol (127) (76.4 mg, 124 µmol).
MS (ESI) m/z: 616.40 [M+H]⁺

### (19-16-4) Synthesis of Pyrene (128)

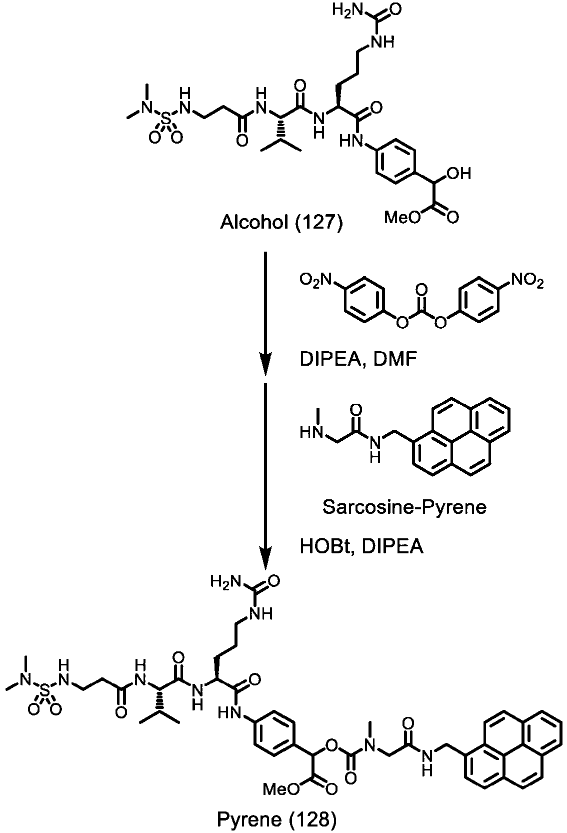

Alcohol (127) (7.2 mg, 11.7 µmol) was dissolved in N,N-dimethylformamide (117 µL) and stirred under ice cooling for 5 minutes, then bis(4-nitrophenyl) carbonate (10.7 mg, 35.1 µmol) and N,N-diisopropylethylamine (4.5 µL, 26.3 µmol) were added, and the mixture was stirred at room temperature for 1 hour. Subsequently, the mixture was cooled on ice, then Sarcosine-Pyrene (10.6 mg, 35.1 µmol), 1-hydroxybenzotriazole (2.4 mg, 17.6 µmol), and N,N-diisopropylethylamine (7.5 µL, 43.9 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain Pyrene (128) (7.5 mg, 7.9 µmol).
MS (ESI) m/z: 944.60 [M+H]⁺

### (19-16-5) Synthesis of Pyrene (129)

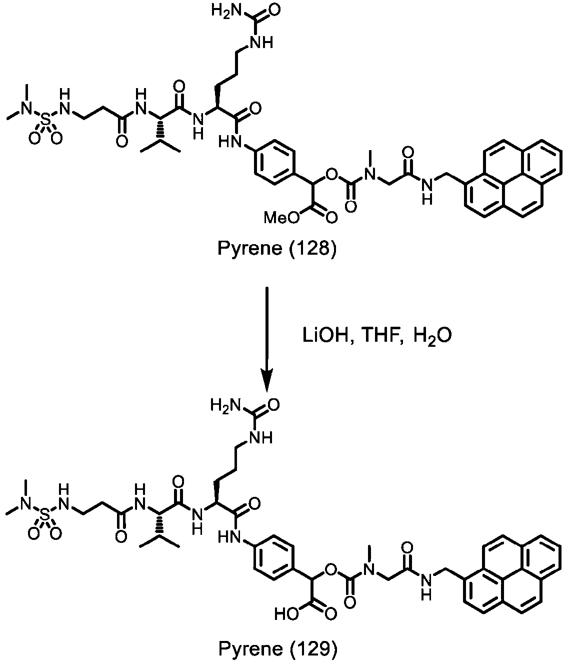

Pyrene (128) (29.0 mg, 30.7 µmol) was dissolved in tetrahydrofuran (2.33 mL) and water (775 µL) and stirred under ice cooling for 5 minutes, then 1 M aqueous lithium hydroxide solution (73.7 µL, 73.7 µmol) was added, and the mixture was stirred at room temperature for 2.5 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain Pyrene (129) (26.1 mg, 28.1 µmol).
MS (ESI) m/z: 930.40 [M+H]⁺

### (19-16-6) Synthesis of Linker-payload mimic (124)

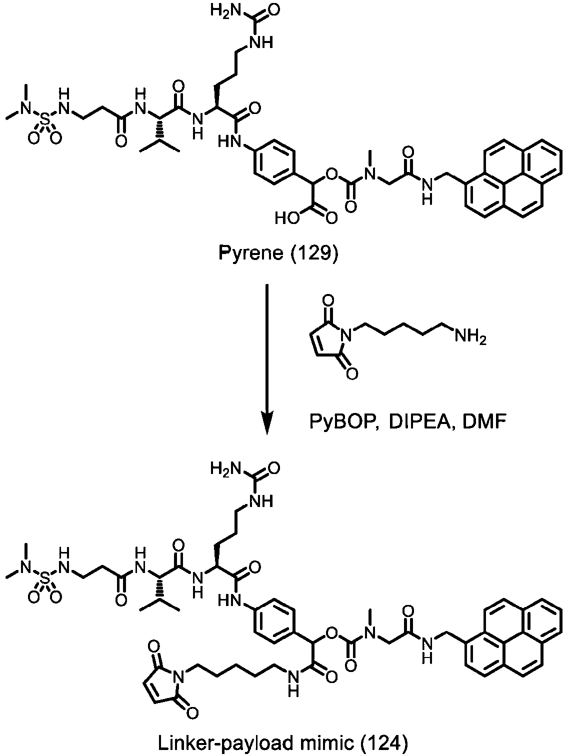

Pyrene (129) (12.9 mg, 13.9 µmol) was dissolved in N,N-dimethylformamide (278 µL) and cooled on ice, then N,N-diisopropylethylamine (4.7 µL, 27.8 µmol) and 1H-benzotriazol-1-yloxytripyrrolidinophosphonium hexafluorophosphate (10.8 mg, 20.8 µmol) were added. Next, N-(5-aminopentyl)maleimide hydrochloride (4.5 mg, 20.8 µmol) was added, and the mixture was returned to room temperature and stirred for 2 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (124) (10.5 mg, 9.6 µmol).
MS (ESI) m/z: 1094.50 [M+H]⁺

### (19-17) Synthesis of Linker-payload (130)

Linker-payload (130) was synthesized as follows.

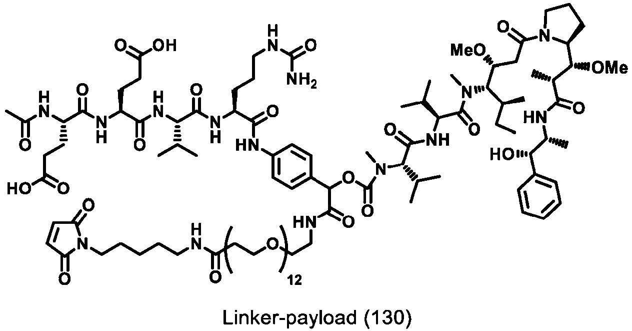

### (19-17-1) Synthesis of Compound (131)

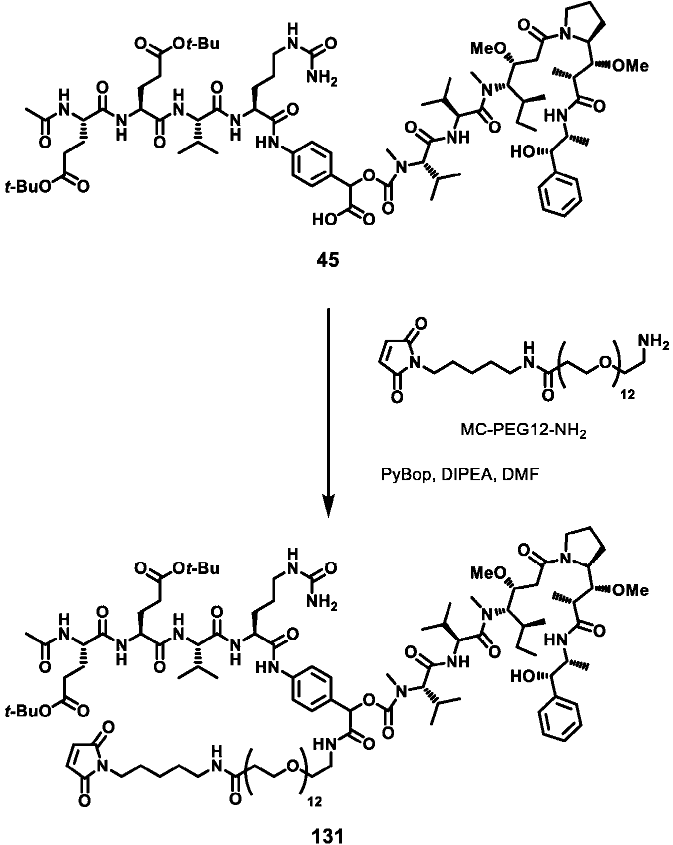

Compound (45) (50.0 mg, 31.6 µmol) was dissolved in N,N-dimethylformamide (1000 µL), and N,N-diisopropylethylamine (28 mg, 160 µmol) and 1H-benzotriazol-1-yloxytripyrrolidinophosphonium hexafluorophosphate (32.9 mg, 63 µmol) were added. Then, MC-PEG12-NH2 (56.0 mg, 63 µmol) was added, and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Compound (131) (51.0 mg, 21.8 µmol).
MS (ESI) m/z: 2344.43 [M+H]⁺

### (19-17-2) Synthesis of Linker-payload (130)

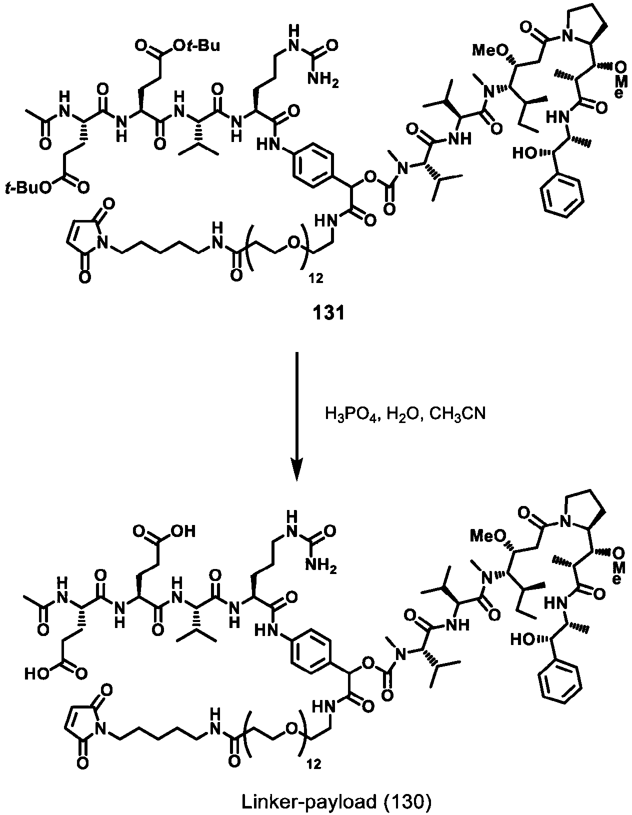

To Compound (131) (51.0 mg, 21.8 µmol) was added acetonitrile (1000 µL), and 85% phosphoric acid solution (1000 µL) was added under ice cooling, and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Linker-payload (130) (29.0 mg, 13.0 µmol).
MS (ESI) m/z: 2231.35 [M+H]⁺

### (19-18) Synthesis of Linker-payload (132)

Linker-payload (132) was synthesized as follows.

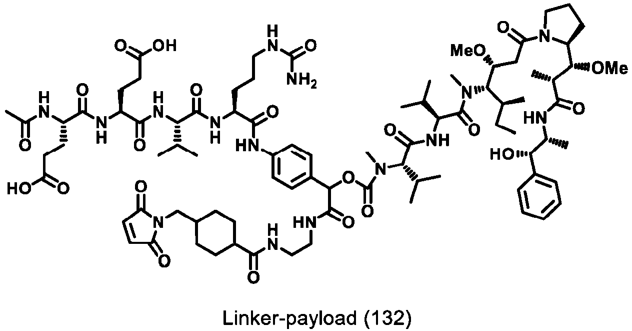

### (19-18-1) Synthesis of Compound (133)

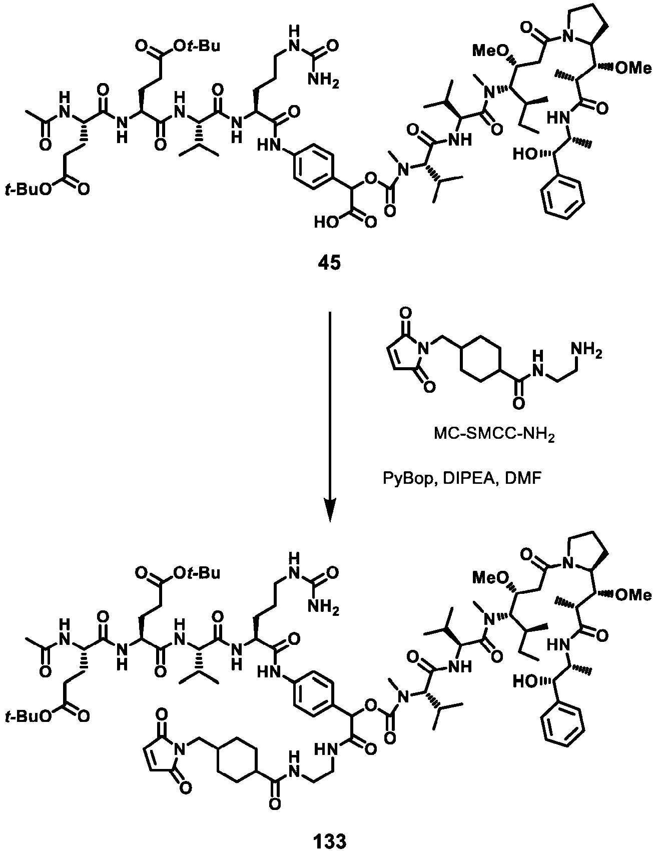

Compound (45) (50.0 mg, 31.6 µmol) was dissolved in N,N-dimethylformamide (1000 µL), and N,N-diisopropylethylamine (29 µL, 160 µmol) and 1H-benzotriazol-1-yloxytripyrrolidinophosphonium hexafluorophosphate (32.8 mg, 63 µmol) were added. Then, MC-SMCC-NH2 (35.3 mg, 63 µmol) was added, and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Compound (133) (23.0 mg, 12.5 µmol).
MS (ESI) m/z: 1841.98 [M+H]⁺

### (19-18-2) Synthesis of Linker-payload (132)

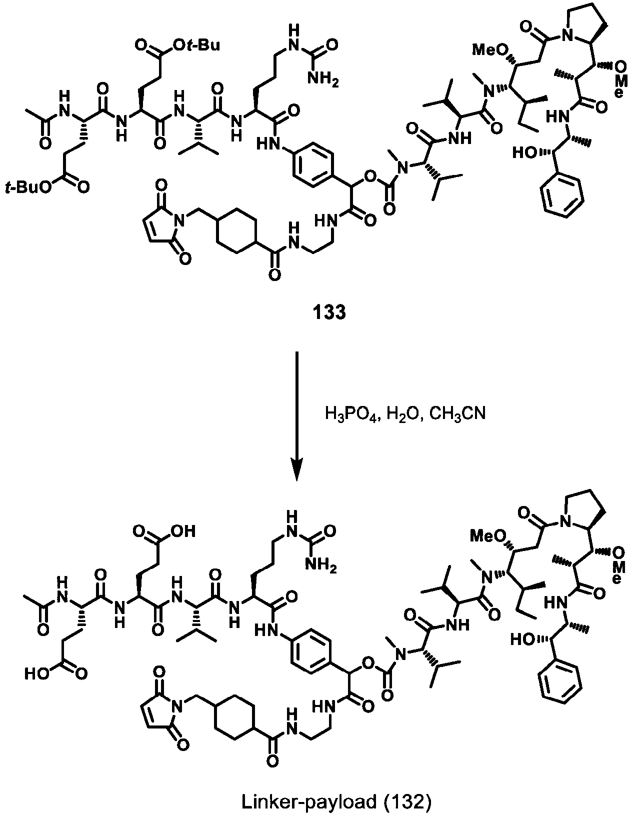

To Compound (133) (23.0 mg, 12.5 µmol) was added acetonitrile (1000 µL), and 85% phosphoric acid solution (1000 µL) was added under ice cooling, and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Linker-payload (132) (15.0 mg, 8.7 µmol).
MS (ESI) m/z: 1729.02 [M+H]⁺

### (19-19) Synthesis of Linker-payload (134)

Linker-payload (134) was synthesized as follows.

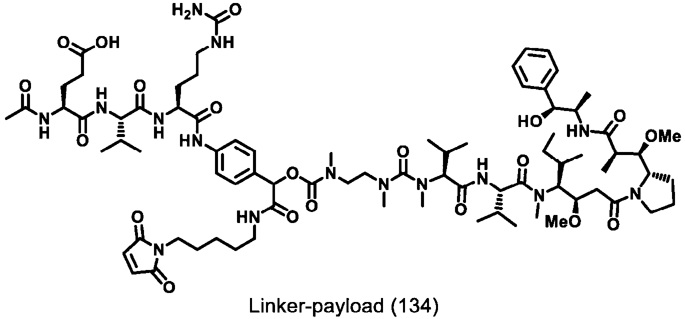

### (19-19-1) Synthesis of Compound (136)

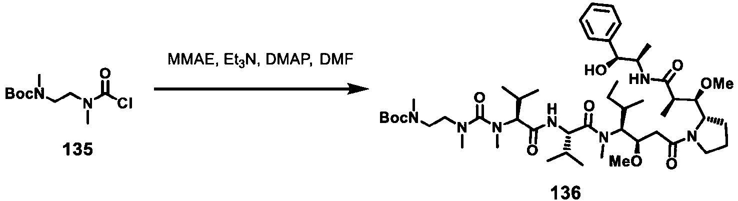

MMAE (106 mg, 0.084 mmol) and DMAP (2.0 mg, 0.016 mmol) were dissolved in N,N-dimethylformamide (1 mL), to which triethylamine (75 mg, 0.742 mmol) was then added at room temperature. Then, Compound (135) (74 mg, 0.285 mmol) was added, and the mixture was stirred at room temperature overnight. Subsequently, Compound (135) (74 mg, 0.285 mmol) was added after 24 hours, and Compound (135) (74 mg, 0.285 mmol) was further added after 24 hours. After the reaction solution was concentrated under reduced pressure, the residue was purified by preparative reversed-phase chromatography. Fractions containing the product were collected, and acetonitrile was removed by concentration under reduced pressure, followed by lyophilization to obtain Compound (136) (26.0 mg, 27.9 µmol).
MS (ESI) m/z: 932.73 [M+H]⁺

### (19-19-2) Synthesis of Compound (137)

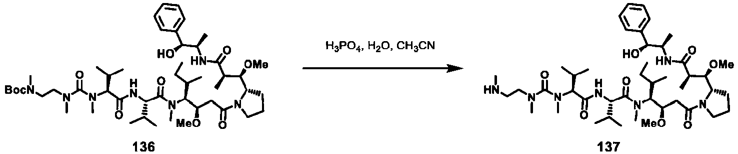

To Compound (136) (32.5 mg, 35 µmol) was added acetonitrile (1000 µL), and 85% phosphoric acid solution (1000 µL, 880 µmol) was added under ice cooling, and the mixture was stirred at room temperature for 2 hours. After the reaction was completed, the reaction mixture was purified by preparative reversed-phase chromatography. Fractions containing the product were collected, and acetonitrile was removed by concentration under reduced pressure, followed by lyophilization to obtain Compound (137) (22.0 mg, 26.5 µmol).
MS (ESI) m/z: 832.39 [M+H]⁺

### (19-19-3) Synthesis of Compound (138)

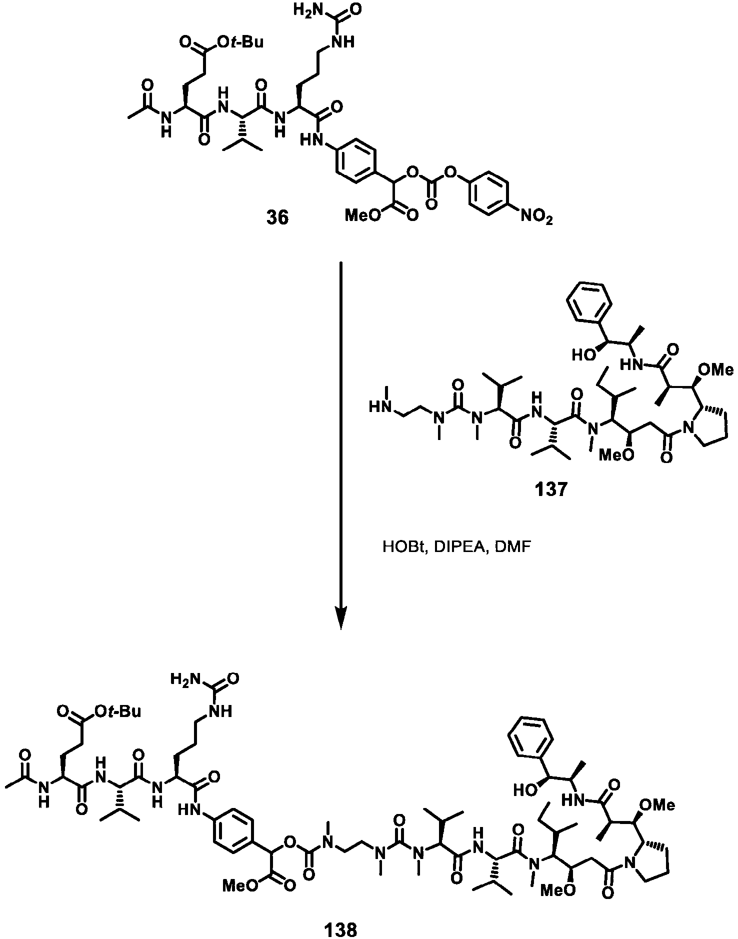

Compound (36) (18.0 mg, 22 µmol), Compound (137) (16.0 mg, 18 µmol), and HOBt (4.0 mg, 27 µmol) were dissolved in DMF (1 mL) at room temperature. To the reaction mixture was added DIPEA (12.0 mg, 93 µmol), and the mixture was stirred at room temperature for 1 hour. After the reaction was completed, the reaction mixture was purified by preparative reversed-phase chromatography. Fractions containing the product were collected, and acetonitrile was removed by concentration under reduced pressure, followed by lyophilization to obtain Compound (138) (22.0 mg, 14.4 µmol).
MS (ESI) m/z: 1523.60 [M+H]⁺

### (19-19-4) Synthesis of Compound (139)

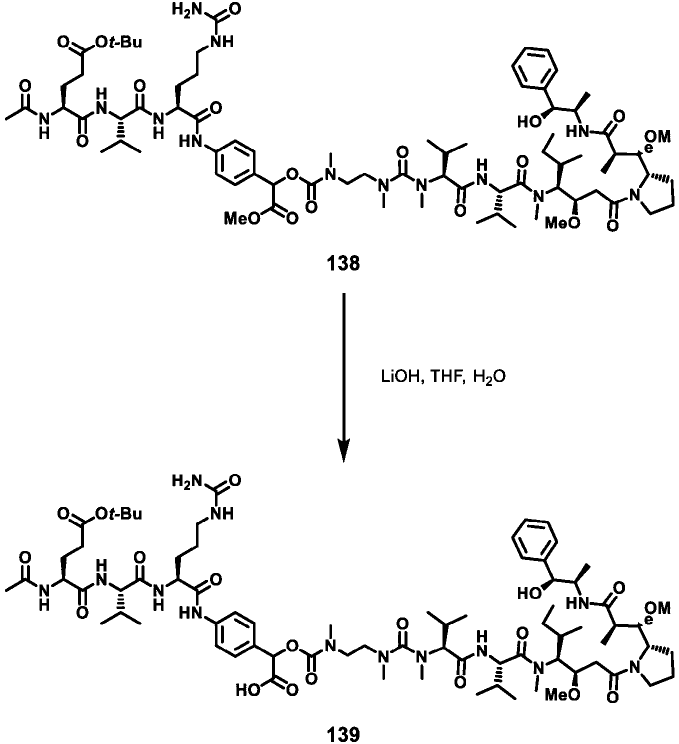

Compound (138) (22 mg, 14 µmol) was dissolved in tetrahydrofuran (1.6 mL) and water (0.4 mL) and stirred under ice cooling for 10 minutes, then lithium hydroxide (0.35 mg, 14 µmol) was added, and the mixture was stirred at room temperature for 10 hours. After the reaction was completed, the pH was adjusted to about 7 with formic acid, and the reaction mixture was purified by preparative reversed-phase chromatography. Fractions containing the product were collected, and acetonitrile was removed by concentration under reduced pressure, followed by lyophilization to obtain Compound (139) (14.5 mg, 9.6 µmol).
MS (ESI) m/z: 1508.47 [M+H]⁺

### (19-19-5) Synthesis of Compound (140)

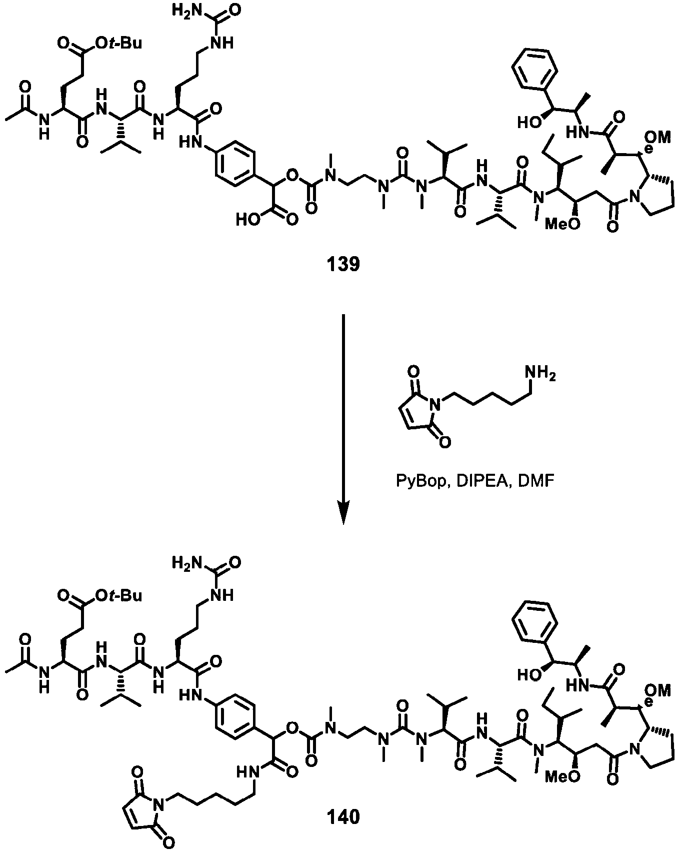

Compound (139) (14.5 mg, 9.6 µmol) was dissolved in N,N-dimethylformamide (1 mL), and N,N-diisopropylethylamine (7.0 µL, 38 µmol) and 1H-benzotriazol-1-yloxytripyrrolidinophosphonium hexafluorophosphate (10.0 mg, 19 µmol) were added. Next, N-(5-aminopentyl)maleimide hydrochloride (4.2 mg, 19 µmol) was added, and the mixture was stirred at room temperature for 1 hour. After the reaction was completed, the reaction mixture was purified by preparative reversed-phase chromatography. Fractions containing the product were collected, and acetonitrile was removed by concentration under reduced pressure, followed by lyophilization to obtain Compound (140) (15.0 mg, 9.0 µmol).
MS (ESI) m/z: 1673.74 [M+H]⁺

### (19-19-6) Synthesis of Linker-payload (134)

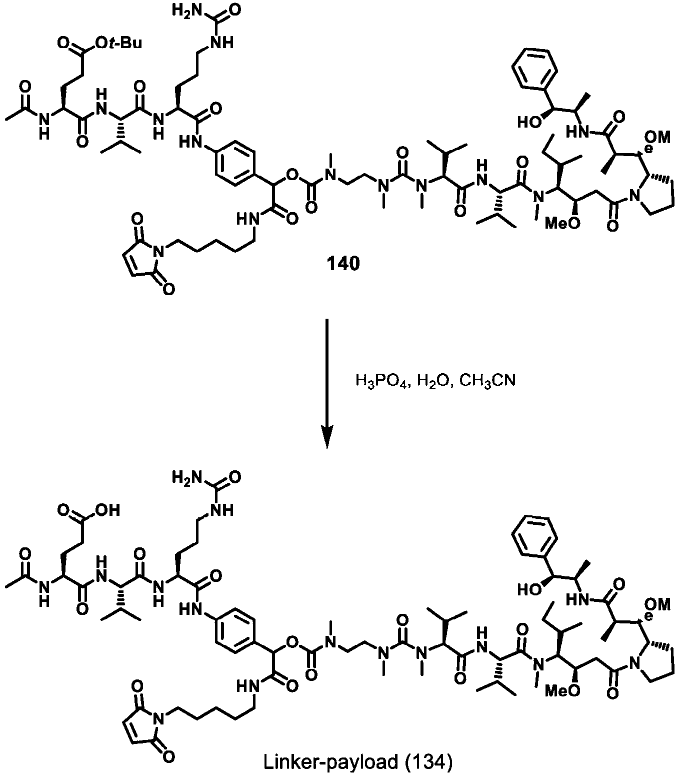

To Compound (140) (15.0 mg, 9.0 µmol) was added acetonitrile (0.4 mL), and 85% phosphoric acid solution (0.4 mL) was added under ice cooling, and the mixture was stirred at room temperature for 3 hours. Subsequently, the reaction mixture was purified by preparative reversed-phase chromatography. Fractions containing the product were collected, and acetonitrile was removed by concentration under reduced pressure, followed by lyophilization to obtain Linker-payload (134) (1.7 mg, 1.1 µmol).
MS (ESI) m/z: 1617.58 [M+H]⁺

### (19-20) Synthesis of Linker-payload (141)

Linker-payload (141) was synthesized as follows.

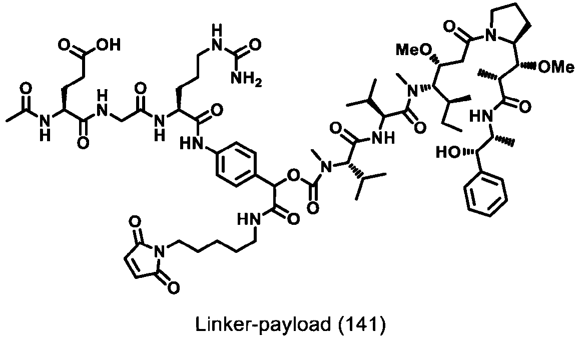

### (19-20-1) Synthesis of Compound (142)

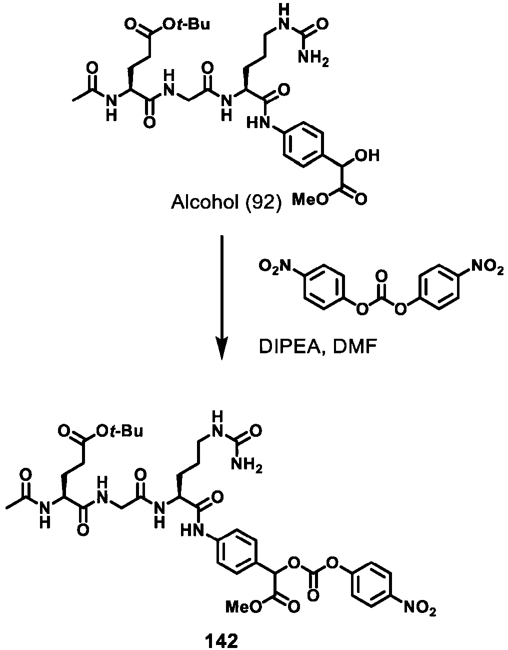

Alcohol (92) (95 mg, 150 µmol), bis(4-nitrophenyl) carbonate (116 mg, 380 µmol), and N,N-diisopropylethylamine (93 µL, 530 µmol) were dissolved in N,N-dimethylformamide (2 mL), and the mixture was stirred at room temperature overnight. After the reaction was completed, the reaction mixture was purified by normal-phase preparative chromatography. Fractions containing the product were collected and concentrated under reduced pressure to obtain the above-described Compound (142) (67 mg, 85 µmol).
MS (ESI) m/z: 788.27 [M+H]⁺

### (19-20-2) Synthesis of Compound (143)

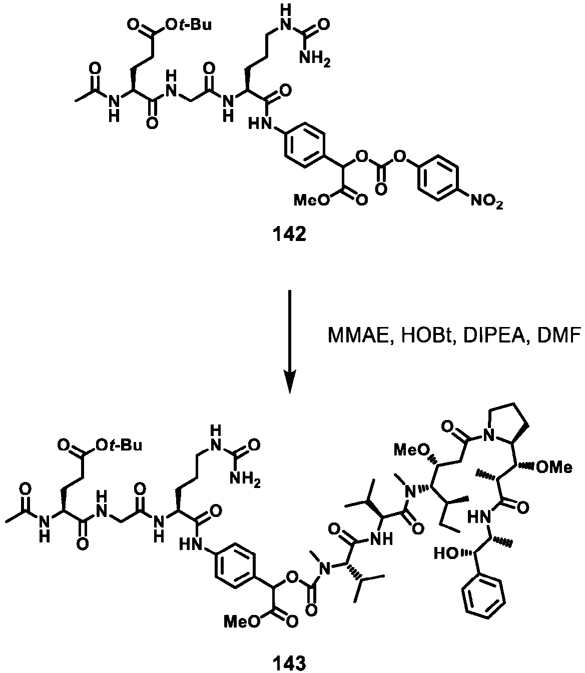

Compound (142) (70 mg, 80 µmol), MMAE (67 mg, 93 µmol), HOBt (18 mg, 132 µmol), and N,N-diisopropylethylamine (77 µL, 440 µmol) were dissolved in N,N-dimethylformamide (2 mL), and the mixture was stirred at room temperature overnight. After the reaction was completed, the reaction mixture was purified by normal-phase preparative chromatography. Fractions containing the product were collected and concentrated under reduced pressure to obtain the above-described Compound (143) (96 mg, 70 µmol).
MS (ESI) m/z: 1367.61 [M+H]⁺

### (19-20-3) Synthesis of Compound (144)

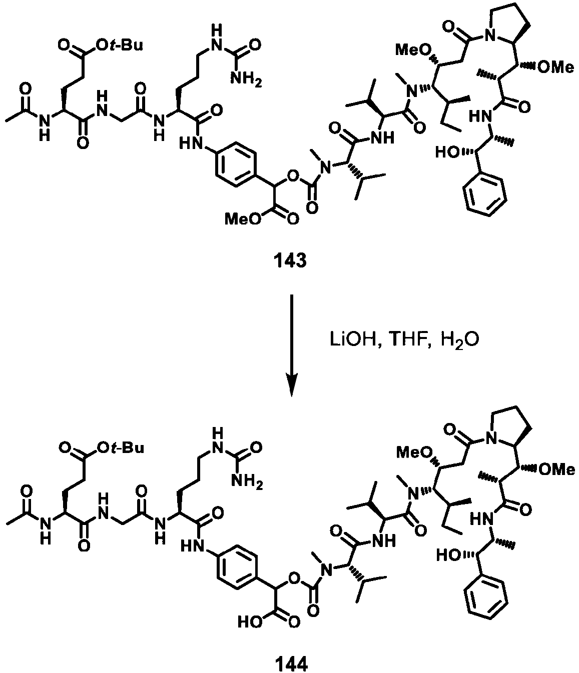

Compound (143) (96 mg, 70 µmol) was dissolved in tetrahydrofuran (1.0 mL) and water (0.3 mL) and stirred under ice cooling for 5 minutes, then 1 M aqueous lithium hydroxide solution (70 µL, 70 µmol) was added, and the mixture was stirred at room temperature for 1 hour. 1 M aqueous lithium hydroxide solution (35 µL, 35 µmol) was further added, and the mixture was stirred at room temperature for 30 minutes. After the reaction was completed, the pH was adjusted to about 7 with formic acid, and the reaction mixture was purified by preparative reversed-phase chromatography. Fractions containing the product were collected, and acetonitrile was removed by concentration under reduced pressure, followed by lyophilization to obtain Compound (144) (57 mg, 42 µmol).
MS (ESI) m/z: 1352.76 [M+H]⁺

### (19-20-4) Synthesis of Compound (145)

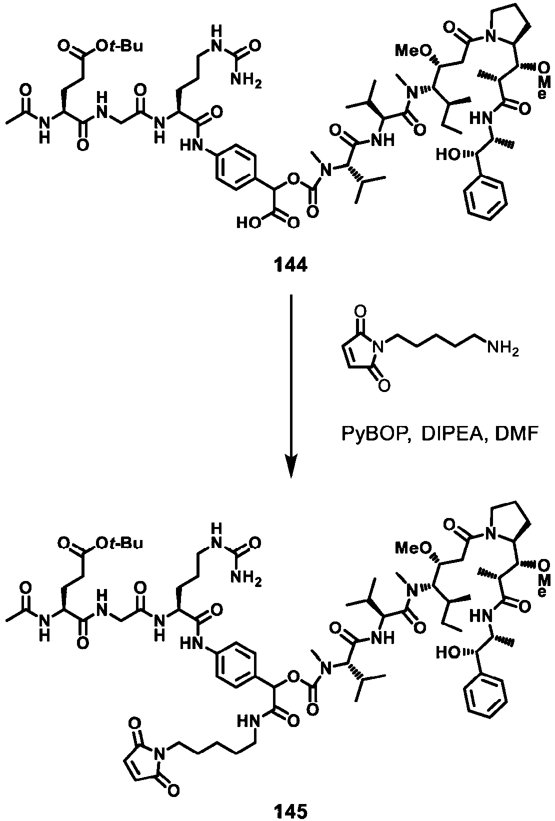

Compound (144) (57 mg, 42 µmol) was dissolved in N,N-dimethylformamide (1 mL) and cooled on ice, then N,N-diisopropylethylamine (37 µL, 210 µmol) and 1H-benzotriazol-1-yloxytripyrrolidinophosphonium hexafluorophosphate (44 mg, 84 µmol) were added. Next, N-(5-aminopentyl)maleimide hydrochloride (18.5 mg, 84 µmol) was added, and the mixture was returned to room temperature and stirred overnight. After the reaction was completed, the reaction mixture was purified by preparative reversed-phase chromatography. Fractions containing the product were collected, and acetonitrile was removed by concentration under reduced pressure, followed by lyophilization to obtain Compound (145) (43 mg, 28 µmol).
MS (ESI) m/z: 1516.65 [M+H]⁺

### (19-20-5) Synthesis of Linker-payload (141)

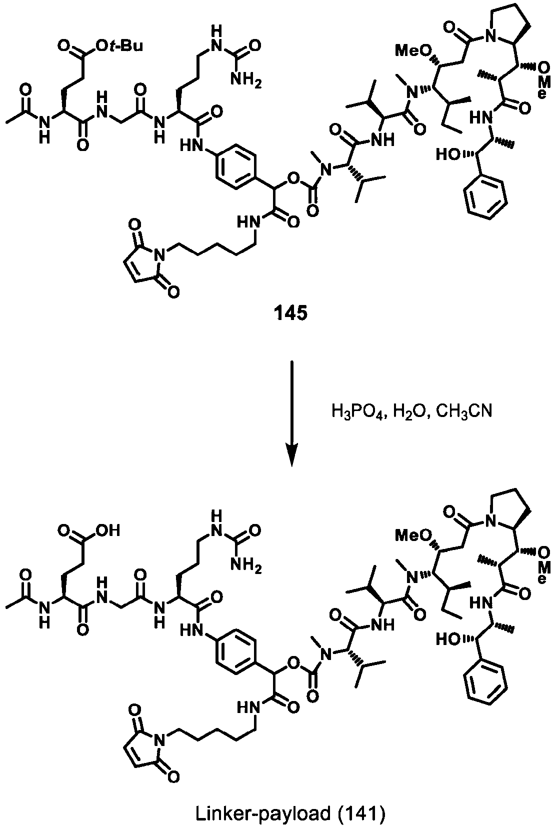

To Compound (145) (43 mg, 28 µmol) was added acetonitrile (1 mL), and 85% phosphoric acid solution (1 mL) was added under ice cooling, and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Linker-payload (141) (26 mg, 18 µmol).
MS (ESI) m/z: 1460.47 [M+H]⁺

### (19-21) Synthesis of Linker-payload (146)

Linker-payload (146) was synthesized as follows.

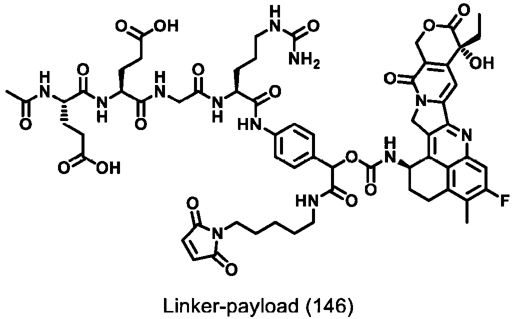

### (19-21-1) Synthesis of Compound (148)

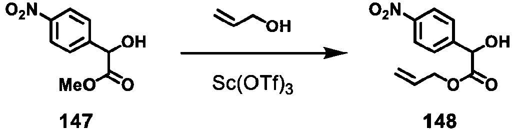

Compound (147) (CAS: 13305-09-4, 1.00 g, 4.74 mmol) was dissolved in allyl alcohol (50 mL), to which Sc(OTf)3 (233 mg, 0.474 mmol) was added. The reaction solution was stirred at 120°C for 3 hours, then cooled to room temperature and filtered through silica gel. The filtrate was concentrated under reduced pressure, and the residue was purified by normal-phase preparative chromatography (hexane: ethyl acetate = 4:1). Fractions containing the product were collected and concentrated under reduced pressure to obtain Compound (148) (793 mg, 3.34 mmol).
MS (ESI) m/z: 238.10 [M+H]⁺

### (19-21-2) Synthesis of Compound (149)

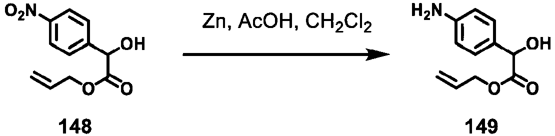

To Compound (148) (793 mg, 3.34 mmol) and acetic acid (670 µL, 11.7 mmol), dichloromethane (16.7 mL) was added at room temperature, followed by addition of zinc (4.37 g, 66.9 mmol), and the mixture was stirred for 2 hours. Water (50 mL) was added to the reaction solution, and extraction was performed with diethyl ether. The obtained organic layer was washed with saturated aqueous sodium bicarbonate and dried over magnesium sulfate. Magnesium sulfate was filtered off, the filtrate was concentrated under reduced pressure, and the residue was purified by normal-phase preparative chromatography (hexane: ethyl acetate = 90:10 to 67:33 to 50:50). Fractions containing the product were collected and concentrated under reduced pressure to obtain Compound (149) (546 mg, 2.63 mmol).
MS (ESI) m/z: 208.10 [M+H]⁺

### (19-21-3) Synthesis of Compound (150)

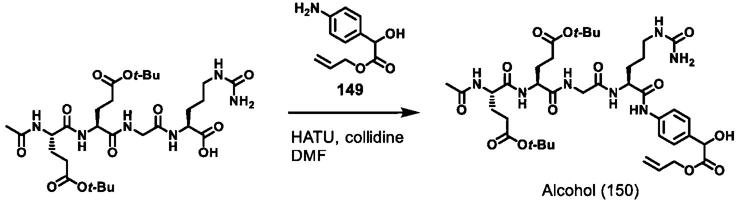

Ac-Glu(OtBu)-Glu(OtBu)-Gly-Cit-OH (200 mg, 310 µmol) and Compound (149) (154 mg, 745 µmol) were dissolved in N,N-dimethylformamide (1.55 mL). 1-[bis(dimethylamino)methylene]-1H-1,2,3,triazolo[4,5-b]pyridinium 3-oxide hexafluorophosphate (142 mg, 372 µmol) and 2,4,6-trimethylpyridine (49 µL, 372 µmol) were added, and the mixture was stirred at room temperature for 17 hours and then purified by preparative reversed-phase chromatography. Fractions containing the product were collected, and acetonitrile was removed by concentration under reduced pressure, followed by lyophilization to obtain Alcohol (150) (133 mg, 159 µmol).
MS (ESI) m/z: 834.45 [M+H]⁺

### (19-21-4) Synthesis of Compound (151)

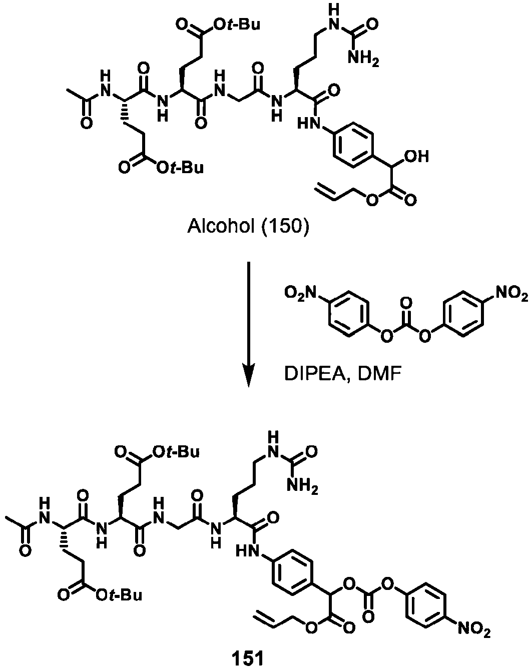

Alcohol (150) (100 mg, 120 µmol), bis(4-nitrophenyl) carbonate (78.5 mg, 258 µmol) and N,N-diisopropylethylamine (73 µL, 420 µmol) were dissolved in N,N-dimethylformamide (3.5 mL), and the mixture was stirred at room temperature overnight. After the reaction was completed, the reaction mixture was purified by preparative reversed-phase chromatography. Fractions containing the product were collected, and acetonitrile was removed by concentration under reduced pressure, followed by lyophilization to obtain Compound (151) (70.2 mg, 70.2 µmol).
MS (ESI) m/z: 999.49 [M+H]⁺

### (19-21-5) Synthesis of Compound (152)

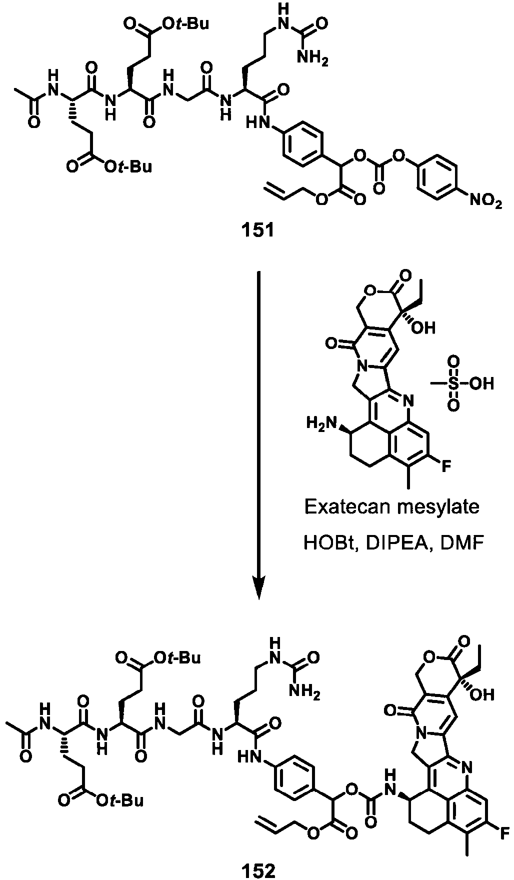

Compound (151) (70 mg, 80 µmol), exatecan mesylate (39 mg, 74 µmol), HOBt (16 mg, 120 µmol), and N,N-diisopropylethylamine (54 µL, 310 µmol) were dissolved in N,N-dimethylformamide (2.5 mL), and the mixture was stirred at room temperature for 2.5 hours. After the reaction was completed, the reaction mixture was purified by normal-phase preparative chromatography. Fractions containing the product were collected and concentrated under reduced pressure to obtain the above-described Compound (152) (80 mg, 62 µmol).
MS (ESI) m/z: 1295.43 [M+H]⁺

### (19-21-6) Synthesis of Compound (153)

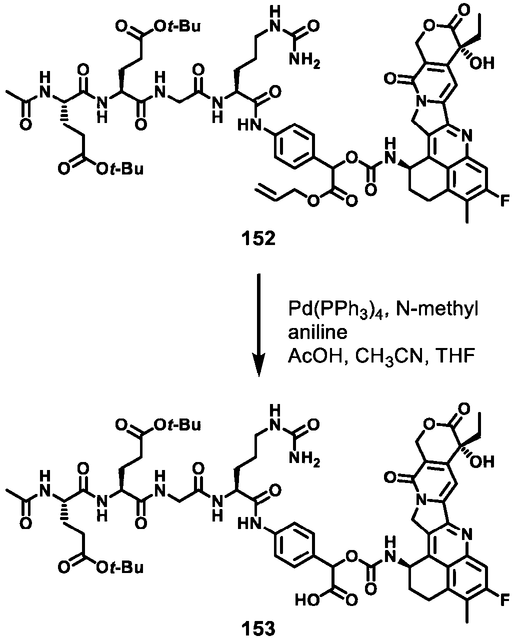

Compound (152) (80 mg, 62 µmol) was dissolved in tetrahydrofuran (1.5 mL) and acetonitrile (1.5 mL), and the mixture was stirred at room temperature for 10 minutes. To the reaction solution were added acetic acid (70 µL, 620 µmol), N-methylaniline (106 µL, 1.85 mmol), and Pd(PPh3)4 (3.6 mg, 3.1 µmol), and the mixture was stirred at room temperature for 25 hours. After the reaction was completed, the pH was adjusted to about 7 with formic acid, and the reaction mixture was purified by preparative reversed-phase chromatography. Fractions containing the product were collected, and acetonitrile was removed by concentration under reduced pressure, followed by lyophilization to obtain Compound (153) (54 mg, 43 µmol).
MS (ESI) m/z: 1255.50 [M+H]⁺

### (19-21-7) Synthesis of Compound (154)

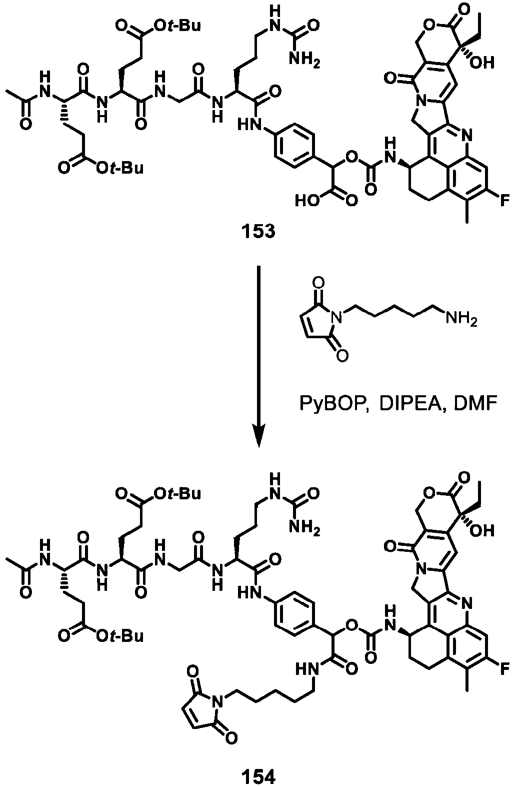

Compound (153) (54 mg, 43 µmol) was dissolved in N,N-dimethylformamide (2.5 mL) and cooled on ice, then N,N-diisopropylethylamine (31 µL, 172 µmol) and 1H-benzotriazol-1-yloxytripyrrolidinophosphonium hexafluorophosphate (45 mg, 86 µmol) were added. Next, N-(5-aminopentyl)maleimide hydrochloride (19 mg, 86 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Compound (154) (33 mg, 23 µmol).
MS (ESI) m/z: 1419.50 [M+H]⁺

### (19-21-8) Synthesis of Linker-payload (146)

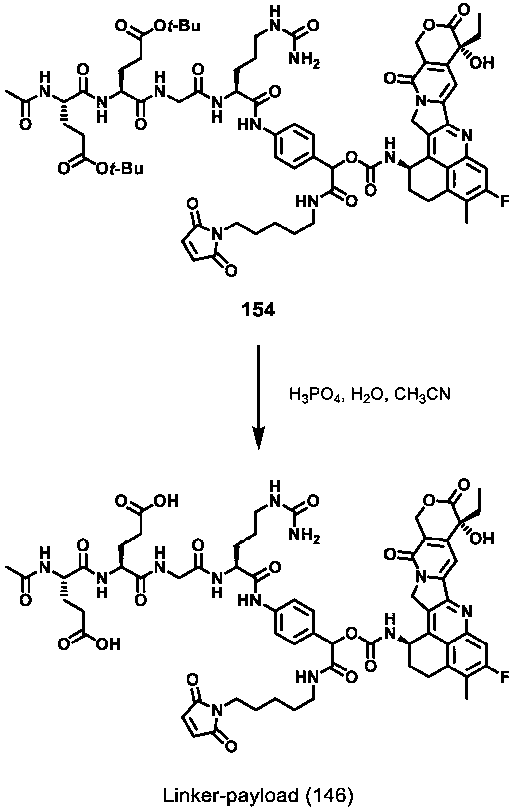

To Compound (154) (33 mg, 23 µmol) was added acetonitrile (0.7 mL), and 85% phosphoric acid solution (0.7 mL) was added under ice cooling, and the mixture was stirred at room temperature for 6 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload (146) (20 mg, 15 µmol).
MS (ESI) m/z: 1307.39 [M+H]⁺

### (19-22) Synthesis of Linker-payload mimic (155)

Linker-payload mimic (155) was synthesized as follows.

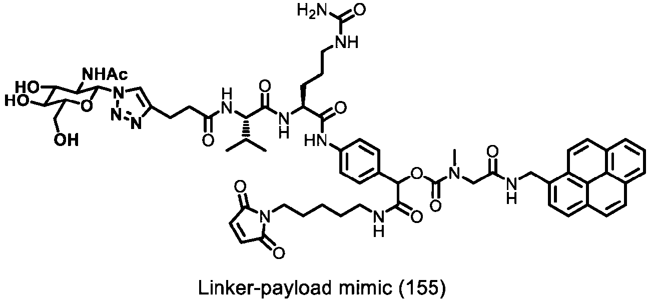

### (19-22-1) Synthesis of H-Val-Cit-OH

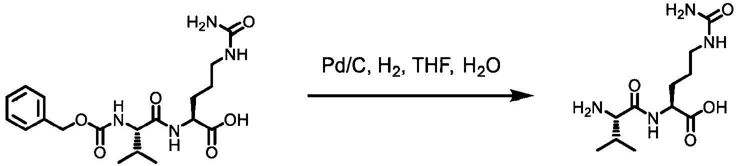

Z-Val-Cit-OH (50.0 mg, 122 µmol) was dissolved in ethyl acetate (875 µL), purified water (750 µL), and methanol (730 µL), then palladium on carbon catalyst (0.72 mg, 6.73 µmol) was added, and the atmosphere was replaced with hydrogen. The mixture was stirred at room temperature for 26 hours. After the reaction, the mixture was filtered through Celite, the filtrate was concentrated under reduced pressure to remove ethyl acetate and methanol, and then lyophilized to obtain the above-described H-Val-Cit-OH (35.0 mg, 12.8 µmol).
MS (ESI) m/z: 275.00 [M+H]⁺

### (19-22-2) Synthesis of Compound (156)

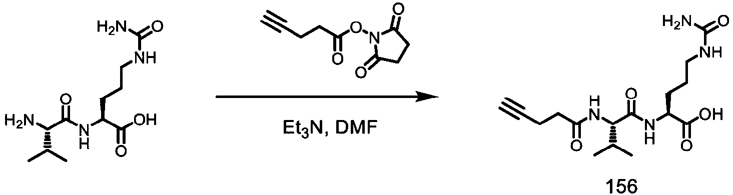

H-Val-Cit-OH (62.0 mg, 226 µmol) was dissolved in N,N-dimethylformamide (1.13 mL), to which 2, 5-dioxopyrrolidine-1-yl pent-4-ynoate (CAS No. 132178-37-1) (52.9 mg, 271 µmol) and N,N-diisopropylethylamine (34.0 µL, 249 µmol) were added. The mixture was stirred at room temperature for 2.5 hours, and then purified by preparative reversed-phase chromatography. Fractions containing the product were collected, and acetonitrile was removed by concentration under reduced pressure, followed by lyophilization to obtain the above-described Compound (156) (71.0 mg, 200 µmol).
MS (ESI) m/z: 355.10 [M+H]⁺

### (19-22-3) Synthesis of Alcohol (157)

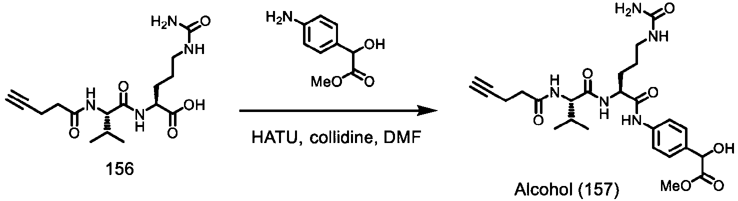

Compound (156) (30.5 mg, 86.1 µmol) was dissolved in N,N-dimethylformamide (430 µL), and 1-[bis(dimethylamino)methylene]-1H-1,2,3,triazolo[4,5-b]pyridinium 3-oxide hexafluorophosphate (39.3 mg, 103 µmol) and 2,4,6-trimethylpyridine (14.0 µL, 103 µmol) were added, followed by stirring at room temperature for 10 minutes. Subsequently, methyl 4-aminomandelate (18.7 mg, 103 µmol) was added, and the mixture was stirred at room temperature for 1.5 hours, followed by purification by preparative reversed-phase chromatography. Fractions containing the product were collected, and acetonitrile was removed by concentration under reduced pressure, followed by lyophilization to obtain the above-described Alcohol (157) (30.0 mg, 58.0 µmol).
MS (ESI) m/z: 518.25 [M+H]⁺

### (19-22-4) Synthesis of Alcohol (158)

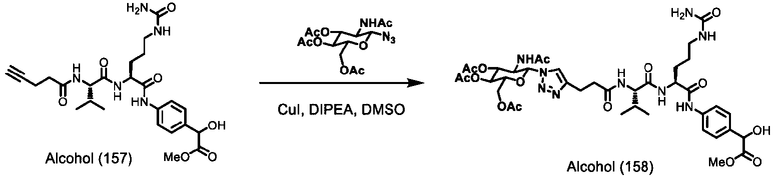

Alcohol (157) (17.0 mg, 32.8 µmol) and 2-acetamido-3,4,6-tri-O-acetyl-2-deoxy-β-D-glucopyranosyl azide (CAS No. 6205-69-2) (12.2 mg, 32.8 µmol) were dissolved in DMSO (164 µL), and N,N-diisopropylethylamine (9.8 µL, 57.5 µmol) and copper iodide (1.25 mg, 6.57 µmol) were added, and the mixture was stirred at room temperature for 1 hour. After the reaction was completed, the reaction mixture was purified by preparative reversed-phase chromatography. Fractions containing the product were collected, and acetonitrile was removed by concentration under reduced pressure, followed by lyophilization to obtain Alcohol (158) (12.0 mg, 13.5 µmol).
MS (ESI) m/z: 890.40 [M+H]⁺

### (19-22-5) Synthesis of Pyrene (159)

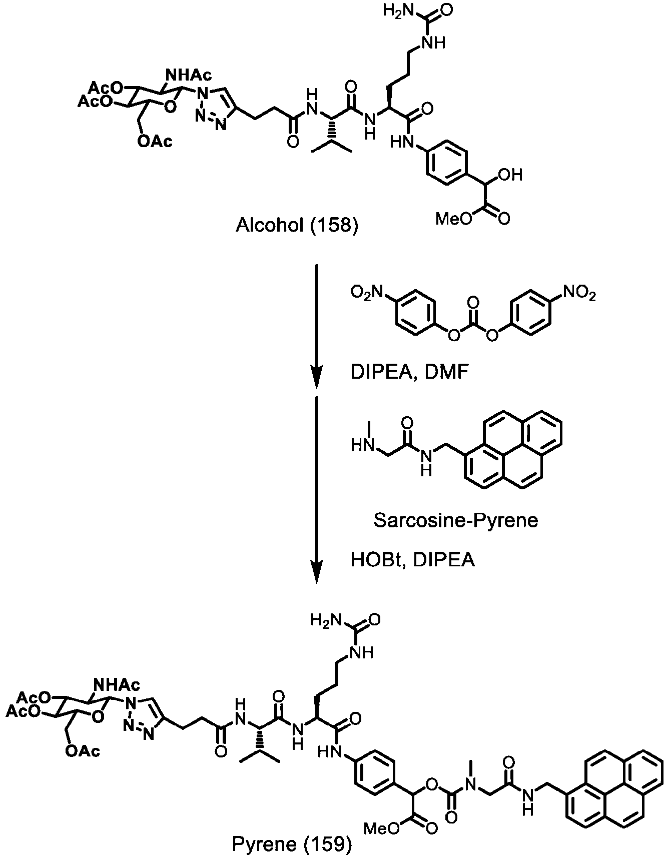

Alcohol (158) (11.0 mg, 12.4 µmol) was dissolved in N,N-dimethylformamide (124 µL) and stirred under ice cooling for 5 minutes, then bis(4-nitrophenyl) carbonate (11.3 mg, 37.1 µmol) and N,N-diisopropylethylamine (4.70 µL, 27.8 µmol) were added, and the mixture was stirred at room temperature for 2 hours. Subsequently, the mixture was cooled on ice, then Sarcosine-Pyrene (11.2 mg, 37.1 µmol), 1-hydroxybenzotriazole (2.52 mg, 18.5 µmol), and N,N-diisopropylethylamine (7.90 µL, 46.4 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain the above-described Pyrene (159) (9.5 mg, 7.8 µmol).
MS (ESI) m/z: 1218.60 [M+H]⁺

### (19-22-6) Synthesis of Pyrene (160)

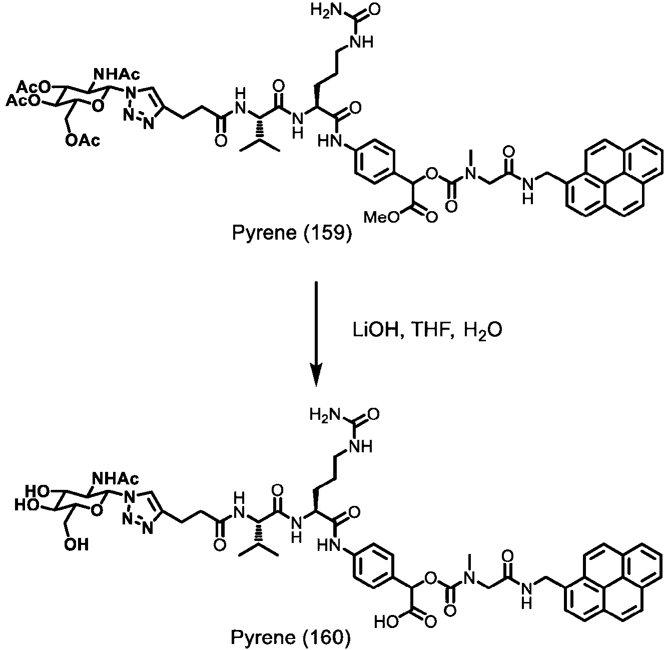

Pyrene (159) (8.5 mg, 7.0 µmol) was dissolved in tetrahydrofuran (450 µL) and water (150 µL) and stirred under ice cooling for 5 minutes, then 1 M aqueous lithium hydroxide solution (16.7 µL, 16.7 µmol) was added, and the mixture was stirred at room temperature for 2 hours. After the reaction was completed, the pH was adjusted to about 6 with 1 M hydrochloric acid, and the reaction mixture was purified by preparative reversed-phase chromatography. Fractions containing the product were collected, and acetonitrile was removed by concentration under reduced pressure, followed by lyophilization to obtain the above-described Pyrene (160) (5.2 mg, 4.8 µmol).
MS (ESI) m/z: 1078.45 [M+H]⁺

### (19-22-7) Synthesis of Linker-payload mimic (155)

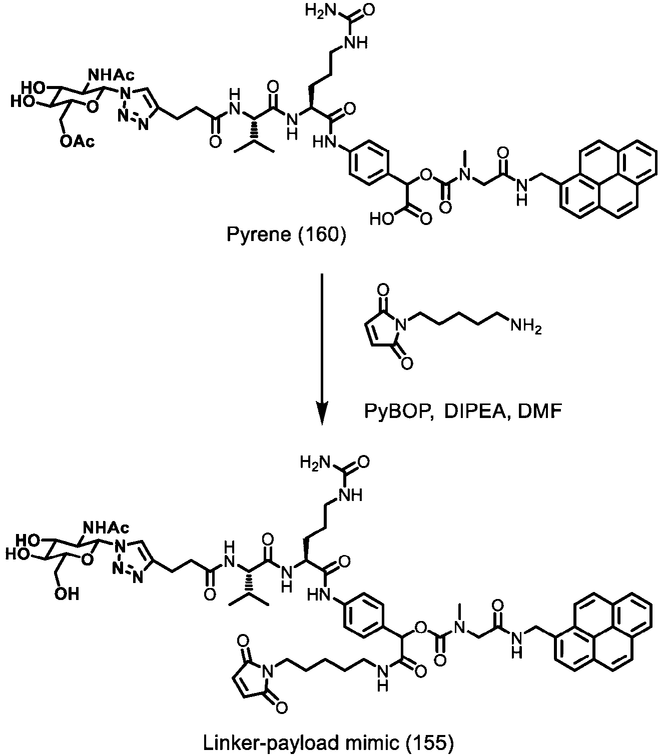

Pyrene (160) (4.2 mg, 3.9 µmol) was dissolved in N,N-dimethylformamide (100 µL) and cooled on ice, then N,N-diisopropylethylamine (0.99 µL, 5.84 µmol) and 1H-benzotriazol-1-yloxytripyrrolidinophosphonium hexafluorophosphate (781 µg, 4.29 µmol) were added. Next, N-(5-aminopentyl)maleimide hydrochloride (3.0 mg, 5.8 µmol) was added, and the mixture was returned to room temperature and stirred for 3 hours. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (155) (3.2 mg, 2.6 µmol).
MS (ESI) m/z: 1242.65 [M+H]⁺

### (19-23) Synthesis of Linker-payload mimic (161)

Linker-payload mimic (161) was synthesized as follows.

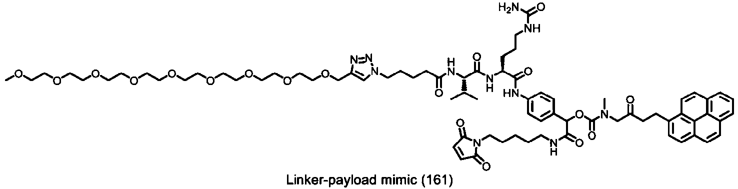

### (19-23-1) Synthesis of Compound (162)

By solid-phase peptide synthesis using Cl-TCP (Cl) ProTide Resin (CEM Corporation) by the Fmoc method, those capped with 5-azopentanoic acid at the N-terminus were prepared, and then stirred overnight in a 20% HFIP / dichloromethane solution to cleave the peptides from the resin while maintaining the protection of the amino acid side chains. The resin was removed by filtration, and the solution was concentrated, which was then purified by preparative HPLC to obtain Compound (162).
MS (ESI) m/z: 400.55 [M+H]⁺

### (19-23-2) Synthesis of Alcohol (163)

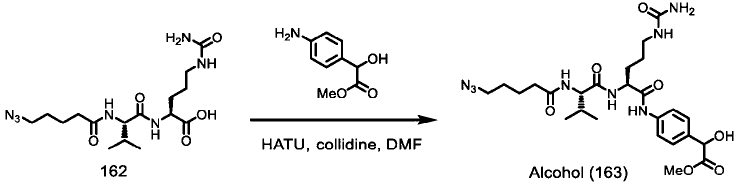

Compound (162) (50.0 mg, 125 µmol) was dissolved in N,N-dimethylformamide (626 µL), and 1-[bis(dimethylamino)methylene]-1H-1,2,3,triazolo[4,5-b]pyridinium 3-oxide hexafluorophosphate (57.1 mg, 150 µmol) and 2,4,6-trimethylpyridine (20.0 µL, 150 µmol) were added, followed by stirring at room temperature for 10 minutes. Subsequently, methyl 4-aminomandelate (27.2 mg, 150 µmol) was added, and the mixture was stirred at room temperature for 25 hours, followed by purification by preparative reversed-phase chromatography. Fractions containing the product were collected, and acetonitrile was removed by concentration under reduced pressure, followed by lyophilization to obtain the above-described Alcohol (163) (57.8 mg, 103 µmol).
MS (ESI) m/z: 563.30 [M+H]⁺

### (19-23-3) Synthesis of Alcohol (164)

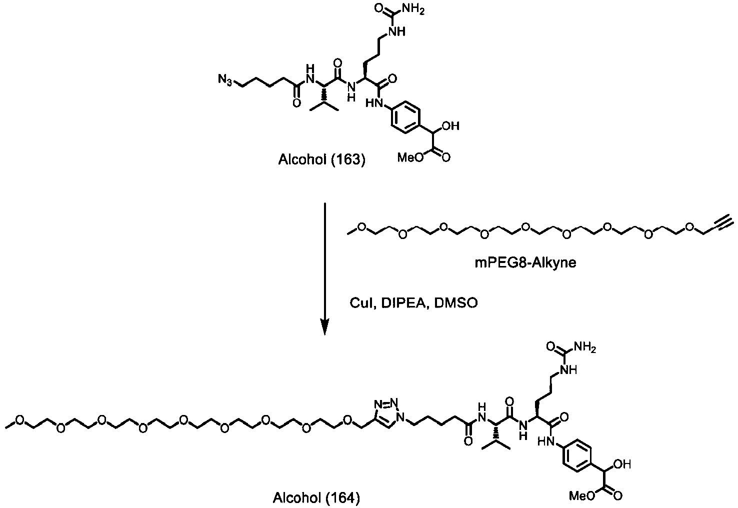

Alcohol (163) (46.0 mg, 81.8 µmol) and mPEG8-Alkyne (34.5 mg, 81.8 µmol) were dissolved in DMSO (409 µL), to which N,N-diisopropylethylamine (24 µL, 143 µmol) and copper iodide (7.79 mg, 40.9 µmol) were added, and the mixture was stirred at room temperature for 1 hour. After the reaction was completed, the reaction mixture was purified by preparative reversed-phase chromatography. Fractions containing the product were collected, and acetonitrile was removed by concentration under reduced pressure, followed by lyophilization to obtain Alcohol (164) (31.0 mg, 31.0 µmol).
MS (ESI) m/z: 985.55 [M+H]⁺

### (19-23-4) Synthesis of Pyrene (165)

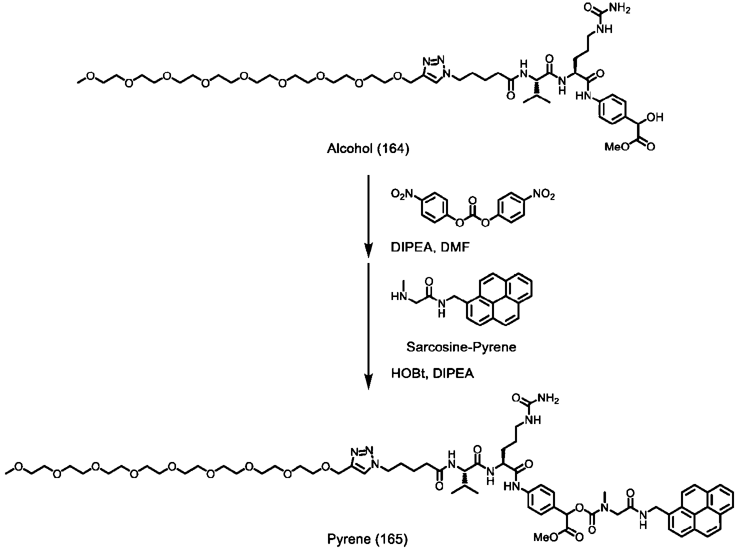

Alcohol (164) (31.0 mg, 31.5 µmol) was dissolved in N,N-dimethylformamide (315 µL) and stirred under ice cooling for 5 minutes, then bis(4-nitrophenyl) carbonate (28.7 mg, 94.4 µmol) and N,N-diisopropylethylamine (12.0 µL, 70.8 µmol) were added, and the mixture was stirred at room temperature for 2 hours. Subsequently, the mixture was cooled on ice, then Sarcosine-Pyrene (28.5 mg, 94.4 µmol), 1-hydroxybenzotriazole (6.42 mg, 47.2 µmol), and N,N-diisopropylethylamine (20.0 µL, 118 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain the above-described Pyrene (165) (29.0 mg, 22.1 µmol).
MS (ESI) m/z: 1313.80 [M+H]⁺

### (19-23-5) Synthesis of Pyrene (166)

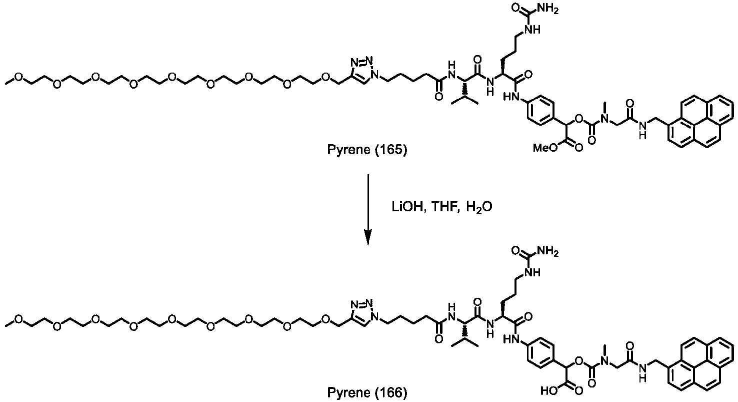

Pyrene (165) (29.0 mg, 22.1 µmol) was dissolved in tetrahydrofuran (1.55 mL) and water (0.55 mL) and stirred under ice cooling for 5 minutes, then 1 M aqueous lithium hydroxide solution (53.0 µL, 53.0 µmol) was added, and the mixture was stirred at room temperature for 1 hour. After the reaction was completed, the pH was adjusted to about 6 with acetic acid, and the reaction mixture was purified by preparative reversed-phase chromatography. Fractions containing the product were collected, and acetonitrile was removed by concentration under reduced pressure, followed by lyophilization to obtain the above-described Pyrene (166) (24.0 mg, 18.5 µmol).
MS (ESI) m/z: 1299.75 [M+H]⁺

### (19-23-6) Synthesis of Linker-payload mimic (161)

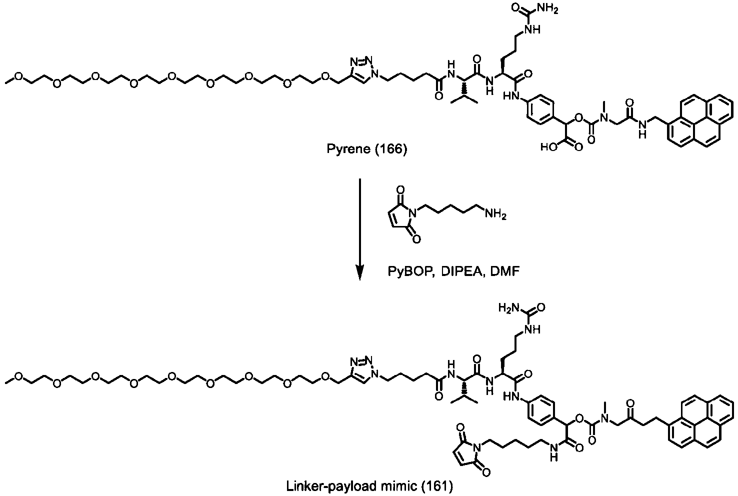

Pyrene (166) (23.0 mg, 17.7 µmol) was dissolved in N,N-dimethylformamide (200 µL) and cooled on ice, then N,N-diisopropylethylamine (6.0 µL, 4.58 µmol) and 1-[bis(dimethylamino)methylene]-1H-1,2,3,triazolo[4,5-b]pyridinium 3-oxide hexafluorophosphate (18.4 mg, 35.4 µmol) were added. Next, N-(5-aminopentyl)maleimide hydrochloride (4.84 mg, 35.4 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (161) (16.0 mg, 17.7 µmol).
MS (ESI) m/z: 1463.90 [M+H]⁺

### (19-24) Synthesis of Linker-payload mimic (167)

Linker-payload mimic (167) was synthesized as follows.

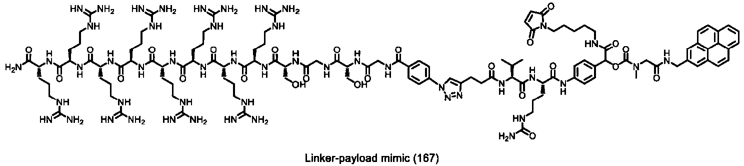

### (19-24-1) Synthesis of Pyrene (168)

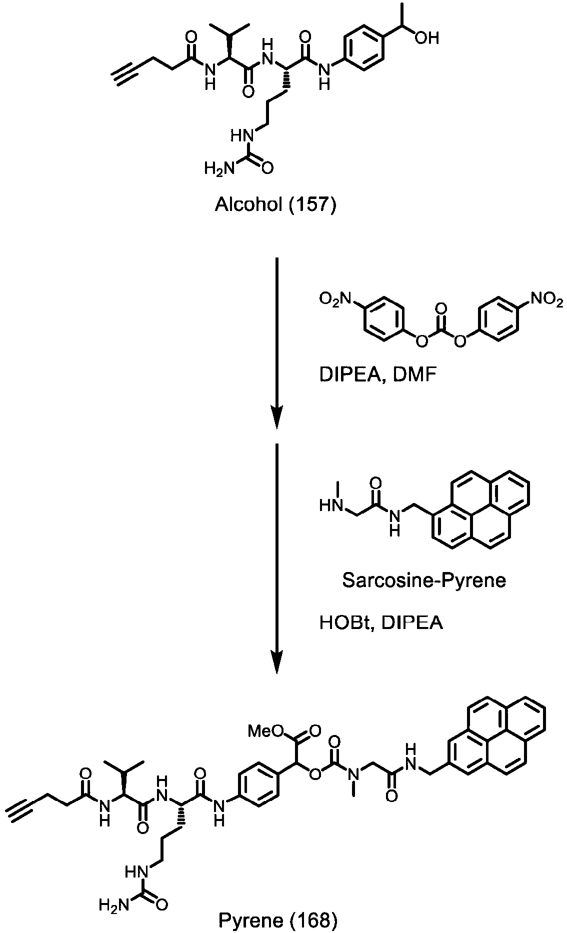

Alcohol (157) (26.0 mg, 50.2 µmol) was dissolved in N,N-dimethylformamide (502 µL) and stirred under ice cooling for 5 minutes, then bis(4-nitrophenyl) carbonate (45.8 mg, 151 µmol) and N,N-diisopropylethylamine (19.0 µL, 113 µmol) were added, and the mixture was stirred at room temperature for 2 hours. Subsequently, the mixture was cooled on ice, then Sarcosine-Pyrene (45.6 mg, 151 µmol), 1-hydroxybenzotriazole (10.3 mg, 75.4 µmol), and N,N-diisopropylethylamine (32.0 µL, 188 µmol) were added, and the mixture was stirred at room temperature for 1 hour. After the reaction, the reaction mixture was purified by preparative reversed-phase chromatography. Fractions containing the product were collected, and acetonitrile was removed by concentration under reduced pressure, followed by lyophilization to obtain the above-described Pyrene (168) (22 mg, 26 µmol).
MS (ESI) m/z: 846.40 [M+H]⁺

### (19-24-2) Synthesis of Pyrene (169)

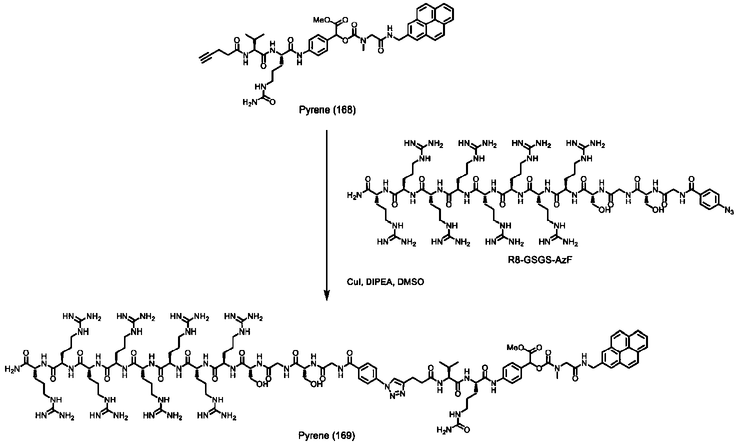

Pyrene (168) (10.0 mg, 11.8 µmol) and R₈-GSGS-AzF (20.1 mg, 11.8 µmol) were dissolved in DMSO (118 µL), to which N,N-diisopropylethylamine (3.5 µL, 21 µmol) and copper iodide (0.45 mg, 2.4 µmol) were added, and the mixture was stirred at room temperature for 1 hour. After the reaction was completed, the reaction mixture was purified by preparative reversed-phase chromatography. Fractions containing the product were collected, and acetonitrile was removed by concentration under reduced pressure, followed by lyophilization to obtain Pyrene (169) (19.2 mg, 7.54 µmol).
MS (ESI) m/z: 1273.65 [M+2H]²⁺, 849.55 [M+3H]³⁺, 637.45 [M+4H]⁴⁺, 510.10 [M+5H]⁵⁺

### (19-24-3) Synthesis of Pyrene (170)

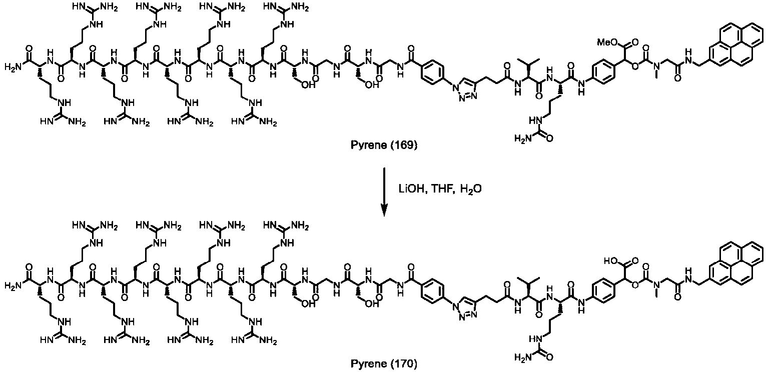

Pyrene (169) (15.6 mg, 6.13 µmol) was dissolved in tetrahydrofuran (234 µL) and water (110 µL) and stirred under ice cooling for 5 minutes, then 1 M aqueous lithium hydroxide solution (14.7 µL, 14.7 µmol) was added, and the mixture was stirred at room temperature for 1 hour. After the reaction was completed, the pH was adjusted to about 6 with acetic acid, and the reaction mixture was purified by preparative reversed-phase chromatography. Fractions containing the product were collected, and acetonitrile was removed by concentration under reduced pressure, followed by lyophilization to obtain the above-described Pyrene (170) (8.8 mg, 3.5 µmol).
MS (ESI) m/z: 1266.70 [M+2H]²⁺, 844.90 [M+3H]³⁺, 633.90 [M+4H]⁴⁺, 507.30 [M+5H]⁵⁺

### (19-24-4) Synthesis of Linker-payload mimic (167)

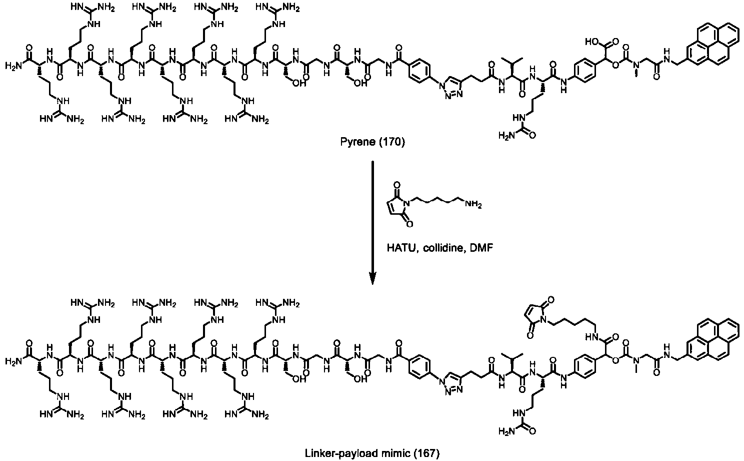

Pyrene (170) (8 mg, 3 µmol) was dissolved in N,N-dimethylformamide (30 µL) and cooled on ice, then N,N-diisopropylethylamine (1 µL, 6 µmol) and 1-[bis(dimethylamino)methylene]-1H-1,2,3,triazolo[4,5-b]pyridinium 3-oxide hexafluorophosphate (2 mg, 5 µmol) were added. Next, N-(5-aminopentyl)maleimide hydrochloride (1 mg, 5 µmol) was added, and the mixture was returned to room temperature and stirred for 1 hour. After the reaction was completed, the reaction mixture was purified by preparative reversed-phase chromatography. Fractions containing the product were collected, and acetonitrile was removed by concentration under reduced pressure, followed by lyophilization to obtain Linker-payload mimic (167) (4.1 mg, 1.5 µmol).
MS (ESI) m/z: 899.65 [M+3H]³⁺, 675.00 [M+4H]⁴⁺, 540.20 [M+5H]⁵⁺

### (19-25) Synthesis of Linker-payload mimic (171)

Linker-payload mimic (171) was synthesized as follows.

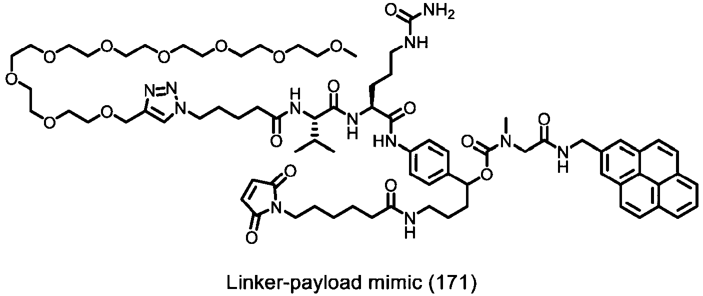

### (19-25-1) Synthesis of Compound (173)

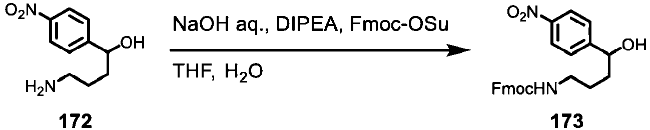

Compound (172) (97.0 mg, 461 µmol) was dissolved in tetrahydrofuran (4.25 mL) and water (4.02 mL). Under ice cooling, 2 N aqueous sodium hydroxide solution (233 µL, 466 µmol), N,N-diisopropylethylamine (103 µL, 600.0 µmol), and N-(Fmoc-oxy)succinimide (203 mg, 601 µmol) were added. After the mixture was returned to room temperature and stirred for 1 hour, the reaction solution was concentrated under reduced pressure to remove tetrahydrofuran. Ethyl acetate (20 mL) and saturated brine (2 mL) were added to the residue and the mixture was extracted, and the aqueous layer was re-extracted with ethyl acetate (10 mL). The combined organic layers were washed with saturated brine (5 mL) and concentrated under reduced pressure, then the resulting residue was purified by column chromatography on silica gel (hexane: ethyl acetate), and the fractions containing the desired product were concentrated to dryness to obtain Compound (173) (178 mg, 412 µmol).
MS (ESI) m/z: 455.1 [M+Na]⁺

### (19-25-2) Synthesis of Compound (174)

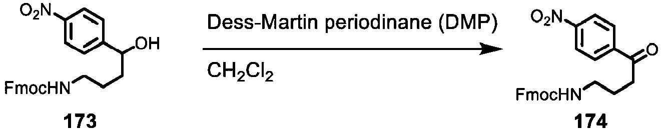

Compound (173) (91.9 mg, 212 µmol) was dissolved in dichloromethane (1.6 mL), to which DMP (133 mg, 297 µmol) was added under ice cooling. The mixture was returned to room temperature and stirred, and after confirming the disappearance of the starting material by TLC, the reaction solution was concentrated under reduced pressure to remove dichloromethane. Ethyl acetate (15 mL), water (3 mL), 10% aqueous sodium hydrogen carbonate solution (2 mL), and saturated brine (1 mL) were added to the residue for extraction, and the aqueous layer was re-extracted with ethyl acetate (10 mL). The organic layers were combined, washed with saturated brine (10 mL), and then concentrated to dryness to obtain Compound (174) (91.2 mg, 212 µmol).
MS (ESI) m/z: 453.1 [M+Na]⁺

### (19-25-3) Synthesis of Compound (175)

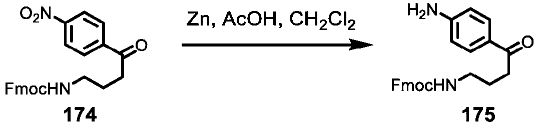

Compound (174) (90.2 mg, 210 µmol) was dissolved in dichloromethane (46 mL), to which acetic acid (84.0 µL, 1.467 mmol) and zinc powder (139 mg, 2.12 mmol) were added. The mixture was heated to 40°C using a heat block, and after 1 hour, LC-MS analysis showed that the starting material remained. Therefore, acetic acid (42.0 µL, 0.727 mmol) and zinc powder (68.6 mg, 1.05 mmol) were added. However, since the starting material still remained, acetic acid (21.0 µL, 0.367 mmol) and zinc powder (35.0 mg, 0.535 mmol) were added again. After confirming the disappearance of the starting material, the reaction solution was cooled, water (10 mL) was added, and the mixture was concentrated under reduced pressure to remove dichloromethane. Water (10 mL) was added to the residue, and 10% aqueous sodium hydrogen carbonate solution (4.0 mL) was added to adjust the pH to about 9. Ethyl acetate (30 mL) and saturated brine (1 mL) were added for extraction, and the aqueous layer was re-extracted twice with ethyl acetate (15 mL). The organic layers were combined, washed with saturated brine (10 mL) and 10% aqueous sodium hydrogen carbonate solution (1.0 mL), and then concentrated under reduced pressure. The resulting residue was diluted and dissolved in ethyl acetate and purified by column chromatography on silica gel (hexane: ethyl acetate). Fractions containing the product were concentrated to dryness to obtain Compound (175) (69.7 mg, 174 µmol).
MS (ESI) m/z: 401.1 [M+H]⁺

### (19-25-4) Synthesis of Compound (176)

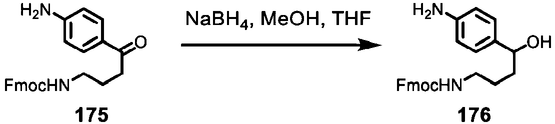

Compound (175) (67.7 mg, 169 µmol) was dissolved in tetrahydrofuran (8.1 mL) and methanol (2.7 mL). Under ice cooling, sodium borohydride (61.3 mg, 1.63 mmol) was added. After 15 minutes, LC-MS analysis showed that the starting material remained. Therefore, about the same amount of sodium borohydride was added three times until the starting material disappeared (total 275 mg, 6.69 mmol). After confirming the disappearance of the starting material, water (15 mL) was added to the reaction solution, the mixture was returned to room temperature, and concentrated under reduced pressure to remove the organic solvent. Ethyl acetate (50 mL) and saturated brine (5 mL) were added to the obtained solution for extraction, and the organic layer was concentrated under reduced pressure. The residue was purified by column chromatography on silica gel (hexane: ethyl acetate), and the fractions containing the desired product were concentrated to dryness to obtain Compound (176) (47.3 mg, 118 µmol).
MS (ESI) m/z: 426.1 [M+Na]⁺

### (19-25-5) Synthesis of Compound (177)

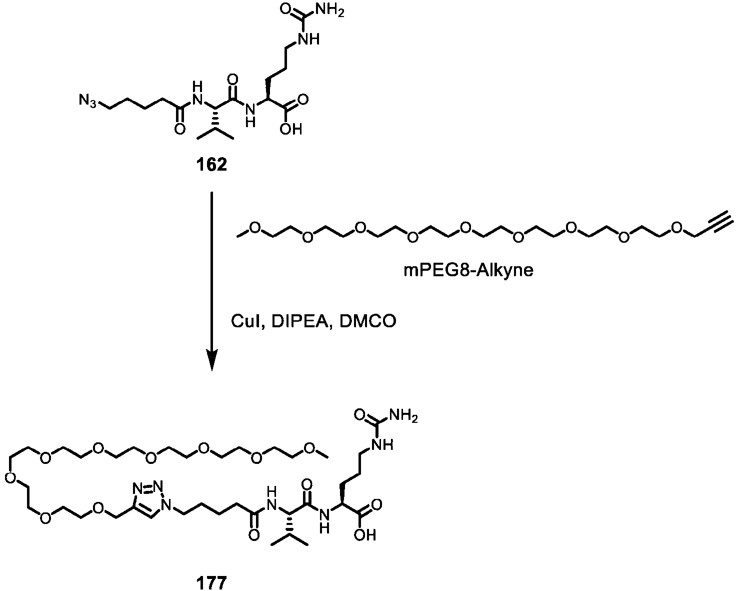

mPEG8-Alkyne (65.0 mg, 154 µmol) was dissolved in dimethyl sulfoxide (768 µL), and Compound (162) (61.4 mg, 154 µmol), N,N-diisopropylethylamine (46.2 µL, 270 µmol), and copper iodide (6.2 mg, 33.0 µmol) were added. After confirming the disappearance of the starting material by LC-MS analysis, the mixture was diluted by adding acetonitrile (384 µL) and 50% aqueous acetonitrile solution (384 µL). The filtered solution was purified by preparative HPLC, and the fractions containing the desired product were collected. After removing acetonitrile by concentration under reduced pressure, the residue was lyophilized to obtain Compound (177) (90.4 mg, 110 µmol).
MS (ESI) m/z: 822.6 [M+H]⁺

### (19-25-6) Synthesis of Alcohol (178)

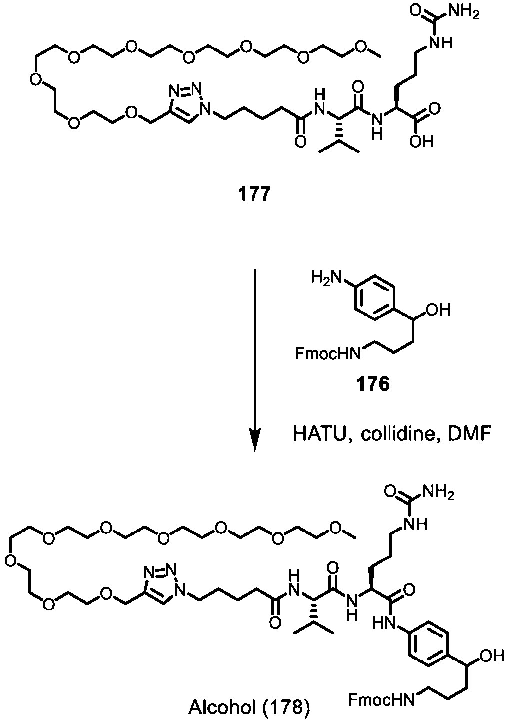

Compound (177) (63.5 mg, 77.3 µmol) was dissolved in N,N-dimethylformamide (400 µL). HATU (35.8 mg, 94.2 µmol) and 2,4,6-trimethylpyridine (12.9 µL, 96.0 µmol) were added and the mixture was stirred at room temperature for 10 minutes. Compound (176) (23.1 mg, 57.4 µmol) dissolved in N,N-dimethylformamide (300 µL) was added, and the mixture was allowed to react for 1 hour. After confirming the disappearance of the starting material by LC-MS analysis, the reaction mixture was diluted by adding acetonitrile (700 µL). The filtered solution was purified by preparative HPLC, and the fractions containing the desired product were collected. After removing acetonitrile by concentration under reduced pressure, the residue was lyophilized to obtain Alcohol (178) (51.2 mg, 57.4 µmol).
MS (ESI) m/z: 1206.8 [M+H]⁺

### (19-25-7) Synthesis of Pyrene (179)

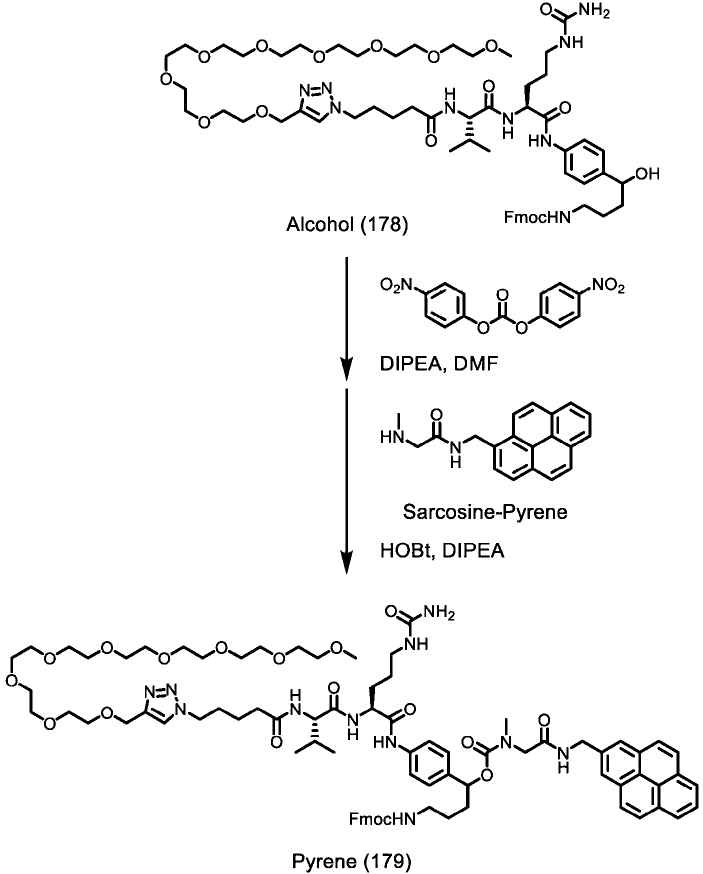

Alcohol (178) (36.7 mg, 30.4 µmol) was dissolved in N,N-dimethylformamide (174 µL), and then bis(4-nitrophenyl) carbonate (23.5 mg, 75.7 µmol) and N,N-diisopropylethylamine (10.4 µL, 60.5 µmol) were added. LC-MS analysis revealed the presence of remaining starting material, and therefore bis(4-nitrophenyl) carbonate (24.0 mg, 77.3 µmol) and N,N-diisopropylethylamine (10.4 µL, 60.5 µmol) were added. After 1 hour, 2-(methylamino)-N-(pyrene-2-ylmethyl)acetamide (16.3 mg, 53.9 µmol), HOBt (6.2 mg, 46.0 µmol), and N,N-diisopropylethylamine (20.9 µL, 121.6 µmol) were added and the reaction was continued. After about 45 minutes, 2-(methylamino)-N-(pyrene-2-ylmethyl)acetamide (7.7 mg, 25.4 µmol) was added. Acetonitrile (348 µL) was added to the reaction solution for dilution, then filtered, and the resulting solution was purified by preparative HPLC, and the fractions containing the desired product were collected. After removing acetonitrile by concentration under reduced pressure, the residue was lyophilized to obtain Pyrene (179) (3.8 mg, 2.5 µmol).
MS (ESI) m/z: 1536.1 [M+H]⁺

### (19-25-8) Synthesis of Pyrene (180)

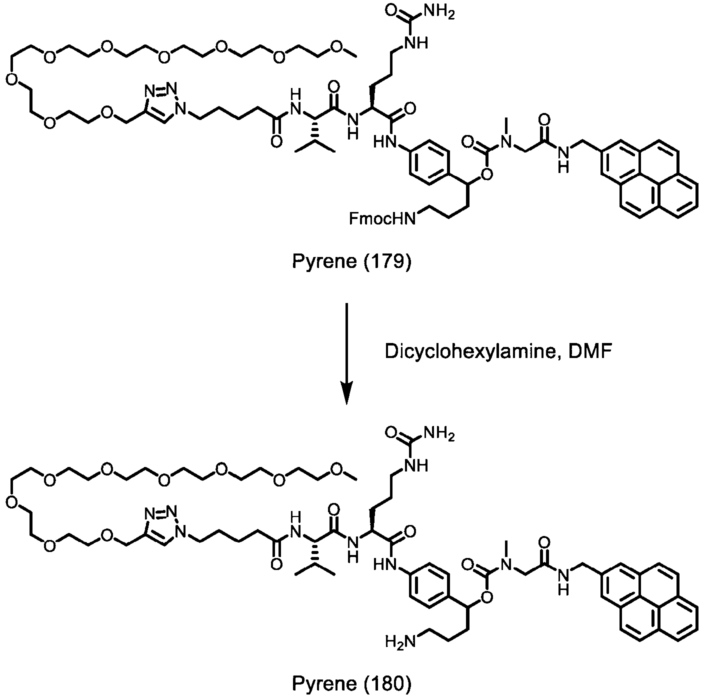

Pyrene (179) (3.5 mg, 2.3 µmol) was dissolved in N,N-dimethylformamide (200 µL), and dicyclohexylamine (40 µL) was added and the mixture was stirred at room temperature. If the starting material remained according to LC-MS analysis, dicyclohexylamine was added. In the end, a total of 120 µL of dicyclohexylamine was used. After adding acetonitrile (200 µL) to dilute the reaction solution, the filtered solution was purified by preparative HPLC. Fractions containing the desired product were collected, and acetonitrile was removed by concentration under reduced pressure, followed by lyophilization to obtain Pyrene (180) (3.0 mg, 2.3 µmol).
MS (ESI) m/z: 1310.8 [M-H]⁻

### (19-25-9) Synthesis of Linker-payload mimic (171)

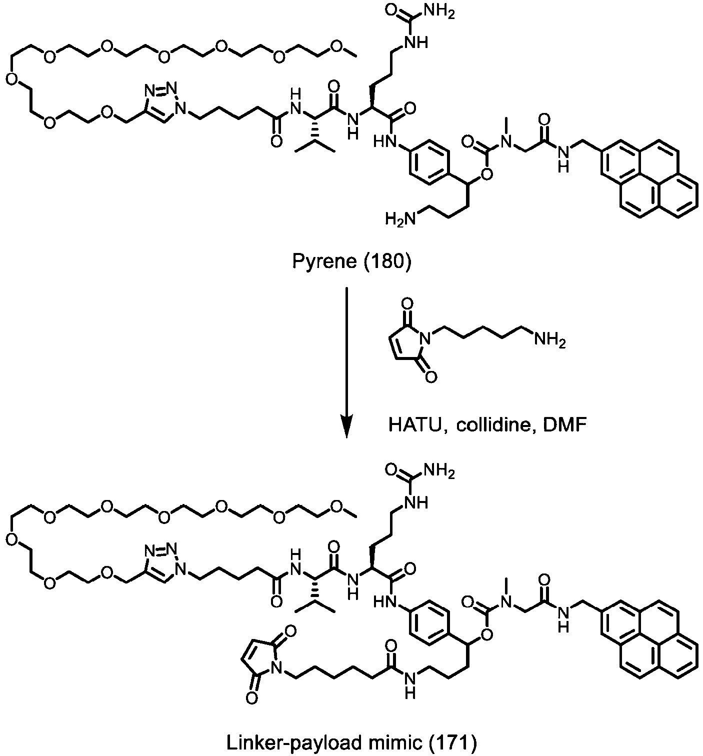

6-maleimidehexanoic acid (0.93 mg, 4.31 µmol) was dissolved in N,N-dimethylformamide (110 µL), to which HATU (1.71 mg, 4.50 µmol) and 2,4,6-trimethylpyridine (0.65 µL, 4.8 µmol) were added, and the mixture was stirred at room temperature for 12 minutes. Pyrene (180) (3.0 mg, 2.3 µmol) dissolved in N,N-dimethylformamide (220 µL) was added. After 30 minutes, 6-maleimide hexanoic acid (0.25 mg, 1.16 µmol) was added. After confirming the disappearance of the starting material by LC-MS analysis, acetonitrile (330 µL) was added to dilute the reaction solution, which was then filtered and purified by preparative HPLC. Fractions containing the desired product were collected, and acetonitrile was removed by concentration under reduced pressure, followed by lyophilization to obtain Linker-payload mimic (171) (2.9 mg, 1.9 µmol).
MS (ESI) m/z: 1506.1 [M+H]⁺

### Example 20: Synthesis and Analysis of ADC mimic

### (20-1) Synthesis of ADC mimic

In the following examples, ADC mimics were prepared in the same manner as in Example 2.

### (20-1-1) Synthesis of ADC mimic 21

From Linker-payload mimic (51) synthesized in Example 19-1 and the thiol-containing antibody, ADC mimic 21 with the following structure was synthesized. ESI-TOFMS analysis was performed, and a peak was confirmed at 150122 for the reaction product, into which two molecules of Linker-payload mimic (51) were introduced.

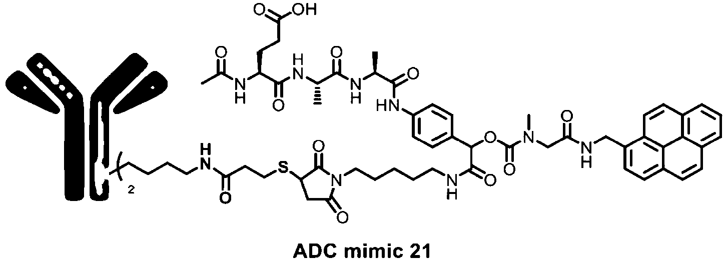

### (20-1-2) Synthesis of ADC mimic 22

From Linker-payload mimic (56) synthesized in Example 19-2 and the thiol-containing antibody, ADC mimic 22 with the following structure was synthesized. ESI-TOFMS analysis was performed, and a peak was confirmed at 150322 for the reaction product, into which two molecules of Linker-payload mimic (56) were introduced.

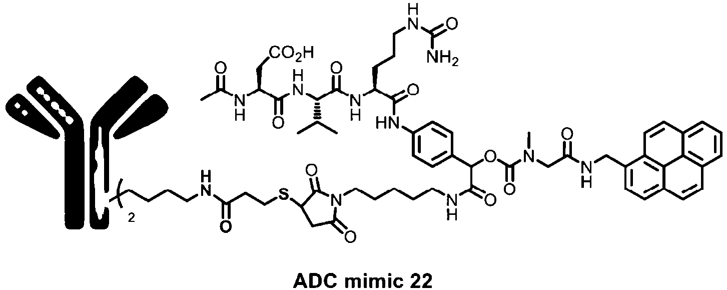

### (20-1-3) Synthesis of ADC mimic 23

From Linker-payload mimic (61) synthesized in Example 19-3 and the thiol-containing antibody, ADC mimic 23 with the following structure was synthesized. ESI-TOFMS analysis was performed, and a peak was confirmed at 150474 for the reaction product, into which two molecules of Linker-payload mimic (61) were introduced.

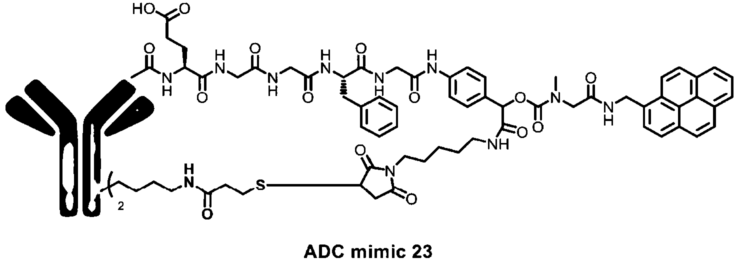

### (20-1-4) Synthesis of ADC mimic 24

From Linker-payload mimic (66) synthesized in Example 19-4 and the thiol-containing antibody, ADC mimic 24 with the following structure was synthesized. ESI-TOFMS analysis was performed, and a peak was confirmed at 151125 for the reaction product, into which two molecules of Linker-payload mimic (66) were introduced.

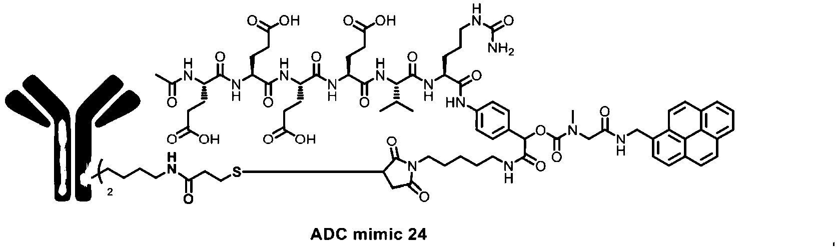

### (20-1-5) Synthesis of ADC mimic 25

From Linker-payload mimic (71) synthesized in Example 19-5 and the thiol-containing antibody, ADC mimic 25 with the following structure was synthesized. ESI-TOFMS analysis was performed, and a peak was confirmed at 150389 for the reaction product, into which two molecules of Linker-payload mimic (71) were introduced.

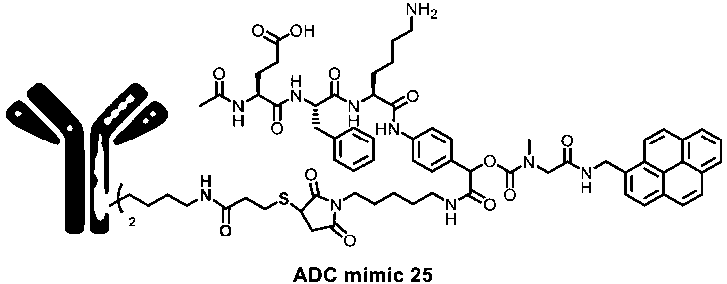

### (20-1-6) Synthesis of ADC mimic 26

From Linker-payload mimic (76) synthesized in Example 19-6 and the thiol-containing antibody, ADC mimic 26 with the following structure was synthesized. ESI-TOFMS analysis was performed, and a peak was confirmed at 150647 for the reaction product, into which two molecules of Linker-payload mimic (76) were introduced.

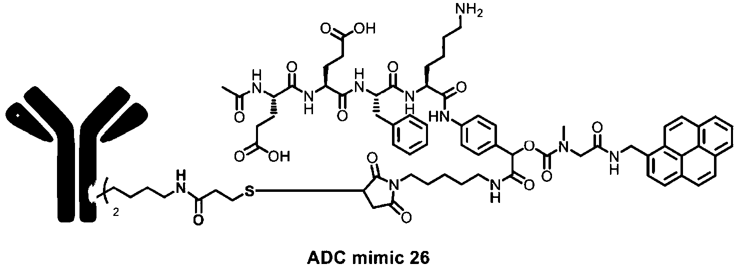

### (20-1-7) Synthesis of ADC mimic 27

From Linker-payload mimic (81) synthesized in Example 19-7 and the thiol-containing antibody, ADC mimic 27 with the following structure was synthesized. ESI-TOFMS analysis was performed, and a peak was confirmed at 150551 for the reaction product, into which two molecules of Linker-payload mimic (81) were introduced.

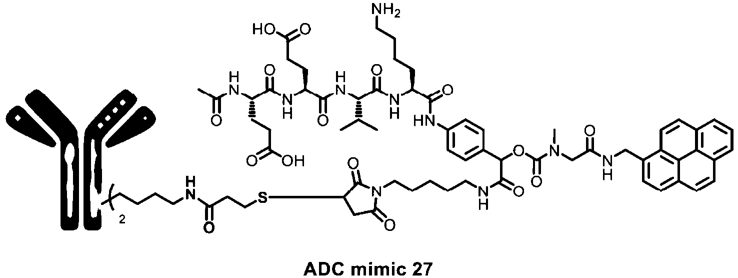

### (20-1-8) Synthesis of ADC mimic 28

From Linker-payload mimic (86) synthesized in Example 19-8 and the thiol-containing antibody, ADC mimic 28 with the following structure was synthesized. ESI-TOFMS analysis was performed, and a peak was confirmed at 150495 for the reaction product, into which two molecules of Linker-payload mimic (86) were introduced.

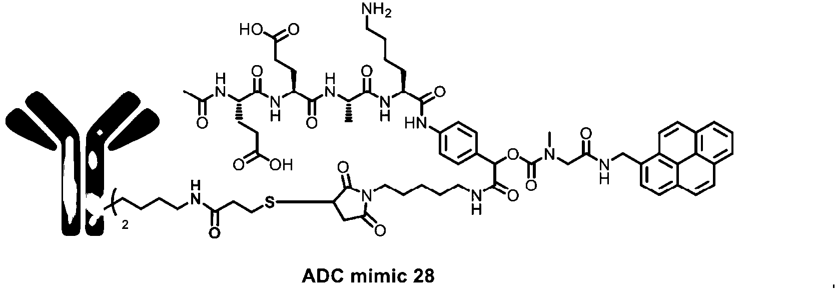

### (20-1-9) Synthesis of ADC mimic 29

From Linker-payload mimic (91) synthesized in Example 19-9 and the thiol-containing antibody, ADC mimic 29 with the following structure was synthesized. ESI-TOFMS analysis was performed, and a peak was confirmed at 150208 for the reaction product, into which two molecules of Linker-payload mimic (91) were introduced.

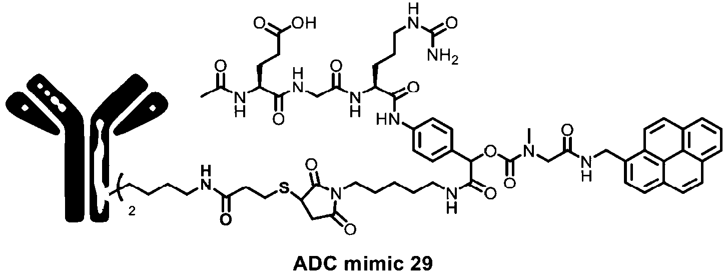

### (20-1-10) Synthesis of ADC mimic 30

From Linker-payload mimic (99) synthesized in Example 19-11 and the thiol-containing antibody, ADC mimic 30 with the following structure was synthesized. ESI-TOFMS analysis was performed, and a peak was confirmed at 150776 for the reaction product, into which two molecules of Linker-payload mimic (99) were introduced.

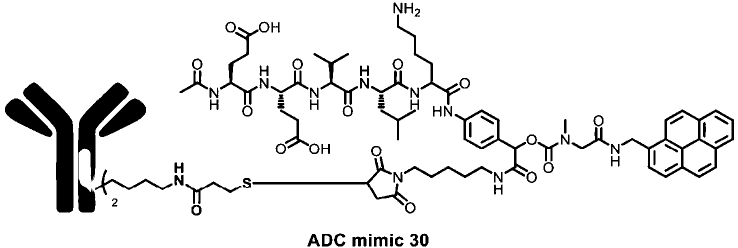

### (20-1-11) Synthesis of ADC mimic 31

From Linker-payload mimic (104) synthesized in Example 19-12 and the thiol-containing antibody, ADC mimic 31 with the following structure was synthesized. ESI-TOFMS analysis was performed, and a peak was confirmed at 150292 for the reaction product, into which two molecules of Linker-payload mimic (104) were introduced.

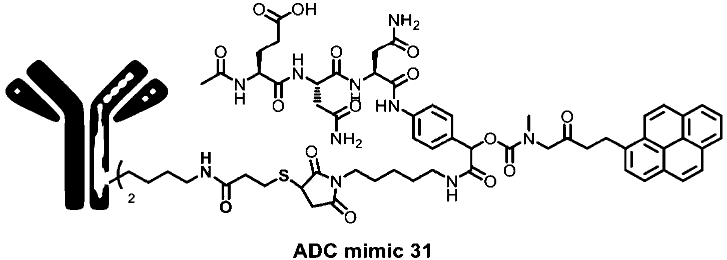

### (20-1-12) Synthesis of ADC mimic 32

From Linker-payload mimic (115) synthesized in Example 19-14 and the thiol-containing antibody, ADC mimic 32 with the following structure was synthesized. ESI-TOFMS analysis was performed, and a peak was confirmed at 150524 for the reaction product, into which two molecules of Linker-payload mimic (115) were introduced.

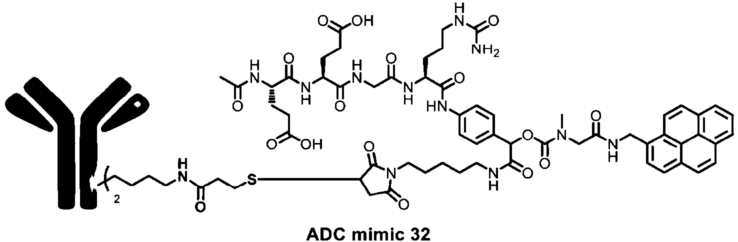

### (20-1-13) Synthesis of ADC mimic 33

From Linker-payload mimic (120) synthesized in Example 19-15 and the thiol-containing antibody, ADC mimic 33 with the following structure was synthesized. ESI-TOFMS analysis was performed, and a peak was confirmed at 151279 for the reaction product, into which two molecules of Linker-payload mimic (120) were introduced.

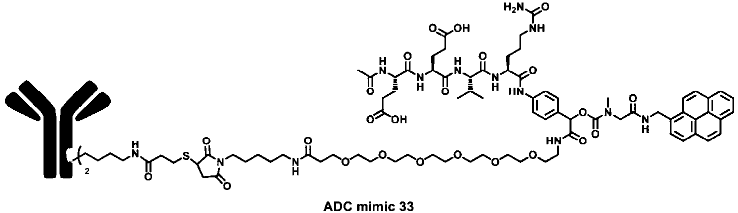

### (20-1-14) Synthesis of ADC mimic 34

From Linker-payload mimic (124) synthesized in Example 19-16 and the thiol-containing antibody, ADC mimic 34 with the following structure was synthesized. ESI-TOFMS analysis was performed, and a peak was confirmed at 150365 for the reaction product, into which two molecules of Linker-payload mimic (124) were introduced.

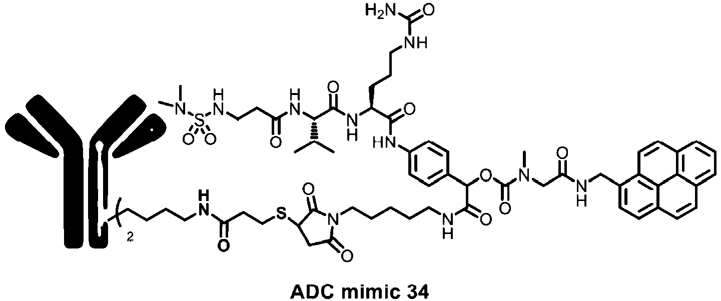

### (20-1-15) Synthesis of ADC mimic 35

From Linker-payload mimic (155) synthesized in Example 19-22 and the thiol-containing antibody, ADC mimic 35 with the following structure was synthesized. ESI-TOFMS analysis was performed, and a peak was confirmed at 150661 for the reaction product, into which two molecules of Linker-payload mimic (155) were introduced.

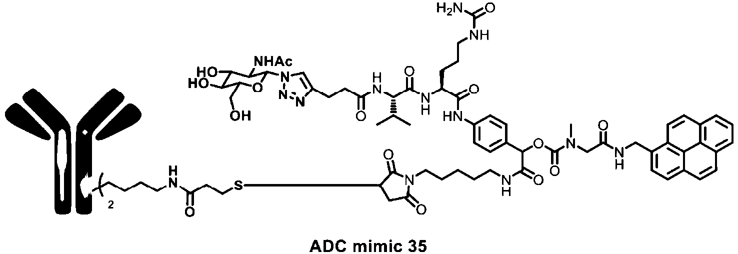

### (20-1-16) Synthesis of ADC mimic 36

From Linker-payload mimic (161) synthesized in Example 19-23 and the thiol-containing antibody, ADC mimic 36 with the following structure was synthesized. ESI-TOFMS analysis was performed, and a peak was confirmed at 151102 for the reaction product, into which two molecules of Linker-payload mimic (161) were introduced.

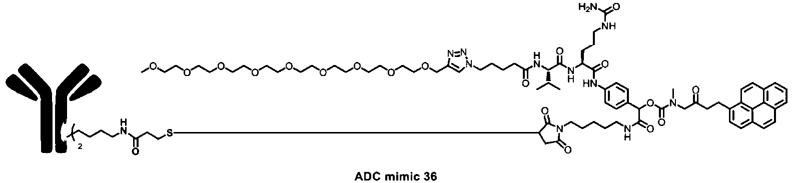

### (20-1-17) Synthesis of ADC mimic 37

From Linker-payload mimic (167) synthesized in Example 19-24 and the thiol-containing antibody, ADC mimic 37 with the following structure was synthesized. ESI-TOFMS analysis was performed, and a peak was confirmed at 153568 for the reaction product, into which two molecules of Linker-payload mimic (167) were introduced.

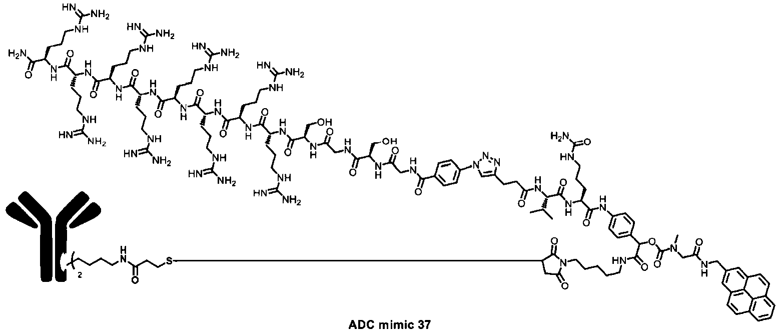

### (20-1-18) Synthesis of ADC mimic 38

From Linker-payload mimic (171) synthesized in Example 19-25 and the thiol-containing antibody, ADC mimic 38 with the following structure was synthesized. ESI-TOFMS analysis was performed, and a peak was confirmed at 151188 for the reaction product, into which two molecules of Linker-payload mimic (171) were introduced.

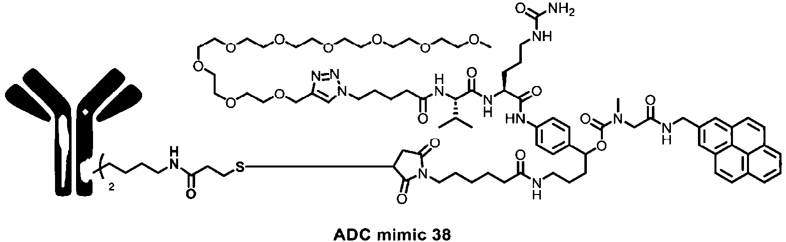

### (20-2) DAR analysis of ADC mimic

ESI-TOFMS analysis of the ADC mimic synthesized in Example 20-1 was performed according to the previous report (WO2019/240287A1), confirming that the DAR was 2.

**Table 19: DAR of ADC mimics**

| | Linker-payload mimic | Example/Comparable Example | DAR |
|---|---|---|---|
| ADC mimic 21 | Linker-payload mimic (51) | Example 20-1-1 | 2 |
| ADC mimic 22 | Linker-payload mimic (56) | Example 20-1-2 | 2 |
| ADC mimic 23 | Linker-payload mimic (61) | Example 20-1-3 | 2 |
| ADC mimic 24 | Linker-payload mimic (66) | Example 20-1-4 | 2 |
| ADC mimic 25 | Linker-payload mimic (76) | Example 20-1-5 | 2 |
| ADC mimic 26 | Linker-payload mimic (76) | Example 20-1-6 | 2 |
| ADC mimic 27 | Linker-payload mimic (81) | Example 20-1-7 | 2 |
| ADC mimic 28 | Linker-payload mimic (86) | Example 20-1-8 | 2 |
| ADC mimic 29 | Linker-payload mimic (91) | Example 20-1-9 | 2 |
| ADC mimic 30 | Linker-payload mimic (99) | Example 20-1-10 | 2 |
| ADC mimic 31 | Linker-payload mimic (104) | Example 20-1-11 | 2 |
| ADC mimic 32 | Linker-payload mimic (115) | Example 20-1-12 | 2 |
| ADC mimic 33 | Linker-payload mimic (120) | Example 20-1-13 | 2 |
| ADC mimic 34 | Linker-payload mimic (124) | Example 20-1-14 | 2 |
| ADC mimic 35 | Linker-payload mimic (155) | Example 20-1-15 | 2 |
| ADC mimic 36 | Linker-payload mimic (161) | Example 20-1-16 | 2 |
| ADC mimic 37 | Linker-payload mimic (167) | Example 20-1-17 | 2 |
| ADC mimic 38 | Linker-payload mimic (171) | Example 20-1-18 | 2 |
| ADC mimic 5 | Linker-payload mimic (26) | Comparable Example 1 | 2 |

### Example 21: Evaluation of degree of hydrophobicity of ADC and ADC mimic by hydrophobic interaction chromatography (HIC-HPLC)

HIC-HPLC analysis was performed according to the previous report (Anal. Chem., 2019, 91, 20, 12724-12732). The measurement was performed using the following conditions. The degree of hydrophobicity of the ADC can be evaluated based on the retention time of the ADC in the HIC chromatogram.
Measurement system: Chromaster^{®} (Hitachi, Ltd.)
Column: Tosoh Biobuthyl NPR 2.5 µm 4.6 × 35 mm column manufactured by Tosoh Bioscience LLC
Gradient: Linear gradient of eluent A/B
Flow rate: 0.8 mL/min
Eluent A: 1.1 M (NH₄)₂SO₄, 25 mM Na₂HPO₄/NaH₂PO₄ (pH6.0)
Eluent B: 25 mM Na₂HPO₄/NaH₂PO₄ (pH6.0, 25 v/v% isopropanol added)
Detector: UV (280 nm)

**Table 20: Evaluation of degree of hydrophobicity of ADCs and ADC mimics using HIC-HPLC**

| | Linker-payload mimic | Example/Comparable Example | Retention Time |
|---|---|---|---|
| ADC mimic 21 | Linker-payload mimic (51) | Example 20-1-1 | 10.5 min |
| ADC mimic 22 | Linker-payload mimic (56) | Example 20-1-2 | 10.8 min |
| ADC mimic 23 | Linker-payload mimic (61) | Example 20-1-3 | 11.5 min |
| ADC mimic 24 | Linker-payload mimic (66) | Example 20-1-4 | 9.9 min |
| ADC mimic 25 | Linker-payload mimic (71) | Example 20-1-5 | 10.5 min |
| ADC mimic 26 | Linker-payload mimic (76) | Example 20-1-6 | 10.5 min |
| ADC mimic 27 | Linker-payload mimic (81) | Example 20-1-7 | 9.9 min |
| ADC mimic 28 | Linker-payload mimic (86) | Example 20-1-8 | 9.7 min |
| ADC mimic 29 | Linker-payload mimic (91) | Example 20-1-9 | 10.3 min |
| ADC mimic 30 | Linker-payload mimic (99) | Example 20-1-10 | 10.5 min |
| ADC mimic 31 | Linker-payload mimic (104) | Example 20-1-11 | 9.9 min |
| ADC mimic 33 | Linker-payload mimic (120) | Example 20-1-13 | 9.2 min |
| ADC mimic 34 | Linker-payload mimic (124) | Example 20-1-14 | 11.5 min |
| ADC mimic 35 | Linker-payload mimic (155) | Example 20-1-15 | 10.8 min |
| ADC mimic 36 | Linker-payload mimic (161) | Example 20-1-16 | 11.2 min |
| ADC mimic 37 | Linker-payload mimic (167) | Example 20-1-17 | 8.9 min |
| ADC mimic 38 | Linker-payload mimic (171) | Example 20-1-18 | 11.6 min |
| ADC mimic 5 | Linker-payload mimic (26) | Comparable Example 1 | 13.6 min |

As a result, it was confirmed that the ADC mimics synthesized in Examples 20-1-1 to 20-1-18 tended to have a short retention time, indicating a high degree of hydrophilicity. Therefore, it was confirmed that the ADC mimics synthesized in Examples 20-1-1 to 20-1-18 are preferable ADCs because they are considered to have slow plasma clearance and a long residence time in the body.

### Example 22: Evaluation of aggregation rate of ADC and ADC mimic by size exclusion chromatography (SEC-HPLC)

SEC-HPLC analysis was performed according to the previous report (ChemistrySelect, 2020, 5, 8435-8439). The measurement was performed using the following conditions.
Measurement system: 1260 HPLC system (Agilent Technologies, Inc.)
Column: AdvanceBio SEC 300Å 2.7 µm, 4.6 mm × 150 mm manufactured by Agilent Technologies, Inc.
Flow rate: 0.25 mL/min
Eluent: 100 mM sodium dihydrogen phosphate/disodium hydrogen phosphate, 250 mM aqueous sodium chloride solution (pH 6.8), 10%v/v isopropanol
Detector: UV (280 nm)

**Table 21: Evaluation of aggregation rate of ADCs and ADC mimics using SEC-HPLC**

| | Linker-payload mimic | Example/Comparable Example | Aggregation Rate |
|---|---|---|---|
| ADC mimic 21 | Linker-payload mimic (51) | Example 20-1-1 | 2.7 % |
| ADC mimic 22 | Linker-payload mimic (56) | Example 20-1-2 | 1.7 % |
| ADC mimic 23 | Linker-payload mimic (61) | Example 20-1-3 | 2.0 % |
| ADC mimic 24 | Linker-payload mimic (66) | Example 20-1-4 | 1.2 % |
| ADC mimic 25 | Linker-payload mimic (71) | Example 20-1-5 | 1.7 % |
| ADC mimic 26 | Linker-payload mimic (76) | Example 20-1-6 | 2.0 % |
| ADC mimic 27 | Linker-payload mimic (81) | Example 20-1-7 | 2.2 % |
| ADC mimic 28 | Linker-payload mimic (86) | Example 20-1-8 | 2.2 % |
| ADC mimic 29 | Linker-payload mimic (91) | Example 20-1-9 | 2.1 % |
| ADC mimic 30 | Linker-payload mimic (99) | Example 20-1-10 | 1.4 % |
| ADC mimic 31 | Linker-payload mimic (104) | Example 20-1-11 | 2.0 % |
| ADC mimic 33 | Linker-payload mimic (120) | Example 20-1-13 | 1.3 % |
| ADC mimic 34 | Linker-payload mimic (124) | Example 20-1-14 | 2.4 % |
| ADC mimic 35 | Linker-payload mimic (155) | Example 20-1-15 | 0.9 % |
| ADC mimic 36 | Linker-payload mimic (161) | Example 20-1-16 | 0.9 % |
| ADC mimic 37 | Linker-payload mimic (167) | Example 20-1-17 | 0.1 % |
| ADC mimic 38 | Linker-payload mimic (171) | Example 20-1-18 | 0.5 % |
| ADC mimic 5 | Linker-payload mimic (26) | Comparable Example 1 | 2.7 % |

### Example 23: Evaluation of ADC mimic using cathepsin B enzyme

The cleavability of various ADC mimics by cathepsin B was evaluated by analyzing the amounts of fluorescent molecules detached from the ADC mimic as described below.

### (23-1) Cathepsin B cleavability test

The test was performed in the same manner as in Example 5.

### (23-2) Quantitation of detached fluorescent molecules using HPLC analysis

The test and analysis were performed in the same manner as in Example 5.

**Table 22: Evaluation of cleavability of ADC mimics by cathepsin B**

| | Linker-payload mimic | Example/Comparable Example | Detachment rate of fluorescent molecules at 6 h |
|---|---|---|---|
| ADC mimic 21 | Linker-payload mimic (51) | Example 20-1-1 | 41 % |
| ADC mimic 22 | Linker-payload mimic (56) | Example 20-1-2 | 180 % |
| ADC mimic 23 | Linker-payload mimic (61) | Example 20-1-3 | 200 % |
| ADC mimic 24 | Linker-payload mimic (66) | Example 20-1-4 | 163 % |
| ADC mimic 25 | Linker-payload mimic (71) | Example 20-1-5 | 89 % |
| ADC mimic 26 | Linker-payload mimic (76) | Example 20-1-6 | 200 % |
| ADC mimic 28 | Linker-payload mimic (86) | Example 20-1-8 | 124 % |
| ADC mimic 33 | Linker-payload mimic (120) | Example 20-1-13 | 200 % |
| ADC mimic 34 | Linker-payload mimic (124) | Example 20-1-14 | 200 % |
| ADC mimic 5 | Linker-payload mimic (26) | Comparable Example 1 | 200 % |

As shown in Table 22, it was found that the synthesized ADC mimics have sufficient cleavability by cathepsin B.

### Example 24: Evaluation of ADC mimic using mouse plasma

### (24-1) Plasma stability test of ADC mimic

The test was performed in the same manner as in Example 6.

### (24-2) Quantitation of detached fluorescent molecules using HPLC analysis

The test and analysis were performed in the same manner as in Example 6.

**Table 23: Results of plasma stability test using ADCs mimics**

| | Linker-payload mimic | Example/Comparable Example | Detachment rate of payload at Day 4 |
|---|---|---|---|
| ADC mimic 21 | Linker-payload mimic (51) | Example 20-1-1 | 3% |
| ADC mimic 22 | Linker-payload mimic (56) | Example 20-1-2 | 3% |
| ADC mimic 23 | Linker-payload mimic (61) | Example 20-1-3 | 8% |
| ADC mimic 24 | Linker-payload mimic (66) | Example 20-1-4 | 4% |
| ADC mimic 25 | Linker-payload mimic (71) | Example 20-1-5 | 22 % |
| ADC mimic 26 | Linker-payload mimic (76) | Example 20-1-6 | 3% |
| ADC mimic 28 | Linker-payload mimic (86) | Example 20-1-8 | 4% |
| ADC mimic 31 | Linker-payload mimic (104) | Example 20-1-13 | 10 % |
| ADC mimic 33 | Linker-payload mimic (120) | Example 20-1-14 | 12 % |
| ADC mimic 34 | Linker-payload mimic (124) | Example 20-1-14 | 2% |
| ADC mimic 5 | Linker-payload mimic (26) | Comparable Example 1 | 200 % |

As a result, compared to the ADC mimic synthesized in Comparative Example 1, the ADC mimics synthesized in Examples 20-1-5 and 20-1-14 showed 2-fold or higher stability, the ADC mimics synthesized in Examples 20-1-3 and 20-1-13 showed 5-fold or higher stability, and the ADC mimics synthesized in Examples 20-1-1, 20-1-2, 20-1-4, 20-1-6, 20-1-8, and 20-1-14 showed 10-fold or higher stability.

### Example 25: Synthesis of Linker-payload

The NMR spectral data of Linker-payload (42) synthesized in Example (12-3-5) were as follows.

¹H NMR (300MHz; DMSO-d₆) δ12.06 (brs, 2H), 10.05-10.07 (m, 1H), 8.47-8.28 (m, 1H), 8.29-8.19 (m, 1H), 8.13-8.04 (m, 3H), 7.91-7.56 (m, 5H), 7.39-7.17 (m, 7H), 6.99 (s, 2H), 5.99 (s, 1H), 5.86-5.67 (m, 1H), 5.43-5.35 (m, 3H), 4.77-4.16 (m, 6H), 4.00-3.98 (m, 2H), 3.80-3.76 (m, 1H), 3.52-3.18 (m, 12H), 3.01-2.73 (m, 9H), 2.26-2.14 (m, 6H), 2.12-2.09 (m, 2H), 1.97-1.90 (m, 2H), 1.84 (s, 6H), 1.76-1.69 (m, 5H), 1.43-1.35 (m, 10H), 1.23-1.14 (m, 3H), 1.01-0.96 (m, 7H), 0.83-0.68 (m, 24H).

### Example 26: Synthesis of ADC

### (26-1-1) Synthesis of Linker intermediate (115)

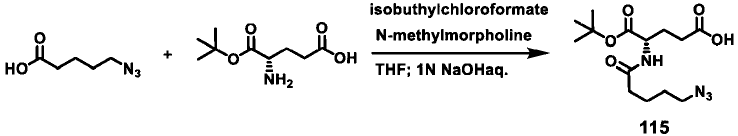

5-azidovaleric acid (800 mg, 5.59 mmol) was dissolved in THF (14 mL), to which isobutyl chloroformate (808 µL, 6.15 mmol) and N-methylmorpholine (873 µL, 8.39 mmol) were added followed by stirring at 0°C for 30 minutes, and then hydrazine hydrate (1.36 g, 6.71 mmol) dissolved in 1 M NaOH aqueous solution (4 mL) was added followed by stirring at room temperature for 3 hours. After concentration under reduced pressure, 1 M NaOH aqueous solution was added to adjust the pH in the system to pH10. After washing with ethyl acetate, 1 M HCl aqueous solution was added to the aqueous layer to adjust the pH in the system to 3.0. After washing with ethyl acetate, sodium sulfate was added to the resulting ethyl acetate solution. Sodium sulfate was removed by filtration, and the resulting solution was concentrated under reduced pressure, purified by column chromatography (dichloromethane: methanol = 10:1). Fractions containing the product were collected and concentrated under reduced pressure to obtain Linker intermediate (115).

¹H NMR (400 MHz, Chloroform-d) δ6.29 (d, J = 7.7 Hz, 1H), 4.56 (td, J = 8.0, 4.9 Hz, 1H), 3.32 (t, J = 6.6 Hz, 2H), 2.53-2.38 (m, 3H), 2.36-2.16 (m, 3H), 2.12 (s, 2H), 1.96 (dq, J = 14.7, 7.6 Hz, 1H), 1.84-1.59 (m, 4H), 1.50 (s, 9H).
MS (ESI) m/z: 329 [M+H]⁺

### (26-1-2) Synthesis of Linker intermediate (117)

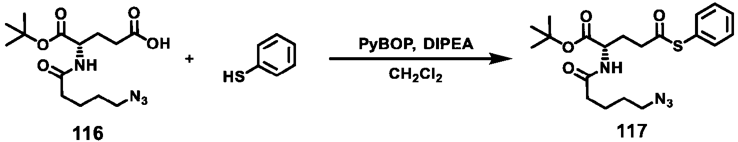

Linker intermediate (116) (2.41g, 5.59 mmol) was dissolved in dichloromethane (28 mL), and thiophenol (627 µL, 6.15 mmol), benzotriazol-1-yloxy (3.49g, 6.71 mmol), and DIPEA (1.42 mL, 8.39 mmol) were added, and the mixture was stirred at room temperature for 2 hours. Thereafter, the resulting product was concentrated under reduced pressure and purified by column chromatography (hexane: ethyl acetate = 4:1). Fractions containing the product were collected and concentrated under reduced pressure to obtain Linker intermediate (117) (2.20 g, 5.23 mmol).

¹H NMR (400 MHz, Chloroform-d) δ7.43 (s, 5H), 6.10 (d, J = 7.8 Hz, 1H), 4.55 (td, J = 7.7, 4.9 Hz, 1H), 3.31 (t, J = 6.7 Hz, 2H), 2.87-2.63 (m, 2H), 2.28 (dd, J = 8.7, 5.9 Hz, 2H), 2.16-1.98 (m, 1H), 1.83-1.58 (m, 4H), 1.50 (s, 9H), 1.37-1.22 (m, 2H), 0.91 (t, J = 6.7 Hz, 1H).
MS (ESI) m/z: 421 [M+H]⁺

### (26-1-3) Synthesis of Linker intermediate (118)

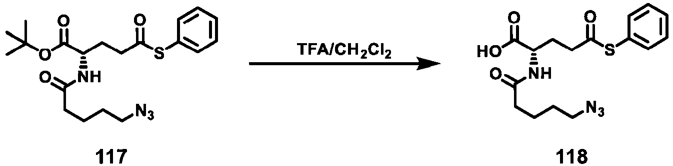

The linker intermediate (117) (2.20 g, 5.23 mmol) was dissolved in dichloromethane (10 mL), to which trifluoroacetic acid (10mL) was added and stirred at room temperature for 1 hour. Then, the resulting product was concentrated under reduced pressure to remove dichloromethane, and water was added for lyophilization to obtain a linker intermediate (118) (1.98 g, 5.43 mmol).

¹H NMR (400 MHz, Chloroform-d) δ7.44 (s, J = 6.3, 4.6, 2.4 Hz, 5H), 6.76 (s, 1H), 4.62 (td, J = 7.5, 4.9 Hz, 1H), 3.31 (t, J = 6.6 Hz, 2H), 2.88 (qt, J = 16.8, 6.8 Hz, 2H), 2.33 (dt, J = 12.4, 6.8 Hz, 3H), 2.18 (dq, J = 14.4, 7.4 Hz, 1H), 1.74 (dq, J = 11.8, 7.5, 6.9 Hz, 2H), 1.63 (ddd, J = 17.7, 10.5, 4.8 Hz, 2H).
MS (ESI) m/z: 365 [M+H]⁺

### (26-1-4) Synthesis of Linker intermediate (119)

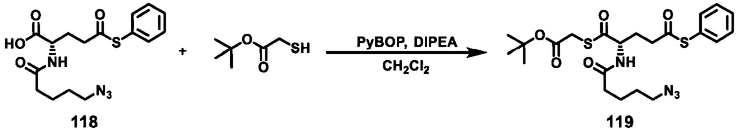

The linker intermediate (118) (100 mg, 0.274 mmol) was dissolved in dichloromethane (3 mL), and (40.6 µL, 0.280 mmol), benzotriazol-1-yloxy (150 mg, 0.288 mmol), DIPEA (70.1 µL, 0.412 mmol) were added and stirred at room temperature for 2 hours. 1 M HCl aqueous solution was added to adjust the pH of the system to 3, followed by dilution with dichloromethane. The mixture was then washed with water and brine, and sodium sulfate was added. Sodium sulfate was removed by filtration, and the resulting solution was concentrated under reduced pressure and purified by column chromatography (hexane: ethyl acetate = 4:1). Fractions containing the product were collected and concentrated under reduced pressure to obtain a linker intermediate (119) (84.7 mg, 0.171 mmol).

¹H NMR (400 MHz, Chloroform-d) δ7.50-7.38 (m, 5H), 6.33 (d, J = 8.4 Hz, 1H), 4.78 (tdd, J = 7.8, 4.6, 3.0 Hz, 1H), 3.70-3.54 (m, 2H), 3.32 (dt, J = 9.1, 6.7 Hz, 2H), 2.96-2.67 (m, 2H), 2.30 (pd, J = 7.1, 4.5 Hz, 2H), 1.85-1.60 (m, 6H), 1.49 (d, J = 2.8 Hz, 9H).
MS (ESI) m/z: 495 [M+H]⁺

### (26-1-5) Synthesis of Linker intermediate (120)

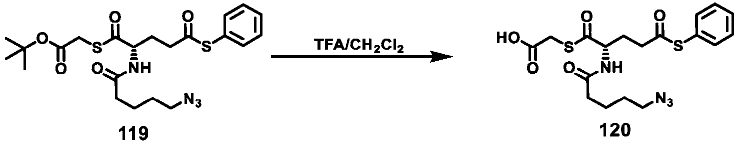

The linker intermediate (119) (84.7 mg, 0.171 mmol) was dissolved in dichloromethane (5 mL), to which trifluoroacetic acid (5 mL) was added and stirred at room temperature for 1 hour. Then, the resulting product was concentrated under reduced pressure to remove dichloromethane, and water was added for lyophilization, and purified by column chromatography (dichloromethane: methanol = 10:1) Fractions containing the product were collected and concentrated under reduced pressure to obtain a linker intermediate (120) (46.8 mg, 0.107 mmol).

¹H NMR (400 MHz, Methanol-d4) δ7.44 (dq, J = 2.3, 1.5 Hz, 5H), 4.69-4.57 (m, 1H), 3.79-3.67 (m, 2H), 3.40-3.30 (m, 2H), 2.89-2.71 (m, 2H), 2.44-2.23 (m, 4H), 2.08-1.95 (m, 1H), 1.82-1.61 (m, 4H).
MS (ESI) m/z: 439 [M+H]⁺

### (26-2) Preparation of affinity reagent (18) having an azido group

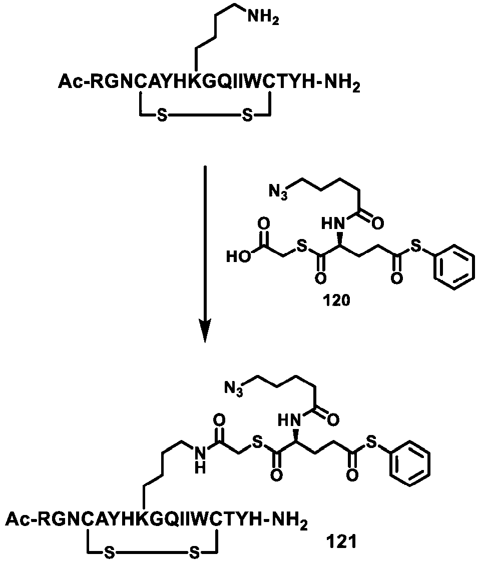

(The aforementioned amino acid sequence is the amino acid sequence of SEQ ID NO: 1.)

Ac-RGNCAYHKGQIIWCTYH-NH2 described in the previous report (WO2019/240287A1) (SEQ ID NO: 1, 30.9 mg, 14.9 µmol, where the two cysteines at positions 4 and 14 form a disulfide bond with each other in the molecule) was dissolved in dimethylformamide (468 µL), and then the linker intermediate (120) synthesized in Example 6-1-5 (46.8 mg, 0.107 mmol) and WSC·HCl (29.7 mg, 0.155 mmol) were added, and the mixture was stirred at room temperature for 5 hours and eluted by reversed phase preparative chromatography. Fractions containing the product were collected, and acetonitrile was removed by concentration under reduced pressure, followed by lyophilization to obtain the modifying reagent (121) (15.1 mg, 6.02 µmol).

### (26-3) Introduction of two peptide reagent molecules to trastuzumab

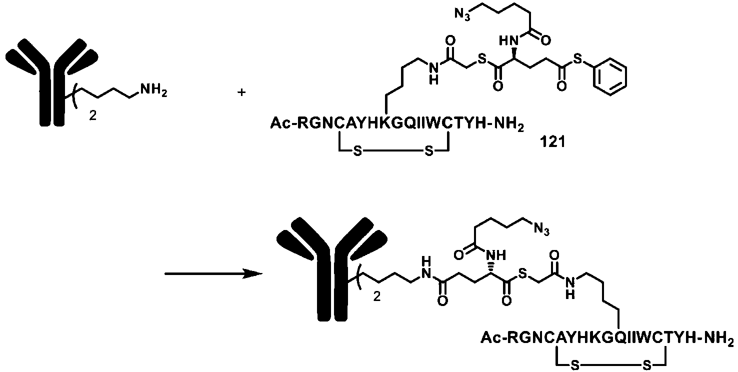

(The aforementioned amino acid sequence is the amino acid sequence of SEQ ID NO: 1.)

Subsequently, a conjugation reaction was performed on trastuzumab using the peptide reagent (121) prepared in Example 26-2 according to the method in the previous report (WO2019/240287A1). As a result, an antibody with the modifying reagent (121) introduced was obtained. For DAR analysis of the antibody with the peptide reagent (121) introduced, HIC-HPLC analysis was performed according to the previous report (Anal.Chem., 2019, 91, 20, 12724-12732) to confirm that two peptide reagents were introduced.

### (26-4) Synthesis of trastuzumab with azido group introduced (T-1)

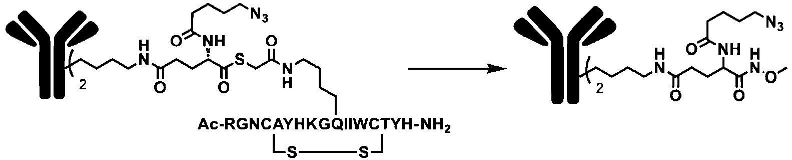

(The aforementioned amino acid sequence is the amino acid sequence of SEQ ID NO: 1.)

A cleavage reaction was performed on the antibody with the modifying reagent (121) introduced obtained in Example 26-3, with reference to the technique in the previous report (WO2019/240287A1), by adding a methoxyamine solution and shaking the mixture at room temperature for 3 hours. As a result, an antibody with an azido group introduced was obtained. HIC-HPLC analysis was performed according to the previous report (Anal. Chem., 2019, 91, 20, 12724-12732) to confirm that an azido group was introduced.

### (26-4) Synthesis of ADC 7

Linker-payload (40) was added to the above-described azide-introduced antibody to obtain ADC (7). ESI-TOFMS analysis was performed, and a peak was confirmed at 151276 for the reaction product, into which two molecules of Linker-payload (40) were introduced. ESI-TOFMS analysis was performed according to the previous report (WO2019/240287A1), confirming that the DAR was 2.

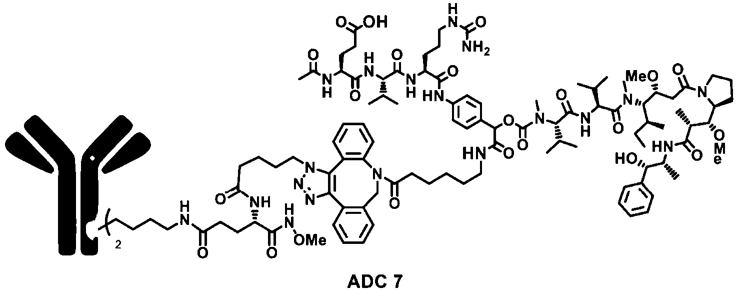

### (26-5) Synthesis of ADC 18

On a compound in which a thiol group was regioselectively introduced into trastuzumab (humanized IgG1 antibody) via the amino group of the side chain of the lysine residue at position 246 or 248 of the antibody heavy chain prepared by the method described in Example 16, the method described in Example 19-6 of WO2022/154127A1 was performed to obtain the following antibody intermediate.

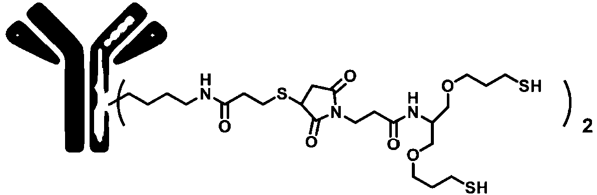

Linker-payload (42) was added to the above-described antibody intermediate to obtain ADC (18). ESI-TOFMS analysis was performed, and a peak was confirmed at 155485 for the reaction product, into which four molecules of Linker-payload (42) were introduced. ESI-TOFMS analysis was performed according to the previous report (WO2019/240287A1), confirming that the DAR was 4.

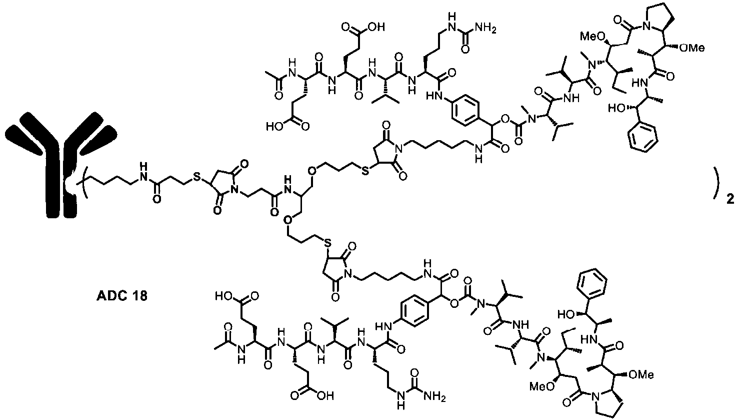

### (26-6-1) Synthesis of antibody with two thiol group molecules bound to Lys288 of the antibody (Q1)

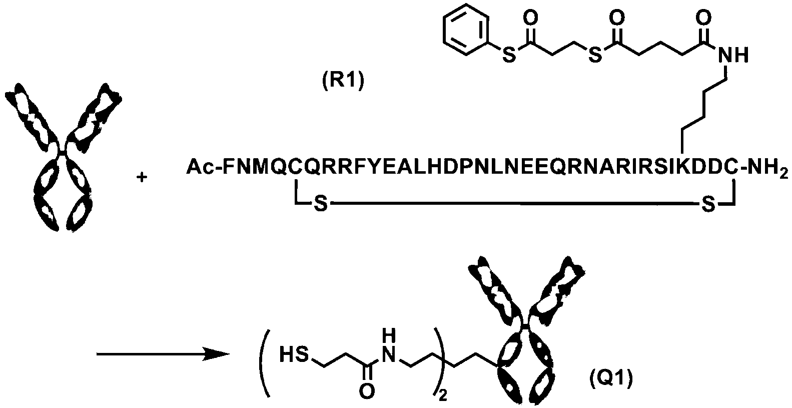

(The SEQ ID NO of the above-described amino acid sequence is SEQ ID NO: 13)

An anti-human HER2 monoclonal antibody Trastuzumab (Chugai Pharmaceutical Co., Ltd.) and a peptide reagent (R1) described in the previous report (WO2022/154116A1) were used following the method described in the previous report (WO2022/154116A1) to obtain an antibody with two thiol group molecules introduced. HIC-HPLC analysis was performed according to the previous report (Anal. Chem., 2019, 91, 20, 12724-12732) to confirm that two thiol group molecules were introduced.

### (26-6-2) Synthesis of antibody with two thiol group molecules bound to Lys248 of the antibody (Q2)

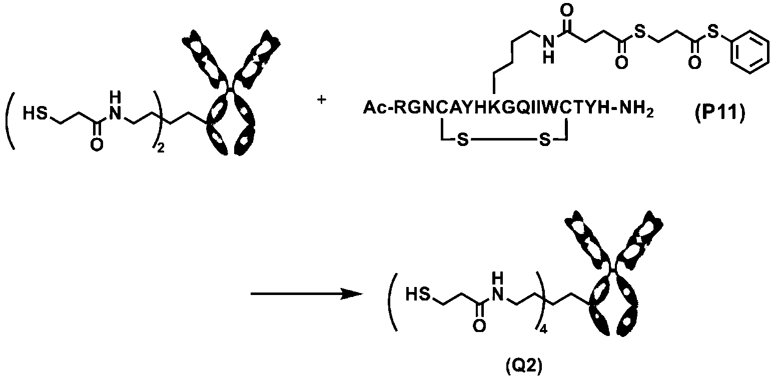

(The SEQ ID NO of the above-described amino acid sequence is SEQ ID NO: 1)

The antibody with two thiol group molecules bound synthesized in Example 26-7-1 (Q1) and a peptide reagent (P11) described in the previous report (WO2019/240287A1) were used following the method described in the previous report (WO2019/240287A1) to obtain an antibody with four thiol group molecules introduced. HIC-HPLC analysis was performed according to the previous report (Anal. Chem., 2019, 91, 20, 12724-12732) to confirm that four thiol group molecules were introduced.

### (26-6-3) Synthesis of ADC19

To the above-described antibody with four thiol group molecules bound (Q2), Linker-payload (42) was added to obtain ADC (19). ESI-TOFMS analysis was performed, and a peak was confirmed at 154880 for the reaction product, into which four molecules of Linker-payload (42) were introduced. ESI-TOFMS analysis was performed according to the previous report (WO2019/240287A1), confirming that the DAR was 4.

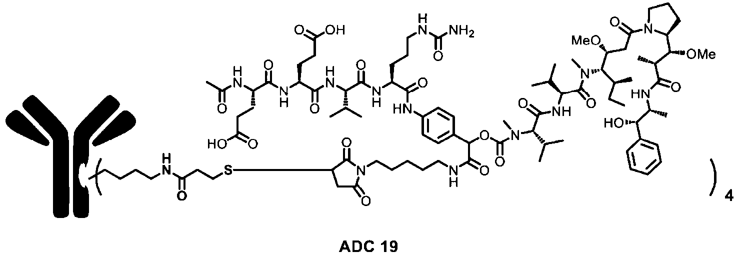

### Example 27: Evaluation of ADC using mouse plasma

### (27-1) Plasma stability test of ADC

To 500 µL of mouse plasma (Charles River Laboratories), ADC was added to a concentration of 0.1 mg/mL, followed by sterile filtration. This solution was dispensed in 50 µL aliquots into six Eppendorf tubes. Three of the six samples were stored in an incubator set at 37°C for 4 days. The remaining three were similarly stored in a freezer at -80°C for 4 days. To each sample, 100 µL of acetonitrile was added, vortexed, and then centrifuged to obtain a precipitate. The resulting supernatant was collected and subjected to HPLC analysis.

### (27-2) Quantitation of detached payload using HPLC analysis

The quantitation was performed by measuring the amount of the payload detached from ADC using a liquid chromatography/mass spectrometry (including tandem mass spectrometry). The samples stored similarly for 4 days in a -80°C freezer in Example 27-1 were designated as Day 0 samples, and the three samples incubated at 37°C for 4 days in Example 27-1 were designated as Day 4 samples, and the MS intensities of the payload detected from the Day 4 and Day 0 samples were calculated by extracted ion chromatogram, and the difference between the intensities was determined.

Separately, using MMAE, the correlation between the HPLC TIC area and the concentration was calculated. Using that calculation formula, the TIC derived from the fluorescence intensity of each of the above ADCs was converted to concentration. The ratio of the aforementioned difference in the ion chromatogram, with the concentration at Day 0 as 100%, was calculated as the detachment rate.

**Table 24: Results of plasma stability test using ADCs**

| | Linker-payload | Example/Comparable Example | Detachment rate of payload at Day 4 |
|---|---|---|---|
| ADC 4 | Linker-payload (35) | Example 16-1 | 5 % |
| ADC 5 | Linker-payload (42) | Example 16-2 | 0% |
| ADC 7 | Linker-payload (40) | Example 26-4 | 2% |
| ADC 12 | Linker-payload (130) | Example 16-6 | 0% |
| ADC 13 | Linker-payload (132) | Example 16-7 | 0% |
| ADC 15 | Linker-payload (141) | Example 16-9 | 1% |
| ADC 18 | Linker-payload (42) | Example 26-5 | 1% |
| ADC 19 | Linker-payload (42) | Example 26-6-3 | 1% |

As a result, it was confirmed that the ADCs synthesized in Examples 16-1, 16-2, 26-4, 16-6, 16-7, 16-9, 26-5, and 26-6-3 have high stability.

### Example 28: Evaluation of ADC using mouse plasma

### (28-1) Plasma stability test of ADC

To 500 µL of mouse plasma (Charles River Laboratories), ADC was added to a concentration of 0.1 mg/mL, followed by sterile filtration. This solution was dispensed in 50 µL aliquots into six Eppendorf tubes. Three of the six samples were stored in an incubator set at 37°C for 4 days. The remaining three were similarly stored in a freezer at -80°C for 4 days. To each sample, 100 µL of acetonitrile was added, vortexed, and then centrifuged to obtain a precipitate. The resulting supernatant was collected and subjected to HPLC analysis.

### (28-2) Quantitation of detached payload using HPLC analysis

The quantitation was performed by measuring the amount of the payload detached from ADC using a liquid chromatography/mass spectrometry (including tandem mass spectrometry). The samples stored similarly for 4 days in a -80°C freezer in Example 28-1 were designated as Day 0 samples, and the three samples incubated at 37°C for 4 days in Example 28-1 were designated as Day 4 samples, and the MS intensities of the payload detected from the Day 4 and Day 0 samples were calculated by extracted ion chromatograms, and the difference between the intensities was determined. The detachment rate of the payload was calculated according to Example 27-2.

**Table 25: Results of plasma stability test using ADCs**

| | Linker-payload mimic | Example/Comparable Example | Detachment rate of payload at Day 4 |
|---|---|---|---|
| ADC 2 | Linker-payload (42) | Example 13-2 | 7 % |
| ADC 9 | Linker-payload (125) | Example 13-4 | 14 % |

As a result, it was confirmed that the ADCs synthesized in Examples 13-2 and 13-4 have high stability.

## Claims

1. A conjugate of an antibody and a functional substance, or a salt thereof, comprising a structural unit represented by the following formula (1): 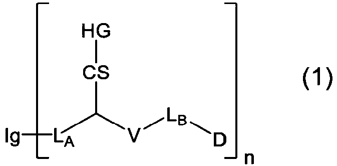 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

2. The conjugate or a salt thereof of claim 1, wherein the immunoglobulin unit is a human immunoglobulin unit.

3. The conjugate or a salt thereof of claim 2, wherein the human immunoglobulin unit is a human IgG antibody.

4. The conjugate or a salt thereof of claim 1, wherein the monovalent group comprising a hydrophilic group (HG-) is a monovalent group represented by the following formula (A):
N(R_{HG1})(R_{HG2})-L_{HG}- (A)
wherein
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more moieties selected from the group consisting of L_{HG}, R_{HG1}, and R_{HG2}.

5. The regioselective conjugate or a salt thereof of claim 4, wherein the divalent group optionally comprising a hydrophilic group (-L_{HG}-) is a divalent group represented by the following formula (a):
-(C(R_{HG})₂)ₙ₁-(C=O)ₙ₂-(NR_{HG})ₙ₃-(C(R_{HG})₂)ₙ₄- (a)
wherein
the plurality of R_{HG}s each independently represent a hydrogen atom, a hydrophilic group, or a monovalent group optionally comprising a hydrophilic group,
n1 is an integer of 0 to 3,
n2 is an integer of 0 or 1,
n3 is an integer of 0 or 1, and
n4 is an integer of 0 to 3.

6. The conjugate or a salt thereof of claim 5, wherein the divalent group represented by formula (a) is a divalent group represented by the following formula (a1), (a2), or (a3):
(a1) -(C(R_{HG})₂)-;
(a2) -(C(R_{HG})₂)-(C=O)-(NR_{HG})-(C(R_{HG})₂)-; or
(a3) -(C=O)-(C(R_{HG})₂)₂-;
wherein
the plurality of R_{HG}s each independently represent a hydrogen atom, a hydrophilic group, or a C1-6 alkyl group comprising a hydrophilic group.

7. The conjugate or a salt thereof of claim 1, wherein the hydrophilic group is one or more groups selected from the group consisting of a carboxylic acid group, a sulfonic acid group, a sulfamide group, a hydroxyl group, a polyethylene glycol group, a polysarcosine group, and a sugar moiety.

8. The conjugate or a salt thereof of claim 1, wherein the hydrophilic group is one or more groups selected from the group consisting of a sulfonic acid group, a sulfamide group, a hydroxyl group, a polyethylene glycol group, a polysarcosine group, and a sugar moiety.

9. The conjugate or a salt thereof of claim 1, wherein the cleavable site is a site cleavable by an enzyme.

10. The conjugate or a salt thereof of claim 9, wherein the enzyme is one or more enzymes selected from the group consisting of cathepsin B, plasmin, legumain, and caspase.

11. The conjugate or a salt thereof of claim 10, wherein the enzyme is one or more enzymes selected from the group consisting of plasmin, legumain, and caspase.

12. The conjugate or a salt thereof of claim 9, wherein the site cleavable by an enzyme comprises VC, VA, AA, GGFG (SEQ ID NO: 2), FK, VK, AK, GC, VLK, NN, or DDVD (SEQ ID NO: 3).

13. The conjugate or a salt thereof of claim 9, wherein the site cleavable by an enzyme comprises AA, GGFG (SEQ ID NO: 2), FK, VK, AK, GC, VLK, NN, or DDVD (SEQ ID NO: 3).

14. The conjugate or a salt thereof of claim 1, wherein the monovalent group comprising a hydrophilic group comprises one or more amino acid residues comprising a carboxylic acid group in the side chain.

15. The conjugate or a salt thereof of claim 1, wherein the structural unit consisting of HG-CS comprises one or more amino acid residues comprising a carboxylic acid group in the side chain and a peptide moiety comprising a site cleavable by an enzyme.

16. The conjugate or a salt thereof of claim 15, wherein the peptide moiety comprises EVC, EEVC (SEQ ID NO: 4), EEEEVC (SEQ ID NO: 5), DVC, EVA, EAA, EGGFG (SEQ ID NO: 6), EFK, EEFK (SEQ ID NO: 7), EEVK (SEQ ID NO: 8), EEAK (SEQ ID NO: 9), EGC, EEVLK (SEQ ID NO: 10), ENN, EDDVD (SEQ ID NO: 11), β-Ala-VC, or EEGC (SEQ ID NO: 12).

17. The conjugate or a salt thereof of claim 15, wherein the peptide moiety comprises EAA, EGGFG (SEQ ID NO: 6), EFK, EEFK (SEQ ID NO: 7), EEVK (SEQ ID NO: 8), EEAK (SEQ ID NO: 9), EGC, EEVLK (SEQ ID NO: 10), ENN, EDDVD (SEQ ID NO: 11), β-Ala-VC, or EEGC (SEQ ID NO: 12).

18. The conjugate or a salt thereof of claim 1, wherein the cleavable site is cleavable under acidic conditions or reducing conditions.

19. The conjugate or a salt thereof of claim 1, wherein the cleavable site is a disulfide bond.

20. The conjugate or a salt thereof of claim 1, wherein L_{A} is a divalent group having a side chain comprising a hydrophilic group or a functional substance.

21. The conjugate or a salt thereof of claim 1, wherein the functional substance is a pharmaceutical agent, a labeling substance, or a stabilizing agent.

22. The conjugate or a salt thereof of claim 1, wherein the conjugate shows an aggregation rate of 2.6% or less as determined by size exclusion chromatography.

23. The conjugate or a salt thereof of claim 1, wherein the structural unit represented by formula (1) is a structural unit represented by the following formula (1'): 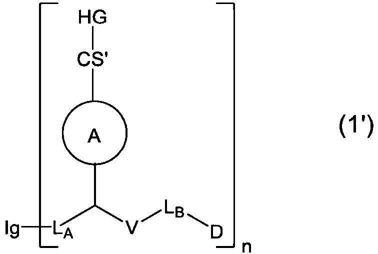 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
D represents a functional substance, and
the average number (n) of said bonds per the immunoglobulin unit is 1.5 or more.

24. The conjugate or a salt thereof of claim 23, wherein Ring A is an optionally substituted phenylene group.

25. The conjugate or a salt thereof of claim 23, wherein the structural unit represented by formula (1') is a structural unit represented by the following formula (1''): 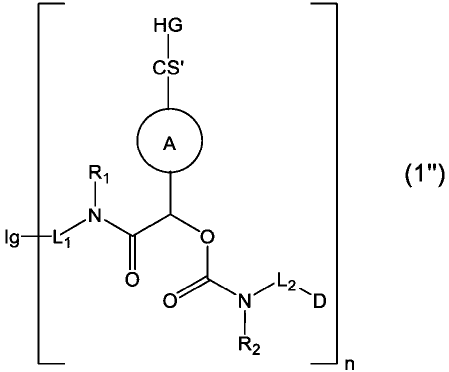 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
D represents a functional substance, and
the average number (n) of said bonds per the immunoglobulin unit is 1.5 or more.

26. The conjugate or a salt thereof of any one of claims 1 to 25, wherein the functional group in the side chain of a specific amino acid residue is the amino group in the side chain of a lysine residue, and n is from 1.5 to 2.5.

27. The conjugate or a salt thereof of claim 26, wherein
L_{A} has a carbonyl group, and
the bond is achieved by an amide bond generated by the bonding between the amino group in the side chain of a lysine residue and the carbonyl group in L_{A}.

28. The conjugate or a salt thereof of claim 26, wherein the lysine residue is present at position 246/248, 288/290, or 317 according to Eu numbering.

29. The conjugate or a salt thereof of claim 25, wherein the structural unit further comprises a modified moiety represented by the following formula (I): 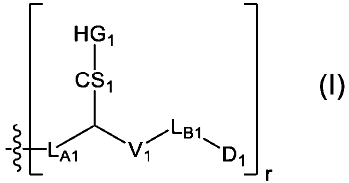 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

30. The conjugate or a salt thereof of claim 29, wherein the modified moiety represented by formula (I) is a modified moiety represented by the following formula (I'): 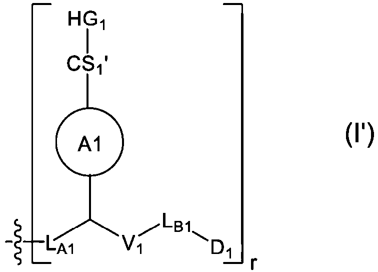 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
D₁ represents a functional substance, and
the average number (r) of said bonds per the immunoglobulin unit is 1.5 to 2.5.

31. The conjugate or a salt thereof of claim 29, wherein the modified moiety represented by formula (I') is a modified moiety represented by the following formula (I''): 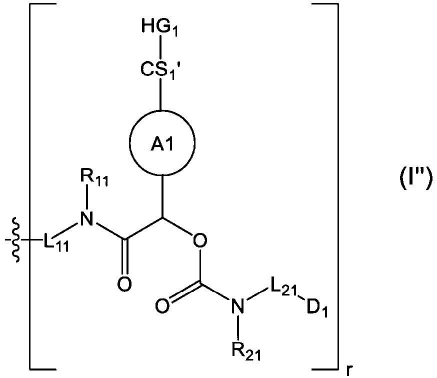 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
D₁ represents a functional substance, and
the average number (r) of said bonds per the immunoglobulin unit is 1.5 to 2.5.

32. The conjugate or a salt thereof of any one of claims 1 to 25, wherein the functional group in the side chain of a specific amino acid residue is the thiol group in the side chain of a cysteine residue, and n is from 2.0 to 8.0.

33. The conjugate or a salt thereof of claim 32, wherein n is 6.0 to 8.0.

34. The conjugate or a salt thereof of claim 33, wherein n is 7.0 to 8.0.

35. An antibody derivative having a bioorthogonal functional group, or a salt thereof, the antibody derivative comprising a structural unit represented by the following formula (2): 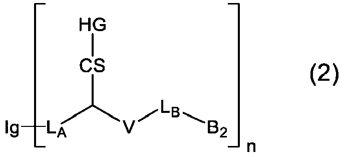 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS represents a divalent group comprising a cleavable site,
V represents an oxygen atom, a sulfur atom, or an amino group (NH),
L_{A} and L_{B} each independently represent a divalent group,
B₂ represents a bioorthogonal functional group, and
the average number (n) of said bonds per the immunoglobulin unit is 1.5 or more.

36. The antibody derivative or a salt thereof of claim 35, wherein the bioorthogonal functional group is a maleimide residue, a thiol residue, a furan residue, a halocarbonyl residue, an alkene residue, an alkyne residue, an azide residue, or a tetrazine residue.

37. The antibody derivative or a salt thereof of claim 35, wherein the structural unit represented by formula (2) is a structural unit represented by the following formula (2'): 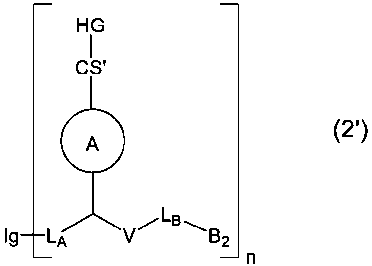 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₂ represents a bioorthogonal functional group, and
the average number (n) of said bonds per the immunoglobulin unit is 1.5 or more.

38. The antibody derivative or a salt thereof of claim 37, wherein the structural unit represented by formula (2') is a structural unit represented by the following formula (2"): 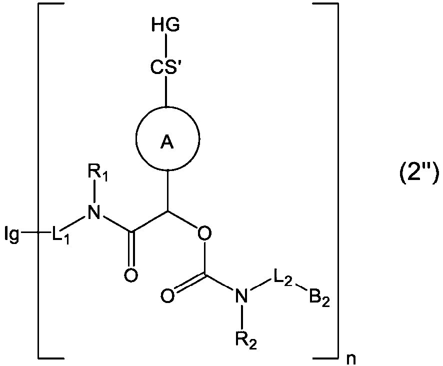 wherein
Ig represents an immunoglobulin unit comprising two heavy chains and two light chains, and is bonded to L_{A} adjacent to Ig via a functional group in the side chain of a specific amino acid residue in Ig,
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₂ represents a bioorthogonal functional group, and
the average number (n) of said bonds per the immunoglobulin unit is 1.5 or more.

39. The antibody derivative or a salt thereof of any one of claims 35 to 38, wherein the functional group in the side chain of a specific amino acid residue is the amino group in the side chain of a lysine residue, and n is from 1.5 to 2.5.

40. The antibody derivative or a salt thereof of claim 39, wherein
L_{A} has a carbonyl group, and
the bond is achieved by an amide bond generated by the bonding between the amino group in the side chain of a lysine residue and the carbonyl group in L_{A}.

41. The antibody derivative or a salt thereof of claim 39, wherein the lysine residue is present at position 246/248, 288/290, or 317 according to Eu numbering.

42. The conjugate or a salt thereof of claim 39, wherein the structural unit further comprises a modified moiety represented by the following formula (II): 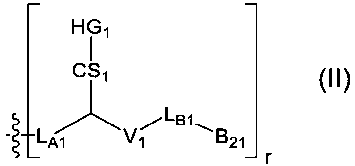 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁ represents a divalent group comprising a cleavable site,
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

43. The antibody derivative or a salt thereof of claim 42, wherein the modified moiety represented by formula (II) is a modified moiety represented by the following formula (II'): 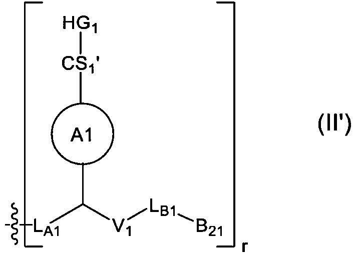 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
V₁ represents an oxygen atom, a sulfur atom, or an amino group (NH),
L_{A1} and L_{B1} each independently represent a divalent group,
B₂₁ represents a bioorthogonal functional group, and
the average number (r) of said bonds per the immunoglobulin unit is 1.5 to 2.5.

44. The antibody derivative or a salt thereof of claim 43, wherein the modified moiety represented by formula (II') is a modified moiety represented by the following formula (II"): 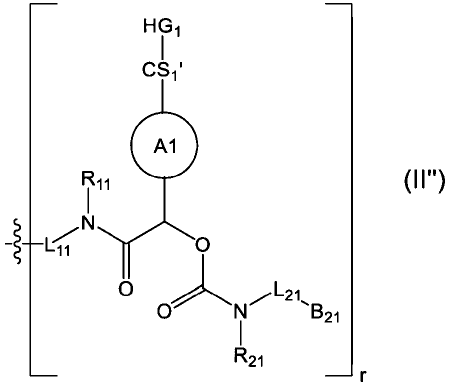 wherein
the hyphen (-) with a wavy line is bonded to the amino group in the side chain of a lysine residue present at position 246/248, 288/290, or 317 according to Eu numbering (wherein the lysine residue is different from the lysine residue having the amino group to which L_{A} is bonded in formula (1)),
HG₁ represents a hydrophilic group or a monovalent group comprising a hydrophilic group,
CS₁' represents a divalent group comprising a cleavable site,
Ring A1 represents an optionally substituted divalent aromatic ring group (wherein the divalent aromatic ring group forms a pi electron conjugated system with the cleavable site),
R₁₁ and R₂₁ each independently represent a hydrogen atom or a monovalent group,
L₁₁ and L₂₁ each independently represent a divalent group,
B₂₁ represents a bioorthogonal functional group, and
the average number (r) of said bonds per the immunoglobulin unit is 1.5 to 2.5.

45. The antibody derivative or a salt thereof of any one of claims 35 to 38, wherein the functional group in the side chain of a specific amino acid residue is the thiol group in the side chain of a cysteine residue, and n is from 2.0 to 8.0.

46. The antibody derivative or a salt thereof of claim 45, wherein n is 6.0 to 8.0.

47. The antibody derivative or a salt thereof of claim 46, wherein n is 7.0 to 8.0.

48. A compound having a bioorthogonal functional group and a functional substance, or a salt thereof, represented by the following formula (3'): 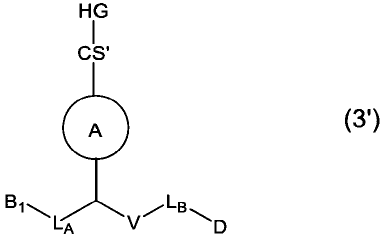 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a bioorthogonal functional group, and
D represents a functional substance.

49. The compound or a salt thereof of claim 48, wherein the structural unit represented by formula (3') is represented by the following formula (3"): 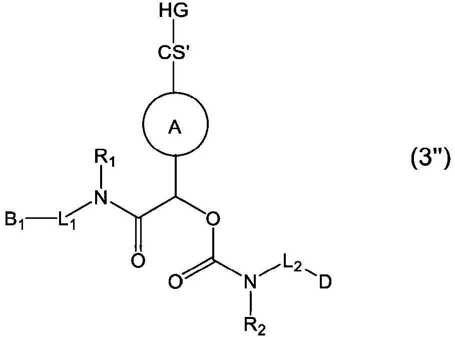 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a bioorthogonal functional group, and
D represents a functional substance.

50. A reagent for derivatizing an antibody, wherein the reagent comprises the compound or a salt thereof of claim 48 or 49.

51. A compound having a first bioorthogonal functional group and a second bioorthogonal functional group, or a salt thereof, represented by the following formula (4'): 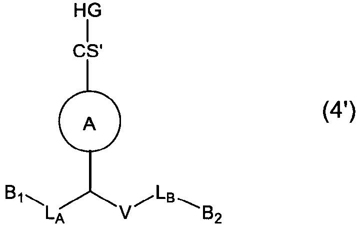 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group.

52. The compound or a salt thereof of claim 51, wherein the compound represented by formula (4') is represented by the following formula (4"): 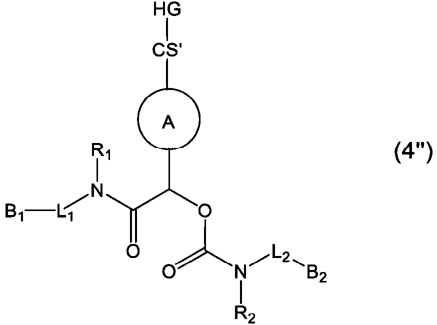 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

53. A reagent for derivatizing an antibody or a functional substance, wherein the reagent comprises the compound or a salt thereof of claim 51 or 52.

54. A compound or a salt thereof, which is of the following (I'), (II'), or (III'):
(I') a compound, or a salt thereof, represented by the following formula (5'): 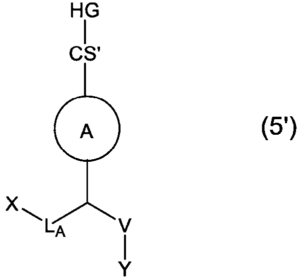 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} represents a divalent group, and
X and Y each independently represent a monovalent group,
(II') a compound, or a salt thereof, represented by the following formula (6'): 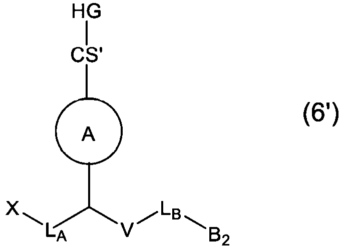 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} and L_{B} each independently represent a divalent group,
X represents a monovalent group, and
B₂ represents a bioorthogonal functional group, or
(III') a compound, or a salt thereof, represented by the following formula (7'): 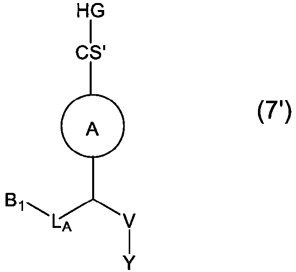 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
L_{A} represents a divalent group,
B₁ represents a bioorthogonal functional group, and
Y represents a monovalent group.

55. The compound or a salt thereof of claim 53, wherein the compound of (I'), (II'), or (III') is a compound of the following (I"), (II''), or (III"), respectively:
(I'') a compound, or a salt thereof, represented by the following formula (5''): 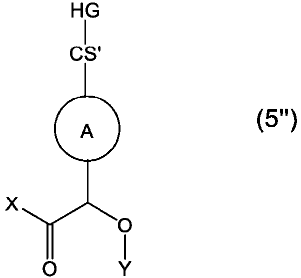 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site), and
X and Y each independently represent a monovalent group,
(II") a compound, or a salt thereof, represented by the following formula (6"): 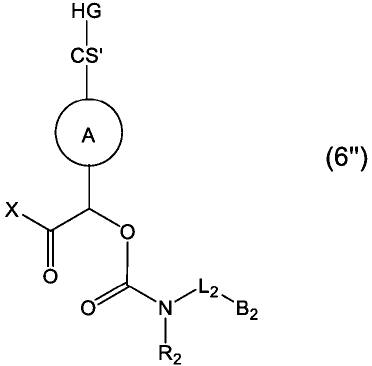 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
R₂ represents a hydrogen atom or a monovalent group,
L₂ represents a divalent group,
X represents a monovalent group, and
B₂ represents a bioorthogonal functional group, or
(III") a compound, or a salt thereof, represented by the following formula (7"): 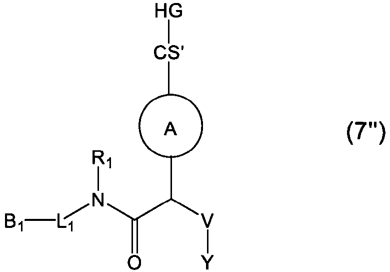 wherein
HG represents a hydrophilic group or a monovalent group comprising a hydrophilic group,
CS' represents a divalent group comprising a cleavable site,
Ring A represents an optionally substituted divalent aromatic ring group (wherein the divalent aromatic ring group forms a pi electron conjugated system with the cleavable site),
V represents an oxygen atom, a sulfur atom, or an amino group (NH),
R₁ represents a hydrogen atom or a monovalent group,
L₁ represents a divalent group,
B₁ represents a bioorthogonal functional group, and
Y represents a monovalent group.
